(19) Europäisches Patentamt / European Patent Office / Office européen des brevets

(11) EP 3 052 523 B1

(12) **EUROPEAN PATENT SPECIFICATION**

(45) Date of publication and mention of the grant of the patent:
**10.03.2021 Bulletin 2021/10**

(21) Application number: **14777632.2**

(22) Date of filing: **01.10.2014**

(51) Int Cl.:
***C07K 16/28*** *(2006.01)* ***C07K 16/32*** *(2006.01)*
***A61P 35/00*** *(2006.01)* ***A61K 39/00*** *(2006.01)*
***A61P 43/00*** *(2006.01)*

(86) International application number:
**PCT/EP2014/071028**

(87) International publication number:
**WO 2015/049280 (09.04.2015 Gazette 2015/14)**

(54) **METHODS OF TREATING AND DIAGNOSING ALPHA-V-BETA-6 OVEREXPRESSING CANCER**

VERFAHREN ZUR DIAGNOSE UND BEHANDLUNG VON ALPHA-V-BETA-6-ÜBEREXPRIMIERENDEN KREBS

PROCÉDÉS DE TRAITEMENT ET DE DIAGNOSTIC D’UN CANCER SUREXPRIMANT ALPHA-V-BÊTA-6

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.10.2013 US 201361885302 P**

(43) Date of publication of application:
**10.08.2016 Bulletin 2016/32**

(73) Proprietor: **Medimmune Limited**
**Cambridge, Cambridgeshire CB21 6GH (GB)**

(72) Inventors:
- **BARRY, Simon T**
  **Macclesfield**
  **Cheshire SK10 4TG (GB)**
- **MARSHALL, John F**
  **London**
  **Greater London EC1M 6BQ (GB)**
- **MOORE, Kate M**
  **London**
  **Greater London EC1M 6BQ (GB)**

(74) Representative: **AstraZeneca**
**Milstein Building**
**Granta Park**
**Cambridge CB21 6GH (GB)**

(56) References cited:
**WO-A2-03/100033** **WO-A2-2007/008712**
**WO-A2-2013/123152**

- **ANTONIO SAHA ET AL: "High-resolution in vivo imaging of breast cancer by targeting the pro-invasive integrin [alpha]v[beta]6", THE JOURNAL OF PATHOLOGY, 13 July 2010 (2010-07-13), pages n/a-n/a, XP055157647, ISSN: 0022-3417, DOI: 10.1002/path.2745**
- **J. J. BARRON ET AL: "HER2 Testing and Subsequent Trastuzumab Treatment for Breast Cancer in a Managed Care Environment", THE ONCOLOGIST, 14 August 2009 (2009-08-14), XP055157656, ISSN: 1083-7159, DOI: 10.1634/theoncologist.2008-0288**
- **K. M. MOORE ET AL: "Therapeutic Targeting of Integrin v 6 in Breast Cancer", JNCI JOURNAL OF THE NATIONAL CANCER INSTITUTE, vol. 106, no. 8, 28 June 2014 (2014-06-28) , pages dju169-dju169, XP055157642, ISSN: 0027-8874, DOI: 10.1093/jnci/dju169**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 052 523 B1

SEQUENCE LISTING

<110> MedImmune Limited

<120> Methods of Treating and Diagnosing Alpha-V-Beta-6 Overexpressing Cancer

<130> A5B6-101WO1

<150> US 61/885,302
<151> 2013-10-01

<160> 97

<170> PatentIn version 3.4

<210> 1
<211> 366
<212> DNA
<213> Homo sapiens

<400> 1

```
caggtgcagc tgcaggagtc gggcccagga ctggtgaagc cttcacagac cctgtccctc     60
acctgcactg tcgctggtgg ctccatcaga agtggtgatt actactggag ctggatccgc    120
cagcacccag ggaagggcct ggagtggatt gggaacatct attacagtgg gagcacctac    180
tacaacccgt ccctcaagag tcgaattacc atttcagtag ccacgtctag gaaccagttc    240
tccctgaagc tgacctctgt gactgccgcg gacacggccg tgtattactg tgcgagaggg    300
ggagctatta cgatttttgg agtgtttgac tactggggcc agggaaccct ggtcaccgtc    360
tcctca                                                               366
```

<210> 2
<211> 122
<212> PRT
<213> Homo sapiens

<400> 2

```
Gln Val Gln Leu Gln Glu Ser Gly Pro Gly Leu Val Lys Pro Ser Gln
1               5                   10                  15

Thr Leu Ser Leu Thr Cys Thr Val Ala Gly Gly Ser Ile Arg Ser Gly
            20                  25                  30

Asp Tyr Tyr Trp Ser Trp Ile Arg Gln His Pro Gly Lys Gly Leu Glu
        35                  40                  45

Trp Ile Gly Asn Ile Tyr Tyr Ser Gly Ser Thr Tyr Tyr Asn Pro Ser
    50                  55                  60

Leu Lys Ser Arg Ile Thr Ile Ser Val Ala Thr Ser Arg Asn Gln Phe
65                  70                  75                  80

Ser Leu Lys Leu Thr Ser Val Thr Ala Ala Asp Thr Ala Val Tyr Tyr
                85                  90                  95

Cys Ala Arg Gly Gly Ala Ile Thr Ile Phe Gly Val Phe Asp Tyr Trp
            100                 105                 110

Gly Gln Gly Thr Leu Val Thr Val Ser Ser
        115                 120
```

<210> 3
<211> 324
<212> DNA
<213> Homo sapiens

<400> 3

gaaattgtgt tgacgcagtc tccaggcacc ctgtctttgt ctccagggga aagagccacc 60

ctctcctgca gggccagtca gagtgttagc agcagctact tagcctggta ccagcagaaa 120

cctggccagg ctcccaggct cctcatctat ggtgcatcca gcagggccac tggcatccca 180

gacaggttca gtggcagtgg gtctgggaca gacttcactc tcaccatcag cagactggag 240

cctgaagatt ttgcagtgta ttactgtcag cagtatggta gctcaccgtg cagttttggc 300

caggggacca agctggagat caaa 324

<210> 4
<211> 108
<212> PRT
<213> Homo sapiens

<400> 4

Glu Ile Val Leu Thr Gln Ser Pro Gly Thr Leu Ser Leu Ser Pro Gly
1 5 10 15

Glu Arg Ala Thr Leu Ser Cys Arg Ala Ser Gln Ser Val Ser Ser Ser
20 25 30

Tyr Leu Ala Trp Tyr Gln Gln Lys Pro Gly Gln Ala Pro Arg Leu Leu
35 40 45

Ile Tyr Gly Ala Ser Ser Arg Ala Thr Gly Ile Pro Asp Arg Phe Ser
50 55 60

Gly Ser Gly Ser Gly Thr Asp Phe Thr Leu Thr Ile Ser Arg Leu Glu
65 70 75 80

Pro Glu Asp Phe Ala Val Tyr Tyr Cys Gln Gln Tyr Gly Ser Ser Pro
85 90 95

Cys Ser Phe Gly Gln Gly Thr Lys Leu Glu Ile Lys
100 105

<210> 5
<211> 366
<212> DNA
<213> Homo sapiens

<400> 5

```
gaggtgcagc tgttggagtc tgggggaggc ttggtacagc ctggggggtc cctgagactc     60
tcctgtgcag cctctggatt cacctttagc agctatgtca tgagctgggt ccgccaggct    120
ccagggaagg ggctggagtg ggtctcagct attagtggta gtggtggtag cacatactac    180
gcagactccg tgaagggccg gttcaccatc tccagagaca attccaagaa cacgctgtat    240
ctgcaaatga acagcctgag agccgaggac acggccgtat attactgtgc gaaaggtgtg    300
gatacagcta tggttaccta cggtatggac gtctggggcc aagggaccac ggtcaccgtc    360
tcctca                                                               366
```

<210> 6
<211> 122
<212> PRT
<213> Homo sapiens

<400> 6

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Val Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Val Asp Thr Ala Met Val Thr Tyr Gly Met Asp Val Trp
            100                 105                 110

Gly Gln Gly Thr Thr Val Thr Val Ser Ser
        115                 120
```

<210> 7
<211> 324
<212> DNA
<213> Homo sapiens

<400> 7

```
tcctatgagc tgacacagcc accctcggtg tcagtgtccc caggacaaac ggccaggatc      60
acctgctctg gagatgcatt gccaaaaaaa tatgcttatt ggtaccagca gaagtcaggc     120
caggcccctg tgctggtcat ctatgacgac agcaaacgac cctccgggat ccctgagaga     180
ttctctggct ccagctcagg gacaatggcc accttgacta tcagtggggc ccaggtggag     240
gatgaagctg actactactg ttactcaaca gacagcagtg gtaatcatag ggtgttcggc     300
ggagggacca agctgaccgt ccta                                            324
```

<210> 8
<211> 108
<212> PRT
<213> Homo sapiens

<400> 8

```
Ser Tyr Glu Leu Thr Gln Pro Pro Ser Val Ser Val Ser Pro Gly Gln
1               5                   10                  15

Thr Ala Arg Ile Thr Cys Ser Gly Asp Ala Leu Pro Lys Lys Tyr Ala
            20                  25                  30

Tyr Trp Tyr Gln Gln Lys Ser Gly Gln Ala Pro Val Leu Val Ile Tyr
        35                  40                  45

Asp Asp Ser Lys Arg Pro Ser Gly Ile Pro Glu Arg Phe Ser Gly Ser
    50                  55                  60

Ser Ser Gly Thr Met Ala Thr Leu Thr Ile Ser Gly Ala Gln Val Glu
65                  70                  75                  80

Asp Glu Ala Asp Tyr Tyr Cys Tyr Ser Thr Asp Ser Ser Gly Asn His
                85                  90                  95

Arg Val Phe Gly Gly Gly Thr Lys Leu Thr Val Leu
            100                 105
```

<210> 9
<211> 369
<212> DNA
<213> Homo sapiens

<220>
<221> misc_feature
<222> (369)..(369)
<223> n is a, c, g, or t

<400> 9

```
gaggtgcagc tggtgcagtc tggagcagag gtgaaaaagc ccggggagtc tctgaagatc      60
tcctgtaagg gttctggata cagctttacc agctactgga tcggctgggt gcgccagatg     120
cccgggaaag gcctggagtg gatggggatc atctatcctg gtgactctga taccagatac     180
agcccgtcct tccaaggcca ggtcatcctc tcagccgaca agtccatcag caccgcctac     240
ctgcagtgga gcagcctgaa ggcctcggac accgccatgt attactgtgc gagacatgat     300
gaaagtagtg gttattacta tgtttttgat atctggggcc aagggacaat ggtcaccgtc     360
tcttcagcn                                                             369
```

<210> 10
<211> 123
<212> PRT
<213> Homo sapiens

<400> 10

```
Glu Val Gln Leu Val Gln Ser Gly Ala Glu Val Lys Lys Pro Gly Glu
1               5                   10                  15

Ser Leu Lys Ile Ser Cys Lys Gly Ser Gly Tyr Ser Phe Thr Ser Tyr
            20                  25                  30

Trp Ile Gly Trp Val Arg Gln Met Pro Gly Lys Gly Leu Glu Trp Met
        35                  40                  45

Gly Ile Ile Tyr Pro Gly Asp Ser Asp Thr Arg Tyr Ser Pro Ser Phe
    50                  55                  60

Gln Gly Gln Val Ile Leu Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Ala Arg His Asp Glu Ser Ser Gly Tyr Tyr Tyr Val Phe Asp Ile Trp
            100                 105                 110

Gly Gln Gly Thr Met Val Thr Val Ser Ser Ala
        115                 120
```

<210> 11
<211> 327
<212> DNA
<213> Homo sapiens

<220>
<221> misc_feature

<222> (300)..(300)
<223> n is c or t

<400> 11

```
tcctatgagc tgacacaacc accctcggtg tcagtgtccc caggacaaac ggccaggatc     60
acctgctctg gagatgcatt gccaaaaaaa tatgcttatt ggtaccagca gaagtcaggc    120
caggcccctg ttctggtcat ctatgatgac atcaaacgac cctccgggat ccctgagaga    180
ttctctggct ccagctcagg gacaatggcc accttgacta tcagtggggc ccaggtggag    240
gatgaagctg actactactg ttactcaaca gacagcagtg gtaatcattg ggttttcttn    300
ggcggaggga ccaagctgac cgtccta                                        327
```

<210> 12
<211> 109
<212> PRT
<213> Homo sapiens

<400> 12

```
Ser Tyr Glu Leu Thr Gln Pro Pro Ser Val Ser Val Ser Pro Gly Gln
1               5                   10                  15

Thr Ala Arg Ile Thr Cys Ser Gly Asp Ala Leu Pro Lys Lys Tyr Ala
            20                  25                  30

Tyr Trp Tyr Gln Gln Lys Ser Gly Gln Ala Pro Val Leu Val Ile Tyr
        35                  40                  45

Glu Asp Ile Lys Arg Pro Ser Gly Ile Pro Glu Arg Phe Ser Gly Ser
    50                  55                  60

Ser Ser Gly Thr Met Ala Thr Leu Thr Ile Ser Gly Ala Gln Val Glu
65                  70                  75                  80

Asp Glu Ala Asp Tyr Tyr Cys Tyr Ser Thr Asp Ser Ser Gly Asn His
                85                  90                  95

Trp Val Phe Phe Gly Gly Gly Thr Lys Leu Thr Val Leu
            100                 105
```

<210> 13
<211> 339
<212> DNA
<213> Homo sapiens

<400> 13

```
caggtgcagc tggtgcagtc tggggctgag gtgaagaagc ctggggcctc agtgaaggtc      60

tcctgcaagg cttctggata caccttcacc ggctactata tgcactgggt gcgacaggcc     120

cctggacaag ggcttgagtg gatgggatgg atcaacccta aaagtggtga cacaaactat     180

gcacagaagt ttcagggcag ggtcaccctg accagggaca cgtccaccag cacagcctac     240

atggagctga gcaggctgag atctgacgac acggccgtgt attactgtgc gagaaggttg     300

gacgtctggg gccaagggac cacggtcacc gtctcctca                            339
```

<210> 14
<211> 113
<212> PRT
<213> Homo sapiens

<400> 14

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
        35                  40                  45

Gly Trp Ile Asn Pro Lys Ser Gly Asp Thr Asn Tyr Ala Gln Lys Phe
    50                  55                  60

Gln Gly Arg Val Thr Leu Thr Arg Asp Thr Ser Thr Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Arg Leu Asp Val Trp Gly Gln Gly Thr Thr Val Thr Val Ser
            100                 105                 110

Ser
```

<210> 15
<211> 330
<212> DNA
<213> Homo sapiens

<400> 15

```
cagtctgtgt tgacgcagcc gccctcagtg tctgcggccc caggacagaa ggtcaccatc     60

tcctgctctg gaagcagctc caacattggg aataattatg tatcctggta ccagcagctc    120

ccaggaacag cccccaaact cctcatttat gacaataata agcgaccctc aggaattcct    180

gaccgattct ctggctccaa gtctggcacg tcagccaccc tgggcatcac cggactccag    240

actggggacg aggccgatta ttactgcgga acatggaata gcagcctgag tgctggttat    300

gtcttcggaa ctgggaccaa ggtcaccgtc                                     330
```

<210> 16
<211> 110
<212> PRT
<213> Homo sapiens

<400> 16

```
Gln Ser Val Leu Thr Gln Pro Pro Ser Val Ser Ala Ala Pro Gly Gln
1               5                   10                  15

Lys Val Thr Ile Ser Cys Ser Gly Ser Ser Ser Asn Ile Gly Asn Asn
            20                  25                  30

Tyr Val Ser Trp Tyr Gln Gln Leu Pro Gly Thr Ala Pro Lys Leu Leu
        35                  40                  45

Ile Tyr Asp Asn Asn Lys Arg Pro Ser Gly Ile Pro Asp Arg Phe Ser
    50                  55                  60

Gly Ser Lys Ser Gly Thr Ser Ala Thr Leu Gly Ile Thr Gly Leu Gln
65                  70                  75                  80

Thr Gly Asp Glu Ala Asp Tyr Tyr Cys Gly Thr Trp Asn Ser Ser Leu
                85                  90                  95

Ser Ala Gly Tyr Val Phe Gly Thr Gly Thr Lys Val Thr Val
            100                 105                 110
```

<210> 17
<211> 375
<212> DNA
<213> Homo sapiens

<220>
<221> misc_feature
<222> (375)..(375)
<223> n is a, c, g, or t

<400> 17

```
gaggtgcagc tggtgcagtc tggagcagag gtgaaaaagc ccggggagtc tctgaagatc      60

tcctgtaagg gttctggata cagctttacc agctactgga tcggctgggt gcgccagatg     120

cccgggaaag gcctggagtg gatggggatc atctatcctg gtgactctga taccagatat     180

agtccgtcct tccaaggcca ggtcaccatc tcagccgaca agtccatcag caccgcctac     240

ctgcagtgga gcagcctgaa ggcctcggac accgccatgt attactgtgc gagacatggt     300

atagcagcag ctggtttcta ctactactat atggacgtct ggggccaagg gaccacggtc     360

accgtctcct cagcn                                                      375
```

<210> 18
<211> 125
<212> PRT
<213> Homo sapiens

<400> 18

```
Glu Val Gln Leu Val Gln Ser Gly Ala Glu Val Lys Lys Pro Gly Glu
1               5                   10                  15

Ser Leu Lys Ile Ser Cys Lys Gly Ser Gly Tyr Ser Phe Thr Ser Tyr
            20                  25                  30

Trp Ile Gly Trp Val Arg Gln Met Pro Gly Lys Gly Leu Glu Trp Met
        35                  40                  45

Gly Ile Ile Tyr Pro Gly Asp Ser Asp Thr Arg Tyr Ser Pro Ser Phe
    50                  55                  60

Gln Gly Gln Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Ala Arg His Gly Ile Ala Ala Ala Gly Phe Tyr Tyr Tyr Tyr Met Asp
            100                 105                 110

Val Trp Gly Gln Gly Thr Thr Val Thr Val Ser Ser Ala
        115                 120                 125
```

<210> 19
<211> 327
<212> DNA
<213> Homo sapiens

<220>

<221> misc_feature
<222> (325)..(325)
<223> n is a or c

<220>
<221> misc_feature
<222> (327)..(327)
<223> n is a, c, g, or t

<400> 19

```
gaaattgtgt tgacgcagtc cccagacacc ctgtctttgt ctccagggga aagagcctcc      60

ctctcctgca gggccagtca gaatgttaac aggaactact tagtctggta ccagcagaaa     120

cctggccagg ctcccaggct cctcatctat ggtacatcca acagggccac tggcatccca     180

gacaggttca gtggcagtgg gtctgggaca gacttcactc tcaccatcag cagactggag     240

cctgaagatt ttgcagttta ttactgtcag cagtgtggta gtttaccatt cactttcggc     300

cctgggacca aagtggatat caaangn                                         327
```

<210> 20
<211> 109
<212> PRT
<213> Homo sapiens

<400> 20

```
Glu Ile Val Leu Thr Gln Ser Pro Asp Thr Leu Ser Leu Ser Pro Gly
1               5                   10                  15

Glu Arg Ala Ser Leu Ser Cys Arg Ala Ser Gln Asn Val Asn Arg Asn
            20                  25                  30

Tyr Leu Val Trp Tyr Gln Gln Lys Pro Gly Gln Ala Pro Arg Leu Leu
        35                  40                  45

Ile Tyr Gly Thr Ser Asn Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Cys Gly Ser Leu Pro
                85                  90                  95

Phe Thr Phe Gly Pro Gly Thr Lys Val Asp Ile Lys Arg
            100                 105
```

<210> 21

<211> 372
<212> DNA
<213> Homo sapiens

<400> 21

```
caggtgcagc tgcaggagtc gggcccagga ctggtgaagc cttcacagac cctgtccctc     60
acctgcactg tctctggtgg ctccatcagc agtggtgttt actactggac ctggatccgc    120
cagcacccag ggaacggcct ggagtggatt ggctacatct attacagtgg gagcacctcc    180
tacaacccgt ccctcaagag tcgagttacc atatcagtag acacgtctaa gaaacagttc    240
tccctgaacc tgacctctgt gactgccgcg gacacggccg tgtattactg tgcgagagaa    300
ggaccactac ggggggacta ctactacggt ctggacgtct ggggccaagg gaccacggtc    360
accgtctcct ca                                                        372
```

<210> 22
<211> 124
<212> PRT
<213> Homo sapiens

<400> 22

```
Gln Val Gln Leu Gln Glu Ser Gly Pro Gly Leu Val Lys Pro Ser Gln
1               5                   10                  15

Thr Leu Ser Leu Thr Cys Thr Val Ser Gly Gly Ser Ile Ser Ser Gly
            20                  25                  30

Val Tyr Tyr Trp Thr Trp Ile Arg Gln His Pro Gly Asn Gly Leu Glu
        35                  40                  45

Trp Ile Gly Tyr Ile Tyr Tyr Ser Gly Ser Thr Ser Tyr Asn Pro Ser
    50                  55                  60

Leu Lys Ser Arg Val Thr Ile Ser Val Asp Thr Ser Lys Lys Gln Phe
65                  70                  75                  80

Ser Leu Asn Leu Thr Ser Val Thr Ala Ala Asp Thr Ala Val Tyr Tyr
                85                  90                  95

Cys Ala Arg Glu Gly Pro Leu Arg Gly Asp Tyr Tyr Tyr Gly Leu Asp
            100                 105                 110

Val Trp Gly Gln Gly Thr Thr Val Thr Val Ser Ser
        115                 120
```

<210> 23
<211> 324
<212> DNA
<213> Homo sapiens

<400> 23

```
gaaattgtgt tgacgcagtc tccaggcacc ctgtctttgt ctccagggga aagagccacc     60
ctctcctgca gggccggtca gactattagc agtcgctact tagcctggta ccagcagaaa    120
cctggccagg ctcccaggcc cctcatctat ggtgcatcca gcagggccac tggcatccca    180
gacaggttca gtggcagtgg gtctgggaca gacttcactc tcaccatcag cagactggag    240
cctgaagatt ttgcagtgta ttactgtcag cagtatggta gctcacctcg gacgttcggc    300
caagggacca aggtggaaat caaa                                           324
```

<210> 24
<211> 108
<212> PRT
<213> Homo sapiens

<400> 24

```
Glu Ile Val Leu Thr Gln Ser Pro Gly Thr Leu Ser Leu Ser Pro Gly
1               5                   10                  15

Glu Arg Ala Thr Leu Ser Cys Arg Ala Gly Gln Thr Ile Ser Ser Arg
            20                  25                  30

Tyr Leu Ala Trp Tyr Gln Gln Lys Pro Gly Gln Ala Pro Arg Pro Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Ser Ser Pro
                85                  90                  95

Arg Thr Phe Gly Gln Gly Thr Lys Val Glu Ile Lys
            100                 105
```

<210> 25
<211> 384
<212> DNA
<213> Homo sapiens

<400> 25

```
caggtgcagc tgcaggagtc gggcccagga ctggtgaagc cttcacagac cctgtccctc     60
acctgcactg tctctggtgg ctccatcagc agtggtggtt actactggag ctggatccgc    120
cagcacccag ggaagggcct ggagtggatt gggtacatct attacagtgg gagcacctac    180
tacaacccgt ccctcaagag tcgagttacc atatcagtag acacgtctaa gaaccagttc    240
tccctgaagc tgagctctgt gactgccgcg gacacggcca tgtattactg tgcgagatat    300
cgaggaccag cggctgggcg gggagacttc tactacttcg gtatggacgt ctggggccaa    360
gggaccacgg tcaccgtctc ctca                                          384
```

<210> 26
<211> 128
<212> PRT
<213> Homo sapiens

<400> 26

```
Gln Val Gln Leu Gln Glu Ser Gly Pro Gly Leu Val Lys Pro Ser Gln
1               5                   10                  15

Thr Leu Ser Leu Thr Cys Thr Val Ser Gly Gly Ser Ile Ser Ser Gly
            20                  25                  30

Gly Tyr Tyr Trp Ser Trp Ile Arg Gln His Pro Gly Lys Gly Leu Glu
        35                  40                  45

Trp Ile Gly Tyr Ile Tyr Tyr Ser Gly Ser Thr Tyr Tyr Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Ser Ser Val Thr Ala Ala Asp Thr Ala Met Tyr Tyr
                85                  90                  95

Cys Ala Arg Tyr Arg Gly Pro Ala Ala Gly Arg Gly Asp Phe Tyr Tyr
            100                 105                 110

Phe Gly Met Asp Val Trp Gly Gln Gly Thr Thr Val Thr Val Ser Ser
        115                 120                 125
```

<210> 27
<211> 339
<212> DNA
<213> Homo sapiens

<220>
<221> misc_feature
<222> (312) .. (312)
<223> n is c or t

<400> 27

```
gatattgtga tgacccagac tccactctct ctgtccgtca cccctggaca gccggcctcc      60
atcttctgca agtctagtca gagcctcctg aacagtgatg gaaagaccta tttgtgttgg     120
tacctgcaga agccaggcca gcctccacag ctcctgatct atgaagtttc caaccggttc     180
tctggagtgc cagataggtt cagtggcagc gggtcaggga cagatttcac actgaaaatc     240
agccgggtgg aggctgagga tgttggggtt tattactgca tgcaaggtat acagcttccg     300
tgggcgttct tnggccaagg gaccaaggtg gaaatcaaa                            339
```

<210> 28
<211> 113
<212> PRT
<213> Homo sapiens

<400> 28

```
Asp Ile Val Met Thr Gln Thr Pro Leu Ser Leu Ser Val Thr Pro Gly
1               5                   10                  15

Gln Pro Ala Ser Ile Phe Cys Lys Ser Ser Gln Ser Leu Leu Asn Ser
            20                  25                  30

Asp Gly Lys Thr Tyr Leu Cys Trp Tyr Leu Gln Lys Pro Gly Gln Pro
        35                  40                  45

Pro Gln Leu Leu Ile Tyr Glu Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Met Gln Gly
                85                  90                  95

Ile Gln Leu Pro Trp Ala Phe Phe Gly Gln Gly Thr Lys Val Glu Ile
            100                 105                 110

Lys
```

<210> 29
<211> 383
<212> DNA

<213> Homo sapiens

<400> 29

```
caggtgcagc tgcaggagtc gggcccagga ctggtgaagc cttcacagac cctgtccctc     60
acctgcactg tctctggtgg ctccatcagc agtggtggtt actactggag ctggatccgc    120
cagcacccag ggaagggcct ggagtggatt gggtacatct attacagtgg gagaacctac    180
aacaacccgt ccctcaagag tcgagttacc atatcagtag acacgtctaa gaaccagttc    240
tccctgaagt tgagttctgt gactgccgcg gacacggccg tgtattactg tgcgagagtg    300
gctacgggga gaggggacta ccacttctac gctatggacg tctggggcca agggaccacg    360
gtcaccgtct cctcagcctc cac                                            383
```

<210> 30
<211> 125
<212> PRT
<213> Homo sapiens

<400> 30

```
Gln Val Gln Leu Gln Glu Ser Gly Pro Gly Leu Val Lys Pro Ser Gln
1               5                   10                  15

Thr Leu Ser Leu Thr Cys Thr Val Ser Gly Gly Ser Ile Ser Ser Gly
            20                  25                  30

Gly Tyr Tyr Trp Ser Trp Ile Arg Gln His Pro Gly Lys Gly Leu Glu
        35                  40                  45

Trp Ile Gly Tyr Ile Tyr Tyr Ser Gly Arg Thr Tyr Asn Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Ser Ser Val Thr Ala Ala Asp Thr Ala Val Tyr Tyr
                85                  90                  95

Cys Ala Arg Val Ala Thr Gly Arg Gly Asp Tyr His Phe Tyr Ala Met
            100                 105                 110

Asp Val Trp Gly Gln Gly Thr Thr Val Thr Val Ser Ser
        115                 120                 125
```

<210> 31
<211> 318
<212> DNA
<213> Homo sapiens

<400> 31

```
tcctatgagc tgacacagcc atcctcagtg tcagtgtctc cgggacagac agccaggatc      60
acctgctcag gagatgtact ggcaaaaaag tctgctcggt ggttccacca gaagccaggc     120
caggcccctg tactggtgat ttataaagac agtgagcggc cctcagggat ccctgagcgc     180
ttctccggct ccagctcagg gaccacagtc accttgacca tcagcggggc ccaggttgag     240
gatgaggctg cctattactg ttactctgcg gctgacaaca atctggtatt cggcggaggg     300
accaagctga ccgtccta                                                   318
```

<210> 32
<211> 106
<212> PRT
<213> Homo sapiens

<400> 32

```
Ser Tyr Glu Leu Thr Gln Pro Ser Ser Val Ser Val Ser Pro Gly Gln
1               5                   10                  15

Thr Ala Arg Ile Thr Cys Ser Gly Asp Val Leu Ala Lys Lys Ser Ala
            20                  25                  30

Arg Trp Phe His Gln Lys Pro Gly Gln Ala Pro Val Leu Val Ile Tyr
        35                  40                  45

Lys Asp Ser Glu Arg Pro Ser Gly Ile Pro Glu Arg Phe Ser Gly Ser
    50                  55                  60

Ser Ser Gly Thr Thr Val Thr Leu Thr Ile Ser Gly Ala Gln Val Glu
65                  70                  75                  80

Asp Glu Ala Ala Tyr Tyr Cys Tyr Ser Ala Ala Asp Asn Asn Leu Val
                85                  90                  95

Phe Gly Gly Gly Thr Lys Leu Thr Val Leu
            100                 105
```

<210> 33
<211> 354
<212> DNA
<213> Homo sapiens

<400> 33

```
gaggtgcagc tggtgcagtc tggagcagag gtgaaaaagc ccggggagtc tctgaagatc     60

tcctgtaagg gttctggata cagctttccc agctactgga tcggctgggt gcgccagatg    120

cccgggaagg gcctggagtg gatggggatc atctatcctg gtgactctga taccagatac    180

agcccgtcct tccaaggcca ggtcaccatc tcagctgaca agtccatcag caccgcctac    240

ctgcagtgga gcagcctgaa ggcctcggac accgccatgt attactgtgc gagacacccct   300

atggaggacg gtatggacgt ctggggccaa gggaccacgg tcaccgtctc ctca          354
```

<210> 34
<211> 118
<212> PRT
<213> Homo sapiens

<400> 34

```
Glu Val Gln Leu Val Gln Ser Gly Ala Glu Val Lys Lys Pro Gly Glu
1               5                   10                  15

Ser Leu Lys Ile Ser Cys Lys Gly Ser Gly Tyr Ser Phe Pro Ser Tyr
            20                  25                  30

Trp Ile Gly Trp Val Arg Gln Met Pro Gly Lys Gly Leu Glu Trp Met
        35                  40                  45

Gly Ile Ile Tyr Pro Gly Asp Ser Asp Thr Arg Tyr Ser Pro Ser Phe
    50                  55                  60

Gln Gly Gln Val Thr Ile Ser Ala Asp Lys Ser Ile Ser Thr Ala Tyr

65                  70                  75                  80

Leu Gln Trp Ser Ser Leu Lys Ala Ser Asp Thr Ala Met Tyr Tyr Cys
                85                  90                  95

Ala Arg His Pro Met Glu Asp Gly Met Asp Val Trp Gly Gln Gly Thr
            100                 105                 110

Thr Val Thr Val Ser Ser
        115
```

<210> 35
<211> 321
<212> DNA
<213> Homo sapiens

<400> 35

```
tcctatgagc tgacacagcc accctcggtg tcagtgtccc caggacaaac ggccaggatc     60

acctgctctg gagatgcttt gccaaaaaaa tatgcttttt ggtaccagca gaagtcaggc    120

caggcccctg tgctggtcat ctatgacgac aacaaacgac cctccgggat ccctgagaga    180

ttctctggct ccagctcagg gacaatggcc accttgacta tcactggggc ccaggtggag    240

gatgaagctg actactactg ttactcaaca gacagcagtg gtcatcatgt attcggcgga    300

gggaccaagc tgaccgtcct a                                              321
```

<210> 36
<211> 107
<212> PRT
<213> Homo sapiens

<400> 36

```
Ser Tyr Glu Leu Thr Gln Pro Pro Ser Val Ser Val Ser Pro Gly Gln
1               5                   10                  15

Thr Ala Arg Ile Thr Cys Ser Gly Asp Ala Leu Pro Lys Lys Tyr Ala
            20                  25                  30

Phe Trp Tyr Gln Gln Lys Ser Gly Gln Ala Pro Val Leu Val Ile Tyr
        35                  40                  45

Asp Asp Asn Lys Arg Pro Ser Gly Ile Pro Glu Arg Phe Ser Gly Ser
    50                  55                  60

Ser Ser Gly Thr Met Ala Thr Leu Thr Ile Thr Gly Ala Gln Val Glu
65                  70                  75                  80

Asp Glu Ala Asp Tyr Tyr Cys Tyr Ser Thr Asp Ser Ser Gly His His
                85                  90                  95

Val Phe Gly Gly Gly Thr Lys Leu Thr Val Leu
            100                 105
```

<210> 37
<211> 375
<212> DNA
<213> Homo sapiens

<400> 37

caggtgcagc tggtggagtc tgggggaggc gtggtccagc ctgggaggtc cctgagactc 60

tcctgtgcag cgtctggatt caccttcagt agctatggca tgcactgggt ccgccaggct 120

ccaggcaagg ggctggagtg ggtggcagtt atatggtatg gtggaagtaa taaatactat 180

gcagactccg tgaagggccg attcaccatc tccagagaca attccaagaa cacgctgtat 240

ctgcaaatga acagcctgag agccgaggac acggctgtgt attactgtgc gagagatctg 300

gcagctcgtc gggggggacta ctactactac ggtatggacg tctggggcca agggaccacg 360

gtcaccgtct cctca 375

<210> 38
<211> 125
<212> PRT
<213> Homo sapiens

<400> 38

Gln Val Gln Leu Val Glu Ser Gly Gly Gly Val Val Gln Pro Gly Arg
1 5 10 15

Ser Leu Arg Leu Ser Cys Ala Ala Ser Gly Phe Thr Phe Ser Ser Tyr
20 25 30

Gly Met His Trp Val Arg Gln Ala Pro Gly Lys Gly Leu Glu Trp Val
35 40 45

Ala Val Ile Trp Tyr Gly Gly Ser Asn Lys Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Asp Leu Ala Ala Arg Arg Gly Asp Tyr Tyr Tyr Tyr Gly Met
100 105 110

Asp Val Trp Gly Gln Gly Thr Thr Val Thr Val Ser Ser

115 120 125

<210> 39
<211> 321
<212> DNA
<213> Homo sapiens

<400> 39

```
tcttctgagc tgactcagga ccctgttgtg tctgtggcct tgggacagac agtcaggatc      60
acttgccaag gcgacagcct cagaagctat tatttaagct ggtaccagca gaagccagga     120
caggcccctg tacttgtcat ctatggtaaa aacaaccggc cctcagggat cccagaccga     180
ttctctggct ccaactcagg aaacacagct tccttgacca tcactggggc tcaggcggaa     240
gatgaggctg actattactg taattcccgg gacagcagtg gtaaccatct gttcggcgga     300
gggaccaagc tgaccgtcct a                                               321
```

<210> 40
<211> 107
<212> PRT
<213> Homo sapiens

<400> 40

```
Ser Ser Glu Leu Thr Gln Asp Pro Val Val Ser Val Ala Leu Gly Gln
1               5                   10                  15

Thr Val Arg Ile Thr Cys Gln Gly Asp Ser Leu Arg Ser Tyr Tyr Leu
            20                  25                  30

Ser Trp Tyr Gln Gln Lys Pro Gly Gln Ala Pro Val Leu Val Ile Tyr
        35                  40                  45

Gly Lys Asn Asn Arg Pro Ser Gly Ile Pro Asp Arg Phe Ser Gly Ser
    50                  55                  60

Asn Ser Gly Asn Thr Ala Ser Leu Thr Ile Thr Gly Ala Gln Ala Glu
65                  70                  75                  80

Asp Glu Ala Asp Tyr Tyr Cys Asn Ser Arg Asp Ser Ser Gly Asn His
                85                  90                  95

Leu Phe Gly Gly Gly Thr Lys Leu Thr Val Leu
            100                 105
```

<210> 41
<211> 375
<212> DNA
<213> Homo sapiens

<220>
<221> misc_feature
<222> (81)..(81)
<223> n is c or t

<220>

<221> misc_feature
<222> (90) .. (90)
<223> n is a, c, g, or t

<220>
<221> misc_feature
<222> (105)..(105)
<223> n is c or t

<220>
<221> misc_feature
<222> (300)..(300)
<223> n is a, c, g, or t

<400> 41

```
gaggtgcagc tggtggagtc tgggggaggc ctggtcaagc ctgggggtc cctgagactc     60
tcctgtgcag cctctggata naccttcacn aactatatca tgcantgggt ccgccaggct    120
ccagggaagg ggctggagtg ggtctcatcc attagtatta gtagtagtta catatactac    180
gcagactcag tgaagggccg attcaccatc tccagagaca acgccaagaa ctcactgtat    240
ctgcaaatga acagcctgag agccgaggac acggctgtgt attactgtgc gagagatccn    300
gtaccactgg aacgacgcga ctactactac ggtatggacg tctggggcca agggaccacg    360
gtcaccgtct cctca                                                      375
```

<210> 42
<211> 125
<212> PRT
<213> Homo sapiens

<400> 42

```
Glu Val Gln Leu Val Glu Ser Gly Gly Gly Leu Val Lys Pro Gly Gly
1               5                   10                  15

Ser Leu Arg Leu Ser Cys Ala Ala Ser Gly Tyr Thr Phe Thr Asn Tyr
            20                  25                  30

Ile Met His Trp Val Arg Gln Ala Pro Gly Lys Gly Leu Glu Trp Val
        35                  40                  45

Ser Ser Ile Ser Ile Ser Ser Ser Tyr Ile Tyr Tyr Ala Asp Ser Val
    50                  55                  60

Lys Gly Arg Phe Thr Ile Ser Arg Asp Asn Ala Lys Asn Ser Leu Tyr
65                  70                  75                  80
```

Leu Gln Met Asn Ser Leu Arg Ala Glu Asp Thr Ala Val Tyr Tyr Cys
85 90 95

Ala Arg Asp Pro Val Pro Leu Glu Arg Arg Asp Tyr Tyr Tyr Gly Met
100 105 110

Asp Val Trp Gly Gln Gly Thr Thr Val Thr Val Ser Ser
115 120 125

<210> 43
<211> 330
<212> DNA
<213> Homo sapiens

<400> 43

cagtctgtgt tgacgcagcc gccctcaatg tctgcggccc caggacagaa ggtcaccatc 60

tcctgctctg gaagcagctc caacattggg aataattatg tatcctggta ccagcagctc 120

ccaggaacag cccccaaact cctcatttat gacaataata agcgaccctc agggattcct 180

gaccgattct ctggctccaa gtctggcacg tcagccaccc tgggcatcac cggactccag 240

actggggacg aggccgatta ttactgcgga acatgggata gcagcctgag cgctggggta 300

ttcggcggag ggaccaagct gaccgtccta 330

<210> 44
<211> 110
<212> PRT
<213> Homo sapiens

<400> 44

```
Gln Ser Val Leu Thr Gln Pro Pro Ser Met Ser Ala Ala Pro Gly Gln
1               5                   10                  15

Lys Val Thr Ile Ser Cys Ser Gly Ser Ser Ser Asn Ile Gly Asn Asn
            20                  25                  30

Tyr Val Ser Trp Tyr Gln Gln Leu Pro Gly Thr Ala Pro Lys Leu Leu
        35                  40                  45

Ile Tyr Asp Asn Asn Lys Arg Pro Ser Gly Ile Pro Asp Arg Phe Ser
    50                  55                  60

Gly Ser Lys Ser Gly Thr Ser Ala Thr Leu Gly Ile Thr Gly Leu Gln
65                  70                  75                  80

Thr Gly Asp Glu Ala Asp Tyr Tyr Cys Gly Thr Trp Asp Ser Ser Leu
                85                  90                  95

Ser Ala Gly Val Phe Gly Gly Gly Thr Lys Leu Thr Val Leu

          100                 105                 110
```

<210> 45
<211> 375
<212> DNA
<213> Homo sapiens

<400> 45

```
caggtgcagc tggtggagtc tgggggaggc gtggtccagc ctgggaggtc cctgagactc      60

tcctgtgcag cgtctggatt caccttcagt agctatggca tgcactgggt ccgccaggct     120

ccaggcaagg ggctggagtg ggtggcagtt atatggtatg atggaagtaa taaatactac     180

gcagactccg tgaagggccg attcaccatc tccagagaca attccaagaa cacgctgtat     240

ctgcaaatga acagcctgag agccgaggac acggctgtgt attactgtgc gagaacggag     300

ggtatagcag ctcgtctcta ctactactac ggtatggacg tctggggcca agggaccacg     360

gtcaccgtct cctca                                                      375
```

<210> 46
<211> 125
<212> PRT
<213> Homo sapiens

<400> 46

```
Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ala Val Ile Trp Tyr Asp Gly Ser Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Thr Glu Gly Ile Ala Ala Arg Leu Tyr Tyr Tyr Tyr Gly Met
            100                 105                 110

Asp Val Trp Gly Gln Gly Thr Thr Val Thr Val Ser Ser
        115                 120                 125
```

<210> 47
<211> 324
<212> DNA
<213> Homo sapiens

<400> 47

```
gaaattgtgt tgacgcagtc tccaggcacc ctgtctttgt ctccagggga aagagccacc      60

ctctcctgca gggccagtca gagtgttagc agcagctact tagcctggta ccagcagaaa     120

cctggccagg ctcccaggct cctcatctat ggtgcatcca gcagggccac tgacatccca     180

gacaggttca gtggcagtgg gtctgggaca gacttcactc tcaccatcag cagactggag     240

cctgaagatt ttgcagtgta ttactgtcag cagtatggta gctcaccgtg gacgttcggc     300

caagggacca aggtggaaat caaa                                            324
```

<210> 48
<211> 108
<212> PRT
<213> Homo sapiens

<400> 48

Glu Ile Val Leu Thr Gln Ser Pro Gly Thr Leu Ser Leu Ser Pro Gly
1 5 10 15

Glu Arg Ala Thr Leu Ser Cys Arg Ala Ser Gln Ser Val Ser Ser Ser
20 25 30

Tyr Leu Ala Trp Tyr Gln Gln Lys Pro Gly Gln Ala Pro Arg Leu Leu
35 40 45

Ile Tyr Gly Ala Ser Ser Arg Ala Thr Asp Ile Pro Asp Arg Phe Ser
50 55 60

Gly Ser Gly Ser Gly Thr Asp Phe Thr Leu Thr Ile Ser Arg Leu Glu
65 70 75 80

Pro Glu Asp Phe Ala Val Tyr Tyr Cys Gln Gln Tyr Gly Ser Ser Pro
85 90 95

Trp Thr Phe Gly Gln Gly Thr Lys Val Glu Ile Lys
100 105

<210> 49
<211> 111
<212> PRT
<213> Homo sapiens

<400> 49

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Gly Tyr
20 25 30

Tyr Met His Trp Val Arg Gln Ala Pro Gly Gln Gly Leu Glu Trp Met
35 40 45

Gly Trp Ile Asn Pro Asn Ser Gly Gly Thr Asn Tyr Ala Gln Lys Phe
50 55 60

Gln Gly Arg Val Thr Met Thr Arg Asp Thr Ser Ile Ser Thr Ala Tyr
65 70 75 80

Met Glu Leu Ser Arg Leu Arg Ser Asp Asp Thr Ala Val Tyr Tyr Cys
85 90 95

Ala Arg Arg Leu Trp Gly Gln Gly Thr Thr Val Thr Val Ser Ser
100 105 110

<210> 50
<211> 123
<212> PRT
<213> Homo sapiens

<400> 50

```
Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30

Ser Met Asn Trp Val Arg Gln Ala Pro Gly Lys Gly Leu Glu Trp Val
        35                  40                  45

Ser Ser Ile Ser Ser Ser Ser Ser Tyr Ile Tyr Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Val Gln Leu Glu Arg Tyr Tyr Tyr Tyr Tyr Gly Met Asp Val
            100                 105                 110

Trp Gly Gln Gly Thr Thr Val Thr Val Ser Ser
        115                 120
```

<210> 51
<211> 121
<212> PRT
<213> Homo sapiens

<400> 51

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Val Asp Thr Ala Met Val Tyr Tyr Gly Met Asp Val Trp Gly
            100                 105                 110

Gln Gly Thr Thr Val Thr Val Ser Ser
        115                 120
```

<210> 52
<211> 122
<212> PRT
<213> Homo sapiens

<400> 52

```
Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ala Val Ile Trp Tyr Asp Gly Ser Asn Lys Tyr Tyr Ala Asp Ser Val
    50                  55                  60
```

```
Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Ile Ala Ala Arg Tyr Tyr Tyr Tyr Tyr Gly Met Asp Val Trp
            100                 105                 110

Gly Gln Gly Thr Thr Val Thr Val Ser Ser
        115                 120
```

<210> 53
<211> 125
<212> PRT
<213> Homo sapiens

<400> 53

```
Gln Val Gln Leu Gln Glu Ser Gly Pro Gly Leu Val Lys Pro Ser Gln
1               5                   10                  15

Thr Leu Ser Leu Thr Cys Thr Val Ser Gly Gly Ser Ile Ser Ser Gly
            20                  25                  30

Gly Tyr Tyr Trp Ser Trp Ile Arg Gln His Pro Gly Lys Gly Leu Glu
        35                  40                  45

Trp Ile Gly Tyr Ile Tyr Tyr Ser Gly Ser Thr Tyr Tyr Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Ser Ser Val Thr Ala Ala Asp Thr Ala Val Tyr Tyr
                85                  90                  95

Cys Ala Arg Gly Ile Ala Ala Ala Gly Tyr Tyr Tyr Tyr Tyr Gly Met
            100                 105                 110

Asp Val Trp Gly Gln Gly Thr Thr Val Thr Val Ser Ser
        115                 120                 125
```

<210> 54
<211> 119
<212> PRT
<213> Homo sapiens

<400> 54

```
Gln Val Gln Leu Gln Glu Ser Gly Pro Gly Leu Val Lys Pro Ser Gln
1               5                   10                  15

Thr Leu Ser Leu Thr Cys Thr Val Ser Gly Gly Ser Ile Ser Ser Gly
            20                  25                  30

Gly Tyr Tyr Trp Ser Trp Ile Arg Gln His Pro Gly Lys Gly Leu Glu
        35                  40                  45

Trp Ile Gly Tyr Ile Tyr Tyr Ser Gly Ser Thr Tyr Tyr Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Ser Ser Val Thr Ala Ala Asp Thr Ala Val Tyr Tyr
                85                  90                  95

Cys Ala Arg Ile Thr Ile Phe Gly Val Phe Asp Tyr Trp Gly Gln Gly
            100                 105                 110

Thr Leu Val Thr Val Ser Ser
        115
```

<210> 55
<211> 122
<212> PRT
<213> Homo sapiens

<400> 55

```
Gln Val Gln Leu Gln Glu Ser Gly Pro Gly Leu Val Lys Pro Ser Gln
1               5                   10                  15

Thr Leu Ser Leu Thr Cys Thr Val Ser Gly Gly Ser Ile Ser Ser Gly
            20                  25                  30

Gly Tyr Tyr Trp Ser Trp Ile Arg Gln His Pro Gly Lys Gly Leu Glu
        35                  40                  45

Trp Ile Gly Tyr Ile Tyr Tyr Ser Gly Ser Thr Tyr Tyr Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Ser Ser Val Thr Ala Ala Asp Thr Ala Val Tyr Tyr
                85                  90                  95
```

```
Cys Ala Arg Val Ala Thr Tyr Tyr Tyr Tyr Tyr Gly Met Asp Val Trp
            100                 105                 110

Gly Gln Gly Thr Thr Val Thr Val Ser Ser
        115                 120
```

<210> 56
<211> 121
<212> PRT
<213> Homo sapiens

<400> 56

```
Gln Val Gln Leu Gln Glu Ser Gly Pro Gly Leu Val Lys Pro Ser Gln
1               5                   10                  15

Thr Leu Ser Leu Thr Cys Thr Val Ser Gly Gly Ser Ile Ser Ser Gly
            20                  25                  30

Gly Tyr Tyr Trp Ser Trp Ile Arg Gln His Pro Gly Lys Gly Leu Glu
        35                  40                  45

Trp Ile Gly Tyr Ile Tyr Tyr Ser Gly Ser Thr Tyr Tyr Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Ser Ser Val Thr Ala Ala Asp Thr Ala Val Tyr Tyr
                85                  90                  95

Cys Ala Arg Leu Arg Tyr Tyr Tyr Tyr Tyr Gly Met Asp Val Trp Gly
            100                 105                 110

Gln Gly Thr Thr Val Thr Val Ser Ser
        115                 120
```

<210> 57
<211> 120
<212> PRT
<213> Homo sapiens

<400> 57

```
Glu Val Gln Leu Val Gln Ser Gly Ala Glu Val Lys Lys Pro Gly Glu
1               5                   10                  15

Ser Leu Lys Ile Ser Cys Lys Gly Ser Gly Tyr Ser Phe Thr Ser Tyr
            20                  25                  30

Trp Ile Gly Trp Val Arg Gln Met Pro Gly Lys Gly Leu Glu Trp Met

        35                  40                  45

Gly Ile Ile Tyr Pro Gly Asp Ser Asp Thr Arg Tyr Ser Pro Ser Phe
    50                  55                  60

Gln Gly Gln Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Ala Arg Ser Ser Gly Tyr Tyr Tyr Ala Phe Asp Ile Trp Gly Gln Gly
            100                 105                 110

Thr Met Val Thr Val Ser Ser Ala
        115                 120
```

<210> 58
<211> 113
<212> PRT
<213> Homo sapiens

<400> 58

Glu Val Gln Leu Val Gln Ser Gly Ala Glu Val Lys Lys Pro Gly Glu
1 5 10 15

Ser Leu Lys Ile Ser Cys Lys Gly Ser Gly Tyr Ser Phe Thr Ser Tyr
20 25 30

Trp Ile Gly Trp Val Arg Gln Met Pro Gly Lys Gly Leu Glu Trp Met
35 40 45

Gly Ile Ile Tyr Pro Gly Asp Ser Asp Thr Arg Tyr Ser Pro Ser Phe
50 55 60

Gln Gly Gln Val Thr Ile Ser Ala Asp Lys Ser Ile Ser Thr Ala Tyr
65 70 75 80

Leu Gln Trp Ser Ser Leu Lys Ala Ser Asp Thr Ala Met Tyr Tyr Cys
85 90 95

Ala Arg Gly Met Asp Val Trp Gly Gln Gly Thr Thr Val Thr Val Ser
100 105 110

Ser

<210> 59
<211> 123
<212> PRT
<213> Homo sapiens

<400> 59

Glu Val Gln Leu Val Gln Ser Gly Ala Glu Val Lys Lys Pro Gly Glu
1               5                   10                  15

Ser Leu Lys Ile Ser Cys Lys Gly Ser Gly Tyr Ser Phe Thr Ser Tyr
            20                  25                  30

Trp Ile Gly Trp Val Arg Gln Met Pro Gly Lys Gly Leu Glu Trp Met
        35                  40                  45

Gly Ile Ile Tyr Pro Gly Asp Ser Asp Thr Arg Tyr Ser Pro Ser Phe
    50                  55                  60

Gln Gly Gln Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Ala Arg Gly Ile Ala Ala Ala Gly Tyr Tyr Tyr Gly Met Asp Val Trp
            100                 105                 110

Gly Lys Gly Thr Thr Val Thr Val Ser Ser Ala
        115                 120

<210> 60
<211> 112
<212> PRT
<213> Homo sapiens

<400> 60

Asp Ile Val Met Thr Gln Thr Pro Leu Ser Leu Ser Val Thr Pro Gly
1               5                   10                  15

Gln Pro Ala Ser Ile Ser Cys Lys Ser Ser Gln Ser Leu Leu His Ser
            20                  25                  30

Asp Gly Lys Thr Tyr Leu Tyr Trp Tyr Leu Gln Lys Pro Gly Gln Pro
        35                  40                  45

Pro Gln Leu Leu Ile Tyr Glu Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Met Gln Ser
85 90 95

Ile Gln Leu Pro Trp Thr Phe Gly Gln Gly Thr Lys Val Glu Ile Lys
100 105 110

<210> 61
<211> 108
<212> PRT
<213> Homo sapiens

<400> 61

Glu Ile Val Leu Thr Gln Ser Pro Gly Thr Leu Ser Leu Ser Pro Gly
1 5 10 15

Glu Arg Ala Thr Leu Ser Cys Arg Ala Ser Gln Ser Val Ser Ser Ser
20 25 30

Tyr Leu Ala Trp Tyr Gln Gln Lys Pro Gly Gln Ala Pro Arg Leu Leu
35 40 45

Ile Tyr Gly Ala Ser Ser Arg Ala Thr Gly Ile Pro Asp Arg Phe Ser
50 55 60

Gly Ser Gly Ser Gly Thr Asp Phe Thr Leu Thr Ile Ser Arg Leu Glu
65 70 75 80

Pro Glu Asp Phe Ala Val Tyr Tyr Cys Gln Gln Tyr Gly Ser Ser Pro
85 90 95

Trp Thr Phe Gly Gln Gly Thr Lys Val Glu Ile Lys
100 105

<210> 62
<211> 108
<212> PRT
<213> Homo sapiens

<400> 62

Glu Ile Val Leu Thr Gln Ser Pro Gly Thr Leu Ser Leu Ser Pro Gly
1 5 10 15

Glu Arg Ala Thr Leu Ser Cys Arg Ala Ser Gln Ser Val Ser Ser Ser
20 25 30

Tyr Leu Ala Trp Tyr Gln Gln Lys Pro Gly Gln Ala Pro Arg Leu Leu
35 40 45

```
Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Ser Ser Pro
                85                  90                  95

Tyr Thr Phe Gly Gln Gly Thr Lys Leu Glu Ile Lys
            100                 105
```

<210> 63
<211> 109
<212> PRT
<213> Homo sapiens

<400> 63

```
Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Ser Ser Pro
                85                  90                  95

Phe Thr Phe Gly Pro Gly Thr Lys Val Asp Ile Lys Arg
            100                 105
```

<210> 64
<211> 109
<212> PRT
<213> Homo sapiens

<400> 64

```
Gln Ser Val Leu Thr Gln Pro Pro Ser Val Ser Ala Ala Pro Gly Gln
1               5                   10                  15
```

Lys Val Thr Ile Ser Cys Ser Gly Ser Ser Ser Asn Ile Gly Asn Asn
20 25 30

Tyr Val Ser Trp Tyr Gln Gln Leu Pro Gly Thr Ala Pro Lys Leu Leu
35 40 45

Ile Tyr Asp Asn Asn Lys Arg Pro Ser Gly Ile Pro Asp Arg Phe Ser
50 55 60

Gly Ser Lys Ser Gly Thr Ser Ala Thr Leu Gly Ile Thr Gly Leu Gln
65 70 75 80

Thr Gly Asp Glu Ala Asp Tyr Tyr Cys Gly Thr Trp Asp Ser Ser Leu
85 90 95

Ser Ala Tyr Val Phe Gly Thr Gly Thr Lys Val Thr Val
100 105

<210> 65
<211> 110
<212> PRT
<213> Homo sapiens

<400> 65

Gln Ser Val Leu Thr Gln Pro Pro Ser Val Ser Ala Ala Pro Gly Gln
1 5 10 15

Lys Val Thr Ile Ser Cys Ser Gly Ser Ser Ser Asn Ile Gly Asn Asn
20 25 30

Tyr Val Ser Trp Tyr Gln Gln Leu Pro Gly Thr Ala Pro Lys Leu Leu
35 40 45

Ile Tyr Asp Asn Asn Lys Arg Pro Ser Gly Ile Pro Asp Arg Phe Ser
50 55 60

Gly Ser Lys Ser Gly Thr Ser Ala Thr Leu Gly Ile Thr Gly Leu Gln
65 70 75 80

Thr Gly Asp Glu Ala Asp Tyr Tyr Cys Gly Thr Trp Asp Ser Ser Leu
85 90 95

Ser Ala Val Val Phe Gly Gly Gly Thr Lys Leu Thr Val Leu
100 105 110

<210> 66
<211> 108
<212> PRT

<213> Homo sapiens

<400> 66

```
Ser Tyr Glu Leu Thr Gln Pro Pro Ser Val Ser Val Ser Pro Gly Gln
1               5                   10                  15

Thr Ala Arg Ile Thr Cys Ser Gly Asp Ala Leu Pro Lys Lys Tyr Ala
            20                  25                  30

Tyr Trp Tyr Gln Gln Lys Ser Gly Gln Ala Pro Val Leu Val Ile Tyr
        35                  40                  45

Glu Asp Ser Lys Arg Pro Ser Gly Ile Pro Glu Arg Phe Ser Gly Ser
    50                  55                  60

Ser Ser Gly Thr Met Ala Thr Leu Thr Ile Ser Gly Ala Gln Val Glu
65                  70                  75                  80

Asp Glu Ala Asp Tyr Tyr Cys Tyr Ser Thr Asp Ser Ser Gly Asn His
                85                  90                  95

Val Val Phe Gly Gly Gly Thr Lys Leu Thr Val Leu
            100                 105
```

<210> 67
<211> 108
<212> PRT
<213> Homo sapiens

<400> 67

```
Ser Tyr Glu Leu Thr Gln Pro Pro Ser Val Ser Val Ser Pro Gly Gln
1               5                   10                  15

Thr Ala Arg Ile Thr Cys Ser Gly Asp Ala Leu Pro Lys Lys Tyr Ala
            20                  25                  30

Tyr Trp Tyr Gln Gln Lys Ser Gly Gln Ala Pro Val Leu Val Ile Tyr
        35                  40                  45

Glu Asp Ser Lys Arg Pro Ser Gly Ile Pro Glu Arg Phe Ser Gly Ser
    50                  55                  60

Ser Ser Gly Thr Met Ala Thr Leu Thr Ile Ser Gly Ala Gln Val Glu
65                  70                  75                  80

Asp Glu Ala Asp Tyr Tyr Cys Tyr Ser Thr Asp Ser Ser Gly Asn His
                85                  90                  95

Val Val Phe Gly Gly Gly Thr Lys Leu Thr Val Leu

                    100                 105
```

<210> 68
<211> 108
<212> PRT
<213> Homo sapiens

<400> 68

```
Ser Ser Glu Leu Thr Gln Asp Pro Ala Val Ser Val Ala Leu Gly Gln
1               5                   10                  15

Thr Val Arg Ile Thr Cys Gln Gly Asp Ser Leu Arg Ser Tyr Tyr Ala
            20                  25                  30

Ser Trp Tyr Gln Gln Lys Pro Gly Gln Ala Pro Val Leu Val Ile Tyr
        35                  40                  45

Gly Lys Asn Asn Arg Pro Ser Gly Ile Pro Asp Arg Phe Ser Gly Ser
    50                  55                  60

Ser Ser Gly Asn Thr Ala Ser Leu Thr Ile Thr Gly Ala Gln Ala Glu
65                  70                  75                  80

Asp Glu Ala Asp Tyr Tyr Cys Asn Ser Arg Asp Ser Ser Gly Asn His
                85                  90                  95

Val Val Phe Gly Gly Gly Thr Lys Leu Thr Val Leu
            100                 105
```

<210> 69
<211> 106
<212> PRT
<213> Homo sapiens

<400> 69

```
Ser Tyr Glu Leu Thr Gln Pro Ser Ser Val Ser Val Ser Pro Gly Gln
1               5                   10                  15

Thr Ala Arg Ile Thr Cys Ser Gly Asp Val Leu Ala Lys Lys Tyr Ala
            20                  25                  30

Arg Trp Phe Gln Gln Lys Pro Gly Gln Ala Pro Val Leu Val Ile Tyr
        35                  40                  45

Lys Asp Ser Glu Arg Pro Ser Gly Ile Pro Glu Arg Phe Ser Gly Ser
    50                  55                  60

Ser Ser Gly Thr Thr Val Thr Leu Thr Ile Ser Gly Ala Gln Val Glu
65                  70                  75                  80

Asp Glu Ala Asp Tyr Tyr Cys Tyr Ser Ala Ala Asp Asn Asn Val Val
                85                  90                  95

Phe Gly Gly Gly Thr Lys Leu Thr Val Leu
            100                 105
```

<210> 70
<211> 372
<212> DNA
<213> Homo sapiens

<400> 70

```
caggtgcagc tgcaggagtc gggcccagga ctggtgaagc cttcacagac cctgtccctc      60
acctgcactg tctctggtgg ctccatcagc agtggtgttt actactggac ctggatccgc     120
cagcacccag ggaacggcct ggagtggatt ggctacatct attacagtgg gagcacctcc     180
tacaacccgt ccctcaagag tcgagttacc atatcagtag acacgtctaa gaaacagttc     240
tccctgaagc tgacctctgt gactgccgcg gacacggccg tgtattactg tgcgagagaa     300
ggaccactac gggggacta ctactacggt ctggacgtct ggggccaagg gaccacggtc      360
accgtctcct ca                                                         372
```

<210> 71
<211> 124
<212> PRT
<213> Homo sapiens

<400> 71

```
Gln Val Gln Leu Gln Glu Ser Gly Pro Gly Leu Val Lys Pro Ser Gln
1               5                   10                  15

Thr Leu Ser Leu Thr Cys Thr Val Ser Gly Gly Ser Ile Ser Ser Gly
            20                  25                  30

Val Tyr Tyr Trp Thr Trp Ile Arg Gln His Pro Gly Asn Gly Leu Glu
        35                  40                  45

Trp Ile Gly Tyr Ile Tyr Tyr Ser Gly Ser Thr Ser Tyr Asn Pro Ser
    50                  55                  60

Leu Lys Ser Arg Val Thr Ile Ser Val Asp Thr Ser Lys Lys Gln Phe
65                  70                  75                  80

Ser Leu Lys Leu Thr Ser Val Thr Ala Ala Asp Thr Ala Val Tyr Tyr
                85                  90                  95

Cys Ala Arg Glu Gly Pro Leu Arg Gly Asp Tyr Tyr Tyr Gly Leu Asp

                100                 105                 110

    Val Trp Gly Gln Gly Thr Thr Val Thr Val Ser Ser
            115                 120
```

<210> 72
<211> 324
<212> DNA
<213> Homo sapiens

<400> 72

```
gaaattgtgt tgacgcagtc tccaggcacc ctgtctttgt ctccagggga aagagccacc     60
ctctcctgca gggccggtca gactattagc agtcgctact tagcctggta ccagcagaaa    120
cctggccagg ctcccaggcc cctcatctat ggtgcatcca gcagggccac tggcatccca    180
gacaggttca gtggcagtgg gtctgggaca gacttcactc tcaccatcag cagactggag    240
cctgaagatt ttgcagtgta ttactgtcag cagtatggta gctcacctcg gacgttcggc    300
caagggacca aggtggaaat caaa                                           324
```

<210> 73
<211> 108
<212> PRT
<213> Homo sapiens

<400> 73

```
Glu Ile Val Leu Thr Gln Ser Pro Gly Thr Leu Ser Leu Ser Pro Gly
1               5                   10                  15

Glu Arg Ala Thr Leu Ser Cys Arg Ala Gly Gln Thr Ile Ser Ser Arg
            20                  25                  30

Tyr Leu Ala Trp Tyr Gln Gln Lys Pro Gly Gln Ala Pro Arg Pro Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Ser Ser Pro
                85                  90                  95

Arg Thr Phe Gly Gln Gly Thr Lys Val Glu Ile Lys
            100                 105
```

<210> 74
<211> 375
<212> DNA
<213> Homo sapiens

<400> 74

```
caggtgcagc tgcaggagtc gggcccagga ctggtgaagc cttcacagac cctgtccctc      60
acctgcactg tctctggtgg ctccatcagc agtggtggtt actactggag ctggatccgc     120
cagcacccag ggaagggcct ggagtggatt gggtacatct attacagtgg gagaacctac     180
aacaacccgt ccctcaagag tcgagttacc atatcagtag acacgtctaa gaaccagttc     240
tccctgaagt tgagttctgt gactgccgcg gacacggccg tgtattactg tgcgagagtg     300
gctacgggga gagcggacta ccacttctac gctatggacg tctggggcca agggaccacg     360
gtcaccgtct cctca                                                      375
```

<210> 75
<211> 125
<212> PRT
<213> Homo sapiens

<400> 75

```
Gln Val Gln Leu Gln Glu Ser Gly Pro Gly Leu Val Lys Pro Ser Gln
1               5                   10                  15

Thr Leu Ser Leu Thr Cys Thr Val Ser Gly Gly Ser Ile Ser Ser Gly
            20                  25                  30

Gly Tyr Tyr Trp Ser Trp Ile Arg Gln His Pro Gly Lys Gly Leu Glu
        35                  40                  45

Trp Ile Gly Tyr Ile Tyr Tyr Ser Gly Arg Thr Tyr Asn Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Ser Ser Val Thr Ala Ala Asp Thr Ala Val Tyr Tyr
                85                  90                  95

Cys Ala Arg Val Ala Thr Gly Arg Ala Asp Tyr His Phe Tyr Ala Met
            100                 105                 110

Asp Val Trp Gly Gln Gly Thr Thr Val Thr Val Ser Ser
        115                 120                 125
```

<210> 76
<211> 318
<212> DNA
<213> Homo sapiens

<400> 76

```
tcctatgagc tgacacagcc atcctcagtg tcagtgtctc cgggacagac agccaggatc     60
acctgctcag gagatgtact ggcaaaaaag tctgctcggt ggttccacca gaagccaggc    120
caggcccctg tactggtgat ttataaagac agtgagcggc cctcagggat ccctgagcgc    180
ttctccggct ccagctcagg gaccacagtc accttgacca tcagcggggc ccaggttgag    240
gatgaggctg cctattactg ttactctgcg gctgacaaca atctggtatt cggcggaggg    300
accaagctga ccgtccta                                                  318
```

<210> 77
<211> 106
<212> PRT
<213> Homo sapiens

<400> 77

```
Ser Tyr Glu Leu Thr Gln Pro Ser Ser Val Ser Val Ser Pro Gly Gln
1               5                   10                  15

Thr Ala Arg Ile Thr Cys Ser Gly Asp Val Leu Ala Lys Lys Ser Ala
            20                  25                  30

Arg Trp Phe His Gln Lys Pro Gly Gln Ala Pro Val Leu Val Ile Tyr
        35                  40                  45

Lys Asp Ser Glu Arg Pro Ser Gly Ile Pro Glu Arg Phe Ser Gly Ser
    50                  55                  60

Ser Ser Gly Thr Thr Val Thr Leu Thr Ile Ser Gly Ala Gln Val Glu
65                  70                  75                  80

Asp Glu Ala Ala Tyr Tyr Cys Tyr Ser Ala Ala Asp Asn Asn Leu Val
                85                  90                  95

Phe Gly Gly Gly Thr Lys Leu Thr Val Leu
            100                 105
```

<210> 78
<211> 339
<212> DNA
<213> Homo sapiens

<400> 78

caggtgcagc tggtgcagtc tggggctgag gtgaagaagc ctggggcctc agtgaaggtc 60

tcctgcaagg cttctggata caccttcacc ggctactata tgcactgggt gcgacaggcc 120

cctggacaag ggcttgagtg gatgggatgg atcaacccta aaagtggtga cacaaactat 180

gcacagaagt ttcagggcag ggtcaccatg accagggaca cgtccaccag cacagcctac 240

atggagctga gcaggctgag atctgacgac acggccgtgt attactgtgc gagaaggttg 300

gacgtctggg gccaagggac cacggtcacc gtctcctca 339

<210> 79
<211> 113
<212> PRT
<213> Homo sapiens

<400> 79

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Gly Tyr
20 25 30

Tyr Met His Trp Val Arg Gln Ala Pro Gly Gln Gly Leu Glu Trp Met
35 40 45

Gly Trp Ile Asn Pro Lys Ser Gly Asp Thr Asn Tyr Ala Gln Lys Phe
50 55 60

Gln Gly Arg Val Thr Met Thr Arg Asp Thr Ser Thr Ser Thr Ala Tyr
65 70 75 80

Met Glu Leu Ser Arg Leu Arg Ser Asp Asp Thr Ala Val Tyr Tyr Cys
85 90 95

Ala Arg Arg Leu Asp Val Trp Gly Gln Gly Thr Thr Val Thr Val Ser
100 105 110

Ser

<210> 80
<211> 333
<212> DNA
<213> Homo sapiens

<400> 80

```
cagtctgtgt tgacgcagcc gccctcagtg tctgcggccc caggacagaa ggtcaccatc      60
tcctgctctg gaagcagctc caacattggg aataattatg tatcctggta ccagcagctc     120
ccaggaacag cccccaaact cctcatttat gacaataata agcgaccctc aggaattcct     180
gaccgattct ctggctccaa gtctggcacg tcagccaccc tgggcatcac cggactccag     240
actggggacg aggccgatta ttactgcgga acatggaata gcagcctgag tgctggttat     300
gtcttcggaa ctgggaccaa ggtcaccgtc cta                                  333
```

<210> 81
<211> 111
<212> PRT
<213> Homo sapiens

<400> 81

```
Gln Ser Val Leu Thr Gln Pro Pro Ser Val Ser Ala Ala Pro Gly Gln
1               5                   10                  15

Lys Val Thr Ile Ser Cys Ser Gly Ser Ser Ser Asn Ile Gly Asn Asn
            20                  25                  30

Tyr Val Ser Trp Tyr Gln Gln Leu Pro Gly Thr Ala Pro Lys Leu Leu
        35                  40                  45

Ile Tyr Asp Asn Asn Lys Arg Pro Ser Gly Ile Pro Asp Arg Phe Ser
    50                  55                  60

Gly Ser Lys Ser Gly Thr Ser Ala Thr Leu Gly Ile Thr Gly Leu Gln
65                  70                  75                  80

Thr Gly Asp Glu Ala Asp Tyr Tyr Cys Gly Thr Trp Asn Ser Ser Leu
                85                  90                  95

Ser Ala Gly Tyr Val Phe Gly Thr Gly Thr Lys Val Thr Val Leu
            100                 105                 110
```

<210> 82
<211> 339
<212> DNA
<213> Homo sapiens

<400> 82

```
caggtgcagc tggtgcagtc tggggctgag gtgaagaagc ctggggcctc agtgaaggtc     60
tcctgcaagg cttctggata caccttcacc ggctactata tgcactgggt gcgacaggcc    120
cctggacaag ggcttgagtg gatgggatgg atcaacccta aaagtggtga cacaaactat    180
gcacagaagt ttcagggcag ggtcaccctg accagggaca cgtccaccag cacagcctac    240
atggagctga gcaggctgag atctgacgac acggccgtgt attactgtgc gagaaggttg    300
gacgtctggg gccaagggac cacggtcacc gtctcctca                           339
```

<210> 83
<211> 113
<212> PRT
<213> Homo sapiens

<400> 83

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
        35                  40                  45

Gly Trp Ile Asn Pro Lys Ser Gly Asp Thr Asn Tyr Ala Gln Lys Phe
    50                  55                  60

Gln Gly Arg Val Thr Leu Thr Arg Asp Thr Ser Thr Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Arg Leu Asp Val Trp Gly Gln Gly Thr Thr Val Thr Val Ser
            100                 105                 110

Ser
```

<210> 84
<211> 333
<212> DNA
<213> Homo sapiens

<400> 84

```
cagtctgtgt tgacgcagcc gccctcagtg tctgcggccc caggacagaa ggtcaccatc     60
tcctgctctg gaagcagctc caacattggg aataattatg tatcctggta ccagcagctc    120
ccaggaacag cccccaaact cctcatttat gacaataata agcgaccctc aggaattcct    180
gaccgattct ctggctccaa gtctggcacg tcagccaccc tgggcatcac cggactccag    240
actggggacg aggccgatta ttactgcgga acatgggata gcagcctgag tgctggttat    300
gtcttcggaa ctgggaccaa ggtcaccgtc cta                                 333
```

<210> 85
<211> 111
<212> PRT
<213> Homo sapiens

<400> 85

```
Gln Ser Val Leu Thr Gln Pro Pro Ser Val Ser Ala Ala Pro Gly Gln
1               5                   10                  15

Lys Val Thr Ile Ser Cys Ser Gly Ser Ser Ser Asn Ile Gly Asn Asn
            20                  25                  30

Tyr Val Ser Trp Tyr Gln Gln Leu Pro Gly Thr Ala Pro Lys Leu Leu
        35                  40                  45

Ile Tyr Asp Asn Asn Lys Arg Pro Ser Gly Ile Pro Asp Arg Phe Ser
    50                  55                  60

Gly Ser Lys Ser Gly Thr Ser Ala Thr Leu Gly Ile Thr Gly Leu Gln
65                  70                  75                  80

Thr Gly Asp Glu Ala Asp Tyr Tyr Cys Gly Thr Trp Asp Ser Ser Leu
                85                  90                  95

Ser Ala Gly Tyr Val Phe Gly Thr Gly Thr Lys Val Thr Val Leu
            100                 105                 110
```

<210> 86
<211> 339
<212> DNA
<213> Homo sapiens

<400> 86

```
caggtgcagc tggtgcagtc tggggctgag gtgaagaagc ctggggcctc agtgaaggtc      60
tcctgcaagg cttctggata caccttcacc ggctactata tgcactgggt gcgacaggcc     120
cctggacaag ggcttgagtg gatgggatgg atcaacccta aaagtggtga cacaaactat     180
gcacagaagt ttcagggcag ggtcaccatg accagggaca cgtccaccag cacagcctac     240
atggagctga gcaggctgag atctgacgac acggccgtgt attactgtgc gagaaggttg     300
gacgtctggg gccaagggac cacggtcacc gtctcctca                            339
```

<210> 87
<211> 113
<212> PRT
<213> Homo sapiens

<400> 87

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
        35                  40                  45

Gly Trp Ile Asn Pro Lys Ser Gly Asp Thr Asn Tyr Ala Gln Lys Phe
    50                  55                  60

Gln Gly Arg Val Thr Met Thr Arg Asp Thr Ser Thr Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Arg Leu Asp Val Trp Gly Gln Gly Thr Thr Val Thr Val Ser
            100                 105                 110

Ser
```

<210> 88
<211> 333
<212> DNA
<213> Homo sapiens

<400> 88

```
cagtctgtgt tgacgcagcc gccctcagtg tctgcggccc caggacagaa ggtcaccatc     60
tcctgctctg gaagcagctc caacattggg aataattatg tatcctggta ccagcagctc    120
ccaggaacag cccccaaact cctcatttat gacaataata agcgaccctc aggaattcct    180
gaccgattct ctggctccaa gtctggcacg tcagccaccc tgggcatcac cggactccag    240
actggggacg aggccgatta ttactgcgga acatgggata gcagcctgag tgctggttat    300
gtcttcggaa ctgggaccaa ggtcaccgtc cta                                 333
```

<210> 89
<211> 111
<212> PRT
<213> Homo sapiens

<400> 89

```
Gln Ser Val Leu Thr Gln Pro Pro Ser Val Ser Ala Ala Pro Gly Gln
1               5                   10                  15

Lys Val Thr Ile Ser Cys Ser Gly Ser Ser Ser Asn Ile Gly Asn Asn
            20                  25                  30

Tyr Val Ser Trp Tyr Gln Gln Leu Pro Gly Thr Ala Pro Lys Leu Leu
        35                  40                  45

Ile Tyr Asp Asn Asn Lys Arg Pro Ser Gly Ile Pro Asp Arg Phe Ser
    50                  55                  60

Gly Ser Lys Ser Gly Thr Ser Ala Thr Leu Gly Ile Thr Gly Leu Gln
65                  70                  75                  80

Thr Gly Asp Glu Ala Asp Tyr Tyr Cys Gly Thr Trp Asp Ser Ser Leu
                85                  90                  95

Ser Ala Gly Tyr Val Phe Gly Thr Gly Thr Lys Val Thr Val Leu
            100                 105                 110
```

<210> 90
<211> 383
<212> DNA
<213> Homo sapiens

<400> 90

```
caggtgcagc tgcaggagtc gggcccagga ctggtgaagc cttcacagac cctgtccctc      60
acctgcactg tctctggtgg ctccatcagc agtggtggtt actactggag ctggatccgc     120
cagcacccag ggaagggcct ggagtggatt gggtacatct attacagtgg gagaacctac     180
aacaacccgt ccctcaagag tcgagttacc atatcagtag acacgtctaa gaaccagttc     240
tccctgaagt tgagttctgt gactgccgcg gacacggccg tgtattactg tgcgagagtg     300
gctacgggga gaggggacta ccacttctac gctatggacg tctggggcca agggaccacg     360
gtcaccgtct cctcagcctc cac                                             383
```

<210> 91
<211> 125
<212> PRT
<213> Homo sapiens

<400> 91

```
Gln Val Gln Leu Gln Glu Ser Gly Pro Gly Leu Val Lys Pro Ser Gln
1               5                   10                  15

Thr Leu Ser Leu Thr Cys Thr Val Ser Gly Gly Ser Ile Ser Ser Gly
            20                  25                  30

Gly Tyr Tyr Trp Ser Trp Ile Arg Gln His Pro Gly Lys Gly Leu Glu
        35                  40                  45

Trp Ile Gly Tyr Ile Tyr Tyr Ser Gly Arg Thr Tyr Asn Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Ser Ser Val Thr Ala Ala Asp Thr Ala Val Tyr Tyr
                85                  90                  95

Cys Ala Arg Val Ala Thr Gly Arg Gly Asp Tyr His Phe Tyr Ala Met
            100                 105                 110

Asp Val Trp Gly Gln Gly Thr Thr Val Thr Val Ser Ser
        115                 120                 125
```

<210> 92
<211> 318
<212> DNA
<213> Homo sapiens

<400> 92

```
tcctatgagc tgacacagcc atcctcagtg tcagtgtctc cgggacagac agccaggatc     60
acctgctcag gagatgtact ggcaaaaaag tctgctcggt ggttccacca gaagccaggc    120
caggcccctg tactggtgat ttataaagac agtgagcggc cctcagggat ccctgagcgc    180
ttctccggct ccagctcagg gaccacagtc accttgacca tcagcggggc ccaggttgag    240
gatgaggctg actattactg ttactctgcg gctgacaaca atctggtatt cggcggaggg    300
accaagctga ccgtccta                                                  318
```

<210> 93
<211> 106
<212> PRT
<213> Homo sapiens

<400> 93

```
Ser Tyr Glu Leu Thr Gln Pro Ser Ser Val Ser Val Ser Pro Gly Gln
1               5                   10                  15

Thr Ala Arg Ile Thr Cys Ser Gly Asp Val Leu Ala Lys Lys Ser Ala
            20                  25                  30

Arg Trp Phe His Gln Lys Pro Gly Gln Ala Pro Val Leu Val Ile Tyr
        35                  40                  45

Lys Asp Ser Glu Arg Pro Ser Gly Ile Pro Glu Arg Phe Ser Gly Ser
    50                  55                  60

Ser Ser Gly Thr Thr Val Thr Leu Thr Ile Ser Gly Ala Gln Val Glu
65                  70                  75                  80

Asp Glu Ala Asp Tyr Tyr Cys Tyr Ser Ala Ala Asp Asn Asn Leu Val
                85                  90                  95

Phe Gly Gly Gly Thr Lys Leu Thr Val Leu
            100                 105
```

<210> 94
<211> 375
<212> DNA
<213> Homo sapiens

<400> 94

```
caggtgcagc tgcaggagtc gggcccagga ctggtgaagc cttcacagac cctgtccctc      60
acctgcactg tctctggtgg ctccatcagc agtggtggtt actactggag ctggatccgc     120
cagcacccag ggaagggcct ggagtggatt gggtacatct attacagtgg gagaacctac     180
aacaacccgt ccctcaagag tcgagttacc atatcagtag acacgtctaa gaaccagttc     240
tccctgaagt tgagttctgt gactgccgcg gacacggccg tgtattactg tgcgagagtg     300
gctacgggga gagcggacta ccacttctac gctatggacg tctggggcca agggaccacg     360
gtcaccgtct cctca                                                      375
```

<210> 95
<211> 125
<212> PRT
<213> Homo sapiens

<400> 95

```
Gln Val Gln Leu Gln Glu Ser Gly Pro Gly Leu Val Lys Pro Ser Gln
1               5                   10                  15

Thr Leu Ser Leu Thr Cys Thr Val Ser Gly Gly Ser Ile Ser Ser Gly
            20                  25                  30

Gly Tyr Tyr Trp Ser Trp Ile Arg Gln His Pro Gly Lys Gly Leu Glu
        35                  40                  45

Trp Ile Gly Tyr Ile Tyr Tyr Ser Gly Arg Thr Tyr Asn Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Ser Ser Val Thr Ala Ala Asp Thr Ala Val Tyr Tyr
                85                  90                  95

Cys Ala Arg Val Ala Thr Gly Arg Ala Asp Tyr His Phe Tyr Ala Met
            100                 105                 110

Asp Val Trp Gly Gln Gly Thr Thr Val Thr Val Ser Ser
        115                 120                 125
```

<210> 96
<211> 318
<212> DNA
<213> Homo sapiens

<400> 96

```
tcctatgagc tgacacagcc atcctcagtg tcagtgtctc cgggacagac agccaggatc      60

acctgctcag gagatgtact ggcaaaaaag tctgctcggt ggttccacca gaagccaggc     120

caggcccctg tactggtgat ttataaagac agtgagcggc cctcagggat ccctgagcgc     180

ttctccggct ccagctcagg gaccacagtc accttgacca tcagcggggc ccaggttgag     240

gatgaggctg actattactg ttactctgcg gctgacaaca atctggtatt cggcggaggg     300

accaagctga ccgtccta                                                   318
```

<210> 97
<211> 106
<212> PRT
<213> Homo sapiens

<400> 97

```
Ser Tyr Glu Leu Thr Gln Pro Ser Ser Val Ser Val Ser Pro Gly Gln
1               5                   10                  15

Thr Ala Arg Ile Thr Cys Ser Gly Asp Val Leu Ala Lys Lys Ser Ala
            20                  25                  30

Arg Trp Phe His Gln Lys Pro Gly Gln Ala Pro Val Leu Val Ile Tyr
        35                  40                  45

Lys Asp Ser Glu Arg Pro Ser Gly Ile Pro Glu Arg Phe Ser Gly Ser
    50                  55                  60

Ser Ser Gly Thr Thr Val Thr Leu Thr Ile Ser Gly Ala Gln Val Glu
65                  70                  75                  80

Asp Glu Ala Asp Tyr Tyr Cys Tyr Ser Ala Ala Asp Asn Asn Leu Val
                85                  90                  95

Phe Gly Gly Gly Thr Lys Leu Thr Val Leu
            100                 105
```

## Description

### Field

The field relates, in some aspects, to methods of treating and diagnosing αVβ6 overexpressing cancer. In some embodiments, the field relates to methods of treating and diagnosing αVβ6 and HER2 overexpressing cancer. In some embodiments, combination therapy strategies are employed.

### Background

Some of the most aggressive and invasive subtypes of breast cancer are those that overexpress Human Epidermal Growth Factor Receptor 2 (HER2), a member of the receptor tyrosine kinase family of receptors comprising of HER1-HER4. HER2 is overexpressed in 25-30% of breast cancer and is responsible for imparting a more invasive phenotype to breast cancer cells although the actual mechanisms are not fully known. The introduction of the humanized antibody trastuzumab, which blocks downstream signaling from HER2, has resulted in reductions in recurrence and mortality of HER2-positive (HER2+) breast cancer patients. Unfortunately, over 70% of patients either have de novo, or develop, resistance to trastuzumab leaving these patients without molecular-specific treatment options. Thus, identifying improved therapies for women with HER2+ breast cancer is required urgently.

Several studies have implicated dysregulation of the P13K/Akt pathway as a resistance mechanism in HER2+ breast cancer. Akt, however, is involved in many non-cancer related pathways hence inhibition may lead to many off-target and potentially undesirable effects, therefore a more cancer-specific target is desired. Thus, additional mechanisms of how HER2 actually promotes invasion and how the up-regulation of PI3K signaling promotes trastuzumab-resistance must be discovered.

Integrins are αβ heterodimeric transmembrane cell-surface receptors for extracellular proteins including some cell-surface proteins. They integrate the extracellular environment with the intracellular cytoskeletal and signaling machinery, transducing spatial-temporal messages that modulate cell behavior. Thus, integrins are central components of most normal cell processes including adhesion, migration, growth, survival and differentiation. Dysregulation of integrin expression and or signaling correlate with development of cancer through inappropriately regulating the processes above but also mediating invasion and metastasis. The integrin αVβ6, which is expressed only by epithelial cells, usually is only detectable on cells undergoing tissue-remodeling as occurs in wound healing, development, chronic inflammation and cancer. Involvement, however, of integrins, such as αVβ6, in certain cancers, especially breast cancer, has not yet been elucidated.

J. J. Barron et al.: "HER2 Testing and Subsequent Trastuzumab Treatment for Breast Cancer in a Managed Care Environment", The Oncologist, 14 August 2009 suggets HER2 testing and subsequent trastuzumab treatment for breast cancer.

### SUMMARY

The invention is defined in the appended claims. The summary of the disclosure and the detailed technical disclosure set out below may in some respects go beyond the disclosure of the invention per se, and may also provide technical background for related technical developments. It will be appreciated that this technical disclosure is not intended to define the invention as such (which is defined exclusively by the appended claims), but rather to place it in a broader technical context. Accordingly, it will be appreciated that the various aspects described are not intended to portray as part of the invention subject-matter that does not fall within the scope of the appended claims. It has presently been shown that αVβ6 may promote a more aggressive phenotype in breast cancer and offers a novel therapeutic target, in some embodiments for patients with trastuzumab-resistance.

It is accordingly an object to detect and treat cancer cells that are sensitive to αVβ6 inhibition, including, but not limited to, breast cancer and breast cancers resistant to trastuzumab. It is also an object to detect and treat cancer cells that are sensitive to both αVβ6 and HER2 inhibition, including, but not limited to, breast cancer and breast cancers resistant to trastuzumab.

One aspect includes, a method of treating a malignant tumor in an animal comprising administering to said animal in need thereof a therapeutically effective dose of:

a. an αVβ6 targeted binding agent that specifically binds to αVβ6 and inhibits binding of ligands to αVβ6; and
b. optionally a combination therapy agent.

Another aspect includes a method of inhibiting growth of tumor cells comprising administering to the tumor cells a therapeutically effective dose of:

a. an $\alpha V\beta 6$ targeted binding agent that specifically binds to $\alpha V\beta 6$ and inhibits binding of ligands to $\alpha V\beta 6$; and
b. optionally a combination therapy agent.

A further aspect includes a method of suppressing growth of trastuzumab-resistant tumor cells comprising administering to said cells a therapeutically effective dose of:

a. an $\alpha V\beta 6$ targeted binding agent that specifically binds to $\alpha V\beta 6$ and inhibits binding of ligands to $\alpha V\beta 6$; and

b. a HER2 targeted binding agent that specifically binds to HER2 and inhibits binding of ligands to HER2.

Yet another aspect includes a method of diagnosing breast cancer sensitive to $\alpha V\beta 6$ and HER2 inhibition in a patient comprising analyzing a patient sample for the presence or absence of tumor cells overexpressing $\alpha V\beta 6$ and HER2 by measuring the expression levels of $\alpha V\beta 6$ and HER2, wherein the patient is diagnosed with breast cancer sensitive to $\alpha V\beta 6$ and HER2 inhibition if $\alpha V\beta 6$ and HER2 are both overexpressed.

A further embodiment includes a method for diagnosing and treating cancer sensitive to $\alpha V\beta 6$ inhibition in a patient comprising analyzing a patient sample for the presence or absence of cancer cells overexpressing $\alpha V\beta 6$ by measuring the levels of $\alpha V\beta 6$, wherein the patient is diagnosed with cancer sensitive to $\alpha V\beta 6$ inhibition if $\alpha V\beta 6$ is overexpressed, and administering to the diagnosed patient a therapeutically effective dose of:

a. an $\alpha V\beta 6$ targeted binding agent that specifically binds to $\alpha V\beta 6$ and inhibits binding of ligands to $\alpha V\beta 6$.

Moreover, one embodiment includes a method for diagnosing and treating breast cancer sensitive to HER2 inhibition in a patient comprising analyzing a patient sample for the presence or absence of breast cancer cells overexpressing $\alpha V\beta 6$ and HER2 by measuring the levels of the $\alpha V\beta 6$ and HER2, wherein the patient is diagnosed with breast cancer sensitive to $\alpha V\beta 6$ and HER2 inhibition if both $\alpha V\beta 6$ and HER2 are overexpressed, and administering to the diagnosed patient a therapeutically effective dose of:

a. a HER2 targeted binding agent that specifically binds to HER2 and inhibits binding of ligands to HER2.

Another embodiment includes a method for diagnosing and treating breast cancer sensitive to $\alpha V\beta 6$ and HER2 inhibition in a patient comprising analyzing a patient sample for the presence or absence of breast cancer cells overexpressing $\alpha V\beta 6$ and HER2 by measuring the levels of the $\alpha V\beta 6$ and HER2, wherein the patient is diagnosed with breast cancer sensitive to $\alpha V\beta 6$ and HER2 inhibition if both $\alpha V\beta 6$ and HER2 are overexpressed, and administering to the diagnosed patient a therapeutically effective dose of:

a. an $\alpha V\beta 6$ targeted binding agent that specifically binds to $\alpha V\beta 6$ and inhibits binding of ligands to $\alpha V\beta 6$; and

b. a HER2 targeted binding agent that specifically binds to HER2 and inhibits binding of ligands to HER2.

A further aspect includes method for treating cancer sensitive to $\alpha V\beta 6$ inhibition in a patient sample comprising requesting a test to determine whether a patient sample contains cancer cells overexpressing $\alpha V\beta 6$, and administering a therapeutically effective dose of:

a. an $\alpha V\beta 6$ targeted binding agent that specifically binds to $\alpha V\beta 6$ and inhibits binding of ligands to $\alpha V\beta 6$

if the patient sample contains cancer cells overexpressing $\alpha V\beta 6$.

Yet an additional aspect includes a method for treating breast cancer sensitive to $\alpha V\beta 6$ and HER2 inhibition in a patient sample comprising requesting a test to determine whether a patient sample contains cancer cells overexpressing $\alpha V\beta 6$ and HER2, and administering a therapeutically effective dose of:

a. an $\alpha V\beta 6$ targeted binding agent that specifically binds to $\alpha V\beta 6$ and inhibits binding of ligands to $\alpha V\beta 6$; and

b. a HER2 targeted binding agent that specifically binds to HER2 and inhibits binding of ligands to HER2

if the patient sample contains cancer cells overexpressing $\alpha V\beta 6$ and HER2.

An additional embodiment includes a method for diagnosing cancer sensitive to $\alpha V\beta 6$ inhibition in a patient that can be treated by inhibiting $\alpha V\beta 6$ comprising:

a. obtaining a biological sample from the subject;

b. applying an αVβ6 targeted binding agent that specifically binds to αVβ6 to the sample, wherein the presence of αVβ6 creates a αVβ6 targeted binding agent-αVβ6 complex;

c. diagnosing an aggressive form of breast cancer where the complex of step b) is detected at a level indicating αVβ6 overexpression.

Another aspect includes a method for diagnosing breast cancer sensitive to αVβ6 and HER2 inhibition in a patient that can be treated by inhibiting αVβ6 and HER2 comprising:

a. obtaining a biological sample from the subject;

b. applying an αVβ6 targeted binding agent that specifically binds to αVβ6 to the sample, wherein the presence of αVβ6 creates a αVβ6 targeted binding agent-αVβ6 complex;

c. optionally applying a HER2 targeted binding agent that specifically binds to HER2 to the sample, wherein the presence of HER2 creates a HER2 binding agent-HER2 complex; and

d. diagnosing an aggressive form of breast cancer where the complexes of steps b) and c) are detected at a level indicating αVβ6 and HER2 overexpression.

Additional objects and advantages will be set forth in part in the description which follows, and in part will be obvious from the description, or may be learned by practice. The objects and advantages will be realized and attained by means of the elements and combinations particularly pointed out in the appended claims.

It is to be understood that both the foregoing general description and the following detailed description are exemplary and explanatory only and are not restrictive of the claims.

The accompanying drawings, which are incorporated in and constitute a part of this specification, illustrate one (several) embodiment(s) and together with the description, serve to explain the principles described herein.

## BRIEF DESCRIPTION OF THE DRAWINGS

Figures 1A-F are entitled "High co-expression of integrin αVβ6 and HER2 predict poor survival in breast cancer patients." Kaplan-Meier curves by integrin αVβ6 expression status. Tick marks indicate patients who were still alive at the time of analyses or who were censored. All P values refer to log-rank tests. (A) Normal and (B) cancerous breast cancer tissue sections immunohistochemically stained for integrin αVβ6 (brown staining) using 6.2G2 antibody (Biogen Idec). Overall survival in 2 cohorts of breast cancer patients from London (C) and Nottingham (D) by integrin αVβ6 status (high expression in red, low in black). The P value for patients with high integrin αVβ6 versus low expression in tumors is <0.00001. (E) Overall survival of HER2+ patients from the combined London and Nottingham patient cohorts by integrin αVβ6 status. The P value for patients with high integrin αVβ6 status versus low tumors is <0.001. (F) Overall survival of HER2+ patients from the METABRIC cohort by integrin αVβ6 status. The survival of patients with high ITGB6 expressing tumors versus low expressing tumors is significantly lower (P=0.003). Please also see Figures 10 and 11.

Figures 2A-G are entitled "Breast cancer cell line invasion is both integrin αVβ6 and HER2·dependent." (A) Expression of integrin αVβ6 and HER2 in a breast cancer cell line panel assessed by flow cytometry. Isotype controls are shown in black, integrin αVβ6 in blue and HER2 expression in red (see Figure 12 for full panel of cell lines analyzed). (B) Transwell invasion assay of breast cancer cell lines expressing varying levels of integrin αVβ6 and HER2. 5 x $10^4$ cells/well were seeded and the number of cells that invaded was counted after 72h. (C) & (D), Breast cancer cell line invasion is integrin αVβ6-dependent. Cells were subjected to either 30 min incubation with IgG or αVβ6 blocking antibody (β6 Ab)(101Jg/ml) (C) or 72h transfection with control or J36 siRNA (201JM) (D) and subject to a transwell invasion assay as before. (E) & (F), Breast cancer cell line invasion is HER2-dependent. Cells were pre-treated for 30 min with IgG or Trastuzumab (TRA) (1 01Jg/ml) (E) or transfected for 72h with control or HER2 siRNA (201JM) (F) and subject to a transwell invasion assay. (G) Cells were pre-treated for 30 min with IgG, P6 Ab, TRA (all 101Jg/ml) or a combination of the blocking antibodies & subject to a transwell invasion assay. All experiments were performed in triplicate, representative experiments shown (n=6±SD). *P=0.05, **P=0.01, ***P<0.001. Please also see Figure 7.

Figures 3A-C are entitled "HER2-driven invasion is integrin αVβ6-dependent. Transwell invasion assay of cell lines overexpressing integrin αVβ6 and HER2." Cells were pre-treated for 30 min with IgG, HRGβ (1 μM) in the presence and absence of αVβ6 blocking antibody (10 μg/ml) (A) or trastuzumab (TRA) (10 μg/ml) (B) and 5 x $10^4$ cells/well seeded into a Transwell invasion assay. The number of cells invaded was counted after 72h. All experiments were performed in triplicate, representative experiments shown (n=6±SD). * P =0.05, **P=0.01, ***P<0.001. (C) Organotypic invasion of MCF10.CA1a (CAla) cell line. Cells were pre-treated for 30 min with IgG, αVβ6 blocking antibody or TRA (10 μg/ml) or transfected with siRNA to αVβ6 or HER2 for 72h (20 μM) prior to seeding. 5 x $10^4$ cells were seeded on top of a collagen:Matrigel gel containing MRC5/hTERT fibroblasts. Gels were fixed in formal saline after 5-7 days incubation. Gels were paraffin embedded, sectioned and sections subject to H&E staining. Magnification bar = 10 μM. Histograms quantify the invasion of each cell with the aforementioned treatments as invasion index. Experiments were performed in triplicate (n=2/experiment), representative experiments shown. * P=0.05, **P=0.01, ***P<0.001.

Figures 4A-G are entitled "Breast cancer xenograft growth is αVβ6-dependent." (A) Mice bearing human BT -474 tumors were treated with IgG (black), 264RAD (blue), trastuzumab (TRA) (red) or 264RAD+TRA (green)(10 mg/kg; i.p) twice weekly for 2 consecutive weeks. Data are presented as mean tumor volume ±SEM (n≥4 mice/group). Treatment commenced when tumors reached 100mm$^3$. (B) Mice bearing human HER2-18 tumors were treated as in (A). (C) Photographic images of representative BT -474 and HER2-18 xenografts posttreatment outlined in (A). Magnification bar=5mm. (D) BT -474 xenograft protein expression. Xenografts were treated as in (A), harvested, protein extracted and subject to immunoblotting. Blots were probed for indicated proteins. (E) Histograms of relative protein expression from blots shown in (D) determined by optical density (n=3 individual tumors ±SEM). *P=0.05, **P=0.01, ***P<0.001. (F & G) HER2-18 xenograft protein expression and quantification as outlined in (D & E).

Figure 5 is entitled "264RAD enhances the anti-tumorigenicity of trastuzumab and inhibits human xenograft MCF-7/HER2-18 cell growth, prolongs survival and reduces αVβ6, HER2, HER3, Akt2 and Smad2 in SCID mice." Mice bearing human MCF-7/HER2-18 tumors were treated with IgG (black), 264RAD (blue), trastuzumab (TRA) (red) or 264RAD+TRA (green) (10mg/kg; i.p) twice weekly for 6 consecutive weeks. Data are presented as mean tumor volume± SEM (n>5 mice/group). Treatment commenced when tumors were 4mm in any one dimension (A), and when tumors reached 200mm$^3$ (n>6 mice/group) (B). (C) Kaplan-Meier survival plot shows survival of mice from study of larger tumors shown in (B). (D) Tumors from treated mice in (A) were analyzed by immunoblotting for indicated targets (combination therapy treated xenografts were eradicated hence were unavailable for analysis). Actin immunoblot shows equal protein input. (E) Histogram quantifying changes in protein expression levels from (D) (β-actin corrected). (F) Immunohistochemical analysis of αVβ6 expression in HER2-18 tumor xenografts. Xenografts were fixed, sectioned and stained for P6 expression after 6 weeks treatment as outlined in (A) or for 2 weeks with 264RAD+trastuzumab (264RAD+TRA). Magnification bar=101JM.

Figures 6A-D are entitled "High co-expression of integrin αVβ6 and HER2 predict poor long-term survival in breast cancer patients." Kaplan-Meier curves by integrin αVβ6 expression status. The tick marks indicate patients who were still alive at the time of the analyses or who were censored. All P values refer to log-rank tests. 15-year overall survival of breast cancer patients from London (A) and Nottingham (B) cohorts by integrin αVβ6 status. The P value for patients with high integrin αVβ6 (red) versus low expression (black) in tumors is P=0.006 and P=0.002 respectively. (C) 15-year overall survival of HER2-positive patients from the combined London and Nottingham patient cohorts by integrin αVβ6 status. The P value for patients with high integrin αVβ6 status versus low tumors is < 0.001. (D) 15-year overall survival of HER2-positive patients from the METABRIC cohort by ITGB6 gene status. The P value for patients with high integrin αVβ6 status versus low expression tumors is P=0.048.

Figures 7A-C are as follows. (A) 264RAD is as effective as 1005 αVβ6 blocking antibody at inhibiting invasion in HER2-18 and CAla cells. Cells overexpressing integrin αVβ6 and HER2 were pre-treated for 30 min with IgG, or αVβ6 blocking antibodies 1005 or 264RAD (10 μg/ml) and 5 x $10^4$ cells/well seeded into a transwell invasion assay. The number cells invaded was counted after 72h. All experiments were performed in triplicate, representative experiments shown (n=6±SD). *P=0.05, **P=0.01, ***P<0.001. (B) Proliferation was unaffected by αVβ6 and/or HER2 antibody blockade over 7 days. 0·5-2 x $10^3$ cells/well were seeded 24h prior to 48h growth in double-charcoal stripped FCS media. After 48h, cells were treated for 7 days with IgG, αVβ6 blocking antibody 264RAD, trastuzumab (TRA) (all 10 μg/ml) or a combination of the blocking antibodies. Cells were subject to the MTS assay after 7 days and 'proliferation' (representing mitochondrial activity) plotted relative to day 7 IgG treated cells. All experiments were performed in triplicate, representative experiments shown (n=6±SD). (C) αVβ6 and HER2 co-localize in the cell membrane. MCF-7/HER2-18 (HER2-18) and MCF10.CA1a (CAla) cells were labeled with αVβ6 in red (1005, Millipore) and HER2 in green (Cell Signaling Technology) antibodies with secondary conjugates of Alexa-488 and Alexa647 respectively. Nuclear staining was performed using DAPI (blue). Samples were imaged on a Zeiss LSM710 confocal microscope. Magnification bar= 10 μM.

Figure 8 is entitled "Invasion is not TGFβ-dependent and blockade of αVβ6 inhibits invasion in the presence and absence of TGFβ ligand or TGFβRII in vitro." Transwell Matrigel invasion assay of cell lines overexpressing integrin αVβ6 and HER2. Cells were subject to TGFβRII siRNA treatment for 72h prior to treatment with 264RAD (10 μg/ml) in the presence and absence of TGFβ (5ng/ml) and 5 x $10^4$ cells/well seeded into a transwell invasion assay. The number cells invaded was counted after 72h. All experiments were performed in triplicate, representative experiments shown (n=6±SD). *P=0.05, **P=0.01, ***P<0.001.

Figure 9 is entitled :Integrin αVβ6-dependent invasion is via Akt2." Transwell invasion assay of cell lines overexpressing integrin αVβ6 and HER2. Cells were pre-treated for 72h transfection with control or Akt1, Akt2 or Akt1 +2 siRNA (20nM) (A) and 5 x $10^4$ cells/well seeded into a Transwell invasion assay. The number of cells invaded was counted after 72h. All experiments were performed in triplicate, representative experiments shown (n=6±SD). *Insert* Representative immunoblot of siRNA protein knockdown. * P =0.05, **P=0.01. (B) Organotypic invasion of MCF10.CA1a (CAla) cell line. Cells were pre-treated as in (A) prior to seeding. 5 x $10^4$ cells were seeded on top of a collagen:Matrigel gel containing MRC5/hTERT fibroblasts. Gels were fixed in formal saline after 5-7 days incubation. Gels were paraffin embedded, sectioned and sections subject to H&E staining. Magnification bar = 10 μM. Histogram quantifies the invasion with the aforementioned treatments as invasion index. Experiments were performed in triplicate (n=2/experiment), representative experiments shown. * P=0.05, **P=0.01.

Figure 10 is a table entitled "clinicopathological characteristics of the London and Nottingham cohorts of breast cancer patients."

Figure 11 is a table entitled "association of αVβ6 with conventional prognostic indicators in breast cancer."

Figure 12 is a table entitled "αVβ6 and HER2 Expression and receptor status in a panel of breast cancer cell lines." Molecular Subtype & receptor status defined by Neve et al (2006) & Subik et al (2010). Invasive Propensity as determined by invasion through matrigel. Expression determined by flow cytometry as Mean fluorescence Intensity (MFI): 0-10 = -, 11-25 = +, 26-50 = ++, 51-100 = +++, > 100 = ++++, ND, not determined.

Figure 13 is a list of antibodies utilized in a study of αVβ6 and HER2 expression in breast cancer.

Figure 14 is a line graph showing the ability of the purified monoclonal antibodies to bind to αVβ6 and block its binding to a GST-LAP peptide.

Figures 15A and B are line graphs showing a plot of the averaged Geometric Mean Fluorescence (GMF) as a function of molecular mAb concentration, which was used to estimate the binding affinity of one of the antibodies to K562 cells that stably express the human αVβ6 antigen. Shown in Figure 15A is affinity data for mAb 188. Figure 15B shows affinity data for mAb 264 RAD.

Figures 16A-E are bar graphs showing the ability of the purified monoclonal antibodies to mediate complement-dependent cytotoxicity in 293 cells stably expressing αVβ6 integrin.

Figure 17 is a bar graph showing the ability of antibodies 264RAD, 133 and 188 SDM to inhibit tumor growth using the Detroit-562 nasopharyngeal cell line.

Figure 18 is a bar chart showing comparison of the activity of 264 RAD with 264 RAD/ADY.

## SEQUENCE LISTING

Embodiments include the specific anti- αVβ6 antibodies listed below in Table 1. This table reports the identification number of each anti- αVβ6 antibody, along with the SEQ ID number of the variable domain of the corresponding heavy chain and light chain genes. Each antibody has been given an identification number that includes the letters "sc" followed by a number.

**Table 1**

| mAb ID No.: | Sequence | SEQ ID NO: |
|---|---|---|
| sc 49 | Nucleotide sequence encoding the variable region of the heavy chain | 1 |
| | Amino acid sequence encoding the variable region of the heavy chain | 2 |
| | Nucleotide sequence encoding the variable region of the light chain | 3 |
| | Amino acid sequence encoding the variable region of the light chain | 4 |
| sc 58 | Nucleotide sequence encoding the variable region of the heavy chain | 5 |
| | Amino acid sequence encoding the variable region of the heavy chain | 6 |
| | Nucleotide sequence encoding the variable region of the light chain | 7 |
| | Amino acid sequence encoding the variable region of the light chain | 8 |

(continued)

| mAb ID No.: | Sequence | SEQ ID NO: |
|---|---|---|
| sc 97 | Nucleotide sequence encoding the variable region of the heavy chain | 9 |
| | Amino acid sequence encoding the variable region of the heavy chain | 10 |
| | Nucleotide sequence encoding the variable region of the light chain | 11 |
| | Amino acid sequence encoding the variable region of the light chain | 12 |
| sc 133 | Nucleotide sequence encoding the variable region of the heavy chain | 13 |
| | Amino acid sequence encoding the variable region of the heavy chain | 14 |
| | Nucleotide sequence encoding the variable region of the light chain | 15 |
| | Amino acid sequence encoding the variable region of the light chain | 16 |
| sc 161 | Nucleotide sequence encoding the variable region of the heavy chain | 17 |
| | Amino acid sequence encoding the variable region of the heavy chain | 18 |
| | Nucleotide sequence encoding the variable region of the light chain | 19 |
| | Amino acid sequence encoding the variable region of the light chain | 20 |
| sc 188 | Nucleotide sequence encoding the variable region of the heavy chain | 21 |
| | Amino acid sequence encoding the variable region of the heavy chain | 22 |
| | Nucleotide sequence encoding the variable region of the light chain | 23 |
| | Amino acid sequence encoding the variable region of the light chain | 24 |
| sc 254 | Nucleotide sequence encoding the variable region of the heavy chain | 25 |
| | Amino acid sequence encoding the variable region of the heavy chain | 26 |
| | Nucleotide sequence encoding the variable region of the light chain | 27 |
| | Amino acid sequence encoding the variable region of the light chain | 28 |
| sc 264 | Nucleotide sequence encoding the variable region of the heavy chain | 29 |
| | Amino acid sequence encoding the variable region of the heavy chain | 30 |
| | Nucleotide sequence encoding the variable region of the light chain | 31 |
| | Amino acid sequence encoding the variable region of the light chain | 32 |
| sc 277 | Nucleotide sequence encoding the variable region of the heavy chain | 33 |
| | Amino acid sequence encoding the variable region of the heavy chain | 34 |
| | Nucleotide sequence encoding the variable region of the light chain | 35 |
| | Amino acid sequence encoding the variable region of the light chain | 36 |
| sc 298 | Nucleotide sequence encoding the variable region of the heavy chain | 37 |
| | Amino acid sequence encoding the variable region of the heavy chain | 38 |
| | Nucleotide sequence encoding the variable region of the light chain | 39 |
| | Amino acid sequence encoding the variable region of the light chain | 40 |
| sc 320 | Nucleotide sequence encoding the variable region of the heavy chain | 41 |
| | Amino acid sequence encoding the variable region of the heavy chain | 42 |
| | Nucleotide sequence encoding the variable region of the light chain | 43 |
| | Amino acid sequence encoding the variable region of the light chain | 44 |

(continued)

| mAb ID No.: | Sequence | SEQ ID NO: |
|---|---|---|
| sc 374 | Nucleotide sequence encoding the variable region of the heavy chain | 45 |
| | Amino acid sequence encoding the variable region of the heavy chain | 46 |
| | Nucleotide sequence encoding the variable region of the light chain | 47 |
| | Amino acid sequence encoding the variable region of the light chain | 48 |
| sc 188 SDM | Nucleotide sequence encoding the variable region of the heavy chain | 70 |
| | Amino acid sequence encoding the variable region of the heavy chain | 71 |
| | Nucleotide sequence encoding the variable region of the light chain | 72 |
| | Amino acid sequence encoding the variable region of the light chain | 73 |
| sc 264 RAD | Nucleotide sequence encoding the variable region of the heavy chain | 74 |
| | Amino acid sequence encoding the variable region of the heavy chain | 75 |
| | Nucleotide sequence encoding the variable region of the light chain | 76 |
| | Amino acid sequence encoding the variable region of the light chain | 77 |
| sc 133 TMT | Nucleotide sequence encoding the variable region of the heavy chain | 78 |
| | Amino acid sequence encoding the variable region of the heavy chain | 79 |
| | Nucleotide sequence encoding the variable region of the light chain | 80 |
| | Amino acid sequence encoding the variable region of the light chain | 81 |
| sc 133 WDS | Nucleotide sequence encoding the variable region of the heavy chain | 82 |
| | Amino acid sequence encoding the variable region of the heavy chain | 83 |
| | Nucleotide sequence encoding the variable region of the light chain | 84 |
| | Amino acid sequence encoding the variable region of the light chain | 85 |
| sc 133 TMT/W DS | Nucleotide sequence encoding the variable region of the heavy chain | 86 |
| | Amino acid sequence encoding the variable region of the heavy chain | 87 |
| | Nucleotide sequence encoding the variable region of the light chain | 88 |
| | Amino acid sequence encoding the variable region of the light chain | 89 |
| sc 264 ADY | Nucleotide sequence encoding the variable region of the heavy chain | 90 |
| | Amino acid sequence encoding the variable region of the heavy chain | 91 |
| | Nucleotide sequence encoding the variable region of the light chain | 92 |
| | Amino acid sequence encoding the variable region of the light chain | 93 |
| sc 264 RAD/A DY | Nucleotide sequence encoding the variable region of the heavy chain | 94 |
| | Amino acid sequence encoding the variable region of the heavy chain | 95 |
| | Nucleotide sequence encoding the variable region of the light chain | 96 |
| | Amino acid sequence encoding the variable region of the light chain | 97 |

## DESCRIPTION OF THE EMBODIMENTS

Reference will now be made in detail to the present embodiment(s) (exemplary embodiments), an example(s) of which is (are) illustrated in the accompanying drawings. Wherever possible, the same reference numbers will be used throughout the drawings to refer to the same or like parts.

## I. Definitions

Unless otherwise defined, scientific and technical terms used herein shall have the meanings that are commonly understood by those of ordinary skill in the art. Further, unless otherwise required by context, singular terms shall include pluralities and plural terms shall include the singular. Generally, nomenclatures utilized in connection with, and techniques of, cell and tissue culture, molecular biology, and protein and oligo- or polynucleotide chemistry and hybridization described herein are those well-known and commonly used in the art.

Standard techniques are used for recombinant DNA, oligonucleotide synthesis, and tissue culture and transformation (e.g., electroporation, lipofection). Enzymatic reactions and purification techniques are performed according to manufacturer's specifications or as commonly accomplished in the art or as described herein. The foregoing techniques and procedures are generally performed according to conventional methods well known in the art and as described in various general and more specific references that are cited and discussed throughout the present specification. *See e.g.,* Sambrook et al., Molecular Cloning: A Laboratory Manual (3rd ed., Cold Spring Harbor Laboratory Press, Cold Spring Harbor, N.Y. (2001)), which is incorporated herein by reference. The nomenclatures utilized in connection with, and the laboratory procedures and techniques of, analytical chemistry, synthetic organic chemistry, and medicinal and pharmaceutical chemistry described herein are those well-known and commonly used in the art. Standard techniques are used for chemical syntheses, chemical analyses, pharmaceutical preparation, formulation, and delivery, and treatment of patients.

As utilized in accordance with the present disclosure, the following terms, unless otherwise indicated, shall be understood to have the following meanings:

The term "and/or" as used herein is to be taken as specific disclosure of each of the two specified features or components with or without the other. For example "A and/or B" is to be taken as specific disclosure of each of (i) A, (ii) B and (iii) A and B, just as if each is set out individually herein.

An antagonist may be a polypeptide, nucleic acid, carbohydrate, lipid, small molecular weight compound, an oligonucleotide, an oligopeptide, RNA interference (RNAi), antisense, a recombinant protein, an antibody, or conjugates or fusion proteins thereof. For a review of RNAi see Milhavet O, Gary DS, Mattson MP. (Pharmacol Rev. 2003 Dec;55(4):629-48. Review.) and antisense see Opalinska JB, Gewirtz AM. (Sci STKE. 2003 Oct 28;2003 (206):pe47.)

Disease-related aberrant activation or expression of "$\alpha V\beta 6$" may be any abnormal, undesirable or pathological cell adhesion, for example tumor-related cell adhesion. Cell adhesion-related diseases include, but are not limited to, non-solid tumors such as leukemia, multiple myeloma or lymphoma, and also solid tumors such as melanoma, small cell lung cancer, non-small cell lung cancer, glioma, hepatocellular (liver) carcinoma, glioblastoma, carcinoma of the thyroid, bile duct, bone, gastric, brain/CNS, head and neck, hepatic system, stomach, prostate, breast, renal, testicle, ovary, skin, cervix, lung, muscle, neuron, oesophageal, bladder, lung, uterus, vulva, endometrium, kidney, colorectum, pancreas, pleural/peritoneal membranes, salivary gland, and epidermous.

A compound refers to any small molecular weight compound with a molecular weight of less than about 2000 Daltons.

The term "$\alpha V\beta 6$" refers to the heterodimer integrin molecule consisting of an $\alpha V$ chain and a $\beta 6$ chain.

The term "neutralizing" when referring to a targeted binding agent, such as an antibody, relates to the ability of said targeted binding agent to eliminate, or significantly reduce, the activity of a target antigen. Accordingly, a "neutralizing" anti-$\alpha V\beta 6$ antibody is capable of eliminating or significantly reducing the activity of $\alpha V\beta 6$. A neutralizing $\alpha V\beta 6$ antibody may, for example, act by blocking the binding of TGF$\beta$LAP to the integrin $\alpha V\beta 6$. By blocking this binding, $\alpha V\beta 6$ mediated cell adhesion is significantly, or completely, eliminated. Ideally, a neutralizing antibody against $\alpha V\beta 6$ inhibits cell adhesion.

The term "isolated polynucleotide" as used herein shall mean a polynucleotide that has been isolated from its naturally occurring environment. Such polynucleotides may be genomic, cDNA, or synthetic. In some embodiments, isolated polynucleotides not associated with all or a portion of the polynucleotides they associate with in nature. The isolated polynucleotides may be operably linked to another polynucleotide that it is not linked to in nature. In addition, isolated polynucleotides may not occur in nature as part of a larger sequence.

The term "isolated protein" referred to herein means a protein that has been isolated from its naturally occurring environment. Such proteins may be derived from genomic DNA, cDNA, recombinant DNA, recombinant RNA, or synthetic origin or some combination thereof, which by virtue of its origin, or source of derivation, the "isolated protein" (1) is not associated with proteins found in nature, (2) is free of other proteins from the same source, *e.g.* free of murine proteins, (3) is expressed by a cell from a different species, or (4) does not occur in nature.

The term "polypeptide" is used herein as a generic term to refer to native protein, fragments, or analogs of a polypeptide sequence. Hence, native protein, fragments, and analogs are species of the polypeptide genus. Polypeptides may comprise the human heavy chain immunoglobulin molecules and the human kappa light chain immunoglobulin molecules, as well as antibody molecules formed by combinations comprising the heavy chain immunoglobulin molecules with light chain immunoglobulin molecules, such as the kappa or lambda light chain immunoglobulin molecules, and vice versa, as well as fragments and analogs thereof. Polypeptides may also comprise solely the human heavy chain immunoglobulin molecules or fragments thereof.

The term "naturally-occurring" as used herein as applied to an object refers to the fact that an object can be found in nature. For example, a polypeptide or polynucleotide sequence that is present in an organism (including viruses) that can be isolated from a source in nature and which has not been intentionally modified by man in the laboratory or otherwise is naturally-occurring.

The term "operably linked" as used herein refers to positions of components so described that are in a relationship permitting them to function in their intended manner. For example, a control sequence "operably linked" to a coding sequence is connected in such a way that expression of the coding sequence is achieved under conditions compatible with the control sequences.

The term "polynucleotide" as referred to herein means a polymeric form of nucleotides of at least 10 bases in length, either ribonucleotides or deoxynucleotides or a modified form of either type of nucleotide, or RNA-DNA heteroduplexes. The term includes single and double stranded forms of DNA.

The term "oligonucleotide" referred to herein includes naturally occurring, and modified nucleotides linked together by naturally occurring, and non-naturally occurring linkages. Oligonucleotides are a polynucleotide subset generally comprising a length of 200 bases or fewer. Oligonucleotides may be 10 to 60 bases in length, in other embodiments, they may be 12, 13, 14, 15, 16, 17, 18, 19, or 20 to 40 bases in length. Oligonucleotides are usually single stranded, *e.g.* for probes; although oligonucleotides may be double stranded, *e.g.* for use in the construction of a gene mutant. Oligonucleotides can be either sense or antisense oligonucleotides.

The term "naturally occurring nucleotides" referred to herein includes deoxyribonucleotides and ribonucleotides. The term "modified nucleotides" referred to herein includes nucleotides with modified or substituted sugar groups and the like. The term "oligonucleotide linkages" referred to herein includes oligonucleotides linkages such as phosphorothioate, phosphorodithioate, phosphoroselenoate, phosphorodiselenoate, phosphoroanilothioate, phosphoraniladate, phosphoroamidate, and the like. *See e.g.,* LaPlanche et al., Nucl. Acids Res. 14:9081 (1986); Stec et al., J. Am. Chem. Soc. 106:6077 (1984); Stein et al., Nucl. Acids Res. 16:3209 (1988); Zon et al., Anti-Cancer Drug Design 6:539 (1991); Zon et al., Oligonucleotides and Analogues: A Practical Approach, pp. 87-108 (F. Eckstein, Ed., Oxford University Press, Oxford England (1991)); Stec et al., U.S. Patent No. 5,151,510; Uhlmann and Peyman Chemical Reviews 90:543 (1990), the disclosures of which are hereby incorporated by reference. An oligonucleotide can include a label for detection, if desired.

The term "selectively hybridize" referred to herein means to detectably and specifically bind. Polynucleotides, oligonucleotides and fragments thereof selectively hybridize to nucleic acid strands under hybridization and wash conditions that minimize appreciable amounts of detectable binding to nonspecific nucleic acids. High stringency conditions can be used to achieve selective hybridization conditions as known in the art and discussed herein. Generally, the nucleic acid sequence homology between the polynucleotides, oligonucleotides, or antibody fragments and a nucleic acid sequence of interest will be at least 80%, and more typically with increasing homologies of at least 85%, 90%, 95%, 99%, and 100%.

The term "CDR region" or "CDR" is intended to indicate the hypervariable regions of the heavy and light chains of the immunoglobulin as defined by Kabat *et al.*, 1991 (Kabat, E.A. et al., (1991) Sequences of Proteins of Immunological Interest, 5th Edition. US Department of Health and Human Services, Public Service, NIH, Washington), and later editions. An antibody typically contains 3 heavy chain CDRs and 3 light chain CDRs. The term CDR or CDRs is used here in order to indicate, according to the case, one of these regions or several, or even the whole, of these regions which contain the majority of the amino acid residues responsible for the binding by affinity of the antibody for the antigen or the epitope which it recognizes.

Among the six short CDR sequences, the third CDR of the heavy chain (HCDR3) has a greater size variability (greater diversity essentially due to the mechanisms of arrangement of the genes which give rise to it). It may be as short as 2 amino acids although the longest size known is 26. CDR length may also vary according to the length that can be accommodated by the particular underlying framework. Functionally, HCDR3 plays a role in part in the determination of the specificity of the antibody (Segal et al., PNAS, 71:4298-4302, 1974, Amit et al., Science, 233:747-753, 1986, Chothia et al., J. Mol. Biol., 196:901-917, 1987, Chothia et al., Nature, 342:877- 883, 1989, Caton et al., J. Immunol., 144:1965-1968, 1990, Sharon et al., PNAS, 87:4814-4817, 1990, Sharon et al., J. Immunol., 144:4863-4869, 1990, Kabat et al., J. Immunol., 147:1709-1719, 1991).

The term a "set of CDRs" referred to herein comprises CDR1, CDR2 and CDR3. Thus, a set of HCDRs refers to HCDR1, HCDR2 and HCDR3 (HCDR refers to a variable heavy chain CDR), and a set of LCDRs refers to LCDR1, LCDR2 and LCDR3 (LCDR refers to a variable light chain CDR). Unless otherwise stated, a "set of CDRs" includes HCDRs and LCDRs.

Two amino acid sequences are "homologous" if there is a partial or complete identity between their sequences. For example, 85% homology means that 85% of the amino acids are identical when the two sequences are aligned for maximum matching. Gaps (in either of the two sequences being matched) are allowed in maximizing matching; gap lengths of 5 or less are used in some embodiments, with 2 or less being used in other embodiments. Alternatively, two protein sequences (or polypeptide sequences derived from them of at least about 30 amino acids in length) are homologous, as this term is used herein, if they have an alignment score of at more than 5 (in standard deviation units) using the program ALIGN with the mutation data matrix and a gap penalty of 6 or greater. *See* Dayhoff, M.O., in Atlas of Protein Sequence and Structure, pp. 101-110 (Volume 5, National Biomedical Research Foundation (1972)) and Supplement 2 to this volume, pp. 1-10. The two sequences or parts thereof are homologous if their amino acids are greater than or equal to 50% identical when optimally aligned using the ALIGN program. It should be appreciated that there can be differing regions of homology within two orthologous sequences. For example, the functional sites of mouse and human orthologues may have a higher degree of homology than non-functional regions.

The term "corresponds to" is used herein to mean that a polynucleotide sequence is homologous (*i.e.,* is identical, not strictly evolutionarily related) to all or a portion of a reference polynucleotide sequence, or that a polypeptide sequence is identical to a reference polypeptide sequence.

In contradistinction, the term "complementary to" is used herein to mean that the complementary sequence is homologous to all or a portion of a reference polynucleotide sequence. For illustration, the nucleotide sequence "TATAC" corresponds to a reference sequence "TATAC" and is complementary to a reference sequence "GTATA."

The term "sequence identity" means that two polynucleotide or amino acid sequences are identical (*i.e.,* on a nucleotide-by-nucleotide or residue-by-residue basis) over the comparison window. The term "percentage of sequence identity" is calculated by comparing two optimally aligned sequences over the window of comparison, determining the number of positions at which the identical nucleic acid base (*e.g.,* A, T, C, G, U, or I) or amino acid residue occurs in both sequences to yield the number of matched positions, dividing the number of matched positions by the total number of positions in the comparison window (*i.e.,* the window size), and multiplying the result by 100 to yield the percentage of sequence identity. The terms "substantial identity" as used herein denotes a characteristic of a polynucleotide or amino acid sequence, wherein the polynucleotide or amino acid comprises a sequence that has at least 85 percent sequence identity, at least 90 to 95 percent sequence identity, or at least 99 percent sequence identity, as compared to a reference sequence over a comparison window of at least 18 nucleotide (6 amino acid) positions, frequently over a window of at least 24-48 nucleotide (8-16 amino acid) positions, wherein the percentage of sequence identity is calculated by comparing the reference sequence to the sequence which may include deletions or additions which total 20 percent or less of the reference sequence over the comparison window. The reference sequence may be a subset of a larger sequence.

As used herein, the twenty conventional amino acids and their abbreviations follow conventional usage. *See* Immunology - A Synthesis (2nd Edition, E.S. Golub and D.R. Gren, Eds., Sinauer Associates, Sunderland, Mass. (1991)), which is incorporated herein by reference. Stereoisomers (*e.g.*, D-amino acids) of the twenty conventional amino acids, unnatural amino acids such as $\alpha$-, $\alpha$-disubstituted amino acids, N-alkyl amino acids, lactic acid, and other unconventional amino acids may also be suitable components for polypeptides herein. Examples of unconventional amino acids include: 4-hydroxyproline, $\gamma$-carboxyglutamate, $\varepsilon$-N,N,N-trimethyllysine, $\varepsilon$-N-acetyllysine, O-phosphoserine, N-acetylserine, N-formylmethionine, 3-methylhistidine, 5-hydroxylysine, $\sigma$-N-methylarginine, and other similar amino acids and imino acids (*e.g., 4*-hydroxyproline). In the polypeptide notation used herein, the left-hand direction is the amino terminal direction and the right-hand direction is the carboxy-terminal direction, in accordance with standard usage and convention.

Similarly, unless specified otherwise, the left-hand end of single-stranded polynucleotide sequences is the 5' end; the left-hand direction of doublestranded polynucleotide sequences is referred to as the 5' direction. The direction of 5' to 3' addition of nascent RNA transcripts is referred to as the transcription direction; sequence regions on the DNA strand having the same sequence as the RNA and which are 5' to the 5' end of the RNA transcript are referred to as "upstream sequences"; sequence regions on the DNA strand having the same sequence as the RNA and which are 3' to the 3' end of the RNA transcript are referred to as "downstream sequences".

As applied to polypeptides, the term "substantial identity" means that two peptide sequences, when optimally aligned, such as by the programs GAP or BESTFIT using default gap weights, share at least 80 percent sequence identity, at least 90 percent sequence identity, at least 95 percent sequence identity, or at least 99 percent sequence identity. Residue positions that are not identical differ by conservative amino acid substitutions. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. Conservative amino acids substitution groups are: valine-leucine-isoleucine, phenylalanine-tyrosine, lysine-arginine, alanine-valine, glutamic-aspartic, and asparagine-glutamine.

As discussed herein, minor variations in the amino acid sequences of antibodies or immunoglobulin molecules are contemplated, providing that the variations in the amino acid sequence maintain at least about 75%, at least 80%, 90%, 95%, or about 99% sequence identity to the antibodies or immunoglobulin molecules described herein. In particular, conservative amino acid replacements are contemplated. Conservative replacements are those that take place within a family of amino acids that have related side chains. Genetically encoded amino acids are generally divided into families: (1) acidic=aspartate, glutamate; (2) basic=lysine, arginine, histidine; (3) non-polar=alanine, valine, leucine, isoleucine, proline, phenylalanine, methionine, tryptophan; and (4) uncharged polar=glycine, asparagine, glutamine, cysteine, serine, threonine, tyrosine. In one embodiment, families are: serine and threonine are an aliphatic-hydroxy family; asparagine and glutamine are an amide-containing family; alanine, valine, leucine and isoleucine are an aliphatic family; and phenylalanine, tryptophan, and tyrosine are an aromatic family. For example, it is reasonable to expect that an isolated replacement of a leucine with an isoleucine or valine, an aspartate with a glutamate, a threonine with a serine, or a similar replacement of an amino acid with a structurally related amino acid will not have a major effect on the binding function or properties of the resulting molecule, especially if the replacement does not involve an amino acid within a framework site.

Whether an amino acid change results in a functional peptide can readily be determined by assaying the specific activity of the polypeptide derivative. Assays are described in detail herein. Fragments or analogs of antibodies or immunoglobulin molecules can be readily prepared by those of ordinary skill in the art. In one embodiment, amino- and carboxy-termini of fragments or analogs occur near boundaries of functional domains. Structural and functional domains can be identified by comparison of the nucleotide and/or amino acid sequence data to public or proprietary sequence databases. Computerized comparison methods are used to identify sequence motifs or predicted protein conformation domains that occur in other proteins of known structure and/or function. Methods to identify protein sequences that fold into a known three-dimensional structure are known. Bowie et al., (1991) Science 253:164. Thus, the foregoing examples demonstrate that those of skill in the art can recognize sequence motifs and structural conformations that may be used to define structural and functional domains in accordance with the antibodies described herein.

A further aspect is a targeting binding agent or an antibody molecule comprising a VH domain that has at least about 60, 70, 80, 85, 90, 95, 98 or about 99% amino acid sequence identity with a VH domain of any of antibodies shown in Table 1, the appended sequence listing, an antibody described herein, or with an HCDR (*e.g.,* HCDR1, HCDR2, or HCDR3) shown in Table 8 or Table 29. The targeting binding agent or antibody molecule may optionally also comprise a VL domain that has at least about 60, 70, 80, 85, 90, 95, 98 or about 99% amino acid sequence identity with a VL domain any of antibodies shown in Table 1, the appended sequence listing, an antibody described herein, or with an LCDR (*e.g.,* LCDR1, LCDR2, or LCDR3) shown in Table 9 or Table 30. Algorithms that can be used to calculate % identity of two amino acid sequences comprise e.g. BLAST (Altschul et al., (1990) J. Mol. Biol. 215: 405-410), FASTA (Pearson and Lipman (1988) PNAS USA 85: 2444-2448), or the Smith-Waterman algorithm (Smith and Waterman (1981) J. Mol Biol. 147: 195-197), e.g. employing default parameters. In some embodiments, the targeting binding agent or antibody that shares amino acid sequence identity as describes above, exhibits substantially the same activity as the antibodies referenced. For instance, substantially the same activity comprises at least one activity that differed from the activity of the references antibodies by no more that about 50%, 40%, 30%, 20%, 10%, 5%, 2%, 1% or less.

An antigen binding site is generally formed by the variable heavy (VH) and variable light (VL) immunoglobulin domains, with the antigen-binding interface formed by six surface polypeptide loops, termed complimentarity determining regions (CDRs). There are three CDRs in each VH (HCDR1, HCDR2, HCDR3) and in each VL (LCDR1, LCDR2, LCDR3), together with framework regions (FRs).

Typically, a VH domain is paired with a VL domain to provide an antibody antigen-binding site, although a VH or VL domain alone may be used to bind antigen. The VH domain (*e.g.* from Table 1) may be paired with the VL domain (*e.g.* from Table 1), so that an antibody antigen-binding site is formed comprising both the VH and VL domains. Analogous embodiments are provided for the other VH and VL domains disclosed herein. In other embodiments, VH chains in Table 8 or Table 29 are paired with a heterologous VL domain. Light-chain promiscuity is well established in the art. Again, analogous embodiments are provided for the other VH and VL domains disclosed herein. Thus, the VH of the parent or of any of antibodies chain on Table 9 or Table 30 may be paired with the VL of the parent or of any of antibodies on Table 1 or other antibody.

An antigen binding site may comprise a set of H and/or L CDRs of the parent antibody or any of antibodies in Table 1 with as many as twenty, sixteen, ten, nine or fewer, *e.g.* one, two, three, four or five, amino acid additions, substitutions, deletions, and/or insertions within the disclosed set of H and/or L CDRs. Alternatively, an antigen binding site may comprise a set of H and/or L CDRs of the parent antibody or any of antibodies Table 1 with as many as twenty, sixteen, ten, nine or fewer, *e.g.* one, two, three, four or five, amino acid substitutions within the disclosed set of H and/or L CDRs. Such modifications may potentially be made at any residue within the set of CDRs.

In one embodiment, amino acid substitutions are those which: (1) reduce susceptibility to proteolysis, (2) reduce susceptibility to oxidation, (3) alter binding affinity for forming protein complexes, (4) alter binding affinities, and (4) confer or modify other physicochemical or functional properties of such analogs. Analogs can include various muteins of a sequence other than the naturally-occurring peptide sequence. For example, single or multiple amino acid substitutions (in one embodiment, conservative amino acid substitutions) may be made in the naturally-occurring sequence (in one embodiment, in the portion of the polypeptide outside the domain(s) forming intermolecular contacts. A conservative amino acid substitution should not substantially change the structural characteristics of the parent sequence (*e.g.,* a replacement amino acid should not tend to break a helix that occurs in the parent sequence, or disrupt other types of secondary structure that characterizes the parent sequence). Examples of art-recognized polypeptide secondary and tertiary structures are described in Proteins, Structures and Molecular Principles (Creighton, Ed., W. H. Freeman and Company, New York (1984)); Introduction to Protein Structure (C. Branden and J. Tooze, eds., Garland Publishing, New York, N.Y. (1991)); and Thornton et at. Nature 354:105 (1991), which are each incorporated herein by reference.

A further aspect is an antibody molecule comprising a VH domain that has at least about 60, 70, 80, 85, 90, 95, 98 or about 99 % amino acid sequence identity with a VH domain of any of antibodies listed in Table 1, the appended sequence listing or described herein, or with an HCDR (*e.g.,* HCDR1, HCDR2, or HCDR3) shown in Table 8 or Table 29. The antibody molecule may optionally also comprise a VL domain that has at least 60, 70, 80, 85, 90, 95, 98 or 99 % amino acid sequence identity with a VL domain of any of the antibodies shown in Table 1, the appended sequence listing or described herein, or with an LCDR (*e.g.,* LCDR1, LCDR2, or LCDR3) shown in Table 9 or Table 30. Algorithms that can be used to calculate % identity of two amino acid sequences comprise *e.g*. BLAST (Altschul et al., (1990) J. Mol. Biol. 215: 405-410), FASTA (Pearson and Lipman (1988) PNAS USA 85: 2444-2448), or the Smith-Waterman algorithm (Smith and Waterman (1981) J. Mol Biol. 147: 195-197), *e.g*. employing default parameters.

Variants of the VH and VL domains and CDRs, including those for which amino acid sequences are set out herein, and which can be employed in targeting agents and antibodies for $\alpha V\beta 6$ can be obtained by means of methods of sequence alteration or mutation and screening for antigen targeting with desired characteristics. Examples of desired characteristics include but are not limited to: increased binding affinity for antigen relative to known antibodies which are specific for the antigen; increased neutralization of an antigen activity relative to known antibodies which are specific for the antigen if the activity is known; specified competitive ability with a known antibody or ligand to the antigen at a specific molar ratio; ability to immunoprecipitate complex; ability to bind to a specified epitope; linear epitope, *e.g*. peptide sequence identified using peptide-binding scan as described herein, *e.g*. using peptides screened in linear and/or constrained conformation; conformational epitope, formed by non-continuous residues; ability to modulate a new biological activity of $\alpha V\beta 6$, or downstream molecule. Such methods are also provided herein.

Variants of antibody molecules disclosed herein may be produced and used herein. Following the lead of computational chemistry in applying multivariate data analysis techniques to the structure/property-activity relationships (Wold, et al., Multivariate data analysis in chemistry. Chemometrics -Mathematics and Statistics in Chemistry (Ed.: B. Kowalski), D. Reidel Publishing Company, Dordrecht, Holland, 1984) quantitative activity-property relationships of antibodies can be derived using well-known mathematical techniques, such as statistical regression, pattern recognition and classification (Norman et al., Applied Regression Analysis. Wiley-Interscience; 3rd edition (April 1998); Kandel, Abraham & Backer, Eric. Computer-Assisted Reasoning in Cluster Analysis. Prentice Hall PTR, (May 11, 1995); Krzanowski, Wojtek. Principles of Multivariate Analysis: A User's Perspective (Oxford Statistical Science Series, No 22 (Paper)). Oxford University Press; (December 2000); Witten, Ian H. & Frank, Eibe. Data Mining: Practical Machine Learning Tools and Techniques with Java Implementations. Morgan Kaufmann; (October 11, 1999);Denison David G. T. (Editor), Christopher C. Holmes, Bani K. Mallick, Adrian F. M. Smith. Bayesian Methods for Nonlinear Classification and Regression (Wiley Series in Probability and Statistics). John Wiley & Sons; (July 2002); Ghose, Arup K. & Viswanadhan, Vellarkad N. Combinatorial Library Design and Evaluation Principles, Software, Tools, and Applications in Drug Discovery). The properties of antibodies can be derived from empirical and theoretical models (for example, analysis of likely contact residues or calculated physicochemical property) of antibody sequence, functional and three-dimensional structures and these properties can be considered singly and in combination.

An antibody antigen-binding site composed of a VH domain and a VL domain is typically formed by six loops of polypeptide: three from the light chain variable domain (VL) and three from the heavy chain variable domain (VH). Analysis of antibodies of known atomic structure has elucidated relationships between the sequence and three-dimensional structure of antibody combining sites. These relationships imply that, except for the third region (loop) in VH domains, binding site loops have one of a small number of main-chain conformations: canonical structures. The canonical structure formed in a particular loop has been shown to be determined by its size and the presence of certain residues at key sites in both the loop and in framework regions.

This study of sequence-structure relationship can be used for prediction of those residues in an antibody of known sequence, but of an unknown three-dimensional structure, which are important in maintaining the three-dimensional structure of its CDR loops and hence maintain binding specificity. These predictions can be backed up by comparison of the predictions to the output from lead optimization experiments. In a structural approach, a model can be created of the antibody molecule using any freely available or commercial package, such as WAM. A protein visualisation and analysis software package, such as Insight II (Accelrys, Inc.) or Deep View may then be used to evaluate possible substitutions at each position in the CDR. This information may then be used to make substitutions likely to have a minimal or beneficial effect on activity.

The techniques required to make substitutions within amino acid sequences of CDRs, antibody VH or VL domains and/or binding agents generally are available in the art. Variant sequences may be made, with substitutions that may or may not be predicted to have a minimal or beneficial effect on activity, and tested for ability to bind and/or neutralize and/or for any other desired property.

Variable domain amino acid sequence variants of any of the VH and VL domains whose sequences are specifically disclosed herein may be employed, as discussed.

The term "polypeptide fragment" as used herein refers to a polypeptide that has an amino-terminal and/or carboxy-terminal deletion, but where the remaining amino acid sequence is identical to the corresponding positions in the naturally-occurring sequence deduced, for example, from a full-length cDNA sequence. Fragments typically are at least about 5, 6, 8 or 10 amino acids long, in one embodiment at least about 14 amino acids long, at least about 20 amino acids long, at least about 50 amino acids long, or at least about 70 amino acids long. The term "analog" as used herein refers to polypeptides which are comprised of a segment of at least about 25 amino acids that has substantial identity to a portion of a deduced amino acid sequence and which has at least one of the following properties: (1) specific binding to αVβ6, under suitable binding conditions, (2) ability to block appropriate ligand/αVβ6 binding, or (3) ability to inhibit αVβ6 activity. Typically, polypeptide analogs comprise a conservative amino acid substitution (or addition or deletion) with respect to the naturally-occurring sequence. Analogs typically are at least 20 amino acids long, at least 50 amino acids long or longer, and can often be as long as a full-length naturally-occurring polypeptide.

Peptide analogs are commonly used in the pharmaceutical industry as non-peptide drugs with properties analogous to those of the template peptide. These types of non-peptide compound are termed "peptide mimetics" or "peptidomimetics." Fauchere, J. Adv. Drug Res. 15:29 (1986); Veber and Freidinger TINS p.392 (1985); and Evans et al., J. Med. Chem. 30:1229 (1987), which are incorporated herein by reference. Such compounds are often developed with the aid of computerized molecular modeling. Peptide mimetics that are structurally similar to therapeutically useful peptides may be used to produce an equivalent therapeutic or prophylactic effect. Generally, peptidomimetics are structurally similar to a paradigm polypeptide (*i.e.,* a polypeptide that has a biochemical property or pharmacological activity), such as human antibody, but have one or more peptide linkages optionally replaced by a linkage chosen from: $--CH_2NH--$, $--CH_2S--$, $--CH_2-CH_2--$, --CH=CH--(cis and trans), $--COCH_2--$, $--CH(OH)CH_2--$, and $-CH_2SO--$, by methods well known in the art. Systematic substitution of one or more amino acids of a consensus sequence with a D-amino acid of the same type (*e.g*., D-lysine in place of L-lysine) may be used to generate more stable peptides. In addition, constrained peptides comprising a consensus sequence or a substantially identical consensus sequence variation may be generated by methods known in the art (Rizo and Gierasch Ann. Rev. Biochem. 61:387 (1992), incorporated herein by reference); for example, by adding internal cysteine residues capable of forming intramolecular disulfide bridges which cyclize the peptide.

As used herein, the term "antibody" refers to a polypeptide or group of polypeptides that are comprised of at least one binding domain that is formed from the folding of polypeptide chains having three-dimensional binding spaces with internal surface shapes and charge distributions complementary to the features of an antigenic determinant of an antigen. An antibody typically has a tetrameric form, comprising two identical pairs of polypeptide chains, each pair having one "light" and one "heavy" chain. The variable regions of each light/heavy chain pair form an antibody binding site.

As used herein, a "targeted binding agent" is an agent, *e.g*. antibody, or binding fragment thereof, that may bind to a target site. In one embodiment, the targeted binding agent is specific for only one target site. In other embodiments, the targeted binding agent is specific for more than one target site. In one embodiment, the targeted binding agent may be a monoclonal antibody and the target site may be an epitope. As described below, a targeted binding agent may comprise at least one antigen binding domain of an antibody, wherein said domain is fused or contained within a heterologous protein.

"Binding fragments" of an antibody are produced by recombinant DNA techniques, or by enzymatic or chemical cleavage of intact antibodies. Binding fragments include Fab, Fab', $F(ab')_2$, Fv, and single-chain antibodies. An antibody other than a "bispecific" or "bifunctional" antibody is understood to have each of its binding sites identical. An antibody substantially inhibits adhesion of a receptor to a counter-receptor when an excess of antibody reduces the quantity of receptor bound to counter-receptor by at least about 20%, 40%, 60% or 80%, and more usually greater than about 85% (as measured in an *in vitro* competitive binding assay).

An antibody may be oligoclonal, a polyclonal antibody, a monoclonal antibody, a chimeric antibody, a CDR-grafted antibody, a multispecific antibody, a bi-specific antibody, a catalytic antibody, a chimeric antibody, a humanized antibody, a fully human antibody, an anti-idiotypic antibody and antibodies that can be labeled in soluble or bound form as well as fragments, variants or derivatives thereof, either alone or in combination with other amino acid sequences provided by known techniques. An antibody may be from any species. The term antibody also includes binding fragments of the antibodies herein; exemplary fragments include Fv, Fab, Fab', single stranded antibody (svFC), dimeric variable region (Diabody) and disulphide stabilized variable region (dsFv).

It has been shown that fragments of a whole antibody can perform the function of binding antigens. Examples of binding fragments are (Ward, E.S. et al., (1989) Nature 341, 544-546) the Fab fragment consisting of VL, VH, CL and CHI domains; (McCafferty et al., (1990) Nature, 348, 552-554) the Fd fragment consisting of the VH and CHI domains; (Holt et al., (2003) Trends in Biotechnology 21, 484-490) the Fv fragment consisting of the VL and VH domains of a single antibody; (iv) the dAb fragment (Ward, E.S. et al., Nature 341, 544-546 (1989), McCafferty et al., (1990) Nature, 348, 552-554, Holt et al., (2003) Trends in Biotechnology 21, 484-490], which consists of a VH or a VL domain; (v) isolated CDR regions; (vi) F(ab')2 fragments, a bivalent fragment comprising two linked Fab fragments (vii) single chain Fv molecules (scFv), wherein a VH domain and a VL domain are linked by a peptide linker which allows the two domains to associate to form an antigen binding site (Bird et al., (1988) Science, 242, 423-426, , Huston et al., (1988) PNAS USA, 85, 5879-5883); (viii) bispecific single chain Fv dimers (PCT/US92/09965) and (ix) "diabodies", multivalent or multispecific fragments constructed by gene fusion (WO94/13804; Holliger, P. (1993) et al., Proc. Natl. Acad. Sci. USA 90 6444-6448,). Fv, scFv or diabody molecules may be stabilized by the incorporation of disulphide bridges linking the VH and VL domains (Reiter, Y. et al., Nature Biotech, 14, 1239-1245, 1996). Minibodies comprising a scFv joined to a CH3 domain may also be made (Hu, S. et al., (1996) Cancer Res., 56, 3055-3061). Other examples of binding fragments are Fab', which differs from Fab fragments by the addition of a few residues at the carboxyl terminus of the heavy chain CHI domain, including one or more cysteines from the antibody hinge region, and Fab'-SH, which is a Fab' fragment in which the cysteine residue(s) of the constant domains bear a free thiol group.

The term "epitope" includes any protein determinant capable of specific binding to an immunoglobulin or T-cell receptor. Epitopic determinants usually consist of chemically active surface groupings of molecules such as amino acids or sugar side chains and may, but not always, have specific three-dimensional structural characteristics, as well as specific charge characteristics. An antibody is said to specifically bind an antigen when the dissociation constant is $\leq 1$ $\mu M$, $\leq 100$ nM, or $\leq 10$ nM.

The term "agent" is used herein to denote a chemical compound, a mixture of chemical compounds, a biological macromolecule, or an extract made from biological materials.

"Active" or "activity" in regard to a $\alpha V\beta 6$ heterodimeric polypeptide refers to a portion of an $\alpha V\beta 6$ heterodimeric polypeptide that has a biological or an immunological activity of a native $\alpha V\beta 6$ polypeptide. "Biological" when used herein refers to a biological function that results from the activity of the native $\alpha V\beta 6$ polypeptide. A $\alpha V\beta 6$ biological activity includes, for example, $\alpha V\beta 6$ induced cell adhesion.

"Mammal" when used herein refers to any animal that is considered a mammal. In one embodiment, the mammal is human.

Digestion of antibodies with the enzyme, papain, results in two identical antigen-binding fragments, known also as "Fab" fragments, and a "Fc" fragment, having no antigen-binding activity but having the ability to crystallize. Digestion of antibodies with the enzyme, pepsin, results in the a $F(ab')_2$ fragment in which the two arms of the antibody molecule remain linked and comprise two-antigen binding sites. The $F(ab')_2$ fragment has the ability to crosslink antigen.

"Fv" when used herein refers to the minimum fragment of an antibody that retains both antigen-recognition and antigen-binding sites.

"Fab" when used herein refers to a fragment of an antibody that comprises the constant domain of the light chain and the CHI domain of the heavy chain.

The term "mAb" refers to monoclonal antibody.

"Liposome" when used herein refers to a small vesicle that may be useful for delivery of drugs that may include the $\alpha V\beta 6$ polypeptide or antibodies to such an $\alpha V\beta 6$ polypeptide to a mammal.

"Label" or "labeled" as used herein refers to the addition of a detectable moiety to a polypeptide, for example, a radiolabel, fluorescent label, enzymatic label chemiluminescent labeled or a biotinyl group. Radioisotopes or radionuclides may include $^{3}H$, $^{14}C$, $^{15}N$, $^{35}S$, $^{90}Y$, $^{99}Tc$, $^{111}In$, $^{125}I$, $^{131}I$, fluorescent labels may include rhodamine, lanthanide phosphors or FITC and enzymatic labels may include horseradish peroxidase, $\beta$-galactosidase, luciferase, alkaline phosphatase.

Additional labels include, by way of illustration and not limitation: enzymes, such as glucose-6-phosphate dehydrogenase ("G6PDH"), alpha-D-galactosidase, glucose oxydase, glucose amylase, carbonic anhydrase, acetylcholinesterase, lysozyme, malate dehydrogenase and peroxidase; dyes; additional fluorescent labels or fluorescers include, such as fluorescein and its derivatives, fluorochrome, GFP (GFP for "Green Fluorescent Protein"), dansyl, umbelliferone, phycoerythrin, phycocyanin, allophycocyanin, o-phthaldehyde, and fluorescamine; fluorophores such as lanthanide cryptates and chelates e.g. Europium etc (Perkin Elmer and Cis Biointernational); chemoluminescent labels or chemiluminescers, such as isoluminol, luminol and the dioxetanes; sensitizers; coenzymes; enzyme substrates; particles, such as latex or carbon particles; metal sol; crystallite; liposomes; cells, etc., which may be further labelled with a dye, catalyst or other detectable group; molecules such as biotin, digoxygenin or 5-bromodeoxyuridine; toxin moieties, such as for example a toxin moiety selected from a group of Pseudomonas exotoxin (PE or a cytotoxic fragment or mutant thereof), Diptheria toxin or a cytotoxic fragment or mutant thereof, a botulinum toxin A, B, C, D, E or F, ricin or a cytotoxic fragment thereof *e.g.* ricin A, abrin or a cytotoxic fragment thereof, saporin or a cytotoxic fragment thereof, pokeweed antiviral toxin or a cytotoxic fragment thereof and bryodin 1 or a cytotoxic fragment thereof.

The term "pharmaceutical agent or drug" as used herein refers to a chemical compound or composition capable of inducing a desired therapeutic effect when properly administered to a patient. Other chemistry terms herein are used according to conventional usage in the art, as exemplified by The McGraw-Hill Dictionary of Chemical Terms (Parker, S., Ed., McGraw-Hill, San Francisco (1985)), (incorporated herein by reference).

As used herein, "substantially pure" means an object species is the predominant species present (*i.e.,* on a molar basis it is more abundant than any other individual species in the composition), and a substantially purified fraction may be a composition wherein the object species comprises at least about 50 percent (on a molar basis) of all macromolecular species present. Generally, a substantially pure composition will comprise more than about 80 percent of all macromolecular species present in the composition, or may comprise at least about 85%, 90%, 95%, and 99%. In one aspect, the object species is purified to essential homogeneity (contaminant species cannot be detected in the composition by conventional detection methods) wherein the composition consists essentially of a single macromolecular species.

The term "patient" includes human and veterinary subjects.

## II. Methods of Treatment

### A. Overview of Treatment Methods for αVβ6 Overexpressing Cancer Cells

Understanding the role of αVβ6 in certain cancers, αVβ6 may be inhibited by administering an αVβ6 targeted binding agent to a patient or to cancer cells may be used to treat cancer or inhibit growth of tumor cells, including, but not limited to, cancer cells overexpressing αVβ6.

An αVβ6 targeted binding agent that specifically binds to αVβ6 and inhibits binding of ligands to αVβ6 may be used in a method of treating a malignant tumor in an animal, including, but not limited to, breast cancer. Alternatively, the malignant tumor may be ovarian cancer, pancreatic cancer, lung cancer, colorectal cancer, head and neck cancer, oesophageal cancer, gastric cancer, and hepatocellular cancer. In another embodiment, the αVβ6 targeted binding agent may be used to inhibit growth of tumor cells, including, but not limited to, tumor cells from the types of cancer recited in this paragraph.

In one embodiment, the animal may be a mammal. In another embodiment, the animal may be a human.

In such a treatment, one or more αVβ6 targeted binding agents may be used. Thus, the use of singular "a" includes the plural.

Such methods may be used in isolation or they may be used in combination with a diagnosis that the malignant tumor or the tumor cells overexpress αVβ6.

In one embodiment, such methods employ the αVβ6 targeted binding agents described in Section IV within the dosage range described. In one embodiment, the αVβ6 targeted binding agent is a monoclonal antibody. In another embodiment, it is a fully human monoclonal embodiment. In yet another embodiment, it is sc 264RAD

In one embodiment, the level of at least one downstream target of αVβ6 downregulated. In one embodiment, the level of at least one of Akt2 and Smad2 is downregulated. In one embodiment, the total level of the target is downregulated. In another embodiment, the phospho level of the target is downregulated. Downregulation may be measured by determining the level of a protein or downregulation may be measured by determining the level of an mRNA. Downregulation and/or inhibition includes a reduction of at least 10%, 20%, 30%, 40%, 50%, 60%, 70%, 80%, 90%, 95%, 99%, or 100% compared to before treatment.

In one embodiment, the breast cancer cells are resistant to trastuzumab. Thus, one embodiment includes a method of suppressing growth of trastuzumab-resistant tumor cells comprising administering to said cells a therapeutically effective dose of an αVβ6 targeted binding agent that specifically binds to αVβ6 and inhibits binding of ligands to αVβ6.

### B. Combination Therapy

When αVβ6 inhibitors are used to treat a malignant tumor or to inhibit growth of tumor cells, the αVβ6 targeted binding agent may be administered as a sole therapy or it may be administered in a combination therapy regime, with conventional surgery or radiotherapy or chemotherapy. Such conjoint treatment may be achieved by way of the simultaneous, sequential, or separate dosing of the individual components of the treatment. Where the administration is sequential or separate, the delay in administering the second component should not be such as to lose the beneficial effect of the combination.

One or more combination therapy agents may be used in addition to a αVβ6 targeted binding agent; likewise, one or more αVβ6 targeted binding agents may be used. Thus, the use of singular "a" includes the plural. Such combination products employ a αVβ6 targeted binding agent described herein within the dosage range described and the combination therapy agent within its approved dosage range.

#### 1. Combination Therapy for Breast Cancer

Combination therapy may be employed in the treatment of a breast cancer tumor or to inhibit growth of breast cancer tumor cells.

Such methods may be used in isolation or they may be used in combination with a diagnosis that the malignant tumor or the tumor cells overexpress αVβ6, overexpress HER2, or overexpress αVβ6 and HER2.

Such methods may be used in isolation or they may be used in combination with a diagnosis that the malignant tumor or the tumor cells overexpress αVβ6.

In one embodiment, the breast cancer cells are resistant to trastuzumab. Thus, one embodiment includes a method of suppressing growth of trastuzumab-resistant tumor cells comprising administering to said cells a therapeutically effective dose of an αVβ6 targeted binding agent that specifically binds to αVβ6 and inhibits binding of ligands to αVβ6 and a HER2 targeted binding agent that specifically binds to HER2 and inhibits binding of ligands to HER2.

In one embodiment, the combination therapy agent may be trastuzumab. In another embodiment, the combination therapy agent is a HER2 targeted binding agent that specifically binds to HER2 and inhibits binding of ligands to HER2.

In another embodiment, the combination therapy agent may be gemcitabine, docetaxel, EGFR inhibitor, HER-2 inhibitor (including but not limited to trastuzumab or Herceptin®), PI3K inhibitor (ATK inhibitor (such as AZD5363, MK2206), rapalogue (such as everolimus), AZD2014, PI3Kα inhibitor, PI3Kβ inhibitor (AZD8186, GSK2636771, SAR 260301), Pan PI3K inhibitor (GDC0941, GDC0942)), MEK/RAF inhibitor (such as vemurafanib (RAF inhibitor), seluemetinib (MEK inhibitor), trametinib (MEK inhibitor)), PD-1 inhibitor, PDL1 inhibitor, or CTLA4 inhibitor.

In one embodiment, the level of at least one downstream target of αVβ6 and/or HER2 is downregulated. In one embodiment, the level of at least one of Akt2 and Smad2 is downregulated. In one embodiment, the total level of the target is downregulated. In another embodiment, the phospho level of the target is downregulated. Downregulation may be measured by determining the level of a protein or downregulation may be measured by determining the level of an mRNA. Downregulation includes a reduction of at least 10%, 20%, 30%, 40%, 50%, 60%, 70%, 80%, 90%, 95%, 99%, or 100% compared to before treatment.

### 2. Combination Therapy for Ovarian Cancer

Combination therapy may be employed in the treatment of an ovarian cancer tumor or to inhibit growth of ovarian cancer tumor cells.

Such methods may be used in isolation or they may be used in combination with a diagnosis that the malignant tumor or the tumor cells overexpress αVβ6, overexpress HER2, or overexpress αVβ6 and HER2.

Such methods may be used in isolation or they may be used in combination with a diagnosis that the malignant tumor or the tumor cells overexpress αVβ6.

In one embodiment, the ovarian cancer cells are resistant to trastuzumab. Thus, one embodiment includes a method of suppressing growth of trastuzumab-resistant tumor cells comprising administering to said cells a therapeutically effective dose of an αVβ6 targeted binding agent that specifically binds to αVβ6 and inhibits binding of ligands to αVβ6 and a HER2 targeted binding agent that specifically binds to HER2 and inhibits binding of ligands to HER2.

In one embodiment, the combination therapy agent may be trastuzumab.

In another embodiment, the combination therapy agent may be gemcitabine, docetaxel, EGFR inhibitor, HER-2 inhibitor (including but not limited to trastuzumab or Herceptin®), PI3K inhibitor (ATK inhibitor (such as AZD5363, MK2206), rapalogue (such as everolimus), AZD2014, PI3Kα inhibitor, PI3Kβ inhibitor (AZD8186, GSK2636771, SAR 260301), Pan PI3K inhibitor (GDC0941, GDC0942)), MEK/RAF inhibitor (such as vemurafanib (RAF inhibitor), seluemetinib (MEK inhibitor), trametinib (MEK inhibitor)), PD-1 inhibitor, PDL1 inhibitor, or CTLA4 inhibitor.

### 3. Combination Therapy for Pancreatic Cancer

Combination therapy may be employed in the treatment of a pancreatic cancer tumor or to inhibit growth of pancreatic cancer cells.

In one embodiment, the combination therapy agent may be gemcitabine, abraxane, folfirinox (a combination therapy approach using 5-fluorouracil, oxaliplatin, irinotecan, and leucovorin), EGFR inhibitor, HER-2 inhibitor (including but not limited to trastuzumab or Herceptin®), PI3K inhibitor (ATK inhibitor (such as AZD5363, MK2206), rapalogue (such as everolimus), AZD2014, PI3Kα inhibitor, PI3Kβ inhibitor (AZD8186, GSK2636771, SAR 260301), Pan PI3K inhibitor (GDC0941, GDC0942)), MEK/RAF inhibitor (such as vemurafanib (RAF inhibitor), seluemetinib (MEK inhibitor), trametinib (MEK inhibitor)), PD-1 inhibitor, PDL1 inhibitor, or CTLA4 inhibitor.

### 4. Combination Therapy for Lung Cancer

Combination therapy may be employed in the treatment of a lung cancer tumor or to inhibit growth of lung cancer cells. In one embodiment, the cancer may be adenocarcinoma, squamous cell carcinoma, or small cell lung cancer.

In one embodiment, the combination therapy agent may be gefitinib (Iressa®), AZD9291, erlotinib (Tarceva®), platinum-based cytotoxics, docetaxel, PI3K inhibitor (ATK inhibitor (such as AZD5363, MK2206), rapalogue (such as everolimus), AZD2014, PI3Kα inhibitor, PI3Kβ inhibitor (AZD8186, GSK2636771, SAR 260301), Pan PI3K inhibitor (GDC0941, GDC0942)), MEK/RAF inhibitor (such as vemurafanib (RAF inhibitor), seluemetinib (MEK inhibitor), trametinib (MEK inhibitor)), PD-1 inhibitor, PDL1 inhibitor, or CTLA4 inhibitor.

### 5. Combination Therapy for Colorectal Cancer

Combination therapy may be employed in the treatment of a colorectal cancer tumor or to inhibit growth of colorectal cancer cells.

In one embodiment, the combination therapy agent may be gemcitabine, folfirinox, docetaxel, platinum-based triplets, 5-fluorouracil, cetuximab (Erbitux®), rapalogue (such as everolimus), ATK inhibitor (such as AZD5363, MK2206), rapalogue (such as everolimus), AZD2014, PI3Kα inhibitor, PI3Kβ inhibitor (AZD8186, GSK2636771, SAR 260301), Pan PI3K inhibitor (GDC0941, GDC0942)), MEK/RAF inhibitor (such as vemurafanib (RAF inhibitor), seluemetinib (MEK inhibitor), trametinib (MEK inhibitor)), PD-1 inhibitor, PDL1 inhibitor, or CTLA4 inhibitor..

### 6. Combination Therapy for Head and Neck Cancer

Combination therapy may be employed in the treatment of head and neck cancer or to inhibit growth of head and neck cancer cells.

In one embodiment, the combination therapy agent may be gemcitabine, platinum-based cytotoxics, docetaxel, radiation, cetuximab (Erbitux®), PI3K inhibitor (ATK inhibitor (such as AZD5363, MK2206), rapalogue (such as everolimus ATK inhibitor (such as AZD5363, MK2206), rapalogue (such as everolimus), AZD2014, PI3Kα inhibitor, PI3Kβ inhibitor (AZD8186, GSK2636771, SAR 260301), Pan PI3K inhibitor (GDC0941, GDC0942)), MEK/RAF inhibitor (such as vemurafanib (RAF inhibitor), seluemetinib (MEK inhibitor), trametinib (MEK inhibitor)), PD-1 inhibitor, PDL1 inhibitor, or CTLA4 inhibitor.

### 7. Combination Therapy for Oesophageal Cancer

Combination therapy may be employed in the treatment of oesophageal cancer or to inhibit growth of oesophageal cancer cells.

In one embodiment, the combination therapy agent may be radiation or standard chemotherapeutics, which are further elaborated in section II.B.10, below.

### 8. Combination Therapy for Gastric Cancer

Combination therapy may be employed in the treatment of gastric cancer or to inhibit growth of gastric cancer cells.

In one embodiment, the combination therapy agent may be triplet chemotherapy (paclitaxel, cisplatin, and S-1).

### 9. Combination Therapy for Hepatocellular Cancer

Combination therapy may be employed in the treatment of hepatocellular cancer or to inhibit growth of hepatocellular cancer cells.

In one embodiment, the combination therapy agent may be sorafanib or TACE (TNFα convertase enzyme) inhibitor.

### 10. Combination Therapy Generally

The anti-tumor treatment defined herein may be applied as a sole therapy or may involve, in addition to the compounds herein, conventional surgery or radiotherapy or chemotherapy.

The compounds may be used in the methods herein as either a single agent by itself or in combination with other clinically relevant agents or techniques. For example, the anti-cancer treatment defined herein may be applied as a sole therapy or may involve, in addition to the compounds herein, conventional surgery or radiotherapy or chemotherapy. Such radiotherapy may include one or more of the following categories of radiation:

(i) external radiation therapy using electromagnetic radiation, and intraoperative radiation therapy using electromagnetic radiation;

(ii) internal radiation therapy or brachytherapy; including interstitial radiation therapy or intraluminal radiation therapy;

or

(iii) systemic radiation therapy, including but not limited to iodine 131 and strontium 89;

Such chemotherapy may include one or more of the following categories of anti-tumor agents:

Antiproliferative/antineoplastic drugs and combinations thereof, as used in medical oncology, such as DNA alkylating agents (for example cisplatin, oxaliplatin, carboplatin, cyclophosphamide, nitrogen mustard, melphalan, chlorambucil, busulphan, temozolamide and nitrosoureas); antimetabolites (for example gemcitabine, fludarabine, capecitabine and antifolates such as fluoropyrimidines like 5-fluorouracil and pemetrexed, tegafur, raltitrexed, methotrexate, cytosine arabinoside, and hydroxyurea); antitumour antibiotics (for example anthracyclines like adriamycin, bleomycin, doxorubicin, liposomal doxorubicin, daunomycin, epirubicin, idarubicin, mitomycin-C, dactinomycin and mithramycin); and topoisomerase inhibitors (for example epipodophyllotoxins like etoposide and teniposide, amsacrine, topotecan, camptothecin and irinotecan); inhibitors of DNA repair mechanisms such as CHK kinase; DNA-dependent protein kinase inhibitors; inhibitors of poly (ADP-ribose) polymerase (PARP inhibitors, including for example Olaparib); and Hsp90 inhibitors such as tanespimycin and retaspimycin;

Compounds that inhibit progression through the cell cycle such as antimitotic agents (for example vinca alkaloids like vincristine, vinblastine, vindesine and vinorelbine; epothilones such as ixabepilone and patupilone; taxoids like taxol and docetaxel; polo-like kinase inhibitors; and inhibitors of kinesin motor proteins such as Eg5 protein inhibitors); aurora kinase inhibitors (for example AZD1152, PH739358, VX-680, MLN8054, R763, MP235, MP529, VX-528 AND AX39459); cyclin dependent kinase inhibitors such as CDK2 and/or CDK4 inhibitors; and inhibitors of centromeric protein function such as CENP-E inhibitors;

Cytostatic agents that alter hormone-dependent growth such as antiestrogens (for example tamoxifen, fulvestrant, toremifene, raloxifene, droloxifene and iodoxyfene), antiandrogens (for example enzalutamide, bicalutamide, flutamide, nilutamide and cyproterone acetate); LHRH antagonists or LHRH agonists (for example goserelin, leuprorelide and buserelin); progestogens (for example megestrol acetate); aromatase inhibitors (for example as anastrozole, letrozole, vorazole and exemestane); and inhibitors of 5$\alpha$-reductase such as finasteride; CYP17A1 inhibitors such as abiraterone acetate;

Anti-invasion agents such as c-Src kinase family inhibitors like AZD0530, dasatinib or BMS-354825; bosutinib (SKI-606), metalloproteinase inhibitors like marimastat; inhibitors of urokinase plasminogen activator receptor function; antibodies to heparanase, inhibitors of FAK or focal-adhesion kinase; small molecule inhibitors of MET receptor kinase (for example volitinib); antibodies to MET receptor kinase or the MET ligand hepatocyte growth factor (for example onartuzumab);

Inhibitors of tumor, tumor stem cell, and endothelial cell precursor migration, including chemokines and chemokine receptors, such as SDF1, MCP-1, CXCR2 and CXCR4;

Inhibitors of growth factor signaling: for example such inhibitors include growth factor antibodies and growth factor receptor antibodies (for example the anti-erbB2 antibody trastuzumab [Herceptin™], the anti-EGFR antibodies panitumumab and cetuximab [Erbitux, C225] and any growth factor or growth factor receptor antibodies disclosed by Stern et al. Critical reviews in oncology/haematology, 2005, Vol. 54, pp11-29); such inhibitors also include tyrosine kinase inhibitors, for example inhibitors of the epidermal growth factor family and their receptors (for example EGFR family tyrosine kinase inhibitors such as gefitinib, *i.e.,* ZD1839, erlotinib, *i.e.,* OSI-774, and CI 1033; combined EGFR and erbB2 tyrosine kinase inhibitors such as lapatinib; mixed erbB 1/2 inhibitors such as afatanib; and irreversible inhibitors of EGFR and Her2 such as HKI-272, irreversible inhibitors of EGFR such as AZD9291; inhibitors of the hepatocyte growth factor family and their receptors; inhibitors of the insulin growth factor family including small molecule kinase inhibitors and antibodies directed to insulin-like growth factors and insulin-like growth factor receptors; inhibitors of the platelet-derived growth factor family and their receptors such as imatinib and/or nilotinib (AMN107); c-kit inhibitors, AnLK inhibitors, Flt3 kinase inhibitors, c-abl kinase inhibitors, and inhibitors of CSF-1R or TRK kinase;

Inhibitors of signal transduction kinases such as FGFR (for example AZD4547), PIM (for example AZD1208), MEK (for example Selumetinib (AZD6244), AKT (for example AZD5363), inhibitors of TOR kinases (including TORC1 and TORC2, for example AZD2014), and inhibitors of PI3 kinase, including isoforms such as PI3K-$\alpha$, PI3K-$\beta$ or PI3K-$\delta$ (for example AZD8186); inhibitors of serine/threonine kinases such as Ras or Raf kinases (for example sorafenib or vemurafenib); Inhibitors of PDK, SGK, PI4K or PIP5K, JAK, STAT (including STAT3, an inhibitor of which is AZD9150) and IRAK4; ATR inhibitors (for example AZD6738) or ATM inhibitors; ABL inhibitors such as imatinib or nilotinib, BTK inhibitors such as ibrutinib, SYK inhibitors such as fostamatinib, and cyclin dependent kinase inhibitors; farnesyl transferase inhibitors such as tipifarnib (R115777) and lonafarnib (SCH66336));

Antiangiogenic agents such as those that inhibit the effects of vascular endothelial growth factor [for example the anti-vascular endothelial cell growth factor antibody bevacizumab (Avastin™) and for example, a VEGF receptor tyrosine kinase inhibitor such as vandetanib (ZD6474), sorafenib, vatalanib (PTK787), sunitinib (SU11248), axitinib (AG-013736), pazopanib (GW 786034) and cediranib (AZD2171); compounds such as those disclosed in International Patent Applications WO97/22596, WO 97/30035, WO 97/32856 and WO 98/13354; and compounds that work by other mechanisms (for example linomide, inhibitors of integrin $\alpha v\beta 3$ function and angiostatin)], or inhibitors of angiopoietins and their receptors (Tie-1 and Tie-2), inhibitors of PLGF, inhibitors of delta-like ligand (DLL-4);

Vascular damaging agents such as Combretastatin A4 and compounds disclosed in International Patent Applications WO 99/02166, WO 00/40529, WO 00/41669, WO 01/92224, WO 02/04434 and WO 02/08213;

An endothelin receptor antagonist, for example zibotentan (ZD4054) or atrasentan;

Antisense therapies, for example those that are directed to the targets listed above, such as ISIS 2503, an anti-ras antisense, an oblimerson sodium, an anti-2 antisense, or antisense to XIAP such as AEG35156;

Gene therapy approaches, including for example approaches to replace aberrant genes such as aberrant p53 or aberrant BRCA1 or BRCA2, GDEPT (gene-directed enzyme pro-drug therapy); approaches such as those using cytosine deaminase, thymidine kinase or a bacterial nitroreductase enzyme; and approaches to increase patient tolerance to chemotherapy or radiotherapy, such as multi-drug resistance gene therapy;

Immunotherapy approaches, including for example ex-vivo and in-vivo approaches to increase the immunogenicity of patient tumor cells, such as transfection with cytokines such as interleukin 2, interleukin 4 or granulocyte-macrophage colony stimulating factor; approaches to decrease T-cell anergy or regulatory T-cell function; approaches that enhance T-cell responses to tumors, such as blocking antibodies to CTLA4 (for example ipilimumab and tremelimumab), B7H1, PD-1 (for example BMS-936558), and agonist antibodies to CD137; approaches using transfected immune cells such as cytokine-transfected dendritic cells; approaches using cytokine-transfected tumor cell lines, approaches using antibodies to tumor associated antigens, and antibodies that deplete target cell types (*e.g.*, unconjugated anti-CD20 antibodies such as Rituximab, radiolabeled anti-CD20 antibodies Bexxar and Zevalin, and anti-CD54 antibody Campath); approaches using anti-idiotypic antibodies; approaches that enhance Natural Killer cell function; and approaches that utilize antibody-toxin conjugates (e.g. anti-CD33 antibody Mylotarg); immunotoxins such as moxetumumab pasudotox; agonists of toll-like receptor 7 or toll-like receptor 9;

Apoptosis-inducing approaches, including antibodies to death receptor 4 or death receptor 5 or cross reactive antibodies binding to both death receptor 4 and death receptor 5; and inhibitors of XIAP and cIAP1 and cIAP2; antibodies to FAS;

Cytokine treatment, including tumor necrosis factor alpha, and recombinant Trail protein, and small molecule or protein mimetics of the Trail protein; FAS or Tweak ligands, or mimetics of these ligands;

Inhibitors of proteasome mediated protein degradation including but not limited to proteasome inhibitors such as Velcade™, inhibitors of ubiquitin ligases, inhibitors of ubiquitin proteases, inhibitors of protein Neddylation, and inhibitors of protein sumoylation; or

Efficacy enhancers, such as leucovorin.

According to a further embodiment, there is provided a kit comprising a $\alpha v\beta 6$ binding agent in combination with an anti-tumor agent chosen from the listing above. In certain embodiments, the kit additionally comprises instructions for the use of said compound(s) in the treatment of cancer or inhibiting the growth of tumor cells.

According to a further embodiment, there is provided a kit comprising:

a) an $\alpha V\beta 6$ targeted binding agent in a first unit dosage form;
b) an anti-tumor agent chosen from the list above in a second unit dosage form; and
c) container means for containing said first and second dosage forms.

**III. Diagnostic Methods**

In one embodiment, a method of diagnosing breast cancer sensitive to $\alpha V\beta 6$ and HER2 inhibition in a patient comprises analyzing a patient sample for the presence or absence of tumor cells overexpressing $\alpha V\beta 6$ and HER2 by measuring the expression levels of $\alpha V\beta 6$ and HER2, wherein the patient is diagnosed with breast cancer sensitive to $\alpha V\beta 6$ and HER2 inhibition if $\alpha V\beta 6$ and HER2 are both overexpressed. By sensitive to inhibition, this includes improvement of at least 10%, 20%, 30%, 40%, 50%, 60%, 70%, 80%, 90%, 95%, 99%, or 100% in any parameter of tumor or cancer cell progression, including but not limited to reduction in tumor growth, tumor size, tumor aggression, or tumor invasion, in either patients and/or laboratory experiments, and/or extended survival in either patients and/or laboratory models; and/or reduction in signaling from downstream molecular messengers.

In another embodiment, a method for diagnosing and treating cancer sensitive to $\alpha V\beta 6$ inhibition in a patient comprises analyzing a patient sample for the presence or absence of cancer cells overexpressing $\alpha V\beta 6$ by measuring the levels of $\alpha V\beta 6$, wherein the patient is diagnosed with cancer sensitive to $\alpha V\beta 6$ inhibition if $\alpha V\beta 6$ is overexpressed, and administering to the diagnosed patient a therapeutically effective dose of an $\alpha V\beta 6$ targeted binding agent that specifically binds to $\alpha V\beta 6$ and inhibits binding of ligands to $\alpha V\beta 6$.

In one aspect, the method also comprises measuring the levels of HER2, wherein the patient is diagnosed with a cancer sensitive to HER2 inhibition if HER2 is overexpressed.

In another embodiment, a method for diagnosing and treating breast cancer sensitive to HER2 inhibition in a patient comprises analyzing a patient sample for the presence or absence of breast cancer cells overexpressing αVβ6 and HER2 by measuring the levels of the αVβ6 and HER2, wherein the patient is diagnosed with breast cancer sensitive to αVβ6 and HER2 inhibition if both αVβ6 and HER2 are overexpressed, and administering to the diagnosed patient a therapeutically effective dose of a HER2 targeted binding agent that specifically binds to HER2 and inhibits binding of ligands to HER2.

Another embodiment includes a method for diagnosing and treating breast cancer sensitive to αVβ6 and HER2 inhibition in a patient comprising analyzing a patient sample for the presence or absence of breast cancer cells overexpressing αVβ6 and HER2 by measuring the levels of the αVβ6 and HER2, wherein the patient is diagnosed with breast cancer sensitive to αVβ6 and HER2 inhibition if both αVβ6 and HER2 are overexpressed, and administering to the diagnosed patient a therapeutically effective dose of an αVβ6 targeted binding agent that specifically binds to αVβ6 and inhibits binding of ligands to αVβ6 and a HER2 targeted binding agent that specifically binds to HER2 and inhibits binding of ligands to HER2.

An embodiment yet further includes method for treating cancer sensitive to αVβ6 inhibition in a patient sample comprising requesting a test to determine whether a patient sample contains cancer cells overexpressing αVβ6, and administering a therapeutically effective dose of an αVβ6 targeted binding agent that specifically binds to αVβ6 and inhibits binding of ligands to αVβ6 if the patient sample contains cancer cells overexpressing αVβ6.

One embodiment includes a method for treating breast cancer sensitive to αVβ6 and HER2 inhibition in a patient sample comprising requesting a test to determine whether a patient sample contains cancer cells overexpressing αVβ6 and HER2, and administering a therapeutically effective dose of:

a. an αVβ6 targeted binding agent that specifically binds to αVβ6 and inhibits binding of ligands to αVβ6; and

b. a HER2 targeted binding agent that specifically binds to HER2 and inhibits binding of ligands to HER2

if the patient sample contains cancer cells overexpressing αVβ6 and HER2.

In one embodiment, the expression levels are measured by measuring protein expression. In one method, αVβ6 and/or HER2 are detected by the extent of tumor cell staining and/or the intensity of tumor cell staining. In one embodiment, αVβ6 and/or HER2 are detected by the extent of tumor cells staining using a scoring system where 0=0%, 1=<25%, 2=25-50%, 3=>50%-75%, and 4=>75%. In another embodiment, αVβ6 and/or HER2 are detected by an intensity of tumor cell staining score of 0=negative, 1=weak, 2=moderate, 3=strong. In one embodiment, the αVβ6 is quantified as overexpressed if it has a final score of ≥5 when the score of extent of tumor cell staining and the score of intensity of staining in a scoring are added together. In another embodiment, the HER2 is quantified as overexpressed if it has a final score of ≥5 when the score of extent of tumor cell staining and the score of intensity of staining in a scoring are added together. In one embodiment, each sample is scored by more than one pathologist and the scores are averaged.

In one method, tumors may be classified for αVβ6 positivity by IHC samples were stained for αVβ6, and then using an independent pathologist scoring system tumor classified on a 0-7 staining intensity scale (which is a composite of 0-4 percentage positivity, and then 0-3 for percentage intensity). From this scale scores of 5, 6, 7 were deemed strongly avb6 positive (approximately 15.1% and 16% of total samples across 2 cohorts of tumors). this was performed using two independent pathologists. Tumors over-expressing avb6 integrin can be determined using a scaled pathologies scoring system incorporating both intensity and percentage cell positivity. The scoring system would be transferred from sample set to sample set using reference samples representative of each scoring intensity relative to an internal control. Alternatively automated imaging techniques can be used using reference samples to set thresholds. These platforms commonly include colour deconvolution algorithms, positive pixel counts, combined with pattern recognition software. Examples of such platforms include Aperio Genie(™) and Definiens(™) automated image quantification packages.

In another embodiment, the expression levels are measured by measuring mRNA expression. For example, αVβ6 expression levels are measured by measuring mRNA expression of ITGB6, which is the gene for the β6 subunit. Numerous techniques such as qRT-PCR, Fluidigm, Nanostring, RNAseq (e.g. Illumina), Affymetrix gene profiling may be used by the person skilled in the art using their common general knowledge to measure the RNA levels and these may be calibrated against the IHC analysis to establish suitable scoring levels.

A method for diagnosing cancer sensitive to αVβ6 inhibition in a patient that can be treated by inhibiting αVβ6 comprising:

a. obtaining a biological sample from the subject;

b. applying an αVβ6 targeted binding agent that specifically binds to αVβ6 to the sample, wherein the presence of αVβ6 creates a αVβ6 targeted binding agent-αVβ6 complex;

c. diagnosing an aggressive form of breast cancer where the complex of step b) is detected at a level indicating αVβ6 overexpression.

A method for diagnosing breast cancer sensitive to αVβ6 and HER2 inhibition in a patient that can be treated by inhibiting αVβ6 and HER2 comprising:

a. obtaining a biological sample from the subject;

b. applying an αVβ6 targeted binding agent that specifically binds to αVβ6 to the sample, wherein the presence of αVβ6 creates a αVβ6 targeted binding agent-αVβ6 complex;

c. optionally applying a HER2 targeted binding agent that specifically binds to HER2 to the sample, wherein the presence of HER2 creates a HER2 binding agent-HER2 complex; and

d. diagnosing an aggressive form of breast cancer where the complexes of steps b) and c) are detected at a level indicating αVβ6 and HER2 overexpression.

A complex of αVβ6 targeted binding agent and αVβ6 or a complex of a HER2 targeted binding agent and HER2 may be detected by methods well known in the art. In one embodiment, the extent of tumor cell staining and/or the intensity of tumor cell staining may be used, as described above. In another embodiment, if the targeted binding agent is an antibody, an ELISA assay may be used to measure overexpression. Alternatively, an immunohistochemical analysis may be used. Alternatively, FMAT macroconfocal scanning may be used to detect the complex.

**IV. Further Description of αVβ6 Targeted Binding Agent**

Embodiments relate to targeted binding agents that bind to αVβ6 integrin (αVβ6). In some embodiments, the binding agents bind to αVβ6 and inhibit the binding of ligands to αVβ6. In one embodiment, the targeted binding agents are monoclonal antibodies, or binding fragments thereof. In another embodiment, the antibodies bind only to the β6 chain yet are able to inhibit binding of ligands to αVβ6.

Other embodiments include fully human anti-αVβ6 antibodies, and antibody preparations that are therapeutically useful. In one embodiment, the anti-αVβ6 antibody preparations have desirable therapeutic properties, including strong binding affinity for αVβ6, and the ability to inhibit TGFβLAP mediated cell adhesion *in vitro.*

Embodiments also include fully human isolated binding fragments of anti-αVβ6 antibodies. In one embodiment the binding fragments are derived from fully human anti-αVβ6 antibodies. Exemplary fragments include Fv, Fab' or other well-known antibody fragments, as described in more detail below. Embodiments also include cells that express fully human antibodies against αVβ6. Examples of cells include hybridomas, or recombinantly created cells, such as Chinese hamster ovary (CHO) cells, variants of CHO cells (for example DG44) and NS0 cells that produce antibodies against αVβ6. Additional information about variants of CHO cells can be found in Andersen and Reilly (2004) Current Opinion in Biotechnology 15, 456-462 which is incorporated herein in its entirety by reference.

In addition, embodiments include methods of using these antibodies for treating an αVβ6-related disease or disorder. An αVβ6-related disease or disorder can be any condition arising due to the aberrant activation or expression of αVβ6. Examples of such diseases include where αVβ6 aberrantly interacts with its ligands thereby altering cell-adhesion or cell signaling properties. This alteration in cell adhesion or cell signaling properties can result in neoplastic diseases. Other αVβ6-related diseases or disorders include inflammatory disorders, lung disease, diseases associated with fibrosis and any disease associated with dysregulated TGF-β.

In one example, the αVβ6-related disease is a neoplastic disease such as melanoma, small cell lung cancer, non-small cell lung cancer, glioma, hepatocellular (liver) carcinoma, thyroid tumor, gastric (stomach) cancer, prostate cancer, breast cancer, ovarian cancer, bladder cancer, lung cancer, glioblastoma, endometrial cancer, kidney cancer, colon cancer, pancreatic cancer, oesophageal carcinoma, head and neck cancers, mesothelioma, sarcomas, biliary (cholangiocarcinoma), small bowel adenocarcinoma, pediatric malignancies and epidermoid carcinoma.

In another example, the αVβ6-related disease is an inflammatory disorder such as inflammatory bowel disease; systemic lupus erythematosus; rheumatoid arthritis; juvenile chronic arthritis; spondyloarthropathies; systemic sclerosis, for example, scleroderma; idiopathic inflammatory myopathies for example, dermatomyositis, polymyositis; Sjogren's syndrome; systemic vaculitis; sarcoidosis; thyroiditis, for example, Grave's disease, Hashimoto's thyroiditis, juvenile lymphocytic thyroiditis, atrophic thyroiditis; immune-mediated renal disease, for example, glomerulonephritis, tubulointerstitial nephritis; demyelinating diseases of the central and peripheral nervous systems such as multiple sclerosis, idiopathic polyneuropathy; hepatobiliary diseases such as infectious hepatitis such as hepatitis A, B, C, D, E and other nonhepatotropic viruses; autoimmune chronic active hepatitis; primary biliary cirrhosis; granulomatous hepatitis; and sclerosing cholangitis; inflammatory and fibrotic lung diseases (*e.g.*, cystic fibrosis); gluten-sensitive enteropathy; autoimmune or immune-mediated skin diseases including bullous skin diseases, erythema multiforme and contact dermatitis, psoriasis; allergic diseases of the lung such as eosinophilic pneumonia, idiopathic pulmonary fibrosis, allergic conjunctivitis and hypersensitivity pneumonitis, transplantation associated diseases including graft rejection and graft-versus host disease.

In yet another example, the αVβ6-related disease is fibrosis such as kidney or lung fibrosis.

In yet another example, the αVβ6-related disease is associated with dysregulated TGF-β include cancer and connective tissue (fibrotic) disorders.

Other embodiments include diagnostic assays for specifically determining the quantity of αVβ6 in a biological sample. The assay kit can include anti-αVβ6 antibodies along with the labels for detecting such antibodies. These diagnostic assays are useful to screen for αV related diseases or β6 disorders including, but not limited to, neoplastic diseases, such as, melanoma, small cell lung cancer, non-small cell lung cancer, glioma, hepatocellular (liver) carcinoma, glioblastoma, and carcinoma of the thyroid, stomach, prostate, breast, ovary, bladder, lung, uterus, kidney, colon, and pancreas, salivary gland, and colorectum.

Another aspect is an antagonist of the biological activity of αVβ6 wherein the antagonist binds to αVβ6. In one embodiment, the antagonist is a targeted binding agent, such as an antibody. The antagonist may bind to:

i) β6 alone;
ii) αVβ6; or
iii) the αVβ6/ligand complex,

or a combination of these. In one embodiment the antibody is able to antagonize the biological activity of αVβ6 *in vitro* and *in vivo.* The antibody may be selected from fully human monoclonal antibody *e.g.*, sc 264 RAD, sc 264 RAD/ADY, sc 188 SDM, sc 133, sc 133 TMT, sc 133 WDS, sc 133 TMT/WDS, sc 188, sc 254, sc 264 or sc 298 or variants thereof.

In one embodiment the antagonist of the biological activity of αVβ6 may bind to αVβ6 and thereby prevent TGFβLAP mediated cell adhesion.

One embodiment is an antibody which binds to the same epitope or epitopes as fully human monoclonal antibody c 264 RAD, sc 264 RAD/ADY, sc 188 SDM, sc 133, sc 133 TMT, sc 133 WDS, sc 133 TMT/WDS, sc 188, sc 254, sc 264 or sc 298.

In one embodiment, the targeted binding agent binds αVβ6 with a $K_d$ of less than 100 nanomolar (nM). The targeted binding agent may bind with a $K_d$ less than about 35 nanomolar (nM). The targeted binding agent may bind with a $K_d$ less than about 25 nanomolar (nM). The targeted binding agent may bind with a $K_d$ less than about 10 nanomolar (nM). In another embodiment, the targeted binding agent binds with a $K_d$ of less than about 60 picomolar (pM).

One embodiment is an antibody-secreting plasma cell that produces the light chain and/or the heavy chain of antibody as described hereinabove. In one embodiment the plasma cell produces the light chain and/or the heavy chain of a fully human monoclonal antibody. In another embodiment the plasma cell produces the light chain and/or the heavy chain of the fully human monoclonal antibody c 264 RAD, sc 264 RAD/ADY, sc 188 SDM, sc 133, sc 133 TMT, sc 133 WDS, sc 133 TMT/WDS, sc 188, sc 254, sc 264 or sc 298. Alternatively the plasma cell may produce an antibody which binds to the same epitope or epitopes as fully human monoclonal antibody sc c 264 RAD, sc 264 RAD/ADY, sc 188 SDM, sc 133, sc 133 TMT, sc 133 WDS, sc 133 TMT/WDS, sc 188, sc 254, sc 264 or sc 298.

Another embodiment is a nucleic acid molecule encoding the light chain or the heavy chain of an antibody as described hereinabove. In one embodiment the nucleic acid molecule encodes the light chain or the heavy chain of a fully human monoclonal antibody. Still another embodiment is a nucleic acid molecule encoding the light chain or the heavy chain of a fully human monoclonal antibody selected from antibodies c 264 RAD, sc 264 RAD/ADY, sc 188 SDM, sc 133, sc 133 TMT, sc 133 WDS, sc 133 TMT/WDS, sc 188, sc 254, sc 264 or sc 298.

Another embodiment is a vector comprising a nucleic acid molecule or molecules as described hereinabove, wherein the vector encodes a light chain and/or a heavy chain of an antibody as defined hereinabove.

Yet another embodiment is a host cell comprising a vector as described hereinabove. Alternatively the host cell may comprise more than one vector.

In addition, one embodiment is a method of producing an antibody by culturing host cells under conditions wherein a nucleic acid molecule is expressed to produce the antibody, followed by recovery of the antibody.

One embodiment includes a method of making an antibody by transfecting at least one host cell with at least one nucleic acid molecule encoding the antibody as described hereinabove, expressing the nucleic acid molecule in the host cell and isolating the antibody.

Another aspect includes a method of antagonising the biological activity of αVβ6 comprising administering an antagonist as described herein. The method may include selecting an animal in need of treatment for an αVβ6 related disease or disorder, and administering to the animal a therapeutically effective dose of an antagonist of the biological activity of αVβ6.

Another aspect includes a method of antagonising the biological activity of αVβ6 comprising administering an antibody as described hereinabove. The method may include selecting an animal in need of treatment for an αVβ6 related disease or disorder, and administering to said animal a therapeutically effective dose of an antibody which antagonises the biological activity of αVβ6.

According to another aspect there is provided a method of treating an αVβ6 related disease or disorder in a mammal comprising administering a therapeutically effective amount of an antagonist of the biological activity of αVβ6. The method may include selecting an animal in need of treatment for an αVβ6 related disease or disorder, and administering to said animal a therapeutically effective dose of an antagonist of the biological activity of αVβ6.

According to another aspect there is provided a method of treating an αVβ6 disease or disorder in a mammal comprising administering a therapeutically effective amount of an antibody which antagonizes the biological activity of αVβ6. The method may include selecting an animal in need of treatment for an αVβ6 related disease or disorder, and administering to said animal a therapeutically effective dose of an antibody which antagonises the biological activity of αVβ6. The antibody can be administered alone, or can be administered in combination with additional antibodies or chemotherapeutic drug or radiation therapy.

According to another aspect there is provided a method of treating cancer in a mammal comprising administering a therapeutically effective amount of an antagonist of the biological activity of αVβ6. The method may include selecting an animal in need of treatment for cancer, and administering to said animal a therapeutically effective dose of an antagonist which antagonises the biological activity of αVβ6. The antagonist can be administered alone, or can be administered in combination with additional antibodies or chemotherapeutic drug or radiation therapy.

According to another aspect there is provided a method of treating cancer in a mammal comprising administering a therapeutically effective amount of an antibody which antagonizes the biological activity of αVβ6. The method may include selecting an animal in need of treatment for cancer, and administering to said animal a therapeutically effective dose of an antibody which antagonises the biological activity of αVβ6. The antibody can be administered alone, or can be administered in combination with additional antibodies or chemotherapeutic drug or radiation therapy.

According to another aspect there is provided the use of an antagonist of the biological activity of αVβ6 for the manufacture of a medicament for the treatment of an αVβ6 related disease or disorder.

According to another aspect there is provided the use of an antibody which antagonizes the biological activity of αVβ6 for the manufacture of a medicament for the treatment of an αVβ6 related disease or disorder.

One embodiment is particularly suitable for use in antagonizing αVβ6, in patients with a tumor which is dependent alone, or in part, on αVβ6 integrin.

Another embodiment includes an assay kit for detecting αVβ6 in mammalian tissues, cells, or body fluids to screen for an αVβ6 related disease or disorder. The kit includes an antibody that binds to αVβ6 and a means for indicating the reaction of the antibody with αVβ6, if present. The antibody may be a monoclonal antibody. In one embodiment, the antibody that binds αVβ6 is labeled. In another embodiment the antibody is an unlabeled primary antibody and the kit further includes a means for detecting the primary antibody. In one embodiment, the means includes a labeled second antibody that is an anti-immunoglobulin. In one aspect, the antibody is labeled with a marker chosen from a fluorochrome, an enzyme, a radionuclide and a radio-opaque material.

Further embodiments, features, and the like regarding anti-αVβ6 antibodies are provided in additional detail below.

## A. Human Antibodies and Humanization of Antibodies

Human antibodies avoid some of the problems associated with antibodies that possess murine or rat variable and/or constant regions. The presence of such murine or rat derived proteins can lead to the rapid clearance of the antibodies or can lead to the generation of an immune response against the antibody by a patient. In order to avoid the utilization of murine or rat derived antibodies, fully human antibodies can be generated through the introduction of functional human antibody loci into a rodent, other mammal or animal so that the rodent, other mammal or animal produces fully human antibodies.

One method for generating fully human antibodies is through the use of XenoMouse® strains of mice that have been engineered to contain up to but less than 1000 kb-sized germline configured fragments of the human heavy chain locus and kappa light chain locus. *See* Mendez et al., Nature Genetics 15:146-156 (1997) and Green and Jakobovits J. Exp. Med. 188:483-495 (1998). The XenoMouse® strains are available from Amgen, Inc. (Fremont, CA).

The production of the XenoMouse® strains of mice is further discussed and delineated in U.S. Patent Application Serial Nos. 07/466,008, filed January 12, 1990, 07/610,515, filed November 8, 1990, 07/919,297, filed July 24, 1992, 07/922,649, filed July 30, 1992, 08/031,801, filed March 15, 1993, 08/112,848, filed August 27, 1993, 08/234,145, filed April 28, 1994, 08/376,279, filed January 20, 1995, 08/430, 938, filed April 27, 1995, 08/464,584, filed June 5, 1995, 08/464,582, filed June 5, 1995, 08/463,191, filed June 5, 1995, 08/462,837, filed June 5, 1995, 08/486,853, filed June 5, 1995, 08/486,857, filed June 5, 1995, 08/486,859, filed June 5, 1995, 08/462,513, filed June 5, 1995, 08/724,752, filed October 2, 1996, 08/759,620, filed December 3, 1996, U.S. Publication 2003/0093820, filed November 30, 2001 and U.S. Patent Nos. 6,162,963, 6,150,584, 6,114,598, 6,075,181, and 5,939,598 and Japanese Patent Nos. 3 068 180 B2, 3 068 506 B2, and 3 068 507 B2. *See also* European Patent No., EP 0 463 151 B1, grant published June 12, 1996, International Patent Application No., WO 94/02602, published February 3, 1994, International Patent Application No., WO 96/34096, published October 31, 1996, WO 98/24893, published June 11, 1998, WO 00/76310, published December 21, 2000.

In an alternative approach, others, including GenPharm International, Inc., have utilized a "minilocus" approach. In the minilocus approach, an exogenous Ig locus is mimicked through the inclusion of pieces (individual genes) from the Ig locus. Thus, one or more $V_H$ genes, one or more $D_H$ genes, one or more $J_H$ genes, a mu constant region, and usually a second constant region (optionally a gamma constant region) are formed into a construct for insertion into an animal. This approach is described in U.S. Patent No. 5,545,807 to Surani et al., and U.S. Patent Nos. 5,545,806, 5,625,825, 5,625,126, 5,633,425, 5,661,016, 5,770,429, 5,789,650, 5,814,318, 5,877,397, 5,874,299, and 6,255,458 each to Lonberg and Kay, U.S. Patent No. 5,591,669 and 6,023.010 to Krimpenfort and Berns, U.S. Patent Nos. 5,612,205, 5,721,367, and 5,789,215 to Berns et al., and U.S. Patent No. 5,643,763 to Choi and Dunn, and GenPharm International U.S. Patent Application Serial Nos. 07/574,748, filed August 29, 1990, 07/575,962, filed August 31, 1990, 07/810,279, filed December 17, 1991, 07/853,408, filed March 18, 1992, 07/904,068, filed June 23, 1992, 07/990,860, filed December 16, 1992, 08/053,131, filed April 26, 1993, 08/096,762, filed July 22, 1993, 08/155,301, filed November 18, 1993, 08/161,739, filed December 3, 1993, 08/165,699, filed December 10, 1993, 08/209,741, filed March, 1994. *See also* European Patent No. 0 546 073 B1, International Patent Application Nos. WO 92/03918, WO 92/22645, WO 92/22647, WO 92/22670, WO 93/12227, WO 94/00569, WO 94/25585, WO 96/14436, WO 97/13852, and WO 98/24884 and U.S. Patent No. 5,981,175. *See further* Taylor *et al.,* 1992, *Chen et al.,* 1993, Tuaillon *et al.,* 1993, Choi *et al.,* 1993, Lonberg *et al.,* (1994), Taylor *et al.,* (1994), and Tuaillon *et al.,* (1995), Fishwild *et al.*, (1996).

Kirin has also demonstrated the generation of human antibodies from mice in which, through microcell fusion, large pieces of chromosomes, or entire chromosomes, have been introduced. *See* European Patent Application Nos. 773 288 and 843 961. Additionally, KM™- mice, which are the result of cross-breeding of Kirin's Tc mice with Medarex's minilocus (Humab) mice have been generated. These mice possess the human IgH transchromosome of the Kirin mice and the kappa chain transgene of the Genpharm mice (Ishida et al., Cloning Stem Cells, (2002) 4:91-102).

Human antibodies can also be derived by *in vitro* methods. Suitable examples include but are not limited to phage display (CAT, Morphosys, Dyax, Biosite/Medarex, Xoma, Symphogen, Alexion (formerly Proliferon), Affimed) ribosome display (CAT), yeast display, and the like.

**B. Preparation of Antibodies**

Antibodies, as described herein, were prepared through the utilization of the XenoMouse® technology, as described below. Such mice, then, are capable of producing human immunoglobulin molecules and antibodies and are deficient in the production of murine immunoglobulin molecules and antibodies. Technologies utilized for achieving the same are disclosed in the patents, applications, and references disclosed in the background section herein. In particular, however, an embodiment of transgenic production of mice and antibodies therefrom is disclosed in U.S. Patent Application Serial No. 08/759,620, filed December 3, 1996 and International Patent Application Nos. WO 98/24893, published June 11, 1998 and WO 00/76310, published December 21, 2000. *See also* Mendez et al., Nature Genetics 15:146-156 (1997).

Through the use of such technology, fully human monoclonal antibodies to a variety of antigens have been produced. Essentially, XenoMouse® lines of mice are immunized with an antigen of interest (e.g. $\alpha V\beta 6$), lymphatic cells (such as B-cells) are recovered from the hyper-immunized mice, and the recovered lymphocytes are fused with a myeloid-type cell line to prepare immortal hybridoma cell lines. These hybridoma cell lines are screened and selected to identify hybridoma cell lines that produced antibodies specific to the antigen of interest. Provided herein are methods for the production of multiple hybridoma cell lines that produce antibodies specific to $\alpha V\beta 6$. Further, provided herein are characterization of the antibodies produced by such cell lines, including nucleotide and amino acid sequence analyses of the heavy and light chains of such antibodies.

Alternatively, instead of being fused to myeloma cells to generate hybridomas, B cells can be directly assayed. For example, CD19+ B cells can be isolated from hyperimmune XenoMouse® mice and allowed to proliferate and differentiate into antibody-secreting plasma cells. Antibodies from the cell supernatants are then screened by ELISA for reactivity against the $\alpha V\beta 6$ immunogen. The supernatants might also be screened for immunoreactivity against fragments of $\alpha V\beta 6$ to further map the different antibodies for binding to domains of functional interest on $\alpha V\beta 6$. The antibodies may also be screened against other related human integrins and against the rat, the mouse, and non-human primate, such as Cynomolgus monkey, orthologues of $\alpha V\beta 6$, the last to determine species cross-reactivity. B cells from wells containing antibodies of interest may be immortalized by various methods including fusion to make hybridomas either from individual or from pooled wells, or by infection with EBV or transfection by known immortalizing genes and then plating in suitable medium. Alternatively, single plasma cells secreting antibodies with the desired specificities are then isolated using a $\alpha V\beta 6$-specific hemolytic plaque assay (see for example Babcook et al., Proc. Natl. Acad. Sci. USA 93:7843-48 (1996)).

Cells targeted for lysis may be sheep red blood cells (SRBCs) coated with the αVβ6 antigen.

In the presence of a B-cell culture containing plasma cells secreting the immunoglobulin of interest and complement, the formation of a plaque indicates specific αVβ6-mediated lysis of the sheep red blood cells surrounding the plasma cell of interest. The single antigen-specific plasma cell in the center of the plaque can be isolated and the genetic information that encodes the specificity of the antibody is isolated from the single plasma cell. Using reverse-transcription followed by PCR (RT-PCR), the DNA encoding the heavy and light chain variable regions of the antibody can be cloned. Such cloned DNA can then be further inserted into a suitable expression vector, a vector cassette such as a pcDNA, or a pcDNA vector containing the constant domains of immunglobulin heavy and light chain. The generated vector can then be transfected into host cells, *e.g.,* HEK293 cells, CHO cells, and cultured in conventional nutrient media modified as appropriate for inducing transcription, selecting transformants, or amplifying the genes encoding the desired sequences.

In general, antibodies produced by the fused hybridomas were human IgG2 heavy chains with fully human kappa or lambda light chains. Antibodies described herein possess human IgG4 heavy chains as well as IgG2 heavy chains. Antibodies can also be of other human isotypes, including IgG1. The antibodies possessed high affinities, typically possessing a Kd of from about $10^{-6}$ through about $10^{-12}$ M or below, when measured by solid phase and solution phase techniques. Antibodies possessing a Kd of at least $10^{-11}$ M may inhibit the activity of αVβ6.

As will be appreciated, antibodies can be expressed in cell lines other than hybridoma cell lines. Sequences encoding particular antibodies can be used to transform a suitable mammalian host cell. Transformation can be by any known method for introducing polynucleotides into a host cell, including, for example packaging the polynucleotide in a virus (or into a viral vector) and transducing a host cell with the virus (or vector) or by transfection procedures known in the art, as exemplified by U.S. Patent Nos. 4,399,216, 4,912,040, 4,740,461, and 4,959,455. The transformation procedure used depends upon the host to be transformed. Methods for introducing heterologous polynucleotides into mammalian cells are well known in the art and include dextran-mediated transfection, calcium phosphate precipitation, polybrene mediated transfection, protoplast fusion, electroporation, encapsulation of the polynucleotide(s) in liposomes, and direct microinjection of the DNA into nuclei.

Mammalian cell lines available as hosts for expression are well known in the art and include many immortalized cell lines available from the American Type Culture Collection (ATCC), including but not limited to Chinese hamster ovary (CHO) cells, HeLa cells, baby hamster kidney (BHK) cells, monkey kidney cells (COS), human hepatocellular carcinoma cells (*e.g.,* Hep G2), human epithelial kidney 293 cells, and a number of other cell lines. Cell lines may be selected through determining which cell lines have high expression levels and produce antibodies with constitutive αVβ6 binding properties.

Based on the ability of mAbs to significantly neutralize αVβ6 activity (as demonstrated in the Examples below), these antibodies will have therapeutic effects in treating symptoms and conditions resulting from αVβ6 expression. In specific embodiments, the antibodies and methods herein relate to the treatment of symptoms resulting from αVβ6 induced cell adhesion or signaling induced as a result of αVβ6 interaction with its ligands

According to another aspect there is provided a pharmaceutical composition comprising an antagonist of the biological activity of αVβ6, and a pharmaceutically acceptable carrier. In one embodiment the antagonist comprises an antibody. According to another aspect there is provided a pharmaceutical composition comprising an antagonist of the biological activity of αVβ6, and a pharmaceutically acceptable carrier. In one embodiment the antagonist comprises an antibody.

Anti-αVβ6 antibodies are useful in the detection of αVβ6 in patient samples and accordingly are useful as diagnostics for disease states as described herein. In addition, based on their ability to significantly inhibit αVβ6 activity (as demonstrated in the Examples below), anti-αVβ6 antibodies have therapeutic effects in treating symptoms and conditions resulting from αVβ6 expression. In specific embodiments, the antibodies and methods herein relate to the treatment of symptoms resulting from αVβ6 induced cell adhesion. Further embodiments involve using the antibodies and methods described herein to treat an αVβ6 related disease or disorder including neoplastic diseases, such as, melanoma, small cell lung cancer, non-small cell lung cancer, glioma, hepatocellular (liver) carcinoma, thyroid tumor, gastric (stomach) cancer, prostate cancer, breast cancer, ovarian cancer, bladder cancer, lung cancer, glioblastoma, endometrial cancer, kidney cancer, colon cancer, and pancreatic cancer.

## C. Therapeutic Administration and Formulations

Embodiments include sterile pharmaceutical formulations of anti-αVβ6 antibodies that are useful as treatments for diseases. Such formulations would inhibit the binding of ligands to the αVβ6 integrin, thereby effectively treating pathological conditions where, for example, tissue αVβ6 is abnormally elevated. Anti-αVβ6 antibodies may possess adequate affinity to potently inhibit αVβ6 activity, and may have an adequate duration of action to allow for infrequent dosing in humans. A prolonged duration of action will allow for less frequent and more convenient dosing schedules by alternate parenteral routes such as subcutaneous or intramuscular injection.

Sterile formulations can be created, for example, by filtration through sterile filtration membranes, prior to or following lyophilization and reconstitution of the antibody. The antibody ordinarily will be stored in lyophilized form or in solution. Therapeutic antibody compositions generally are placed into a container having a sterile access port, for example, an intravenous solution bag or vial having an adapter that allows retrieval of the formulation, such as a stopper pierceable by a hypodermic injection needle.

The route of antibody administration is in accord with known methods, *e.g.*, injection or infusion by intravenous, intraperitoneal, intracerebral, intramuscular, intraocular, intraarterial, intrathecal, inhalation or intralesional routes, direct injection to a tumor site, or by sustained release systems as noted below. The antibody may be administered continuously by infusion or by bolus injection.

An effective amount of antibody to be employed therapeutically will depend, for example, upon the therapeutic objectives, the route of administration, and the condition of the patient. Accordingly, the therapist may titer the dosage and modify the route of administration to obtain the optimal therapeutic effect. In one aspect, the clinician will administer antibody until a dosage is reached that achieves the desired effect. The progress of this therapy is easily monitored by conventional assays or by the assays described herein.

Antibodies, as described herein, can be prepared in a mixture with a pharmaceutically acceptable carrier. This therapeutic composition can be administered intravenously or through the nose or lung, optionally as a liquid or powder aerosol (lyophilized). The composition may also be administered parenterally or subcutaneously as desired. When administered systemically, the therapeutic composition should be sterile, pyrogen-free and in a parenterally acceptable solution having due regard for pH, isotonicity, and stability. These conditions are known to those skilled in the art. Briefly, dosage formulations of the compounds described herein are prepared for storage or administration by mixing the compound having the desired degree of purity with pharmaceutically acceptable carriers, excipients, or stabilizers. Such materials are non-toxic to the recipients at the dosages and concentrations employed, and include buffers such as TRIS HCl, phosphate, citrate, acetate and other organic acid salts; antioxidants such as ascorbic acid; low molecular weight (less than about ten residues) peptides such as polyarginine, proteins, such as serum albumin, gelatin, or immunoglobulins; hydrophilic polymers such as polyvinylpyrrolidinone; amino acids such as glycine, glutamic acid, aspartic acid, or arginine; monosaccharides, disaccharides, and other carbohydrates including cellulose or its derivatives, glucose, mannose, or dextrins; chelating agents such as EDTA; sugar alcohols such as mannitol or sorbitol; counterions such as sodium and/or nonionic surfactants such as TWEEN, PLURONICS or polyethyleneglycol.

Sterile compositions for injection can be formulated according to conventional pharmaceutical practice as described in Remington: The Science and Practice of Pharmacy (20th ed, Lippincott Williams & Wilkens Publishers (2003)). For example, dissolution or suspension of the active compound in a pharmaceutically acceptable carrier such as water or naturally occurring vegetable oil like sesame, peanut, or cottonseed oil or a synthetic fatty vehicle like ethyl oleate or the like may be desired. Buffers, preservatives, antioxidants and the like can be incorporated according to accepted pharmaceutical practice.

Suitable examples of sustained-release preparations include semipermeable matrices of solid hydrophobic polymers containing the polypeptide, which matrices are in the form of shaped articles, films or microcapsules. Examples of sustained-release matrices include polyesters, hydrogels (e.g., poly(2-hydroxyethyl-methacrylate) as described by Langer et al., J. BiomedMater. Res., (1981) 15:167-277 and Langer, Chem. Tech., (1982) 12:98-105, or poly(vinylalcohol)), polylactides (U.S. Pat. No. 3,773,919, EP 58,481), copolymers of L-glutamic acid and gamma ethyl-L-glutamate (Sidman et al., Biopolymers, (1983) 22:547-556), non-degradable ethylene-vinyl acetate (Langer *et al., supra*), degradable lactic acid-glycolic acid copolymers such as the LUPRON Depot™ (injectable microspheres composed of lactic acid-glycolic acid copolymer and leuprolide acetate), and poly-D-(-)-3-hydroxybutyric acid (EP 133,988).

While polymers such as ethylene-vinyl acetate and lactic acid-glycolic acid enable release of molecules for over 100 days, certain hydrogels release proteins for shorter time periods. When encapsulated proteins remain in the body for a long time, they may denature or aggregate as a result of exposure to moisture at 37°C, resulting in a loss of biological activity and possible changes in immunogenicity. Rational strategies can be devised for protein stabilization depending on the mechanism involved. For example, if the aggregation mechanism is discovered to be intermolecular S-S bond formation through disulfide interchange, stabilization may be achieved by modifying sulfhydryl residues, lyophilizing from acidic solutions, controlling moisture content, using appropriate additives, and developing specific polymer matrix compositions.

The antibodies also encompass antibodies that have half-lives (*e.g.*, serum half-lives) in a mammal, optionally a human, of greater than that of an unmodified antibody. In one embodiment, said antibody anybody half-life is greater than 15 days, greater than 20 days, greater than 25 days, greater than 30 days, greater than 35 days, greater than 40 days, greater than 45 days, greater than 2 months, greater than 3 months, greater than 4 months, or greater than 5 months. The increased half-lives of the antibodies herein or fragments thereof in a mammal, optionally a human, result in a higher serum titer of said antibodies or antibody fragments in the mammal, and thus, reduce the frequency of the administration of said antibodies or antibody fragments and/or reduces the concentration of said antibodies or antibody fragments to be administered. Antibodies or fragments thereof having increased *in vivo* half-lives can be generated by techniques known to those of skill in the art. For example, antibodies or fragments thereof with increased *in vivo* half-lives can be generated by modifying (*e.g.,* substituting, deleting or adding) amino acid residues identified as involved in the interaction between the Fc domain and the FcRn receptor (see, *e.g.*, International Publication Nos. WO 97/34631 and WO 02/060919). Antibodies or fragments thereof with increased *in vivo* half-lives can be generated by attaching to said antibodies or antibody fragments polymer molecules such as high molecular weight polyethyleneglycol (PEG). PEG can be attached to said antibodies or antibody fragments with or without a multifunctional linker either through site-specific conjugation of the PEG to the Nor C-terminus of said antibodies or antibody fragments or via epsilon-amino groups present on lysine residues. Linear or branched polymer derivatization that results in minimal loss of biological activity will be used. The degree of conjugation will be closely monitored by SDS-PAGE and mass spectrometry to ensure proper conjugation of PEG molecules to the antibodies. Unreacted PEG can be separated from antibody-PEG conjugates by, *e.g.*, size exclusion or ion-exchange chromatography.

Sustained-released compositions also include preparations of crystals of the antibody suspended in suitable formulations capable of maintaining crystals in suspension. These preparations when injected subcutaneously or intraperitonealy can produce a sustained release effect. Other compositions also include liposomally entrapped antibodies. Liposomes containing such antibodies are prepared by methods known per se: U.S. Pat. No. DE 3,218,121; Epstein et al., Proc. Natl. Acad. Sci. USA, (1985) 82:3688-3692; Hwang et al., Proc. Natl. Acad. Sci. USA, (1980) 77:4030-4034; EP 52,322; EP 36,676; EP 88,046; EP 143,949; 142,641; Japanese patent application 83-118008; U.S. Pat. Nos. 4,485,045 and 4,544,545; and EP 102,324.

The dosage of the antibody formulation for a given patient will be determined by the attending physician taking into consideration various factors known to modify the action of drugs including severity and type of disease, body weight, sex, diet, time and route of administration, other medications and other relevant clinical factors. Therapeutically effective dosages may be determined by either *in vitro* or *in vivo* methods.

An effective amount of the antibodies, described herein, to be employed therapeutically will depend, for example, upon the therapeutic objectives, the route of administration, and the condition of the patient. Accordingly, the therapist may titer the dosage and modify the route of administration as required to obtain the optimal therapeutic effect. A typical daily dosage might range from about 0.001mg/kg to up to 100mg/kg or more, depending on the factors mentioned above. Typically, the clinician will administer the therapeutic antibody until a dosage is reached that achieves the desired effect. The progress of this therapy is easily monitored by conventional assays or as described herein.

It will be appreciated that administration of therapeutic entities in accordance with the compositions and methods herein will be administered with suitable carriers, excipients, and other agents that are incorporated into formulations to provide improved transfer, delivery, tolerance, and the like. These formulations include, for example, powders, pastes, ointments, jellies, waxes, oils, lipids, lipid (cationic or anionic) containing vesicles (such as Lipofectin™), DNA conjugates, anhydrous absorption pastes, oil-in-water and water-in-oil emulsions, emulsions carbowax (polyethylene glycols of various molecular weights), semi-solid gels, and semi-solid mixtures containing carbowax. Any of the foregoing mixtures may be appropriate in treatments and therapies, provided that the active ingredient in the formulation is not inactivated by the formulation and the formulation is physiologically compatible and tolerable with the route of administration. *See also* Baldrick P. "Pharmaceutical excipient development: the need for preclinical guidance." Regul. Toxicol. Pharmacol. 32(2):210-8 (2000), Wang W. "Lyophilization and development of solid protein pharmaceuticals." Int. J. Pharm. 203(1-2): 1-60 (2000), Charman WN "Lipids, lipophilic drugs, and oral drug delivery-some emerging concepts." J Pharm Sci .89(8):967-78 (2000), Powell et al., "Compendium of excipients for parenteral formulations" PDA J Pharm Sci Technol. 52:238-311 (1998) and the citations therein for additional information related to formulations, excipients and carriers well known to pharmaceutical chemists.

**D. Design and Generation of Other Therapeutics**

In accordance with the present embodiments and based on the activity of the antibodies that are produced and characterized herein with respect to $\alpha V\beta 6$, the design of other therapeutic modalities beyond antibody moieties is facilitated. Such modalities include, without limitation, advanced antibody therapeutics, such as bispecific antibodies, immunotoxins, and radiolabeled therapeutics, single domain antibodies, generation of peptide therapeutics, $\alpha V\beta 6$ binding domains in novel scaffolds, gene therapies, particularly intrabodies, antisense therapeutics, and small molecules.

In connection with the generation of advanced antibody therapeutics, where complement fixation is a desirable attribute, it may be possible to sidestep the dependence on complement for cell killing through the use of bispecific antibodies, immunotoxins, or radiolabels, for example.

Bispecific antibodies can be generated that comprise (i) two antibodies one with a specificity to $\alpha V\beta 6$ and another to a second molecule that are conjugated together, (ii) a single antibody that has one chain specific to $\alpha V\beta 6$ and a second chain specific to a second molecule, or (iii) a single chain antibody that has specificity to $\alpha V\beta 6$ and the other molecule. Such bispecific antibodies can be generated using techniques that are well known; for example, in connection with (i) and (ii) *see e.g.,* Fanger et al., Immunol Methods 4:72-81 (1994) and Wright and Harris, *supra.* and in connection with (iii) *see e.g.,* Traunecker et al., Int. J. Cancer (Suppl.) 7:51-52 (1992). In each case, the second specificity can be made to the heavy chain activation receptors, including, without limitation, CD16 or CD64 *(see e.g.,* Deo et al., Immunol. Today 18:127 (1997)) or CD89 (*see e.g.,* Valerius et al., Blood 90:4485-4492 (1997)).

In connection with immunotoxins, antibodies can be modified to act as immunotoxins utilizing techniques that are well known in the art. *See e.g.,* Vitetta Immunol Today 14:252 (1993). *See also* U.S. Patent No. 5,194,594. In connection with the preparation of radiolabeled antibodies, such modified antibodies can also be readily prepared utilizing techniques that are well known in the art. *See e.g.,* Junghans et al., in Cancer Chemotherapy and Biotherapy 655-686 (2d edition, Chafner and Longo, eds., Lippincott Raven (1996)). *See also* U.S. Patent Nos. 4,681,581, 4,735,210, 5,101,827, 5,102,990 (RE 35,500), 5,648,471, and 5,697,902.

An antigen binding site may be provided by means of arrangement of CDRs on non-antibody protein scaffolds, such as fibronectin or cytochrome B etc. (Haan & Maggos (2004) BioCentury, 12(5): A1-A6; Koide et al., (1998) Journal of Molecular Biology, 284: 1141-1151; Nygren et al., (1997) Current Opinion in Structural Biology, 7: 463-469) or by randomising or mutating amino acid residues of a loop within a protein scaffold to confer binding specificity for a desired target. Scaffolds for engineering novel binding sites in proteins have been reviewed in detail by Nygren *et al.,* (Nygren et al., (1997) Current Opinion in Structural Biology, 7: 463-469). Protein scaffolds for antibody mimics are disclosed in WO/0034784, in which the inventors describe proteins (antibody mimics) that include a fibronectin type III domain having at least one randomised loop. A suitable scaffold into which to graft one or more CDRs, *e.g.* a set of HCDRs, may be provided by any domain member of the immunoglobulin gene superfamily. The scaffold may be a human or non-human protein. An advantage of a non-antibody protein scaffold is that it may provide an antigen-binding site in a scaffold molecule that is smaller and/or easier to manufacture than at least some antibody molecules. Small size of a binding agent may confer useful physiological properties, such as an ability to enter cells, penetrate deep into tissues or reach targets within other structures, or to bind within protein cavities of the target antigen. Use of antigen binding sites in non-antibody protein scaffolds is reviewed in Wess, 2004 (Wess, L. In: BioCentury, The Bernstein Report on BioBusiness, 12(42), A1-A7, 2004). Typical are proteins having a stable backbone and one or more variable loops, in which the amino acid sequence of the loop or loops is specifically or randomly mutated to create an antigen-binding site that binds the target antigen. Such proteins include the IgG-binding domains of protein A from S. aureus, transferrin, albumin, tetranectin, fibronectin (*e.g.* 10th fibronectin type III domain), lipocalins as well as gamma-crystalline and other Affilin™ scaffolds (Scil Proteins). Examples of other approaches include synthetic "Microbodies" based on cyclotides - small proteins having intra-molecular disulphide bonds, Microproteins (Versabodies™, Amunix) and ankyrin repeat proteins (DARPins, Molecular Partners).

In addition to antibody sequences and/or an antigen-binding site, a binding agent may comprise other amino acids, *e.g.* forming a peptide or polypeptide, such as a folded domain, or to impart to the molecule another functional characteristic in addition to ability to bind antigen. Binding agents may carry a detectable label, or may be conjugated to a toxin or a targeting moiety or enzyme (*e.g.* via a peptidyl bond or linker). For example, a binding agent may comprise a catalytic site (*e.g.* in an enzyme domain) as well as an antigen binding site, wherein the antigen binding site binds to the antigen and thus targets the catalytic site to the antigen. The catalytic site may inhibit biological function of the antigen, *e.g.* by cleavage.

## EXAMPLES

### Example 1. Materials and Methods

#### Clinical samples

Two independent cohorts of breast cancer samples were analysed following REMARK guidelines (23). One comprised 1,795 consecutive cases from the Nottingham Tenovus Breast Carcinoma Series (Nottingham Cohort) of women aged <70 years presenting from 1986-1998 (24,25). Data were available on tumor type, histological grade, size, lymph node (LN) status, ER-, PR- and HER2-status, cytokeratin (CK) profile, recurrence (local, regional and distant) and survival. The second cohort constituted 1,197 invasive cases from Guy's and St. Thomas' Breast Tissue Bank, London (London Cohort). Patients underwent surgery from 1960-1998 (98% from 1975 onwards). Data were available on tumor type, grade, LN status, ER-, PR- and HER2-status, disease free- and overall-survival. A summary of clinicopathological data is presented (Figure 10). All studies were approved by the North East London Research Ethics Committee.

#### Immunohistochemical analysis

Immunohistochemistry utilized 4 $\mu$m, formalin-fixed, paraffin-embedded serial sections of tissue microarrays (TMA). Each sample was represented by a minimum of two x 0.6mm tumor cores. A standard Avidin-Biotin Complex technique (Vectastain Elite ABC Kit, Vector Laboratories, Peterborough, UK) was employed, with citrate buffer microwave antigen retrieval for cytokeratin 5/6 (Sigma, UK) and cytokeratin 14 (Sigma, UK). The protocol used for αvβ6 integrin (mAb 6.2G2, Biogen Idec) was described previously (16). Normal breast (n=15) constituted a positive control for cytokeratin antibodies while mouse IgG represented a negative control. Integrin αvβ6 staining was scored as the sum of the extent of tumor cells staining (0,<25%=1,25-50%=2,50-75%=3,>75%=4) and intensity (0=negative, 1=weak, 2=moderate, 3=strong); giving a final score range of 0-7. An example of strong αvβ6 staining is shown in Figure 1B. Each tumor core was scored by two independent pathologists; final score represents mean of the two readings. A pre-determined cut-off, between cases showing strong expression (scores≥5) and those showing moderate or negative staining (scores<5), was used in all analyses. For CK5/6 and CK14 expression, cases were considered positive if >10% staining occurred (25).

**METABRIC cohort preprocessing**

This study makes use of the METABRIC data generated by the Molecular Taxonomy of Breast Cancer International Consortium (26). Funding for the project was provided by Cancer Research UK and the British Columbia Cancer Agency Branch. Breast cancer METABRIC dataset was preprocessed, summarized and quantile-normalized from the raw expression files generated by Illumina BeadStudio. (R packages: beadarray v2.4.2 and illuminaHuman v3.db_1.12.2). Raw METABRIC files were downloaded from European genome-phenome archive (EGA) (study id: EGAS00000000083). Raw data files of one METABRIC sample was not available at the time of our analysis, therefore it was excluded. All preprocessing was performed in R statistical environment v2.14.1.

**Survival analysis**

HER2+ patients in the London and Nottingham clinical cohorts were dichotomised into low- and high-risk groups using αvβ6 protein expression (Low-risk αvβ6<5, High-risk αvβ6≥5). Survival analysis was performed in R statistical environment v.2.14.1 (R package: survival v2.36-14). Hazard ratio was estimated by fitting univariate Cox proportional hazards model, and significance of difference between the survival of risk groups were computed using Logrank test. Likewise, gene expression-derived HER2+ patients in the METABRIC cohort were analysed using ITGB6 expression profile. The riskgroup dichotomisation threshold for ITGB6 expression in METABRIC was established by using the proportion of low- and high-risk HER2+ patients determined by antibody studies of the London/Nottingham cohorts. Kaplan- Meier survival curves were drawn in R statistical environment v2.14.1.

**Cell lines and drug sources**

Twenty human breast cancer cell lines were analyzed for αVβ6-expression. Genetic identity of all lines was confirmed by LGC STR profiling (data not shown). Human breast cancer cell lines MCF-7 and MDA MB-468 cells were grown in DMEM containing 10% fetal bovine serum (FBS) and L-glutamine. MCF-7/neo-1 and MCF-7/HER2-18 were a kind gift from Prof. Hung, Texas, USA (37) Cell sources and media requirements are as detailed (37, 38, 39, 40). BT-474 cells were grown in RPMI containing 10% FCS, L-glutamine and insulin (10μg/ml).

Mouse monoclonal antibody 6.2G2 was a generous gift from Biogen Idec (Cambridge, MA, USA). IgG and αVβ6-blocking antibody 264RAD were generous gifts from Oncology iMED, AstraZeneca (Maccelsfield, U.K). Trastuzumab was a kind gift from Roche Pharmaceuticals. siRNA was supplied by Dharmacon (SMARTpool: siGENOME, Thermo Scientific). Growth factors were supplied by Peprotech.

**Transwell and mini organotypic invasion assays**

For Transwell invasion assays, 5 x $10^4$ cells were seeded per well posttreatment into 6.5mm diameter, 8 μm pore-sized Transwells® (Corning BV) coated with 70 μl BD Matrigel Basement Membrane matrix (Matrigel): media (1:2 ratio). Cells which invaded through Matrigel were counted after 72h using a CASY counter (Scharfe Systems, Germany). For the organotypic assays, 5 x $10^4$ cells were seeded per well post-treatment onto 6.5mm diameter, 4μm pore-sized Transwells with 120μl collagen (Rat tail Collagen I, Marathon Laboratories):Matrigel mix (70:30) containing 5 x $10^4$ MRC5/hTERT fibroblasts. Media was changed every 2 days for 5-6 days, gels were fixed in formal saline, paraffin embedded and sections hematoxylin and eosin stained. Invasion Index was calculated by multiplying the mean depth at 5 points on each gel by the area occupied by the invading cells. Analysis was performed using ImageJ 1 64 software.

**Immunoblotting**

Cells were lysed in NP-40 buffer post treatment and then subject to western blotting. Briefly, after quantification,

10-50μg of protein was loaded per lane, gels run and transferred to membrane. Non-specific binding was blocked by incubation in 5% non-fat milk in 0.1%TBS-Tween-20, 1h, room temperature. Membranes were incubated with desired primary antibodies, overnight, 4°C. Figure 13 lists antibodies and suppliers. Analysis was performed using ImageJ 1 64 software.

**Human tumor xenograft models**

All animal experiments were approved by and followed Home Office Guidelines. For all animal studies, 264RAD and trastuzumab were dissolved in 1xPBS, at a final concentration of 10mg/kg. Estrogen pellets (0.25mg 60-day release, Innovative Research of America) were implanted subcutaneously into mice 24h prior to tumour cell injection. SCID-mice (a generous gift from Oncology iMED, AstraZeneca, Maccelsfield, U.K) or CD1 nu/nu mice (Charles River Laboratories) were inoculated subcutaneously with either $1x10^6$ MCF-7/HER2-18 cells in 200μl of PBS or $1x10^7$ BT-474 cells in 1:1 PBS/Matrigel. When tumours were palpable ($3-4mm^3$) or reached 100 or $200mm^3$, mice were randomized into treatment groups. Mice received bi-weekly intraperitoneal injections (10mg/kg in 200 μl of PBS) of human IgG, 264RAD, trastuzumab or both 264RAD and trastuzumab. Tumors were measured with calipers bi-weekly in two directions and tumor volume calculated using the formula ($width^2$ x length)/2.

**Statistical analysis**

Statistical significance in drug-treated versus control *in vitro* cultures was determined using the Student's t-test for 2 variables. For 3 or more variables data were analysed using one-way ANOVA with Bonferroni's Multiple Comparison Test using Prism GraphPad software (Systat Software, San Jose, CA, USA). For tumor xenograft models, individual growth curves were plotted and then a linear mixed model (27) was used to test for differences between the treatments. It was fitted by maximum likelihood using the nlme package in the statistical software R (R Development Core Team, 2010) 2.11.1. P values are from Wald tests. Survival of mice was measured using the Log-Rank test in Prism GraphPad. All statistical tests were two-sided.

**Example 2. High co-expression of integrin αVβ6 and HER2 predict poor survival from breast cancer.**

We stained for αVβ6 expression (example staining Figure 1A) on tissue microarrays (TMAs) from two separate cohorts (London and Nottingham) totaling over 2000 women with breast cancer. The clinicopathological parameters and the correlation of αVβ6 expression with these parameters for these two cohorts are in shown in Figure 10 and 11, respectively. Normal breast tissue (n>15) lacked αVβ6 expression whereas high expression of αVβ6 was observed on 15%-16% of invasive ductal carcinoma (Figures 1A, 1B, and 11). There was a significant correlation between high expression of αVβ6 and poor survival (Figure 1C and 1D). Thus, 5-year survival dropped from 71.3% to 57% in the London cohort (Figure 1C; $P=2.9x10^{-6}$) and from 73.5% to 53.2% in the Nottingham cohort (Figure 1 D; $P=4.73x10^{-5}$) and this significant association between poor survival and high expression of αVβ6 extended for at least 15 years (Figure 6). Even after adjusting for tumor stage, size and grade αVβ6 remained an independent predictor of survival (P=0.03; combined cohort data). Data regarding tumor dissemination were available only for the Nottingham series where αVβ6 expression associated significantly with distant spread (P=0.02). Of 1026 αVβ6-negative cases, 317 (31%) had distant metastases, whereas of the corresponding 205 αVβ6-positive cases 81 (40%) had distant metastases. Furthermore, αVβ6-positive cancers were significantly more likely to have spread to bone (P=0.04).

We also noted for both cohorts that there was a strong correlation between HER2 and high αVβ6 expression (P=0.001; Figure 11). Co-expression of high αVβ6 and HER2 proteins significantly reduced survival in the combined London and Nottingham cohorts (Figure 1 E; Hazard Ratio (HR) 3.43; $P=3.98x10^{-12}$). The increased risk appeared to be controlled at the transcriptional level since analysis of the METABRIC Breast cancer expression database (>2000 cases (26)) confirmed that patients who had high ERBB2 (HER2) and ITGB6 (integrin β6 subunit) gene expression had significantly reduced survival (Figure IF; HR=1.94, P=0.003). Thus, as there appeared to be correlations between HER2 and αVβ6 at both protein and mRNA levels predicting poor survival from breast cancer, we investigated whether these two receptors co-operated to promote invasion and cancer.

**Example 3. Integrin αYβ6 and HER2 both promote breast carcinoma invasion.**

Using flow cytometry we screened 20 breast cancer cell lines for expression of αVβ6 and HER2 and their ability to invade through Matrigel (Figures 2A, 2B, and 12). We discovered 80% of cell lines expressed αVβ6 and of these we examined more closely the αVβ6/HER2 double-positive cell lines BT-474, MCF10A.CA1a (CA1a) and the trastuzumab-resistant MCF-7/HER2-18 (HER2-18). Antibody blockade of αVβ6 (264RAD) or HER2 (trastuzumab, TRA) blocked invasion significantly (Figure 2C and 2D). Similarly, siRNA to ITGB6 (Figure 2E) or ERBB2 (Figure 2F) also blocked invasion significantly. Since 264RAD also has some activity against αVβ8 we repeated these experiments with the αVβ6-specific antibody, 10D5, with similar results (Figure 7A). Combined antibody blockade of αVβ6 and HER2 did not decrease invasion beyond that achieved by single antibody blockade (Figure 2G), possibly suggesting that these receptors functioned through the same pathway. Proliferation of HER2-18 or CA1a cells was not significantly changed by treatment with 264RAD, trastuzumab or a combination of both antibodies over the course of the Matrigel assays or after 7 days of treatment (Figure 7B). Proliferation was not significantly reduced in trastuzumab-sensitive BT-474 cells with any treatment over 3 days, although trastuzumab did reduce proliferation by ~30% over 7 days; 264RAD did not significantly affect BT -474 proliferation over 3 or 7 days (data not shown).

Confocal microscopy revealed αVβ6 and HER2 co-localized in breast cancer cells (Figure 7C). However, the two proteins did not co-immunoprecitate, with or without Heregulin β1 (HRGβ) stimulation, even when protein-protein cross-linking agents were added to strengthen any weak associations (data not shown).

**Example 4. Integrin αYβ6 Mediates HER2·Driven Invasion.**

To establish the relationship between αVβ6 and HER2 function we stimulated HER2 invasion by addition of HRGβ to induce HER2/3 heterodimerization and downstream signaling activation. HER3 is the preferred dimerization partner of HER2 in breast cancer (28) and confers poor survival. HRGβ is also a ligand for HER4, however the vast majority of signaling occurred via HER2/3 (data not shown). This was confirmed in tumor xenografts, where P-HER4 expression was negligible with or without HRGβ (data not shown).

Figures 3A and 3B show that HRGβ significantly increased the invasive propensity of both HER2-18 and CA1a cells and that this increased invasion could be inhibited by antibody blockade of HER2 (trastuzumab) or αVβ6 (264RAD). These data suggest that HER2-promoted invasion is mediated by αVβ6. In contrast, addition of HRGβ to BT -474 cells did not enhance invasive ability, suggesting that their HER2-promoted invasive propensity was at a maximum. However, blockade of αVβ6 or HER2 again suppressed their endogenous invasive propensity (Figure 3A and B).

To test invasion in a more physiologically relevant assay we tested our cell lines using the organotypic invasion assay, which allows tumor cells to invade into a fibroblast-rich collagen gel. We found that HER2-18 and BT-474 cells could not be adapted to the organotypic system so we tested CA 1 a cells. Figure 3C shows that both antibody blockade and siRNA knockdown of β6 or HER2 suppresses invasion significantly. Invasion was reduced by 67.45±12.53% with αVβ6 blockade and by 69.81±9.85% with HER2 blockade (invasion quantified as 'Invasion Index' shown in histograms). These data support the Matrigel invasion data (Figure 2). Together, these in vitro data suggest that in breast cancer, αVβ6 co-operates with HER2 to generate intracellular signals required for invasion and further suggests that blockade of αVβ6 function could improve HER2-targeted antibody therapy. Note, 264RAD also has some activity against αVβ8 (29), however MCF-/HER2-18, MCF10A. CA1a and BT -474 do not express this integrin hence the actions of the antibody are specifically against αVβ6 in these cells.

**Example 5. Antibody Blockade of αVβ6 Improves Trastuzumab Efficacy In Vivo**

To test whether αVβ6-blockade could improve trastuzumab antibody therapy we tested the effect of 264RAD on the growth of the trastuzumab sensitive BT -474 cell line in vivo. Figure 4A shows 2-week treatment of mice with BT -474 tumors of $100mm^3$ with 264RAD stopped tumor growth compared to IgG (P<0·0001), whereas trastuzumab (TRA) significantly reduced the growth of tumors by 77.8% (P<0.0001). However, the combination of 264RAD and trastuzumab was more effective than trastuzumab alone, with a reduction in volume of 94.8% compared to IgG after 14 days treatment (P<0.0001).

To assess whether αVβ6-blockade could improve the efficacy of trastuzumab in a trastuzumab-resistant tumor we repeated antibody therapy with the trastuzumab-resistant MCF-7/HER2-18 (HER2-18) cell line in vivo. Tumors were allowed to reach $100mm^3$ before therapy was commenced. Figure 4B shows that in comparison with IgG controls that progressed rapidly, monotherapy with either 264RAD or trastuzumab slowed growth by a similar degree (53.9% (P=0.0006) and 52.1% (P=0.0004) reductions in final volume compared with IgG respectively). Combination therapy reduced tumor volume to a significantly greater extent than either antibody alone with a further 24.14% reduction in tumor volume compared with trastuzumab alone (P<0.0001) and an overall reduction in tumor volume of 76.2% compared with IgG (P<0.0001). Representative images of BT - 474 and HER2-18 xenografts post-treatment with antibodies are shown in Figure 4C.

Next we investigated the molecular mechanisms behind this antitumorigenic effect by analyzing protein expression in post-treatment xenografts.

**Example 6. Molecular response of breast tumors to 264RAD and trastuzumab therapy.**

Residual tumor tissues from BT-474 and HER2-18 xenografts post 2 week treatment were lysed and analyzed for a variety of signaling molecules from the 2-week treatment regime. Protein expression of the direct targets of each antibody, αVβ6, HER2 and HER3, were assessed, as well as downstream targets of these pathways (Total (T)-Akt2) and the αVβ6-associated TGFβ signaling pathway (Total (T) and phospho (P)-Smad2). Immunoblots in Figure 4D (quantified in Figure 4E) show treatment of 3 representative BT -474 xenografts with 264RAD or trastuzumab (TRA) significantly reduced expression of β6; combination therapy almost abolished β6 expression. Combination therapy also enhanced the reduction of expression observed with trastuzumab alone of HER2, HER3, T-Smad2, PSmad2 and T-Akt2, consistent with the enhanced anti-tumorigenicity observed with the combination treatment.

HER2-18 xenografts were subject to the same analysis (Figure 4F and G). Again, β6 levels were significantly reduced with the αVβ6-blocking antibody 264RAD and with the combination treatment. Statistically significant reductions in P-HER2, T-HER3, P-HER3, T-Smad2 and T-Akt2 were only observed after combination therapy. T -HER2 levels were increased in HER2-18 tumors treated with trastuzumab, as has been observed previously. We observed that blockade of αVβ6 with 264RAD also increased HER2 expression. However, combined antibody therapy significantly inhibited signaling via HER2 as seen by reduced P-HER2 levels.

**Example 7. Antibody Blockade of αYβ6 Improves Trastuzumab Efficacy and Extends Survival in a Trastuzumab-resistant Model.**

Trastuzumab-resistance poses a significant clinical problem hence we investigated the enhanced anti-tumorigenicity of the combination therapy further in the HER2-18 trastuzumab-resistant model. In initial experiments the effect of the regime on small tumors was assessed. Subcutaneous xenografts were allowed to reach a palpable size (10-20 $mm^3$) before commencing antibody therapy for 6 weeks. 264RAD reduced growth by over 70% compared with IgG, equivalent to the reduction seen with trastuzumab (both P<0.001) (Figure 5A). More impressively, the combined blockade of αVβ6 and HER2 eradicated HER2-18 tumors in all treated mice.

We next determined whether combination therapy would be as effective on larger xenografts. Tumors were allowed to reach 200mm3 before therapy was commenced. Figure 5B shows that in comparison with IgG controls that progressed rapidly, monotherapy with either 264RAD or trastuzumab again slowed growth by a similar degree (P=0.0019 and P=0.0022 respectively), which was again significantly reduced with combination therapy (P=0.0135 and P=0.0223 respectively). Combination therapy completely suppressed growth of tumors (P<0.0001 compared to IgG), whose size remained static for 50 days. These mice were allowed to progress until their tumors reached the maximum size permissible (according to Home Office regulations) at which point they were killed. Figure 5C shows that compared with IgG, monotherapy with 264RAD or trastuzumab significantly increased survival (P=0.0007 and P=0.018, respectively), combination therapy was even more effective (P<0.0001). In fact, combination therapy was significantly better than monotherapy (P=0.0039 and P=0.0393 compared with 264RAD and trastuzumab respectively). Thus, 264RAD-blockade of αVβ6 suppressed breast cancer growth and enhanced the therapeutic abilities of trastuzumab in both trastuzumab-sensitive and -resistant breast cancer xenografts.

**Example 8. Molecular response of breast tumors to long-term 264RAD and trastuzumab therapy.**

In order to confirm whether monotherapy was operating via similar molecular mechanisms to the combination therapy, we harvested and lysed tumor tissues after 6 weeks treatment (from Figure 5A) and immunoblotted for the same panel of proteins. As the combination treated xenografts were eradicated early on in this study no analysis of the combination therapy could be performed. Figures 5D and E show 264RAD and trastuzumab monotherapy over 6 weeks significantly reduces expression of β6 protein, T-HER2, P-HER2, T-HER3, P-HER3, and T-Akt2, similar to the response of combination therapy for 2 weeks (Figures 4D-G).

Suppression of TGFβ signaling, as measured by reduction in T-Smad2 and P-Smad2, occurred in the (trastuzumab-sensitive) BT -474 cells after 2 weeks monotherapy with either 264RAD or trastuzumab, and this reduction was further reduced by combination therapy (Figure 4D). In contrast, there was limited or no change in T-Smad2 or P-Smad2 after 2-week antibody therapy of HER2-18. However, after 6 weeks monotherapy T-Smad2 and PSmad2 levels were significantly reduced in HER2-18 tumors (Figure 5D and E).

Supporting these data, immunohistochemical analysis of β6 expression in HER2-18 xenografts (Figure 5F) also showed a reduction in β6 expression with monotherapy after 6 weeks, compared to 2 weeks combination therapy where β6 expression was almost eradicated.

Furthermore, this was supported by Matrigel invasion assays in HER2-18, CA1a and BT-474 cells, where cells treated with siRNA to TGF(3RII or antibodies to TGFβ (antibody data not shown as results similar) failed to reduce invasion and 264RAD was able to inhibit invasion, in the presence and absence of TGFβ, to a similar degree (Figure 8).

**Example 9. Discussion**

This study shows conclusively that 1) up regulation of integrin αvβ6 in breast cancer is a prognostic factor predicting a poor prognosis for the patient that is linked with development of distant metastases (P=0.03), 2) co-up regulation of αvβ6 and HER2 identifies one of the worse prognostic sub-groups of breast cancer identified to date and 3) the biological explanation for these clinical observations is that αvβ6 and HER2 co-operate, the integrin αvβ6 mediating the invasive behavior of HER2-promoted cancer. Thus, our data support the proposal that testing of biopsies for αvβ6 expression should become a routine immunopathological procedure to stratify women with breast cancer into this new 'very high' risk αVβ6-positive/HER2+ subgroup. The value of this stratification is that our study also suggests a promising therapeutic strategy for this very high-risk subgroup.

Since its introduction in 1988 the anti-HER2 antibody trastuzumab has been the first line of therapy for women with HER2+ breast cancer, either as an adjuvant therapy for early stage breast cancer or in combination with chemotherapy for metastatic breast cancer (5,30). Thus, in 2012, when over 225,000 women developed breast cancer in the USA, 20-25% would have had HER2 overexpression (NIH statistics) and likely to have received trastuzumab therapy. However, 70% of these women will develop, or have a pre-existing resistance, to trastuzumab (7), which means up to 39,375 American women will develop HER2+ breast cancers for which no specific therapies exist. Our data shows that over 40% of these women with trastuzumab-resistant disease are also likely to express high levels of αvβ6 We suggest that antibody targeting of αvβ6 in these women may offer a therapeutic option and our pre-clinical studies support this proposal. Our data show that in both trastuzumab-sensitive and trastuzumab-resistant HER2-overexpressing human breast cancer xenografts, simultaneous antibody targeting of αvβ6 (with 264RAD) and HER2 (with trastuzumab) significantly improves the therapeutic effect of trastuzumab alone and significantly increases survival time. There is a pressing need to achieve such responses clinically.

The molecular mechanisms of how the antibody-blockade can suppress, or even reduce, breast cancer growth involves, in part, the changing of the tumor phenotype to a lower risk sub-type. Thus, in antibody treated tumors there is consistent down-regulation of expression of αvβ6, HER2 and HER3, three receptors whose up regulation promote breast cancer, reduce survival and therefore drive metastasis. Even monotherapy targeting either αvβ6 or HER2 was able to suppress αvβ6 expression, further showing that these two molecules are co-regulated in breast cancer. Down regulation of HER2 was achieved by two weeks of single antibody therapy in the trastuzumab-sensitive line BT -474, but not the trastuzumab-resistant line HER2-18. However, 6 weeks monotherapy eliminated expression of αvβ6 HER2 and HER3 in HER2-18 trastuzumab-resistant tumors.

The loss of αvβ6 and/or HER2, after antibody-targeting of αvβ6 or HER2, in either BT-474 or HER2-18 tumor models, significantly slowed tumor growth, but did not stop or reduce tumor growth in the same way that combined αvβ6/HER2 targeting did. Thus we looked at signaling pathways implicated in αvβ6 and HER2 behaviour.

Studies have shown that trastuzumab mediates anti-proliferative effects in HER2+ cells by facilitating HER2 degradation (31) and downregulation of PI3-K/Akt signaling (32), data consistent with those observed here in vivo, not only with trastuzumab blockade of HER2, but also with αvβ6-blockade using 264RAD. We determined that our cell lines expressed Akt1 and Akt2 but not Akt3 (data not shown). Moreover, in vitro, siRNA down regulation of Akt2, but not Akt1 suppressed invasion of BT-474, HER2-18 and CA1a cell lines (Figure 9). Thus we analyzed our antibody treated tumors for Akt2 protein and showed that combination therapy for 2 weeks significantly reduced Akt2 expression, whereas monotherapy had little effect. Thus, loss of Akt2, the Akt isoform essential for invasion in 3/3 breast carcinoma cell lines, correlates with the improved in vivo efficacy of combined αvβ6 and HER2 targeting, compared with monotherapy.

We also examined TGFβ signaling since αvβ6 can activate latent TGFβ (16). Moreover, activated TGFβ promotes HER2 tumorigenicity by increasing migration, invasion and metastasis (1 0,11, 12,33). Again, only combination therapy significantly reduced total (T) and activated (P)-Smad2 in BT -474 tumors, whereas monotherapy was not significantly effective. In contrast, in the trastuzumab-resistant tumors, the reduction in TGFβ signaling was moderate, or only a marginal significant reduction in T-Smad2 was observed after combination therapy. Thus, after 2 weeks antibody therapy, down regulation of Akt2, rather than down-regulation of TGFβ signaling, correlates more strongly with the enhanced tumor suppression seen with combination therapy. However this does not negate the likelihood that loss of TGFβ signaling, due to antibody-blockade of αvβ6 contributes to tumor therapy and overall survival seen after 6 weeks therapy.

In summary, we suggest that examining breast cancers for αvβ6 expression should become standard practice as high expression of αvβ6 identifies women with significantly more hazardous types of disease. This is especially true for the 40% of women with HER2/ αvβ6 double-positive cancers who represent one of the worse prognostic breast cancer groups identified to date. Routine determination of αvβ6 expression on breast cancers would stratify women into higher-risk categories requiring therapeutic intervention. In addition, our data also show that antibody blockade of αvβ6 could offer an effective additional therapy for such women, possibly even those with trastuzumab-resistant disease. The fact that human (264RAD (29)) and humanized (STX-100 (36)) αvβ6-blocking antibodies are being developed for human use, shows αvβ6 targeted therapy of breast cancer is feasible and should be a major consideration for the near future.

**Example 10. αVβ6 Binding Agents: Immunization and Titering**

**Immunization**

Immunizations were conducted using soluble αVβ6 and cell-bound αVβ6 (CHO transfectants expressing human αVβ6 at the cell surface), respectively. For the generation of CHO transfectants, human full length αVβ6 cDNA was inserted into the pcDNA 3 expression vector. CHO cells were transiently transfected via electroporation. Expression of human αVβ6 on the cell surface at the level suitable for immunogen purpose was confirmed by Fluorescene-Activated Cell Sorter (FACS) analysis. Ten μg/mouse of soluble protein for Campaign 1, and 3 x $10^6$ cells/mouse of transfected CHO cells for Campaign 2, were used for initial immunization in XenoMouse™ according to the methods disclosed in U.S. Patent Application Serial No. 08/759,620, filed December 3, 1996 and International Patent Application Nos. WO 98/24893, published June 11, 1998 and WO 00/76310, published December 21, 2000, the disclosures of which are hereby incorporated by reference. Following the initial immunization, thirteen subsequent boost immunizations of five μg/mouse were administered for groups one and two (soluble antigen), and nine subsequent boost immunizations of 1.5 x $10^6$ cells/mouse were administered for groups three and four (cell-bound antigen). The immunization programs are summarized in Table 2.

Table 2: Summary of Immunization Programs

| Campaign | Group | Immunogen | Strain | No of mice | Immunization routes |
|---|---|---|---|---|---|
| 1 | 1 | Soluble αVβ6 | XMG2/k | 10 | IP, Tail, BIP, twice/wk, x 6wks |
| 1 | 2 | Soluble αVβ6 | XMG1/kl | 10 | IP, Tail, BIP, twice/wk, x 6wks |
| 2 | 3 | Cell-bound αVβ6 (CHO transfectants) | XMG2/k | 10 | IP, Tail, BIP, twice/wk, x 6wks |
| 2 | 4 | Cell-bound αVβ6 (CHO transfectants) | XMG1/kl | 10 | IP, Tail, BIP, twice/wk, x 6wks |

**Selection of Animals for Harvest by Titer**

Titers of the antibody against human αVβ6 were tested by ELISA assay for mice immunized with soluble antigen. Titers of the antibody for mice immunized with native (cell-bound) antigen were tested by FACS. The ELISA and FACS analyses showed that there were some mice which appeared to be specific for αVβ6. Therefore, at the end of the immunization program, twenty mice were selected for harvest, and lymphocytes were isolated from the spleens and lymph nodes of the immunized mice, as described in the next example.

**Example 11. Recovery of Lymphocytes and B-cell Isolations**

Immunized mice were sacrificed by cervical dislocation, and the draining lymph nodes harvested and pooled from each cohort. The lymphoid cells were dissociated by grinding in DMEM to release the cells from the tissues and the cells were suspended in DMEM. B cells were enriched by negative selection in IgM and positive selection on IgG. The cells were cultured to allow B cell expansion and differentiation into antibody-secreting plasma cells.

Antibody-secreting plasma cells were grown as routine in the selective medium. Exhaustive supernatants collected from the cells that potentially produce anti-human αVβ6 antibodies were subjected to subsequent screening assays as detailed in the examples below.

**Example 12. Binding to Cell-bound αVβ6**

The binding of secreted antibodies to αVβ6 was assessed. Binding to cell-bound αVβ6 was assessed using an FMAT macroconfocal scanner, and binding to soluble αVβ6 was analyzed by ELISA, as described below.

Supernatants collected from harvested cells were tested to assess the binding of secreted antibodies to HEK 293 cells stably overexpressing αVβ6. A parental 293F cell line was used as a negative control. Cells in Freestyle media

(Invitrogen) were seeded into 384-well FMAT plates in a volume of 50 μL/well at a density of 2500 cells/well for the stable transfectants, and at a density of 22,500 cells/well for the parental cells, and cells were incubated overnight at 37°C. Then, 10 μL/well of supernatant was added, and plates were incubated for approximately one hour at 4°C, after which 10 μL/well of anti-human IgG-Cy5 secondary antibody was added at a concentration of 2.8 μg/ml (400ng/ml final concentration). Plates were then incubated for one hour at 4°C, and fluorescence was read using an FMAT macroconfocal scanner (Applied Biosystems). FMAT results for 11 antibodies are summarized in Table 3.

Additionally, antibody binding to soluble αVβ6 was analyzed by ELISA. Costar medium binding 96-well plates (Costar catalog #3368) were coated by incubating overnight at 4°C with αVβ6 at a concentration of 5 μg/ml in TBS/1mM $MgCl_2$ buffer for a total volume of 50 μ/well. Plates were then washed with TBS/1mM $MgCl_2$ buffer, and blocked with 250 μL of IX PBS/1% milk for thirty minutes at room temperature. Ten μL of supernatant was then added to 40 μL TBS/1mM $MgCl_2$/1% milk and incubated for one hour at room temperature. Plates were washed and then incubated with goat-anti-human IgG Fc-peroxidase at 0.400ng/ml in TBS/1mM $MgCl_2$/1% milk, and incubated for one hour at room temperature. Plates were washed and then developed with 1-Step TMB substrate. The ELISA results for one of the antibodies are shown in Table 3.

Table 3: Binding of Supernatants to Cell-Bound and Soluble αVβ6

| mAb | FMAT Data | | | ELISA data |
|---|---|---|---|---|
| | Count | FL1 | FL1XCount | OD |
| sc 049 | 185 | 4377.73 | 809880 | ND |
| sc 058 | ND | ND | ND | 1.79 |
| sc 188 | 127 | 628.04 | 79761 | ND |
| sc 097 | 98 | 1237.18 | 121243 | ND |
| sc 277 | 28 | 382.31 | 10704 | ND |
| sc 133 | 82 | 709.82 | 58205 | ND |
| sc 161 | 23 | 725.21 | 16679 | ND |
| sc 254 | 174 | 9179.65 | 1597259 | ND |
| sc 264 | 63 | 734.29 | 46260 | ND |
| sc 298 | 102 | 2137.94 | 218069 | ND |
| sc 374 | 174 | 4549.65 | 791639 | ND |
| sc 320 | 141 | 3014.63 | 425062 | ND |
| Negative Control (Blank): | 0 | 0 | 0 | 0.21 |
| Positive Control (2077z - 1 ug/mL): | 67 | 659.49 | 44185 | 6.00 |

**Example 13. Inhibition of Cell Adhesion**

In order to determine the relative potency of the different antibody-containing supernatants, the antibodies were assessed for their ability to inhibit TGFβLAP-mediated adhesion of αVβ6-positive HT29 cells. Plates were coated overnight with 10ug/ml TGFβLAP, and pre-blocked with 3% BSA/PBS for 1 hour prior to the assay. Cells were then pelleted and washed twice in HBBS, after which the cells were then resuspended in HBSS at appropriate concentrations. The cells were incubated in the presence of appropriate antibodies at 4°C for 30 minutes in a V-bottom plate. The antigen coating solution was removed and the plates were blocked with 100μL of 3% BSA for one hour at room temperature. Plates were washed twice with PBS or HBSS, and the cell-antibody mixtures were transferred to the coated plate and the plate was incubated at 37°C for 30 minutes. The cells on the coated plates were then washed four times in warm HBSS, and the cells were thereafter frozen at -80°C for one hour. The cells were allowed to thaw at room temperature for one hour, and then 100μL of CyQuant dye/lysis buffer (Molecular Probes) was added to each well according to the manufacturer's instructions. Fluorescence was read at an excitation wavelength of 485 nm and an emission wavelength of 530 nm. The results for twelve antibodies are summarized in Table 4. Those antibodies shown ranged in potency from 62% inhibition to over 100% inhibition, relative to coated and uncoated control wells on the plate which were used to represent the maximum and minimum adhesion values that could be obtained in the assay.

Table 4: Adhesion Assay

| Antibody ID | Assay 1% Inhibition | Assay 2 % Inhibition | Average % Inhibition |
|---|---|---|---|
| sc 049 | 80% | 98% | 89% |
| sc 058 | 77% | 46% | 62% |

(continued)

| Antibody ID | Assay 1% Inhibition | Assay 2 % Inhibition | Average % Inhibition |
|---|---|---|---|
| sc 097 | 96% | 106% | 101% |
| sc 133 | 99% | 106% | 103% |
| sc 161 | 98% | 106% | 102% |
| sc 188 | 99% | 103% | 101% |
| sc 254 | 98% | 106% | 102% |
| sc 264 | 98% | 100% | 99% |
| sc 277 | 98% | 101% | 100% |
| sc 298 | 98% | 102% | 100% |
| sc 320 | 97% | 97% | 97% |
| sc 374 | 118% | 89% | 104% |

**Example 14. Cross-reactivity to Macaque αYβ6 and Human αV**

Cross-reactivity of the antibody-containing supernatants to macaque αVβ6 was tested on the supernatants using FACS analysis on HEK-293 cells transiently transfected with cynomolgus αV and cynomolgus β6.

Cross-reactivity to human αV was also tested. For this assay, cross-reactivity was tested on the supernatants using FACS analysis on parental A375M cells, which express αVβ3 and αVβ5, but not αVβ6. This screen was designed to show that the antibodies were specifically recognizing either the β6 chain or the β6 chain in combination with αV. The human αV assay was run at the same time as the macaque αVβ6 cross-reactivity screen.

The assays were performed as follows. A375M cells that were approximately 75% confluent were labeled with CFSE intracellular dye by dissociating and then pelleting cells (equivalent to 250,000 to 300,000 cells per well) in a falcon tube, then resuspending in 0.125μM CFSE in FACS buffer to a final volume of 100μL for every 250,000 cells, and then by incubating at 37°C for five minutes. The cells were then pelleted, the supernatant discarded, and resuspended in FACS buffer and incubated for 30 minutes at 37°C. Cells were then washed twice with FACS buffer and resuspended in a final volume of 100μL FACS buffer per well.

HEK-293 cells were transiently transfected with cynomolgus αV and cynomolgus β6. After 48 hours, the cells were collected and resuspended in FACS buffer to reach a final concentration of approximately 50,000 cells in 100μL.

Approximately 100,000 cells total, comprising a 50:50 mix of CFSE-labeled A375M cells and transfected 293 cells, were used in each reaction as follows. 100μL of CFSE-labeled A375M cells and 100μL of 293 cells were dispensed into a V-bottom plate. The cells in the plate were pelleted at 1500 rpm for 3 minutes, and then resuspended in 100 μL FACS buffer. The pelleting step was repeated, and the FACS buffer supernatant was removed. The harvested antibody-containing supernatants, or control primary antibodies were added in a volume of 50 μL and the cells were resuspended. Primary antibody controls were murine αVβ6 (Cat#MAB2077z, Chemicon) and an anti-aV recombinant. The plate was incubated on ice for 45 minutes, after which 100 μL FACS buffer was added to dilute the primary antibody. The cells were then pelleted by centrifuging at 1500 rpm for 3 minutes, and the pellet was resuspended in 100 μL FACS buffer. The pelleting step was repeated, and the FACS buffer supernatant was removed. Cells were then resuspended in the appropriate secondary antibody (5 μg/ml) with 7AAD dye (10 μg/ml), and stained on ice for 7 minutes. Then 150 μL of FACS buffer was added and the cells were pelleted at 1500 rpm for 3 minutes, after which the cells were washed in 100 μL FACS buffer, pelleted, and then resuspended in 250 μL buffer and added to FACS tubes. Samples were analyzed on a high throughput FACS machine and analyzed using Cell Quest Pro software.

The results for twelve antibodies are summarized in Table 5, and demonstrate that the antibodies shown were able to specifically bind to macaque αVβ6 but were not able to non-specifically bind human αV on the parental A375M cells.

Table 5. Cross-Reactivity to Macaque αVβ6 and Human αV

| Antibody ID | Mac AVB6 % Cells Shifted | Mac AVB6 GeoMean | A375M % Cells Shifted | A375M GeoMean |
|---|---|---|---|---|
| sc 049 | 23% | 30.19 | 20% | 1.74 |
| sc 058 | 25% | 22.77 | 18% | 1.78 |

(continued)

| Antibody ID | Mac AVB6 % Cells Shifted | Mac AVB6 GeoMean | A375M % Cells Shifted | A375M GeoMean |
|---|---|---|---|---|
| sc 097 | 35% | 37.04 | 24% | 1.84 |
| sc 133 | 32% | 35.22 | 24% | 1.79 |
| sc 161 | 14% | 32.98 | 11% | 16.68 |
| sc 188 | 18% | 23.9 | 13% | 1.65 |
| sc 254 | 59% | 78.49 | 55% | 2.31 |
| sc 264 | 55% | 66.38 | 46% | 2.35 |
| sc 277 | 35% | 33.35 | 23% | 1.86 |
| sc 298 | 53% | 63.08 | 45% | 2.14 |
| sc 320 | 19% | 33.45 | 15% | 23.18 |
| sc 374 | 51% | 61.79 | 39% | 2.14 |
| Human IgG Isotype Control | 1% (day 1)<br>0% (day 2) | 9.54 (day 1)<br>7.39 (day 2) | 5% (day 1)<br>1% (day 2) | 1.66 (day 1)<br>7.23 (day 2) |
| Mouse IgG2 with Murine Secondary Antibody | 1% (day 1)<br>0% (day 2) | 8.85 (day 1)<br>11.21 (day 2) | 4% (day 1)<br>3% (day 2) | 1.67 (day 1)<br>11.16 (day 2) |
| Positive Control 2077z (1ug/ml) | 42% (day 1)<br>11% (day 2) | 55.52 (day 1)<br>28.11 (day 2) | 30% (day 1)<br>5% (day 2) | 2.03 (day 1)<br>15.36 (day 2) |

**Example 15. αVβ6-specific Hemolytic Plaque Assay**

Antibody-secreting plasma cells were selected from each harvest for the production of recombinant antibodies. Here, a fluorescent plaque assay was used to identify single plasma cells expressing antibodies against αVβ6. Then, the single cells were subjected to reverse transcription and polymerase chain reaction to rescue and amplify the variable heavy and variable light chains that encoded the initial antibody specificity, as described in the next example. The preparation of a number of specialized reagents and materials needed to conduct the αVβ6-specific hemolytic plaque assay are described below.

*Biotinylation of Sheep red blood cells (SRBC).* SRBC were stored in RPMI media as a 25% stock. A 250 μl SRBC packed-cell pellet was obtained by aliquoting 1.0mL of the stock into a 15-mL falcon tube, spinning down the cells and removing the supernatant. The cell pellet was then re-suspended in 4.75mL PBS at pH 8.6 in a 50mL tube. In a separate 50mL tube, 2.5 mg of Sulfo-NHS biotin was added to 45mL of PBS at pH 8.6. Once the biotin had completely dissolved, 5mL of SRBCs was added and the tube was rotated at room temperature for 1 hour. The SRBCs were centrifuged at 3000g for 5 minutes. The supernatant was drawn off and 25mL PBS at pH 7.4 was added as a wash. The wash cycle was repeated 3 times, then 4.75mL immune cell media (RPMI 1640 with 10% FCS) was added to the 250 μl biotinylated-SRBC (B-SRBC) pellet to gently re-suspend the B-SRBC (5% B-SRBC stock). The stock was stored at 4° C until needed.

*Streptavidin (SA) coating of B-SRBC.* One mL of the 5% B-SRBC stock was transferred into to a fresh eppendorf tube. The B-SRBC cells were pelleted with a pulse spin at 8000 rpm (6800 rcf) in a microfuge. The supernatant was then drawn off, the pellet was re-suspended in 1.0mL PBS at pH 7.4, and the centrifugation was repeated. The wash cycle was repeated 2 times, then the B-SRBC pellet was resuspended in 1.0 mL of PBS at pH 7.4 to give a final concentration of 5% (v/v). 10 μl of a 10mg/mL Streptavidin (CalBiochem, San Diego, CA) stock solution was added. The tube was mixed and rotated at RT for 20 minutes. The washing steps were repeated and the SA-SRBC were re-suspended in 1 mL PBS pH 7.4 (5% (v/v)).

*Human αVβ6 coating of SA-SRBC.* Soluble antigen (lacking the transmembrane domain) was used for coating the SRBC. Both chains were used because αVβ6 is only presented on the cell surface as a dimer. The SA-SRBC were coated with the biotinylated-αVβ6 at 50μg/mL, mixed and rotated at room temperature for 20 minutes. The SRBC were washed twice with 1.0 mL of PBS at pH 7.4 as above. The Ag-coated SRBC were re-suspended in RPMI (+10%FCS) to a final concentration of 5% (v/v).

*Determination of the quality of αVβ6-SRBC by immunofluorescence (IF).* 10 μl of 5% SA-SRBC and 10 μl of

5% Ag-coated SRBC were each added to separate fresh 1.5mL eppendorf tube containing 40 μl of PBS. Human anti-αVβ6 antibodies were added to each sample of SRBCs at 50 μg/mL. The tubes were rotated at room temperature for 25 min, and the cells were then washed three times with 100 μl of PBS. The cells were re-suspended in 50 μl of PBS and incubated with 2 μg/mL Gt-anti Human IgG Fc antibody conjugated to the photostable fluorescent dye Alexa488 (Molecular Probes, Eugene, OR). The tubes were rotated at room temperature for 25 min, followed by washing with 100 μl PBS and resuspension in 10 μl PBS. 10 μl of the stained cells were spotted onto a clean glass microscope slide, covered with a glass coverslip, observed under fluorescent light, and scored on an arbitrary scale of 0-4 to assess the quality of the isolated cells.

*Preparation of plasma cells.* The contents of a single B cell culture well previously identified as neutralizing for αVβ6 activity (therefore containing a B cell clone secreting the immunoglobulin of interest), was harvested. The B cell culture present in the well was recovered by addition of RPMI +10% FCS at 37°C. The cells were re-suspended by pipetting and then transferred to a fresh 1.5mL eppendorf tube (final volume approximately 500-700 μl). The cells were centrifuged in a microfuge at 1500 rpm (240 rcf) for 2 minutes at room temperature, then the tube was rotated 180 degrees and centrifuged again for 2 minutes at 1500 rpm. The freeze media was drawn off and the immune cells were resuspended in 100 μl RPMI (10% FCS), then centrifuged. This washing with RPMI (10% FCS) was repeated and the cells re-suspended in 60 μl RPMI (FCS) and stored on ice until ready to use.

*Performance of the Hemolytic Plaque Assay.* To the 60 μl sample of immune cells was added 60 μl each of αVβ6-coated SRBC (5% v/v stock), 4x guinea pig complement (Sigma, Oakville, ON) stock prepared in RPMI (FCS), and 4x enhancing sera stock (1:900 in RPMI (FCS)). The mixture (3-5μl) was spotted onto plastic lids from 100 mm Falcon tissue culture plates and the spots were covered with undiluted paraffin oil. The slides were incubated at 37°C for a minimum of 45 minutes.

*Analysis of Plaque assay results.* The coating of the sheep red blood cells with the catalytic domain of human αVβ6 was successful. These Ag-coated red blood cells were then used to identify antigen-specific plasma cells from the wells shown below in Table 6. These cells were then isolated by micromanipulation. After micromanipulation to rescue the antigen-specific plasma cells, the genes encoding the variable region genes were rescued by RT-PCR on a single plasma cell, as described further in the next example.

Table 6. Plaque Assay Results

| **Parent Plate ID** | | | **Plaque Assay** | |
|---|---|---|---|---|
| Plate | Row | Column | Assay | Single Cells |
| 68 | B | 10 | Fluorescent | 45-57 |
| 296 | D | 10 | Fluorescent | 58-59 |
| 318 | F | 1 | Hemolytic | 60-62 |
| 612 | G | 1 | Fluorescent | 187-189 |
| 752 | D | 12 | Fluorescent | 95-100 |
| 762 | D | 8 | Fluorescent | 277-286 |
| 766 | B | 5 | Fluorescent | 132-143, 147-150 |
| 827 | E | 12 | Fluorescent | 159-170 |
| 659 | F | 11 | Fluorescent | 252-263 |
| 761 | H | 3 | Fluorescent | 264-276 |
| 765 | A | 8 | Fluorescent | 287-298 |
| 652 | D | 2 | Fluorescent | 374-379, 392-397 |
| 806 | A | 6 | Fluorescent | 312-321 |

**Example 16. Recombinant Protein Isolation**

After isolation of the desired single plasma cells, mRNA was extracted and reverse transcriptase PCR was conducted to generate cDNA encoding the variable heavy and light chains of the antibody secreted by each cell. The human variable heavy chain cDNA was digested with restriction enzymes that were added during the PCR and the products of this reaction were cloned into an IgG2 expression vector with compatible overhangs for cloning. This vector was generated by cloning the constant domain of human IgG2 into the multiple cloning site of pcDNA3.1+/Hygro (Invitrogen, Burlington, Ontario, Canada). The human variable light chain cDNA was digested with restriction enzymes that were added during the PCR reaction and the products of this reaction were cloned into an IgKappa or IgLamda expression vector with compatible overhangs for cloning. This vector was generated by cloning the constant domain of human IgK or IgL into the multiple cloning site of pcDNA3.1+/Neo (Invitrogen).

The heavy chain and the light chain expression vectors were then co-transfected using lipofectamine into a 60 mm dish of 70% confluent human embryonal kidney (HEK) 293 cells. The transfected cells secreted a recombinant antibody with the identical specificity as the original plasma cell for 24 to 72 hours. The supernatant (3 mL) was harvested from the HEK 293 cells and the secretion of an intact antibody was demonstrated with a sandwich ELISA to specifically detect human IgG. Specificity was confirmed through binding of the recombinant antibody to αVβ6 using ELISA. The rescued clones secreting antibody that could bind to the target antigen are summarized in Table 7.

Table 7. Secretion and Binding Data for the Recombinant Antibodies

| **Parent Plate ID** | | | |
|---|---|---|---|
| Plate | Row | Column | Antibody ID |
| 68 | B | 10 | 49 |
| 296 | D | 10 | 58 |
| 612 | G | 1 | 188 |
| 752 | D | 12 | 97 |
| 762 | D | 8 | 277 |
| 766 | B | 5 | 133 |
| 827 | E | 12 | 161 |
| 659 | F | 11 | 254 |
| 761 | H | 3 | 264 |
| 765 | A | 8 | 298 |
| 652 | D | 2 | 374 |
| 806 | A | 6 | 320 |

### Example 17. Purification of Recombinant Antibodies

For larger scale production of the anti-αVβ6 antibodies, heavy and light chain expression vectors (2.5 μg of each chain/dish) were lipofected into ten 100 mm dishes that were 70% confluent with HEK 293 cells. The transfected cells were incubated at 37°C for 4 days, the supernatant (6 mL) was harvested and replaced with 6 mL of fresh media. At day 7, the supernatant was removed and pooled with the initial harvest (120 mL total from 10 plates). The antibodies were purified from the supernatant using Protein-A Sepharose (Amersham Biosciences, Piscataway, NJ) affinity chromatography (1 mL). The antibodies were eluted from the Protein-A column with 500 μL of 0.1 M Glycine pH 2.5. The eluate was dialyzed in PBS pH 7.4 and filter sterilized. The antibodies were analyzed by nonreducing SDS-PAGE to assess purity and yield. Protein concentration was determined by determining the optical density at 280 nm.

### Example 18. Structural Analysis of αYβ6 Antibodies

The variable heavy chains and the variable light chains of the antibodies were sequenced to determine their DNA sequences. The complete sequence information for the anti-αVβ6 antibodies is provided in the sequence listing with nucleotide and amino acid sequences for each gamma and kappa/lambda chain combination. The variable heavy sequences were analyzed to determine the VH family, the D-region sequence and the J-region sequence. The sequences were then translated to determine the primary amino acid sequence and compared to the germline VH, D and J-region sequences to assess somatic hypermutations.

Table 8 is a table comparing the antibody heavy chain regions to their cognate germ line heavy chain region. Table 9 is a table comparing the antibody kappa or lambda light chain regions to their cognate germ line light chain region.

The variable (V) regions of immunoglobulin chains are encoded by multiple germ line DNA segments, which are joined into functional variable regions ($V_HDJ_H$ or $V_KJ_K$) during B-cell ontogeny. The molecular and genetic diversity of the antibody response to αVβ6 was studied in detail. These assays revealed several points specific to anti-αVβ6 antibodies.

According the sequencing data, the primary structure of the heavy chains of sc 298 and sc 374 are similar, but not identical. sc 254 is structurally different from the other two. It should also be appreciated that where a particular antibody differs from its respective germline sequence at the amino acid level, the antibody sequence can be mutated back to the germline sequence. Such corrective mutations can occur at one, two, three or more positions, or a combination of any of the mutated positions, using standard molecular biological techniques. By way of non-limiting example, Table 9 shows that the light chain sequence of sc 298 (SEQ ID NO.: 40) differs from the corresponding germline sequence (SEQ ID NO.:68) by a Val to Ala mutation (mutation 1) in the FR1 region, via a Leu to Ala mutation (mutation 2) in the CDR1 region and an Asn to Ser in the FR3 region. Thus, the amino acid or nucleotide sequence encoding the light chain of sc 298 can be modified to change mutation 1 to yield the germline sequence at the site of mutation 1. Further, the amino acid or nucleotide sequence encoding the light chain of mAb sc 298 can be modified to change mutation 2 to yield the germline sequence at the site of mutation 2. Still further, the amino acid or nucleotide sequence encoding the light chain of mAb sc 298 can be modified to change mutation 3 to yield the germline sequence at the site of mutation 3. Still further again, the amino acid or nucleotide sequence encoding the light chain of sc 298 can be modified to change mutation 1, mutation 2 and mutation 3 to yield the germline sequence at the sites of mutations 1, 2 and 3. Still further again, the amino acid or nucleotide sequence encoding the light chain of sc 298 can be modified to change any combination of mutation 1, mutation 2 and mutation 3. In another example, heavy chain of sc 264 (SEQ ID NO: 30) differs from its germline (SEQ ID NO: 55) at position 61. Thus the amino acid or nucleotide sequence encoding the heavy chain of sc 264 can be modified from a N to Y to yield the germline sequence. Tables 10-13 below illustrate the position of such variations from the germline for sc 133, sc 188 and sc 264. Each row represents a unique combination of germline and non-germline residues at the position indicated by bold type. Particular examples of an antibody sequence that can be mutated back to the germline sequence include: sc 133 where the L at amino acid 70 of the heavy chain is mutated back to the germline amino acid of M (referred to herein as sc 133 TMT); sc 133 where the N at amino acid 93 of the light chain is mutated back to the germline amino acid of D (referred to herein as sc 133 WDS); and sc 264 where the A at amino acid 84 of the light chain is mutated back to the germline amino acid of D (referred to herein as sc 264 ADY).

One embodiment includes modifying one or more of the amino acids in the CDR regions, *i.e.,* CDR1, CDR2 and/or CDR3. In one example, the CDR3 of the heavy chain of an antibody described herein is modified. Typically, the amino acid is substituted with an amino acid having a similar side chain (a conservative amino acid substitution) or can be substituted with any appropriate amino acid such as an alanine or a leucine. In one embodiment, the sc 264 CDR3, VATGRGDYHFYAMDV (amino acid residues 100-114 of SEQ ID NO: 30), can be modified at one or more amino acids. Applicants have already demonstrated that the CDR3 region can be modified without adversely affecting activity, *i.e.,* see sc 264 RAD where the second G in the CDR3 region is substituted for an A. Other modifications within the CDR3 region are also envisaged. In another embodiment, the sc 133 CDR3 region, RLDV, can be modified at one or more amino acids including substituting the L for an A and/or the V for an A. Means of substituting amino acids are well known in the art and include site-directed mutagenesis.

Another embodiment includes replacing any structural liabilities in the sequence that might affect the heterogeneity or specificity of binding of the antibodies. In one example, the antibody sc 264 has an RGD sequence in the CDR3 region that might cause cross-reactive binding. Therefore the glycine residue in the RGD can be replaced with an alanine (sc 264 RAD).

Table 8. Heavy chain analysis

| Chain Name | SEQ ID NO: | V | D | J | FR1 | CDR1 | FR2 | CDR2 | FR3 | CDR3 | FR4 |
|---|---|---|---|---|---|---|---|---|---|---|---|
| | 49 | Germline | | | QVQLVQSGAEVKKPGASVKVSCKAS | GYTFTGYYMH | WVRQAPGQGLEWMG | WINPNSGGTNYAQKFQG | RVTMTRDTSISTAYMELSRLRSDDTAVYYCAR | RL-- | WGQGTTVTVSS |
| sc 133 | 14 | VH1-2 | 5-12 | JH6 B | QVQLVQSGAEVKKPGASVKVSCKAS | GYTFTGYYMH | WVRQAPGQGLEWMG | WINPKSGDTNYAQKFQG | RVTLTRDTSTSTAYMELSRLRSDDTAVYYCAR | RLDV | WGQGTTVTVSS |
| | 50 | Germline | | | EVQLVESGGGLVKPGGSLRLSCAAS | GFTFSSYSMN | WVRQAPGKGLEWVS | SISSSSSYIYYADSVKG | RFTISRDNAKNSLYLQMNSLRAEDTAVYYCAR | --VQLERYYYYYGMDV | WGQGTTVTVSS |
| sc 320 | 42 | VH3-21 | D1-1 | JH6 B | EVQLVESGGGLVKPGGSLRLSCAAS | GYTFTNYIMH | WVRQAPGKGLEWVS | SISISSSYIYYADSVKG | RFTISRDNAKNSLYLQMNSLRAEDTAVYYCAR | DPVPLERRDYYYGMDV | WGQGTTVTVSS |
| | 51 | Germline | | | EVQLLESGGGLVQPGGSLRLSCAAS | GFTFSSYAMS | WVRQAPGKGLEWVS | AISGSGGSTYYADSVKG | RFTISRDNSKNTLYLQMNSLRAEDTAVYYCAK | -VDTAMVYYGMDV | WGQGTTVTVSS |
| sc 58 | 6 | VH3-23 | D5-5 | JH6 B | EVQLLESGGGLVQPGGSLRLSCAAS | GFTFSSYVMS | WVRQAPGKGLEWVS | AISGSGGSTYYADSVKG | RFTISRDNSKNTLYLQMNSLRAEDTAVYYCAK | GVDTAMVTYGMDV | WGQGTTVTVSS |
| | 52 | Germline | | | QVQLVESGGGVVQPGRSLRLSCAAS | GFTFSSYGMH | WVRQAPGKGLEWVA | VIWYDGSNKYYADSVKG | RFTISRDNSKNTLYLQMNSLRAEDTAVYYCAR | -IAAR--YYYYYGMDV | WGQGTTVTVSS |

(continued)

| Chain Name | SEQ ID NO: | V | D | J | FR1 | CDR1 | FR2 | CDR2 | FR3 | CDR3 | FR4 |
|---|---|---|---|---|---|---|---|---|---|---|---|
| sc 298 | 38 | VH3-3 3 | D6-6 | JH6 B | QVQLVESGG GVVQPGRSL RLSCAAS | GFTFS SYGM H | WVRQ APGKG LEWV A | VIWYGGS NKYYAD SVKG | RFTISRDNSKNTL YLQMNSLRAED TAVYYCAR | DLAARR GDYYYY GMDV | WGQG TTVT VSS |
| sc 374 | 46 | VH3-3 3 | D6-6 | JH6 B | QVQLVESGG GVVQPGRSL RLSCAAS | GFTFS SYGM H | WVRQ APGKG LEWV A | VIWYDGS NKYYAD SVKG | RFTISRDNSKNTL YLQMNSLRAED TAVYYCAR | TEGIAAR LYYYYG MDV | WGQG TTVT VSS |
| | 53 | Germline | | | QVQLQESGP GLVKPSQTLS LTCTVS | GGSIS SGGY YWS | WIRQH PGKGL EWIG | YIYYSGS TYYNPSL KS | RVTISVDTSKNQ FSLKLSSVTAAD TAVYYCAR | -- GIAAAG-- YYYYYG MDV | WGQG TTVT VSS |
| sc 254 | 26 | VH4-3 1 | D6-1 3 | JH6 B | QVQLQESGP GLVKPSQTLS LTCTVS | GGSIS SGGY YWS | WIRQH PGKGL EWIG | YIYYSGS TYYNPSL KS | RVTISVDTSKNQ FSLKLSSVTAAD TAMYYCAR | YRGPAA GRGDFY YFGMDV | WGQG TTVT VSS |
| | 54 | Germline | | | QVQLQESGP GLVKPSQTLS LTCTVS | GGSIS SGGY YWS | WIRQH PGKGL EWIG | YIYYSGS TYYNPSL KS | RVTISVDTSKNQ FSLKLSSVTAAD TAVYYCAR | --- ITIFGVFD Y | WGQG TLVT VSS |
| sc 49 | 2 | VH4-3 1 | D3-3 | JH4 B | QVQLQESGP GLVKPSQTLS LTCTVA | GGSIR SGDY YWS | WIRQH PGKGL EWIG | NIYYSGS TYYNPSL KS | RITISVATSRNQF SLKLTSVTAADT AVYYCAR | GGAITIFG VFDY | WGQG TLVT VSS |
| | 55 | Germline | | | QVQLQESGP GLVKPSQTLS LTCTVS | GGSIS SGGY YWS | WIRQH PGKGL EWIG | YIYYSGS TYYNPSL KS | RVTISVDTSKNQ FSLKLSSVTAAD TAVYYCAR | VAT--- YYYYYG MDV | WGQG TTVT VSS |

(continued)

| Chain Name | SEQ ID NO: | V | D | J | FR1 | CDR1 | FR2 | CDR2 | FR3 | CDR3 | FR4 |
|---|---|---|---|---|---|---|---|---|---|---|---|
| Sc 264 | 30 | VH4-31 | D4-17 | JH6B | QVQLQESGP<br>GLVKPSQTLS<br>LTCTVS | GGSIS<br>SGGY<br>YWS | WIRQH<br>PGKGL<br>EWIG | YIYYSGR<br>TYNNPSL<br>KS | RVTISVDTSKNQ<br>FSLKLSSVTAAD<br>TAVYYCAR | VATGRG<br>DYHFYA<br>MDV | WGQG<br>TTVT<br>VSS |
| | 56 | Germline | | | QVQLQESGP<br>GLVKPSQTLS<br>LTCTVS | GGSIS<br>SGGY<br>YWS | WIRQH<br>PGKGL<br>EWIG | YIYYSGS<br>TYYNPSL<br>KS | RVTISVDTSKNQ<br>FSLKLSSVTAAD<br>TAVYYCAR | ---<br>LRYYYY<br>YGMDV | WGQG<br>TTVT<br>VSS |
| Sc 188 | 22 | VH4-31 | D4-23 | JH6B | QVQLQESGP<br>GLVKPSQTLS<br>LTCTVS | GGSIS<br>SGVY<br>YWT | WIRQH<br>PGNGL<br>EWIG | YIYYSGS<br>TSYNPSL<br>KS | RVTISVDTSKKQ<br>FSLNLTSVTAAD<br>TAVYYCAR | EGPLRGD<br>YYYGLD<br>V | WGQG<br>TTVT<br>VSS |
| | 57 | Germline | | | EVQLVQSGA<br>EVKKPGESLK<br>ISCKGS | GYSFT<br>SYWI<br>G | WVRQ<br>MPGK<br>GLEW<br>MG | IIYPGDSD<br>TRYSPSF<br>QG | QVTISADKSISTA<br>YLQWSSLKASDT<br>AMYYCAR | ---<br>SSGYYYA<br>FDI | WGQG<br>TMVT<br>VSSA |
| Sc 97 | 10 | VH5-51 | D3-22 | JH3B | EVQLVQSGA<br>EVKKPGESLK<br>ISCKGS | GYSFT<br>SYWI<br>G | WVRQ<br>MPGK<br>GLEW<br>MG | IIYPGDSD<br>TRYSPSF<br>QG | QVILSADKSISTA<br>YLQWSSLKASDT<br>AMYYCAR | HDESSGY<br>YYVFDI | WGQG<br>TMVT<br>VSSA |
| | 58 | Germline | | | EVQLVQSGA<br>EVKKPGESLK<br>ISCKGS | GYSFT<br>SYWI<br>G | WVRQ<br>MPGK<br>GLEW<br>MG | IIYPGDSD<br>TRYSPSF<br>QG | QVTISADKSISTA<br>YLQWSSLKASDT<br>AMYYCAR | -----GMDV | WGQG<br>TTVT<br>VSS |
| Sc 277 | 34 | VH5-51 | D3-10 | JH6B | EVQLVQSGA<br>EVKKPGESLK<br>ISCKGS | GYSFP<br>SYWI<br>G | WVRQ<br>MPGK<br>GLEW<br>MG | IIYPGDSD<br>TRYSPSF<br>QG | QVTISADKSISTA<br>YLQWSSLKASDT<br>AMYYCAR | HPMEDG<br>MDV | WGQG<br>TTVT<br>VSS |

(continued)

| Chain Name | SEQ ID NO: | V | D | J | FR1 | CDR1 | FR2 | CDR2 | FR3 | CDR3 | FR4 |
|---|---|---|---|---|---|---|---|---|---|---|---|
| | 59 | Germline | | | EVQLVQSGAEVKKPGESLKISCKGS | GYSFTSYWIG | WVRQMPGKGLEWMG | IIYPGDSDTRYSPSFQG | QVTISADKSISTAYLQWSSLKASDTAMYYCAR | -GIAAAG-YYYGMDV | WGKGTTVTVSSA |
| Sc 161 | 18 | VH5-51 | D6-13 | JH6C | EVQLVQSGAEVKKPGESLKISCKGS | GYSFTSYWIG | WVRQMPGKGLEWMG | IIYPGDSDTRYSPSFQG | QVTISADKSISTAYLQWSSLKASDTAMYYCAR | HGIAAAGFYYYYMDV | WGQGTTVTVSSA |

Table 9. Light chain analysis

| Chain Name | SE QID NO : | V Kappa | J | FR1 | CDR1 | FR2 | CDR 2 | FR3 | CDR3 | J |
|---|---|---|---|---|---|---|---|---|---|---|
| | 60 | Germline | | DIVMTQTPL<br>SLSVTPGQP<br>ASISC | KSSQSLL<br>HSDGKT<br>YLY | WYLQKP<br>GQPPQL<br>LIY | EVS<br>NRF<br>S | GVPDRFSGSGSG<br>TDFTLKISRVEAE<br>DVGVYYC | MQSIQ<br>LPWT | FGQG<br>TKVEI<br>K |
| Sc 254 | 28 | A2 | JK1 | DIVMTQTPL<br>SLSVTPGQP<br>ASIFC | KSSQSLL<br>NSDGKT<br>YLC | WYLQKP<br>GQPPQL<br>LIY | EVS<br>NRF<br>S | GVPDRFSGSGSG<br>TDFTLKISRVEAE<br>DVGVYYC | MQGI<br>QLPW<br>AF | FGQG<br>TKVEI<br>K |
| | 61 | Germline | | EIVLTQSPGT<br>LSLSPGERAT<br>LSC | RASQSV<br>SSSYLA | WYQQK<br>PGQAPR<br>LLIY | GAS<br>SRA<br>T | GIPDRFSGSGSGT<br>DFTLTISRLEPED<br>FAVYYC | QQYG<br>SSPWT | FGQG<br>TKVEI<br>K |
| sc 188 | 24 | A27 | JK1 | EIVLTQSPGT<br>LSLSPGERAT<br>LSC | RAGQTIS<br>SRYLA | WYQQK<br>PGQAPR<br>PLIY | GAS<br>SRA<br>T | GIPDRFSGSGSGT<br>DFTLTISRLEPED<br>FAVYYC | QQYG<br>SSPRT | FGQG<br>TKVEI<br>K |
| sc 374 | 48 | A27 | JK1 | EIVLTQSPGT<br>LSLSPGERAT<br>LSC | RASQSV<br>SSSYLA | WYQQK<br>PGQAPR<br>LLIY | GAS<br>SRA<br>T | DIPDRFSGSGSGT<br>DFTLTISRLEPED<br>FAVYYC | QQYG<br>SSPWT | FGQG<br>TKVEI<br>K |
| | 62 | Germline | | EIVLTQSPGT<br>LSLSPGERAT<br>LSC | RASQSV<br>SSSYLA | WYQQK<br>PGQAPR<br>LLIY | GAS<br>SRA<br>T | GIPDRFSGSGSGT<br>DFTLTISRLEPED<br>FAVYYC | QQYG<br>SSPYT | FGQG<br>TKLEI<br>K |
| Sc 49 | 4 | A27 | JK2 | EIVLTQSPGT<br>LSLSPGERAT<br>LSC | RASQSV<br>SSSYLA | WYQQK<br>PGQAPR<br>LLIY | GAS<br>SRA<br>T | GIPDRFSGSGSGT<br>DFTLTISRLEPED<br>FAVYYC | QQYG<br>SSPCS | FGQG<br>TKLEI<br>K |
| | 63 | Germline | | EIVLTQSPGT | RASQSV | WYQQK | GAS | GIPDRFSGSGSGT | QQYG | FGPGT |
| **Chain Name** | **SE QID NO** | **V Kappa** | **J** | **FR1** | **CDR1** | **FR2** | **CDR 2** | **FR3** | **CDR3** | **J** |
| | | | | LSLSPGERAT<br>LSC | SSSYLA | PGQAPR<br>LLIY | SRA<br>T | DFTLTISRLEPED<br>FAVYYC | SSPFT | KVDIK<br>R |

(continued)

| Chain Name | SEQ ID NO | V Kappa | J | FR1 | CDR1 | FR2 | CDR 2 | FR3 | CDR3 | J |
|---|---|---|---|---|---|---|---|---|---|---|
| sc 161 | 20 | A27 | JK3 | EIVLTQSPDT LSLSPGERAS LSC | RASQNV NRNYLV | WYQQK PGQAPR LLIY | GTS NRA T | GIPDRFSGSGSGT DFTLTISRLEPED FAVYYC | QQCG SLPFT | FGPGT KVDIK R |
| **Chain Name** | **SEQ ID NO** | **V Lambda** | **J** | **FR1** | **CDR1** | **FR2** | **CDR 2** | **FR3** | **CDR3** | **J** |
| | 64 | Germline | | QSVLTQPPS VSAAPGQKV TISC | SGSSSNI GNNYVS | WYQQLP GTAPKL LIY | DNN KRP S | GIPDRFSGSKSGT SATLGITGLQTG DEADYYC | GTWD SSLSA -YV | FGTGT KVTV |
| sc 133 | 16 | V1-19 | JL1 | QSVLTQPPS VSAAPGQKV TISC | SGSSSNI GNNYVS | WYQQLP GTAPKL LIY | DNN KRP S | GIPDRFSGSKSGT SATLGITGLQTG DEADYYC | GTWN SSLSA GYV | FGTGT KVTV |
| | 65 | Germline | | QSVLTQPPS VSAAPGQKV TISC | SGSSSNI GNNYVS | WYQQLP GTAPKL LIY | DNN KRP S | GIPDRFSGSKSGT SATLGITGLQTG DEADYYC | GTWD SSLSA VV | FGGG TKLT VL |
| sc 320 | 44 | V1-19 | JL2 | QSVLTQPPS MSAAPGQK VTISC | SGSSSNI GNNYVS | WYQQLP GTAPKL LIY | DNN KRP S | GIPDRFSGSKSGT SATLGITGLQTG DEADYYC | GTWD SSLSA GV | FGGG TKLT VL |
| | 66 | Germline | | SYELTQPPSV SVSPGQTARI TC | SGDALP KKYAY | WYQQK SGQAPV LVIY | EDS KRP S | GIPERFSGSSSGT MATLTISGAQVE DEADYYC | YSTDS SGNH VV | FGGG TKLT VL |
| sc 277 | 36 | V2-7 | JL2 | SYELTQPPSV SVSPGQTARI TC | SGDALP KKYAF | WYQQK SGQAPV LVIY | DDN KRP S | GIPERFSGSSSGT MATLTITGAQVE DEADYYC | YSTDS SGHH V | FGGG TKLT VL |
| sc 97 | 12 | V2-7 | JL2 | SYELTQPPSV SVSPGQTARI TC | SGDALP KKYAY | WYQQK SGQAPV LVIY | EDIK RPS | GIPERFSGSSSGT MATLTISGAQVE DEADYYC | YSTDS SGNH WVF | FGGG TKLT VL |

(continued)

| Chain Name | SEQ ID NO | V Lambda | J | FR1 | CDR1 | FR2 | CDR 2 | FR3 | CDR3 | J |
|---|---|---|---|---|---|---|---|---|---|---|
| | 67 | Germline | | SYELTQPPSV SVSPGQTARI TC | SGDALP KKYAY | WYQQK SGQAPV LVIY | EDS KRP S | GIPERFSGSSSGT MATLTISGAQVE DEADYYC | YSTDS SGNH VV | FGGG TKLT VL |
| sc 58 | 8 | V2-7 | JL3 | SYELTQPPSV SVSPGQTARI TC | SGDALP KKYAY | WYQQK SGQAPV LVIY | DDS KRP S | GIPERFSGSSSGT MATLTISGAQVE DEADYYC | YSTDS SGNH RV | FGGG TKLT VL |
| | 68 | Germline | | SSELTQDPA VSVALGQTV RITC | QGDSLR SYYAS | WYQQK PGQAPV LVIY | GKN NRP S | GIPDRFSGSSSGN TASLTITGAQAE DEADYYC | NSRDS SGNH VV | FGGG TKLT VL |
| sc 298 | 40 | V2-13 | JL2 | SSELTQDPV VSVALGQTV RITC | QGDSLR SYYLS | WYQQK PGQAPV LVIY | GKN NRP S | GIPDRFSGSNSG NTASLTITGAQA EDEADYYC | NSRDS SGNH L | FGGG TKLT VL |
| | 69 | Germline | | SYELTQPSSV SVSPGQTARI TC | SGDVLA KKYAR | WFQQKP GQAPVL VIY | KDS ERPS | GIPERFSGSSSGT TVTLTISGAQVE DEADYYC | YSAA DNNV V | FGGG TKLT VL |
| sc 264 | 32 | V2-19 | JL2 | SYELTQPSSV SVSPGQTARI TC | SGDVLA KKSAR | WFHQKP GQAPVL VIY | KDS ERPS | GIPERFSGSSSGT TVTLTISGAQVE DEAAYYC | YSAA DNNL V | FGGG TKLT VL |

Table 10: Exemplary Mutations of sc 133 Heavy Chain (SEQ ID NO: 14) to Germline (SEQ ID NO: 49) at the indicated Residue Number

| 54 | 57 | 70 | 76 |
|---|---|---|---|
| N | G | M | T |
| N | G | L | I |
| N | G | L | T |
| N | D | M | I |
| N | D | L | I |
| N | D | M | T |
| N | D | L | T |
| K | G | M | I |
| K | G | M | T |
| K | G | L | I |
| K | G | L | T |
| K | D | M | I |
| K | D | L | I |
| K | D | M | T |

Table 11: Exemplary Mutations of sc 188 Light Chain (SEQ ID NO: 24) to Germline (SEQ ID NO: 61) at the indicated Residue Number

| 26 | 28 | 29 | 32 | 47 |
|---|---|---|---|---|
| G | S | V | S | L |
| G | S | V | S | p |
| G | S | V | R | P |
| G | S | V | R | L |
| G | S | V | R | L |
| G | S | V | S | P |
| G | S | I | R | P |
| G | S | I | R | L |
| G | T | V | R | L |
| G | T | V | S | P |
| G | T | V | S | L |
| G | T | I | R | P |
| G | T | I | R | L |
| G | T | I | S | L |
| S | S | V | S | P |
| S | S | V | R | P |
| S | S | V | R | L |
| S | S | V | R | L |
| S | S | V | S | P |

(continued)

| 26 | 28 | 29 | 32 | 47 |
|---|---|---|---|---|
| S | S | I | R | P |
| S | S | I | R | L |
| S | T | V | R | L |
| S | T | V | S | P |
| S | T | V | S | L |
| S | T | I | R | P |
| S | T | I | R | L |
| S | T | I | S | L |

Table 12: Exemplary Mutations of sc 188 Heavy Chain (SEQ ID NO: 22) to Germline (SEQ ID NO: 56) at the indicated Residue Number

| 33 | 37 | 45 | 60 | 78 | 83 | 85 |
|---|---|---|---|---|---|---|
| G | S | K | Y | N | K | S |
| G | S | K | Y | N | K | T |
| G | S | K | Y | N | N | S |
| G | S | K | Y | N | N | T |
| G | S | K | Y | K | N | S |
| G | S | K | Y | K | N | T |
| G | S | K | Y | K | K | S |
| G | S | K | Y | K | K | T |
| G | S | K | S | N | K | S |
| G | S | K | S | N | K | T |
| G | S | K | S | N | N | S |
| G | S | K | S | N | N | T |
| G | S | K | S | K | N | S |
| G | S | K | S | K | N | T |
| G | S | K | S | K | K | S |
| G | S | K | S | K | K | T |
| G | S | N | Y | N | K | S |
| G | S | N | Y | N | K | T |
| G | S | N | Y | N | N | S |
| G | S | N | Y | N | N | T |
| G | S | N | Y | K | N | S |
| G | S | N | Y | K | N | T |
| G | S | N | Y | K | K | S |
| G | S | N | Y | K | K | T |
| G | S | N | S | N | K | S |
| G | S | N | S | N | K | T |

(continued)

| 33 | 37 | 45 | 60 | 78 | 83 | 85 |
|---|---|---|---|---|---|---|
| G | S | N | S | N | N | S |
| G | S | N | S | N | N | T |
| G | S | N | S | K | N | S |
| G | S | N | S | K | N | T |
| G | S | N | S | K | K | S |
| G | S | N | S | K | K | T |
| V | S | K | Y | N | K | S |
| V | S | K | Y | N | K | T |
| V | S | K | Y | N | N | S |
| V | S | K | Y | N | N | T |
| V | S | K | Y | K | N | S |
| V | S | K | Y | K | N | T |
| V | S | K | Y | K | K | S |
| V | S | K | Y | K | K | T |
| V | S | K | S | N | K | S |
| V | S | K | S | N | K | T |
| V | S | K | S | N | N | S |
| V | S | K | S | N | N | T |
| V | S | K | S | K | N | S |
| V | S | K | S | K | N | T |
| V | S | K | S | K | K | S |
| V | S | K | S | K | K | T |
| V | S | N | Y | N | K | S |
| V | S | N | Y | N | K | T |
| V | S | N | Y | N | N | S |
| V | S | N | Y | N | N | T |
| V | S | N | Y | K | N | S |
| V | S | N | Y | K | N | T |
| V | S | N | Y | K | K | S |
| V | S | N | Y | K | K | T |
| V | S | N | S | N | K | S |
| V | S | N | S | N | K | T |
| V | S | N | S | N | N | S |
| V | S | N | S | N | N | T |
| V | S | N | S | K | N | S |
| V | S | N | S | K | N | T |
| V | S | N | S | K | K | S |
| V | S | N | S | K | K | T |

(continued)

| 33 | 37 | 45 | 60 | 78 | 83 | 85 |
|---|---|---|---|---|---|---|
| G | I | K | Y | N | K | S |
| G | I | K | Y | N | K | T |
| G | I | K | Y | N | N | S |
| G | I | K | Y | N | N | T |
| G | I | K | Y | K | N | S |
| G | I | K | Y | K | N | T |
| G | I | K | Y | K | K | S |
| G | I | K | Y | K | K | T |
| G | I | K | S | N | K | S |
| G | I | K | S | N | K | T |
| G | I | K | S | N | N | S |
| G | I | K | S | N | N | T |
| G | I | K | S | K | N | S |
| G | I | K | S | K | N | T |
| G | I | K | S | K | K | S |
| G | I | K | S | K | K | T |
| G | I | N | Y | N | K | S |
| G | I | N | Y | N | K | T |
| G | I | N | Y | N | N | S |
| G | I | N | Y | N | N | T |
| G | I | N | Y | K | N | S |
| G | I | N | Y | K | N | T |
| G | I | N | Y | K | K | S |
| G | I | N | Y | K | K | T |
| G | I | N | S | N | K | S |
| G | I | N | S | N | K | T |
| G | I | N | S | N | N | S |
| G | I | N | S | N | N | T |
| G | I | N | S | K | N | S |
| G | I | N | S | K | N | T |
| G | I | N | S | K | K | S |
| G | I | N | S | K | K | T |
| V | I | K | Y | N | K | S |
| V | I | K | Y | N | K | T |
| V | I | K | Y | N | N | S |
| V | I | K | Y | N | N | T |
| V | I | K | Y | K | N | S |
| V | I | K | Y | K | N | T |

(continued)

| 33 | 37 | 45 | 60 | 78 | 83 | 85 |
|---|---|---|---|---|---|---|
| V | I | K | Y | K | K | S |
| V | I | K | Y | K | K | T |
| V | I | K | S | N | K | S |
| V | I | K | S | N | K | T |
| V | I | K | S | N | N | S |
| V | I | K | S | N | N | T |
| V | I | K | S | K | N | S |
| V | I | K | S | K | N | T |
| V | I | K | S | K | K | S |
| V | I | K | S | K | K | T |
| V | I | N | Y | N | K | S |
| V | I | N | Y | N | K | T |
| V | I | N | Y | N | N | S |
| V | I | N | Y | N | N | T |
| V | I | N | Y | K | N | S |
| V | I | N | Y | K | N | T |
| V | I | N | Y | K | K | S |
| V | I | N | Y | K | K | T |
| V | I | N | S | N | K | S |
| V | I | N | S | N | K | T |
| V | I | N | S | N | N | S |
| V | I | N | S | N | N | T |
| V | I | N | S | K | N | S |
| V | I | N | S | K | N | T |
| V | I | N | S | K | K | S |
| V | I | N | S | K | K | T |

Table 13: Exemplary Mutations of sc 264 Light Chain (SEQ ID NO: 32) to Germline (SEQ ID NO: 69) at the indicated Residue Number

| 31 | 36 | 84 |
|---|---|---|
| Y | H | A |
| Y | H | D |
| Y | Q | A |
| S | H | D |
| S | Q | D |
| S I | Q | A |

**Example 19. Potency Determination of αVβ6 Antibodies**

To discriminate antibodies based on their ability to prevent the adhesion of HT29 cells to TGFβLAP, the following adhesion assay was performed.

Nunc MaxiSorp (Nunc) plates were coated overnight with 50μL of 10μg/ml TGF Betal LAP (TGFβLAP), and pre-blocked with 3% BSA/PBS for 1 hour prior to the assay. HT29 cells grown to the optimal density were then pelleted and washed twice in HBBS (with 1% BSA and without $Mn^{2+}$), after which the cells were then resuspended in HBSS at 30,000 cell per well. The coating liquid was removed from the plates, which were then blocked with 100μL 3% BSA at room temperature for 1 hour and thereafter washed twice with PBS.

Antibody titrations were prepared in 1:3 serial dilutions in a final volume of 30μL and at two times the final concentration. Care was taken to ensure that the PBS concentration in the control wells matched the PBS concentration in the most dilute antibody well. 30μL of cells were added to each well, and the cells were incubated in the presence of the antibodies at 4°C for 40 minutes in a V-bottom plate. The cell-antibody mixtures were transferred to the coated plate and the plate was incubated at 37°C for 40 minutes. The cells on the coated plates were then washed four times in warm HBSS, and the cells were thereafter frozen at -80°C for 15 minutes. The cells were allowed to thaw at room temperature, and then 100μL of CyQuant dye/lysis buffer (Molecular Probes) was added to each well according to the manufacturer's instructions. Fluorescence was read at an excitation wavelength of 485 nm and an emission wavelength of 530 nm. An estimated $IC_{50}$ value for each mAb was calculated based on the maximal and minimal amount of cell adhesion possible in the assay, as determined by positive and negative control wells. The results for twelve antibodies are summarized in Table 14.

Table 14. Adhesion Assay Results (Estimated $IC_{50}$ Values)

| | n=1 (ng/mL) | n=2 (ng/mL) | n=3 (ng/mL) |
|---|---|---|---|
| sc 049 | >5000 | >5000 | >5000 |
| sc 058 | 4065 | 2028 | 3259 |
| sc 097 | 1006 | 281 | 536 |
| sc 133 | 25 | 16 | 30 |
| sc 161 | 2408 | 137 | ND |
| sc 188 | 41 | 26 | ND |
| sc 254 | 63 | 37 | 37 |
| sc 264 | 26 | 14 | 18 |
| sc 277 | 1455 | 540 | 720 |
| sc 298 | 29 | 25 | 33 |
| sc 320 | 648 | 381 | 415 |
| sc 374 | 277 | 300 | 549 |
| Positive Control 2077Z | 226 | 185 | 286 |

**Example 20. Competition Assay**

To establish that the antibodies were specifically capable of blocking αVβ6 integrin binding to soluble TGFβLAP, a competition assay was run with the purified antibodies to measure their ability to bind to αVβ6 and block its binding to a GST-LAP peptide.

Medium binding 96-well plates (Costar, catalog # 3368) were coated with 50μL/well of 10μg/ml GST-LAP in PBS and 0.05% sodium azide, and incubated overnight at 4°C. The plates were then washed three times using 300μL/well of assay diluent (1% milk in TBS (50mM Tris, 50mM NaCl, 1mM $MgCl_2$ and 1mM $CaCl_2$, pH 6.9), after which the plates were blocked using 300μL/well 5% milk in TBS and incubated for 30 minutes at room temperature. The mAbs (in 1:3 serial dilutions ranging from 10μg/ml to 0.01 μg/ml) were incubated overnight with αVβ6 (250ng/ml in assay diluent containing 0.05% sodium azide). The following day, 50μL/well of the pre-incubated primary antibody was transferred to the GST-LAP peptide-coated plate and incubated for one hour at room temperature. The wells were then washed three times using 300μL/well of assay diluent. Then, to detect the amount of αVβ6 bound to the plates, mAb 2075 (Chemicon) was added at a concentration of 1μg/ml in assay diluent (50μL/well) and incubated for one hour at room temperature.

The wells were then washed three times using 300μL/well of assay diluent, and incubated with goat anti-mouse IgG Fc-peroxidase at 400ng/ml in assay diluent (50μL/well) for one hour at room temperature. The wells were then washed three times using 300μL/well of assay diluent, and developed using 1-step TMB (Neogen) at a total volume of 50μL/well. After 15 minutes, the developing reaction was quenched with 50μL/well of IN Hydrochloric acid. The plates were read at 450nm, and the results for five of the antibodies are summarized in Figure 14, which shows that the antibodies were able to inhibit αVβ6 binding to GST-LAP.

**Example 21. Cross-reactivity to αVβ3 or αVβ5 Integrins**

To establish that the antibodies were functional only against αVβ6 integrin and not αVβ3 or αVβ5 integrins, the following assay was performed to test the ability of the antibodies to inhibit the adhesion of A375M cells to an osteopontin peptide.

Assay plates were coated with osteopontin peptide. Two fragments of osteopontin were used: OPN 17-168 and OPN 17-314. Assay plates were pre-blocked with 3% BSA/PBS for one hour prior to the assay. The A375M cells were removed from a culture flask, pelleted and washed twice with HBSS containing 1% BSA and 1mM $Ca^{2+}$ and 1mM $Mg^{2+}$. The cells were then resuspended in HBSS at a concentration of 30,000 cells per well. The coating liquid containing the osteopontin fragments was removed, and the plates were blocked with 100μL of 3% BSA for one hour at room temperature. The coated plates were washed twice with HBSS containing 1% BSA. Antibody titrations were prepared in 1:4 serial dilutions in a final volume of 30μL and at twice the final concentration. The resuspended cells were added to the wells containing the titrated antibody in a V-bottom plate, and the cells and antibodies were co-incubated at 4°C for 40 minutes. The cell-antibody mixture was then transferred to the coated plate, which was thereafter incubated at 37°C for 40 minutes. The cells on the coated plates were next washed four times in warm HBSS, and the cells in the plates were then frozen at -80°C for 15 minutes. The cells were allowed to thaw at room temperature, and then 100μL of CyQuant dye/lysis buffer (Molecular Probes) was added to each well according to the manufacturer's instructions. Fluorescence was read at an excitation wavelength of 485 nm and an emission wavelength of 530 nm.

The results for five of the antibodies are summarized in Table 15. A commercially available αV integrin specific antibody was used as a positive control in this assay and exhibited about 90% inhibition of adhesion. A commercially available αVβ6 antibody served as a negative control in this assay for adhesion to αVβ3 or αVβ5 integrins. All antibodies were tested at a concentration of 5μg/ml and none of the test antibodies could block adhesion to αVβ3 or αVβ5 integrins.

Table 15. Cross-Reactivity to αVβ3 or αVβ5 Integrins

| Antibodv ID | Percent Inhibition |
|---|---|
| sc 133 | 3 |
| sc 188 | -2 |
| sc 254 | -5 |
| sc 264 | 3 |
| sc 298 | 9 |
| αV Control | 89 |
| αVβ6 Control | 11 |
| Human IgG Control | 3 |
| Mouse IgG Control | 5 |

**Example 22. Cross-reactivity to α4β1 Integrin**

To establish that the antibodies were functional only against the αVβ6 integrin and not the α4β1 integrin, an assay was performed to test the ability of the antibodies to inhibit the adhesion of J6.77 cells to the CS-1 peptide of fibronectin. The assay was performed as described in Example 21 above, with the exception that J6.77 cells were used for binding and the CS-1 peptide of fibronectin was used to coat the assay plates.

The results for 11 of the antibodies are summarized in Table 16. A commercially available β1 integrin specific antibody was used as a positive control in this assay and exhibited 97% inhibition of adhesion. A commercially available αVβ6 specific antibody served as a negative control in this assay for adhesion to α4β1. All antibodies were used at 5μg/ml and none of the test antibodies could block adhesion to α4β1.

Table 16. Cross-Reactivity to $\alpha$**4**$\beta$**1** Integrin

| Antibody at 5ug/ml | Percent Inhibition |
|---|---|
| sc 58 | -14 |
| sc 97 | -7 |
| sc 133 | -15 |
| sc 161 | 12 |
| sc 188 | -10 |
| sc 254 | 0 |
| sc 264 | -8 |
| sc 277 | -17 |
| sc 298 | -7 |
| sc 320 | -8 |
| sc 374 | -8 |
| Human IgG1 | -6 |
| Human IgG2 | -9 |
| Anti-betal integrin antibody | 97 |
| Anti-$\alpha$V$\beta$6 integrin antibody | -15 |
| No CS-1 or antibody on plates | 12 |
| CS-1 fragment coated plates without antibody | 10 |

**Example 23. Cross-reactivity to $\alpha$5$\beta$1 Integrin**

To establish that the antibodies were functional only against the $\alpha$V$\beta$6 integrin and not the $\alpha$5$\beta$1 integrin, an adhesion assay was performed to test the ability of the antibodies to inhibit the adhesion of K562 cells to fibronectin.

Assay plates were coated with the FN9-10 peptide of fibronectin at a concentration of 12.5$\mu$g/mL. Assay plates were pre-blocked with 3% BSA/PBS for one hour prior to the assay. The K562 cells were removed from a culture flask, pelleted and washed twice with HBSS containing 1% BSA and 1mM $Mn^{2+}$. The cells were then resuspended in HBSS at a concentration of 30,000 cells per well. The coating liquid containing the osteopontin fragments was removed, and the plates were blocked with 100$\mu$L of 3% BSA for one hour at room temperature. The coated plates were washed twice with HBSS containing 1% BSA. Antibody titrations were prepared in 1:4 serial dilutions in a final volume of 30$\mu$L and at twice the final concentration. The resuspended cells were added to the wells containing the titrated antibody in a V-bottom plate, and the cells and antibodies were co-incubated at 4°C for 60 minutes. The cell-antibody mixture was then transferred to the coated plate, which was thereafter incubated at 37°C for 40 minutes. The cells on the coated plates were next washed four times in warm HBSS, and the cells in the plates were then frozen at -80°C for 15 minutes. The cells were allowed to thaw at room temperature, and then 100$\mu$L of CyQuant dye/lysis buffer (Molecular Probes) was added to each well according to the manufacturer's instructions. Fluorescence was read at an excitation wavelength of 485 nm and an emission wavelength of 530 nm.

The results for five of the antibodies are summarized in Table 17. Test antibodies were compared to a commercially available $\alpha$5$\beta$1 antibody as a positive control and an $\alpha$V$\beta$6 specific antibody as a negative control. None of the test antibodies were able to block adhesion in the assay at the 5 $\mu$g/ml concentration used in this assay.

Table 17. Cross-Reactivity to $\alpha$**5**$\beta$**1** Integrin

| Antibody ID | Percent Inhibition |
|---|---|
| sc 133 | -12 |
| sc 188 | 5 |
| sc 254 | -9 |

(continued)

| Antibody ID | Percent Inhibition |
|---|---|
| sc 264 | -4 |
| sc 298 | 2 |
| $\alpha V\beta 6$ Control | 7 |
| $\alpha 5\beta 1$ Control | 78 |
| Human IgG Control | 11 |

**Example 24. Cross-reactivity to Murine and Cynomolgus $\alpha V\beta 6$ Integrin**

In order to determine whether the antibodies exhibited cross-reactivity to mouse $\alpha V\beta 6$ or Cynomolgus $\alpha V\beta 6$, the following assay was performed.

Cross-reactivity of the mAbs to macaque and mouse $\alpha V\beta 6$ was tested on the purified mAbs using FACS analysis on HEK-293 cells transiently transfected with cynomolgus or mouse $\alpha V$, $\beta 6$, or $\alpha V\beta 6$. Approximately 48 hours after transfection, the cells were collected and resuspended in FACS buffer to reach a final concentration of approximately 50,000 cells in 100$\mu$L.

Approximately 100,000 cells total, were used in each reaction as follows. 200$\mu$L of 293 cells were dispensed into a V-bottom plate. The cells in the plate were pelleted at 1500 rpm for 3 minutes, and then resuspended in 100 $\mu$L FACS buffer. The pelleting step was repeated, and the FACS buffer supernatant was removed. The purified mAbs, or control primary antibodies were added in a volume of 50 $\mu$L and the cells were resuspended. Primary antibody controls were murine $\alpha V\beta 6$ (Cat#MAB2077z, Chemicon) and anti-aV and anti-$\beta 6$ recombinants. The plate was incubated on ice for 45 minutes, after which 100 $\mu$L FACS buffer was added to dilute the primary antibody. The cells were then pelleted by centrifuging at 1500 rpm for 3 minutes, and the pellet was resuspended in 100 $\mu$L FACS buffer. The pelleting step was repeated, and the FACS buffer supernatant was removed. Cells were then resuspended in the appropriate secondary antibody (5 $\mu$g/ml) with 7AAD dye (10 $\mu$g/ml), and stained on ice for 7 minutes. Then 150 $\mu$L of FACS buffer was added and the cells were pelleted at 1500 rpm for 3 minutes, after which the cells were washed in 100 $\mu$L FACS buffer, pelleted, and then resuspended in 250 $\mu$L buffer and added to FACS tubes. Samples were analyzed on a high throughput FACS machine and analyzed using Cell Quest Pro software.

The results are summarized in Table 18, and demonstrate that mAb sc 133 and mAb sc 188 were clearly cross-reactive with mouse and Cynomolgus $\alpha V\beta 6$ and $\beta 6$. mAb sc 254 appeared to cross-react with $\beta 6$, $\alpha V$, and $\alpha V\beta 6$. mAbs sc 264 and 298 had high levels of binding to parental cells making species cross-reactivity difficult to discern.

Table 18. Cross-Reactivity with Mouse and Cynomolgus $\alpha$**V$\beta$6**

| Antibodies | Parental | Mouse alpha V | Mouse beta6 | Mouse alphaVbeta6 | Cynomolgus alpha V | Cynomolgus beta6 | Cynomolgus alphaVbeta6 |
|---|---|---|---|---|---|---|---|
| Cells alone | 0 | 0 | 0 | 0 | 1 | 0 | 0 |
| Gt anti Mouse | 0 | 0 | 0 | 0 | 0 | 0 | 0 |
| anti alpha Vbeta6 | 0 | 1 | 11 | 45 | 0 | 5 | 20 |
| anti alphaV | 68 | 68 | 63 | 59 | 68 | 69 | 67 |
| anti beta6 | 0 | 0 | 0 | 0 | 0 | 0 | 0 |
| Gt anti Human | 0 | 0 | 0 | 0 | 0 | 0 | 0 |
| Human IgG1 | 0 | 1 | 0 | 1 | 1 | 1 | 0 |
| sc.133 | 2 | 4 | 19 | 49 | 5 | 10 | 28 |
| sc.188 | 1 | 3 | 29 | 51 | 2 | 17 | 27 |
| sc.254 | 8 | 13 | 21 | 50 | 16 | 19 | 26 |

(continued)

| Antibodies | Parental | Mouse alpha V | Mouse beta6 | Mouse alphaVbeta6 | Cynomolgus alpha V | Cynomolgus beta6 | Cynomolgus alphaVbeta6 |
|---|---|---|---|---|---|---|---|
| sc.264 | 74 | 71 | 68 | 63 | 70 | 75 | 54 |
| sc.298 | 49 | 45 | 52 | 53 | 48 | 52 | 38 |
| Data represent percent of cells shifted | | | | | | | |

**Example 25. Internalization Assay**

The internalization of the antibodies was tested using a K562 cell line that stably expressed human αVβ6. Internalization of the purified antibodies was compared to a commercially available αVβ6 antibody that was not internalized in this assay.

The results are summarized in Table 19.

Table 19. Summary of the Internalization Assay

| Antibody | Concentration (ug/mL) | Percent Internalization |
|---|---|---|
| sc 133 | 10 | 28% |
| sc 133 | 1 | 30% |
| sc 188 | 10 | 38% |
| sc 188 | 1 | 34% |
| sc 254 | 10 | 49% |
| sc 254 | 1 | 39% |
| sc 264 | 10 | 76% |
| sc 264 | 1 | 77% |
| sc 298 | 10 | 28% |
| sc 298 | 1 | 26% |

**Example 26. High Resolution Biacore Analysis**

High resolution Biacore analysis using a soluble αVβ6 protein to bind antibodies immobilized on CM5 chips was performed for 5 of the αVβ6 antibodies to estimate their affinity for soluble antigen.

The Biacore analysis was performed as follows. A high-density goat α human IgG antibody surface over two CM5 Biacore chips was prepared using routine amine coupling. Each mAb was diluted in degassed HBS-P running buffer containing 100 μg/ml BSA, 1mM $MgCl_2$, and 1mM $CaCl_2$ to a concentration of approximately 1 μg/mL. More precisely, mAb sc 133 was diluted to 0.98 μg/mL, mAb sc 188 was diluted to 0.96 μg/mL, mAb sc 264 was diluted to 0.94 μg/mL, mAb sc 254.2 was diluted to 0.87 μg/mL, and mAb sc 298 was diluted to 1.6 μg/mL. Then, a capture level protocol was developed for each mAb by capturing each mAb over a separate flow cell at a 10 μL/min flow rate at the concentrations listed above. mAbs sc 133, sc 298, and sc 254.2 were captured for 30 seconds while mAbs sc 188 and sc 264 were captured for 1 minute. A 4-minute wash step at 50 μL/min followed to stabilize the mAb baseline.

Soluble αVβ6 was injected for 4 minutes at a concentration range of 116 - 3.6 nM for mAbs sc 133, sc 188, sc 264, and sc 298, and 233 - 3.6 nM for mAb sc 254.2, with a 2x serial dilution for each concentration range. A 10-minute dissociation followed each antigen injection. The antigen samples were prepared in the HBS-P running described above. All samples were randomly injected in triplicate with several mAb capture/buffer inject cycles interspersed for double referencing. The high-density goat α mouse antibody surfaces were regenerated with one 18-second pulse of 146 mM phosphoric acid (pH 1.5) after each cycle at a flow rate of 100 μL/min. A flow rate of 50 μL/min was used for all antigen injection cycles.

The data were then fit to a 1:1 interaction model with the inclusion of a term for mass transport using CLAMP The resulting binding constants are listed in Table 20. The mAbs are listed from highest to lowest affinity.

Table 20. Affinity Determination Results for Cloned and Purified mAbs Derived from High Resolution Biacore™.

| Antibody | $R_{max}$ | $k_a$ ($M^{-1}s^{-1}$) | $k_d$ ($s^{-1}$) | $K_D$ (nM) |
|---|---|---|---|---|
| sc 264 | 96 | 5.85 X $10^4$ | 3.63 X $10^{-4}$ | 6.2 |
| sc 298 | 77 | 5.65 X $10^4$ | 1.18 X $10^{-3}$ | 21.0 |
| sc 188 | 76 | 4.52 X $10^4$ | 9.56 X $10^{-4}$ | 21.2 |
| sc 133 | 96 | 5.73 X $10^4$ | 1.89 X $10^{-3}$ | 33.0 |
| sc 254.2 | 53,45 | 5.73 X $10^4$ | 5.64 X $10^{-4}$ | 34.9 |

**Example 27. Binding Affinity Analysis Using FACS**

As an alternative to Biacore, FACS analysis was also used to estimate the binding affinity of one of the antibodies to K562 cells that stably express the human $\alpha V\beta 6$ antigen. The amount of antigen was titrated to generate a binding curve and estimate the binding affinity to the antigen.

K562 cells expressing $\alpha V\beta 6$ were resuspended in filtered HBS buffer containing 1 mM of $MgCl_2$ and 1 mM of $CaCl_2$ at a concentration of approximately 6 million cells/mL. The cells were kept on ice. Purified mAb sc 188 was serially diluted by a factor of 1:2 in HBS across 11 wells in a 96-well plate. The 12$^{th}$ well in each row contained buffer only. Titrations were done in triplicate. Additional HBS and cells were added to each well so that the final volume was 300 $\mu$L/well and each well contained approximately 120,000 cells. The final molecular concentration range for mAb sc 188 was 4.9 - 0.019 nM. The plates were placed into a plate shaker for 5 hours at 4°C, after which the plates were spun and washed three times with HBS, following which, 200 $\mu$L of 131 nM Cy5 goat $\alpha$-human polyclonal antibody (Jackson Laboratories, #109-175-008) were added to each well. The plates were then shaken for 40 minutes at 4°C, and thereafter were spun and washed once again three times with HBS. The Geometric Mean Fluorescence (GMF) of 20,000 "events" for each mAb concentration was recorded using a FACSCalibur instrument, and the corresponding triplicate titration points were averaged to give one GMF point for each mAb concentration. A plot of the averaged GMF as a function of molecular mAb concentration with Scientist software was fit nonlinearly using the equation:

$$F = P' \frac{(K_D + L_T + n \cdot M) - \sqrt{(K_D + L_T + n \cdot M)^2 - 4n \cdot M \cdot L_T}}{2} + B$$

In the above equation, F = geometric mean fluorescence, $L_T$ = total molecular mAb concentration, P' = proportionality constant that relates arbitrary fluorescence units to bound mAb, M = cellular concentration in molarity, n = number of receptors per cell, B = background signal, and $\mathbf{K_D}$ = equilibrium dissociation constant. For mAb sc 188 an estimate for $\mathbf{K_D}$ is obtained as P', n, B, and $\mathbf{K_D}$ are allowed to float freely in the nonlinear analysis.

The resulting plot with its nonlinear fits (red line) is shown in Figure 15. Table 21 lists the resulting $\mathbf{K_D}$ for mAb sc 188 along with the 95% confidence interval of the fit. These results for mAb sc 188 indicate binding to one type of receptor.

Binding affinity for sc 188 as determined by FACS was significantly tighter than as determined by Biacore (Example 26). There are at least 2 possible explanations for the difference in $\mathbf{K_D}$ values for sc 188. The first reason is that the two assays used different forms of the antigen for the measurement - Biacore used soluble antigen and the FACs analysis used a cell-bound form of the antigen. The second reason is that the antibodies that were tested were raised against the cell-bound form of the antigen and may not bind with as high an affinity to the soluble antigen as they do to the cell-bound antigen.

Table 21. Binding Affinity Analysis Using FACS

| Antibody | KD (pM) | 95% CI (pM) |
|---|---|---|
| sc 188 | 51.9 | + 22.7 |

**Example 28. CDC Assay**

The purified antibodies described in the examples above are of the IgG1 isotype and can have effector function. In order to determine the ability of these antibodies to mediate complement-dependent cytotoxicity (CDC), the following assay was performed using 293 cells stably expressing αVβ6 (293-10A11) and parental 293 cells (293F).

For calcein staining of cells, aliquots of approximately 25 X 10e6 each of HT29, 293-10A11, and 293F cells were individually resuspended in 3ml serum-free RPMI media. 45μL of 1mM calcein was then added to each 3ml sample of cells, and the samples were incubated at 37°C for 45 minutes. The cells were centrifuged at 1200xRPM for 3 minutes, the supernatant was discarded and the cells were resuspended in each respective cell line's culture media. The centrifugation step was repeated and the cells were resuspended to give a final concentration of about 100,000 cells in 50μL media.

Serial 1:2 dilutions of each antibody were prepared in a v-bottom 96-well plate, with concentrations ranging from 20μg/ml to 0.625μg/ml in a volume of 50 μL. Then, 100,000 of the cells prepared as described above were added in a volume of 50 μL to the antibody-containing plates, and the resulting mixture was incubated on ice for two hours. Following the incubation, the cells were pelleted, and the supernatant was discarded. The cells were resuspended in 100μL of their respective media containing 10% human sera (ABI donor #27), and incubated at 37°C for 30 minutes. The cells were then centrifuged, and 80μL of the supernatant was transferred to a FMAT plate. The plate was read on a Tecan reader using an excitation wavelength of 485nm and an emission wavelength of 530nm.

The results are summarized in Figures 16A-3E, and demonstrate that each purified antibody tested is capable of mediating CDC in 293 cells stably expressing αVβ6 integrin.

**Example 29. Site-directed Mutagenesis**

One of the antibodies (sc 264) prepared from the immunizations (Example 1) showed strong functional blocking activity *in vitro* in the TGFβLAP binding inhibition assay (see Example 4), but exhibited cross-reactive binding to non-αVβ6 expressing cell lines (see Example 24). This antibody, sc 264, has an RGD sequence in the CDR3 region, which is presumed to be responsible for the cross-reactive binding. Therefore, site-directed mutagenesis was used to replace the glycine residue in the RGD with an alanine (sc 264 RAD).

A second antibody (sc 188) has a glycosylation site within the FR3 region. This site was eliminated through site-directed mutagenesis with a substitution from NLT to KLT (sc 188 SDM). The mutated versions of these two antibodies were then expressed and purified as described in Examples 7 and 8, and the purified antibodies were analyzed as described in the following examples.

**Example 30. Binding Assay to Test Cross-Reactive Binding of Mutant Antibodies**

A binding assay was performed to test whether the cross-reactive binding observed in Example 24 was reduced because of site-directed mutagenesis of sc 264. Binding of the antibodies was analyzed on 293T and 293F cell lines to test whether removing the RGD site from sc 264 would result in decreased binding compared with the original antibody.

293T and 293F cells were spun down after collection and resuspended in HBSS with 1% BSA and 1mM $CaCl_2$ and 1mM $MgCl_2$ (wash buffer), so that at least 150,000 cells were used in each reaction. Cells were divided between reactions in a V-bottom 96-well plate (Sarstedt), and the cells in the plate were pelleted at 1500 rpm for 3 minutes, after which the HBSS supernatant was removed. The primary antibody was added at the concentration indicated in Table 19 in a volume of 50μL, and the cells were resuspended and thereafter incubated on ice for 60 minutes. After incubation, the cells were pelleted by centrifugation at 1500 rpm for 3 minutes, resuspended in 100μL wash buffer, and then pelleted again. Cells were then resuspended in the appropriate secondary antibody at 2μg/ml with 10μg/ml 7AAD, and stained on ice for 7 minutes, after which 150 μL of wash buffer was added, and cells were pelleted at 1500 rpm for 3 minutes and then resuspended in 100μL of HBSS with 1% BSA. Samples were read on a FACS machine with a HTS attachment and the data was analyzed using Cell Quest Pro software. The results are summarized in Table 22, and data appears as Geometric Mean Shift values in arbitrary units. These data demonstrate that at all concentrations tested, sc 264 RAD had significantly less binding to parental 293T cells compared to the original mAb sc 264.

Table 22. Cross-reactivity of mutated antibodies to parental cells.

| Antibody | Concentration (ug/ml) | 293T Cells | 293T-αVβ6 Cells |
|---|---|---|---|
| None | n/a | 3 | 2 |
| Mouse IgG2a | 20 | 27 | 8 |
| Human IgG1 | 20 | 4 | 4 |
| Anti-aVb6 | 20 | 4 | 5 |
| sc 264 | 20 | 433 | 6673 |

(continued)

| Antibody | Concentration (ug/ml) | 293T Cells | 293T-$\alpha V\beta 6$ Cells |
|---|---|---|---|
| sc 264 RAD | 20 | 44 | 7241 |
| sc 188 | 20 | 27 | 6167 |
| sc 188 SDM | 20 | 25 | 6758 |
| sc 264 | 5 | 88 | 6418 |
| sc 264 RAD | 5 | 13 | 6840 |
| sc 188 | 5 | 9 | 5822 |
| sc 188 SDM | 5 | 9 | 6822 |
| sc 264 | 1.25 | 24 | 6230 |
| sc 264 RAD | 1.25 | 7 | 4890 |
| sc 188 | 1.25 | 6 | 6395 |
| sc 188 SDM | 1.25 | 5 | 4532 |

**Example 31. Potency Analysis of Mutant Antibodies**

In order to determine the concentration ($IC_{50}$) of mutant $\alpha V\beta 6$ antibodies required to inhibit TGF$\beta$LAP-mediated adhesion of HT-29 cells, the following assay was performed.

Nunc MaxiSorp (Nunc) plates were coated overnight with 50$\mu$L of 10$\mu$g/ml TGF Betal LAP (TGF$\beta$LAP), and pre-blocked with 3% BSA/PBS for 1 hour prior to the assay. HT29 cells grown to the optimal density were then pelleted and washed twice in HBBS (with 1% BSA and with 1mM $Ca^{2+}$ and 1mM $Mg^{2+}$), after which the cells were then resuspended in HBSS at 30,000 cell per well. The coating liquid was removed from the plates, which were then blocked with 100$\mu$L 3% BSA at room temperature for 1 hour and thereafter washed twice with PBS.

Antibody titrations were prepared in 1:4 serial dilutions in a final volume of 30$\mu$L and at two times the final concentration. Care was taken to ensure that the PBS concentration in the control wells matched the PBS concentration in the most dilute antibody well. 30$\mu$L of cells were added to each well, and the cells were incubated in the presence of the antibodies at 4°C for 40 minutes in a V-bottom plate. The cell-antibody mixtures were transferred to the coated plate and the plate was incubated at 37°C for 40 minutes. The cells on the coated plates were then washed four times in warm HBSS, and the cells were thereafter frozen at -80°C for 15 minutes. The cells were allowed to thaw at room temperature, and then 100$\mu$L of CyQuant dye/lysis buffer (Molecular Probes) was added to each well according to the manufacturer's instructions. Fluorescence was read at an excitation wavelength of 485 nm and an emission wavelength of 530 nm. The results for twelve antibodies are summarized in Table 23, and demonstrate that the $IC_{50}$ of the mutant antibodies is consistently less than that of each original antibody.

Table 23. Concentration ($IC_{50}$) of mutant antibodies required to inhibit TGF$\beta$LAP-mediated adhesion of HT29 cells.

| | n=1 (ng/ml) | n=2 (ng/ml) | n=3 (ng/ml) |
|---|---|---|---|
| sc.264 | **113** | **96** | **55** |
| sc.264 | **13** | **13** | **39** |
| RAD | | | |
| sc.264 | **57** | **89** | **46** |
| sc.188 | **125** | **157** | **64** |
| sc.188 SDM | **22** | **24** | **67** |

**Example 32. Cross-Reactivity of Mutant Antibodies to $\alpha 4\beta 1$ Integrin**

To establish that the mutant antibodies were functional only against the $\alpha V\beta 6$ integrin and not the $\alpha 4\beta 1$ integrin, an assay was performed to test the ability of the antibodies to inhibit the adhesion of J6.77 cells to the CS-1 peptide of fibronectin. The assay was performed as described as described below.

Assay plates were coated with the CS-1 peptide of fibronectin. Assay plates were pre-blocked with 3% BSA/PBS for one hour prior to the assay. The J6.77 cells were grown to confluency, then removed from a culture flask, pelleted and washed three times with HBSS. The cells were then resuspended in HBSS at a concentration of 30,000 cells per well. The coating liquid containing the fibronectin fragments was removed, and the plates were blocked with 100μL of 3% BSA for one hour at room temperature. The coated plates were washed three times with HBSS. Antibody titrations were prepared in 1:4 serial dilutions in a final volume of 30μL and at twice the final concentration. The resuspended cells were added to the wells containing the titrated antibody in a V-bottom plate, and the cells and antibodies were co-incubated at 4°C for 40 minutes. The cell-antibody mixture was then transferred to the coated plate, which was thereafter incubated at 37°C for 40 minutes. The cells on the coated plates were next washed four times in warm HBSS, and the cells in the plates were then frozen at -80°C for 15 minutes. The cells were allowed to thaw at room temperature, and then 100μL of CyQuant dye/lysis buffer (Molecular Probes) was added to each well according to the manufacturer's instructions. Fluorescence was read at an excitation wavelength of 485 nm and an emission wavelength of 530 nm.

The results for the two mutant antibodies and their non-mutated counterparts are summarized in Table 24. A commercially available β1 integrin specific antibody was used as a positive control in this assay and exhibited 95% inhibition of adhesion. A commercially available αVβ6 specific antibody served as a negative control in this assay for adhesion to α4β1. All antibodies were used at 5μg/ml and none of the test antibodies could block adhesion to α4β1.

Table 24. Cross-Reactivity to α**4**β**1** Integrin

| Antibody at 5ug/ml | Percent Inhibition |
|---|---|
| sc.188 | 2 |
| sC.188 SDM | -6 |
| sc.264 | -30 |
| sc.264 RAD | -2 |
| Human IgG1 | 26 |
| Human IgG2 | 13 |
| Human IgG4 | 15 |
| Anti-beta 1 Integrin | 95 |

**Example 33. Cross-Reactivity of Mutant Antibodies to α5β1 Integrin**

To establish that the mutant antibodies were functional only against the αVβ6 integrin and not the α5β1 integrin, an assay was performed to test the ability of the antibodies to inhibit the adhesion of K562 cells to fibronectin. The assay was performed as described as described in Example 14. The results are summarized in Table 25, and demonstrate that none of the tested antibodies could block adhesion to α5β1.

Table 25. Cross-Reactivity to α**5**β**1** Integrin.

| Antibody ID | % Inhibition |
|---|---|
| sc 188 | -5 |
| sc 188 SDM | -8 |
| sc 264 | 3 |
| sc 264 RAD | 6 |
| αVβ6 Control | -16 |
| α5β1 Control | 78 |
| Human IgG Control | -12 |

**Example 34. Cross-Reactivity Of Mutant Antibodies To Mouse And Cynomolgus αvβ6 Integrin**

In order to determine if the mutant αVβ6-specific antibodies exhibit cross-reactivity to mouse αVβ6 or Cynomolgus αVβ6, the following assay was performed.

K562 parental cells, or K562 cells expressing Cynomolgus or mouse αVβ6 were spun down after collection and resuspended in HBSS with 1% BSA and 1mM $CaCl_2$ and 1mM $MgCl_2$ (wash buffer), so that at least 150,000 cells were used in each reaction. Cells were divided between reactions in a V-bottom 96-well plate (Sarstedt), and the cells in the plate were pelleted at 1500 rpm for 3 minutes, after which the HBSS supernatant was removed. The primary antibody was added in a volume of 50μL, and the cells were resuspended and thereafter incubated on ice for 60 minutes. After incubation, the cells were pelleted by centrifugation at 1500 rpm for 3 minutes, resuspended in 100μL wash buffer, and then pelleted again. Cells were then resuspended in the appropriate secondary antibody at 2μg/ml with 10μg/ml 7AAD, and stained on ice for 7 minutes, after which 150 μL of wash buffer was added, and cells were pelleted at 1500 rpm for 3 minutes and then resuspended in 100μL of HBSS with 1% BSA. Samples were read on a FACS machine with a HTS attachment and the data was analyzed using Cell Quest Pro software. The results are summarized in Table 26, and data appears as Geometric Mean Shift values in arbitrary units. These data demonstrate that at the concentrations tested, sc 264 RAD and sc 188 SDM exhibit cross-reactivity to mouse and cynomolgus αVβ6.

Table 26. Cross-Reactivity with Mouse and Cynomolgus α**V**β**6**

| Antibodies | Parental | Mouse alphaVbeta6 | Cynomolgus alphaVbeta6 |
|---|---|---|---|
| Cells Alone | 3 | 3 | 3 |
| Gt anti Mouse | 5 | 6 | 7 |
| anti alphaVbeta6 | 15 | 122 | 84 |
| anti alphaV | 109 | 144 | 163 |
| anti beta6 | 26 | 43 | 37 |
| Mouse IgG2a | 23 | 36 | 25 |
| Mouse IgG1 | 12 | 20 | 13 |
| Gt anti Human | 7 | 12 | 7 |
| Human IgG1 | 46 | 108 | 54 |
| sc 133 | 57 | 246 | 154 |
| sc 188 | 55 | 227 | 139 |
| sc 188 SDM | 47 | 219 | 142 |
| sc 254 | 98 | 260 | 190 |
| sc 264 | 33 | 160 | 121 |
| sc 264 RAD | 48 | 196 | 139 |
| sc 298 | 33 | 150 | 97 |

### Example 35. Internalization Assay

The internalization of the mutant antibodies was tested using a K562 cell line that stably expressed human αVβ6. The assay was performed as described in Example 24. Internalization of the purified antibodies was compared to a commercially available αVβ6 antibody that was not internalized in this assay.

The results are summarized in Table 27, and demonstrate that the sc 264 RAD mutant antibody is internalized significantly less than the non-mutated sc 264.

Table 27. Summary of the Internalization Assay

| Antibody | Concentration (ug/ml) | Percent Internalization |
|---|---|---|
| sc 264 | 10 | 75% |
| sc 264 | 1 | 47% |
| sc 264 RAD | 10 | 42% |
| sc 264 RAD | 1 | 31% |
| sc 188 | 10 | 18% |

(continued)

| Antibody | Concentration (ug/ml) | Percent Internalization |
|---|---|---|
| sc 188 | 1 | 27% |
| sc 188 SDM | 10 | 22% |
| sc 188 SDM | 1 | 17% |

**Example 36. Binding Affinity Analysis of sc 264 RAD Using FACS**

The binding affinity to αVβ6 of the sc 264 RAD antibody was measured as described in Example 18. The results of this assay are summarized in Table 28, and demonstrate that the sc 264 RAD antibody has an affinity <50pM.

Table 28. Binding Affinity Analysis Using FACS

| mAb Sample | KD (pM) | 95% CI (pM) |
|---|---|---|
| sc 264 RAD | 46.3 | + 15.9 |

**Example 37. Comparison of the Activity of sc 264 RAD with sc 264 RAD/ADY**

The activity of sc 264 RAD antibody and the germlined (GL) version of 264RAD (containing the mutation A84D in the light chain), 264 RAD/ADY were compared in a Detroit-562 adhesion assay.

Plates were coated with 0.5μg/ml GST-TGF-b LAP fusion protein at 4°C overnight and the following morning, washed, and then blocked with 3% BSA/PBS for 1 hour. Detroit-562 cells (25000 cells per well) were then allowed to adhere to the plates for 45 minutes at 37°C in HBSS containing 2mM $MgCl_2$. After 45 minutes the plates were washed three times in PBS and then fixed in ethanol. Cells were visualized by staining with Hoescht and quantitated by counting the number of cells bound per well on a Cellomics Arrayscan II.

The data shown in Figure 18 indicates that both sc 264 RAD and sc 264 RAD/ADY have similar activity and that the ability to block αVβ6 function is maintained in the modified antibody.

**Example 38. Growth Study**

To establish that the antibodies 264RAD, 133 and 188 SDM block avb6 function *in vivo* each were tested for the ability to inhibit growth of αVβ6 positive tumour xenograft. One such model is the Detroit-562 nasophayngeal cell line, which expresses αVβ6 and also grows as a sub-cutaeneous tumour xenograft.

Detroit 562 cells were cultured in EMEM with Earle's BSS and 2mM L-Glu + 1.0 mM sodium pyruvate, 0.1mM NEAA + 1.5g/L sodium bicarbonate + 10% FBS. Cells were harvested and resuspended in 50% PBS + 50% matrigel. The suspension was then implanted at $5x10^{-6}$ per mouse in a volume of 0.1 ml within the right flank. Animals were 6-8 week old NCR female nude mice. Dosing was initiated when tumours reached 0.1 cm3 and dosed at 20mg/kg once weekly for the duration of the study.

All three antibodies inhibited tumour growth (see figure 17). 264RAD was the most effective, followed by 133, and 188. This data clearly shows that the antibodies 264RAD, 133 and 188 are active *in vivo* and are able reduce the growth of a tumour dependent on αVβ6 signaling for growth.

Table 29 Exemplary Antibody Heavy Chain Amino Acid Sequences

| Chain Name | SEQ ID NO: | FR1 | CDR1 | FR2 | CDR2 | FR3 | CDR3 | FR4 |
|---|---|---|---|---|---|---|---|---|
| sc264RAD | 75 | **QVQLQESGPGLVKPSQTLSLTCTVS** | **GGSISSGGYYWS** | WIRQHPGKGLEWIG | YIYYSGRTYNNPSLKS | RVTISVDTSKNQFSLKLSSVTAADTAVYYCAR | VATGRADYHFYAMDV | WGQGTTVTVSS |
| sc264RAD/ADY | 95 | QVQLQESGPGLVKPSQTLSLTCTVS | GGSISSGGYYWS | WIRQHPGKGLEWIG | YIYYSGRTYNNPSLKS | RVTISVDTSKNQFSLKLSSVTAADTAVYYCAR | VATGRADYHFYAMDV | WGQGTTVTVSS |
| sc188SDM | 71 | QVQLQESGPGLVKPSQTLSLTCTVS | GGSISSGVYYWT | WIRQHPGNGLEWIG | YIYYSGSTSYNPSLKS | RVTISVDTSKKQFSLKLTSVTAADTAVYYCAR | EGPLRGDYYYGLDV | WGQGTTVTVSS |
| sc133TMT | 79 | QVQLVQSGAEVKKPGASVKVSCKAS | GYTFTGYYMH | WVRQAPGQGLEWMG | WINPKSGDTNYAQKFQG | RVTMTRDTSTSTAYMELSRLRSDDTAVYYCAR | RLDV | WGQGTTVTVSS |
| sc133WDS | 83 | QVQLVQSGAEVKKPGASVKVSCKAS | GYTFTGYYMH | WVRQAPGQGLEWMG | WINPKSGDTNYAQKFQG | RVTLTRDTSTSTAYMELSRLRSDDTAVYYCAR | RLDV | WGQGTTVTVSS |
| sc133TMT/WDS | 87 | QVQLVQSGAEVKKPGASVKVSCKAS | GYTFTGYYMH | WVRQAPGQGLEWMG | WINPKSGDTNYAQKFQG | RVTMTRDTSTSTAYMELSRLRSDDTAVYYCAR | RLDV | WGQGTTVTVSS |

Table 30 Exemplary Antibody Light Chain Amino Acid Sequences

| Chain Name | SEQ ID NO: | FR1 | CDR1 | FR2 | CDR2 | FR3 | CDR3 | FR4 |
|---|---|---|---|---|---|---|---|---|
| **sc 264 RAD** | **77** | SYELTQPSSV SVSPGQTARI TC | SGDVL AKKSA R | WFHQKPGQAP VLVIY | KDSERPS | GIPERFSGSSSGTTV TLTISGAQVEDEAA YYC | YSAADN NLV | FGGGTK LTVL |
| **sc 264 RAD/A DY** | **97** | SYELTQPSSV SVSPGQTARI TC | SGDVL AKKSA R | WFHQKPGQAP VLVIY | KDSERPS | GIPERFSGSSSGTTV TLTISGAQVEDEA**D** YYC | YSAADN NLV | FGGGTK LTVL |
| **sc 188 SDM** | **73** | EIVLTQSPGT LSLSPGERAT LSC | RAGQT ISSRYL A | WYQQKPGQAP RPLIY | GASSRAT | GIPDRFSGSGSGTDF TLTISRLEPEDFAVY YC | QQYGSSP RT | FGQGTK VEIK |
| **sc 133 TMT** | **81** | QSVLTQPPSV SAAPGQKVTI SC | SGSSS NIGNN YVS | WYQQLPGTAP KLLIY | DNNKRPS | GIPDRFSGSKSGTSA TLGITGLQTGDEAD YYC | GTWNSSL SAGYV | FGTGTK VTVL |
| **sc 133 WDS** | **85** | QSVLTQPPSV SAAPGQKVTI SC | SGSSS NIGNN YVS | WYQQLPGTAP KLLIY | DNNKRPS | GIPDRFSGSKSGTSA TLGITGLQTGDEAD YYC | GTW**D**SSL SAGYV | FGTGTK VTVL |
| **sc 133 TMT/WDS** | **89** | QSVLTQPPSV SAAPGQKVTI SC | SGSSS NIGNN YVS | WYQQLPGTAP KLLIY | DNNKRPS | GIPDRFSGSKSGTSA TLGITGLQTGDEAD YYC | GTW**D**SSL SAGYV | FGTGTK VTVL |

## REFERENCES

1. Varden Y, Sliwkowski MX. Untangling the ErbB signalling network. Nat Rev Mol Cell Bioi. 2001;2: 127-137.
2. Slamon DJ, Clark GM, Wong SG, et al. Human breast cancer: correlation of relapse and survival with amplification of the HER-2/neu oncogene. Science. 1987;235: 177-182.
3. Slamon DJ, Godolphin W, Jones LA, et al. Studies of the HER-2/neu proto-oncogene in human breast and ovarian cancer. Science. 1989;244: 707-712.
4. Wong AL, Lee SC. Mechanisms of Resistance to Trastuzumab and Novel Therapeutic Strategies in HER2-Positive Breast Cancer. Int J Breast Cancer. 2012;2012: 415170.
5. Piccart-Gebhart MJ, Procter M, Leyland-Jones B, et al. Trastuzumab after adjuvant chemotherapy in HER2-positive breast cancer. N Engl J Med. 2005;353: 1659-1672.
6. Romond EH, Perez EA, Bryant J, et al. Trastuzumab plus adjuvant chemotherapy for operable HER2-positive breast cancer. N Engl J Med. 2005;353: 1673-1684.
7. Arribas J, Baselga J, Pedersen K, Parra-Palau JL. p95HER2 and breast cancer. Cancer Res. 2011 ;71: 1515-1519.
8. Gajria D, Chandarlapaty S. HER2-amplified breast cancer: mechanisms of trastuzumab resistance and novel targeted therapies. Expert Rev Anticancer Ther. 2011 ;11: 263-275.
9. Lindsley CW, Barnett SF, Layton ME, Bilodeau MT. The P13K/Akt pathway: recent progress in the development of ATP-competitive and allosteric Akt kinase inhibitors. Curr Cancer Drug Targets. 2008;8: 7-18.
10. Ueda Y, Wang S, Dumont N, et al. Overexpression of HER2 (erbB2) in human breast epithelial cells unmasks transforming growth factor betainduced cell motility. J Bioi Chem. 2004;279: 24505-24513.
11. Wang SE, Shin I, Wu FY, Friedman DB, Arteaga CL. HER2/Neu (ErbB2) signaling to Rac1-Pak1 is temporally and spatially modulated by transforming growth factor beta. Cancer Res. 2006;66: 9591-9600.
12. Muraoka RS, Koh Y, Roebuck LR, et al. Increased malignancy of Neuinduced mammary tumors overexpressing active transforming growth factor betal. Mol Cell Bioi. 2003;23: 8691-8703.
13. Lyons RM, Gentry LE, Purchio AF, Moses HL. Mechanism of activation of latent recombinant transforming growth factor beta 1 by plasmin. J Cell Bioi. 1990;110: 1361-1367.
14. Munger JS, Huang X, Kawakatsu H, et al. The integrin alpha v beta 6 binds and activates latent TGF beta 1: a mechanism for regulating pulmonary inflammation and fibrosis. Cell. 1999;96: 319-328.
15. Hazelbag S, Kenter GG, Gorter A, et al. Overexpression of the alpha v beta 6 integrin in cervical squamous cell carcinoma is a prognostic factor for decreased survival. J Pathol. 2007;212: 316-324.
16. Bates RC, Bellovin D1, Brown C, et al. Transcriptional activation of integrin beta6 during the epithelial-mesenchymal transition defines a novel prognostic indicator of aggressive colon carcinoma. J Clin Invest. 2005;115: 339-347.
17. Elayadi AN, Samli KN, Prudkin L, et al. A peptide selected by biopanning identifies the integrin alphavbeta6 as a prognostic biomarker for nonsmall cell lung cancer. Cancer Res. 2007;67: 5889-5895.
18. Breuss JM, Gallo J, Delisser HM, et al. Expression of the beta 6 integrin subunit in development, neoplasia and tissue repair suggests a role in epithelial remodeling. J Cell Sci. 1995;108 (Pt 6): 2241-2251.
19. Van Aarsen LA, Leone DR, Ho S, et al. Antibody-mediated blockade of integrin alpha v beta 6 inhibits tumor progression in vivo by a transforming growth factor-beta-regulated mechanism. Cancer Res. 2008;68: 561-570.
20. Hu M, Yao J, Carroll DK, et al. Regulation of in situ to invasive breast carcinoma transition. Cancer Cell. 2008;13: 394-406.
21. Hynes RO. Integrins: bidirectional, allosteric signaling machines. Cell. 2002;110: 673-687.
22. Kumar CC. Signaling by integrin receptors. Oncogene. 1998;17: 1365-1373.
23. McShane LM, Altman DG, Sauerbrei W, et al. Reporting recommendations for tumour MARKer prognostic studies (REMARK). Eur J Cancer. 2005;41 : 1690-1696.
24. Rakha EA, EI-Rehim DA, Paish C, et al. Basal phenotype identifies a poor prognostic subgroup of breast cancer of clinical importance. Eur J Cancer. 2006;42: 3149-3156.
25. Abd EI-Rehim OM, Pinder SE, Paish CE, et al. Expression of luminal and basal cytokeratins in human breast carcinoma. J Pathol. 2004;203: 661-671.
26. Curtis C, Shah SP, Chin SF, et al. The genomic and transcriptomic architecture of 2,000 breast tumours reveals novel subgroups. Nature. 2012;486: 346-352.
27. Crowder MJ HD. Monographs on Statistics and Applied Probability. 1, Editor, editor" editors. London: Chapman & Hall; 1990.
28. Lewis GO, Lofgren JA, McMurtrey AE, et al. Growth regulation of human breast and ovarian tumor cells by heregulin: Evidence for the requirement of ErbB2 as a critical component in mediating heregulin responsiveness. Cancer Res. 1996;56: 1457-1465.

29. Eberlein C, Kendrew J, McDaid K, et al. A human monoclonal antibody 264RAD targeting alphavbeta6 integrin reduces tumour growth and metastasis, and modulates key biomarkers in vivo. Oncogene. 2012.
30. Slamon OJ, Leyland-Janes B, Shak S, et al. Use of chemotherapy plus a monoclonal antibody against HER2 for metastatic breast cancer that overexpresses HER2. N Eng/ J Med. 2001;344: 783-792.
31. Varden V. Biology of HER2 and its importance in breast cancer. Oncology. 2001;61 Suppl2: 1-13.
32. Clark AS, West K, Streicher S, Dennis PA. Constitutive and inducible Akt activity promotes resistance to chemotherapy, trastuzumab, or tamoxifen in breast cancer cells. Mol Cancer Ther. 2002;1: 707-717.
33. Muraoka-Cook RS, Shin I, Vi JV, et al. Activated type I TGFbeta receptor kinase enhances the survival of mammary epithelial cells and accelerates tumor progression. Oncogene. 2006;25: 3408-3423.
34. Falcioni R, Antonini A, Nistico P, et al. Alpha 6 beta 4 and alpha 6 beta 1 integrins associate with ErbB-2 in human carcinoma cell lines. Exp Cell Res. 1997;236: 76-85.
35. Guo W, Pylayeva Y, Pepe A, et al. Beta 4 integrin amplifies ErbB2 signaling to promote mammary tumorigenesis. Cell. 2006;126: 489-502.
36. Violette S, Sheppard, D, Rosas, 10, Arjomandi, M, Rice, T, Gilman, M, Kaminski, N, Prasse, A, Maroni, B. Identification Of Biomarkers To Monitor The Activity Of STX-100, A Humanized Anti-αVβ6 Antibody, In A Phase 2a Trial In Idiopathic Pulmonary Fibrosis. Am J Respir Crit Care Med. 2012;185.
37. Benz CC, Scott GK, Sarup JC, et al. Estrogen-dependent, tamoxifen resistant tumorigenic growth of MCF-7 cells transfected with HER2/neu. Breast Cancer Res Treat. 1992;24{2):85-95.
38. Meyer T, Marshall JF, Hart IR. Expression of alphav integrins and vitronectin receptor identity in breast cancer cells. Br J Cancer. 1998;77(4):530-6.
39. Santner SJ, Dawson PJ, Tait L, et al. Malignant MCF10CA1 cell lines derived from premalignant human breast epithelial MCF10AT cells. Breast Cancer Res Treat. 2001;65(2): 101-10.
40. Nahta R, Esteva FJ. Herceptin: mechanisms of action and resistance. Cancer Lett. 2006;232 (2): 23-38.
41. Neve RM, Chin K, Fridlyand J, et al. A collection of breast cancer cell lines for the study of functionally distinct cancer subtypes. Cancer Cell. 2006; 1 0(6):515-27.
42. Subik K, Lee JF, Baxter L, et al. The Expression Patterns of ER, PR, HER2, CK5/6, EGFR, Ki-67 and AR by Immunohistochemical Analysis in Breast Cancer Cell Lines. Breast Cancer (Auckl). 2010; 4: 35-41.

**EQUIVALENTS**

The foregoing written specification is considered to be sufficient to enable one skilled in the art to practice the embodiments. The foregoing description and Examples detail certain embodiments and describes the best mode contemplated by the inventors. It will be appreciated, however, that no matter how detailed the foregoing may appear in text, the embodiment may be practiced in many ways and should be construed in accordance with the appended claims.

## Claims

1. An αVβ6 antibody for use in a method of treating a malignant tumor in an animal, wherein the αVβ6 antibody is administered simultaneously or sequentially with a HER2 antibody,
wherein the αVβ6 antibody comprises a heavy chain and a light chain, wherein the αVβ6 antibody heavy chain variable region comprises a CDR1 of amino acid sequence GGSISSGGYYWS, a CDR2 of amino acid sequence YIYYSGRTYNNPSLKS, and a CDR3 of amino acid sequence VATGRADYHFYAMDV and wherein the αVβ6 antibody light chain variable region comprises a CDR1 of amino acid sequence SGDVLAKKSAR, a CDR2 of amino acid sequence KDSERPS and a CDR3 of amino acid sequence YSAADNNLV; and
wherein the HER2 antibody is trastuzumab,
wherein the malignant tumor overexpresses αVβ6 and HER2.

2. A HER2 antibody for use in a method of treating a malignant tumor in an animal, wherein the HER2 antibody is administered simultaneously or sequentially with an αVβ6 antibody,
wherein the αVβ6 antibody comprises a heavy chain and a light chain, wherein the αVβ6 antibody heavy chain variable region comprises a CDR1 of amino acid sequence GGSISSGGYYWS, a CDR2 of amino acid sequence YIYYSGRTYNNPSLKS, and a CDR3 of amino acid sequence VATGRADYHFYAMDV and wherein the αVβ6 antibody light chain variable region comprises a CDR1 of amino acid sequence SGDVLAKKSAR, a CDR2 of amino acid sequence KDSERPS and a CDR3 of amino acid sequence YSAADNNLV; and
wherein the HER2 antibody is trastuzumab,
wherein the malignant tumor overexpresses αVβ6 and HER2.

3. An αVβ6 antibody for use in a method of treatment by inhibiting growth of tumor cells in an animal, wherein the αVβ6 antibody is administered simultaneously or sequentially with a HER2 antibody,
wherein the αVβ6 antibody comprises a heavy chain and a light chain, wherein the αVβ6 antibody heavy chain variable region comprises a CDR1 of amino acid sequence GGSISSGGYYWS, a CDR2 of amino acid sequence YIYYSGRTYNNPSLKS, and a CDR3 of amino acid sequence VATGRADYHFYAMDV and wherein the αVβ6 antibody light chain variable region comprises a CDR1 of amino acid sequence SGDVLAKKSAR, a CDR2 of amino acid sequence KDSERPS and a CDR3 of amino acid sequence YSAADNNLV; and
wherein the HER2 antibody is trastuzumab,
wherein the tumor cells overexpresses αVβ6 and HER2.

4. A HER2 antibody for use in a method of treatment by inhibiting growth of tumor cells in an animal, wherein the HER2 antibody is administered simultaneously or sequentially with an αVβ6 antibody,
wherein the αVβ6 antibody comprises a heavy chain and a light chain, wherein the αVβ6 antibody heavy chain variable region comprises a CDR1 of amino acid sequence GGSISSGGYYWS, a CDR2 of amino acid sequence YIYYSGRTYNNPSLKS, and a CDR3 of amino acid sequence VATGRADYHFYAMDV and wherein the αVβ6 antibody light chain variable region comprises a CDR1 of amino acid sequence SGDVLAKKSAR, a CDR2 of amino acid sequence KDSERPS and a CDR3 of amino acid sequence YSAADNNLV; and
wherein the HER2 antibody is trastuzumab,
wherein the tumor cells overexpresses αVβ6 and HER2.

5. An αVβ6 antibody for use in a method of treatment by suppressing growth of trastuzumab-resistant tumor cells in an animal, wherein the αVβ6 antibody is administered simultaneously or sequentially with a HER2 antibody,
wherein the αVβ6 antibody comprises a heavy chain and a light chain, wherein the αVβ6 antibody heavy chain variable region comprises a CDR1 of amino acid sequence GGSISSGGYYWS, a CDR2 of amino acid sequence YIYYSGRTYNNPSLKS, and a CDR3 of amino acid sequence VATGRADYHFYAMDV and wherein the αVβ6 antibody light chain variable region comprises a CDR1 of amino acid sequence SGDVLAKKSAR, a CDR2 of amino acid sequence KDSERPS and a CDR3 of amino acid sequence YSAADNNLV; and
wherein the HER2 antibody is trastuzumab,
wherein the tumor cells overexpresses αVβ6 and HER2.

6. A HER2 antibody for use in a method of treatment by suppressing growth of trastuzumab-resistant tumor cells in an animal, wherein the HER2 antibody is administered simultaneously or sequentially with an αVβ6 antibody,
wherein the αVβ6 antibody comprises a heavy chain and a light chain, wherein the αVβ6 antibody heavy chain variable region comprises a CDR1 of amino acid sequence GGSISSGGYYWS, a CDR2 of amino acid sequence YIYYSGRTYNNPSLKS, and a CDR3 of amino acid sequence VATGRADYHFYAMDV and wherein the αVβ6 antibody light chain variable region comprises a CDR1 of amino acid sequence SGDVLAKKSAR, a CDR2 of amino acid sequence KDSERPS and a CDR3 of amino acid sequence YSAADNNLV; and
wherein the HER2 antibody is trastuzumab,
wherein the tumor cells overexpresses αVβ6 and HER2.

7. The αVβ6 antibody for use of any one of claims 1, 3 or 5, or the HER2 antibody for use of any one of claims 2, 4 or 6, wherein the malignant tumor or tumor cells comprise tumor cells chosen from breast cancer cells, ovarian cancer cells, pancreatic cancer cells, lung cancer cells, colorectal cancer cells, head and neck cancer cells, oesophageal cancer cells, gastric cancer cells, or hepatocellular cancer cells.

8. The αVβ6 antibody for use or the HER2 antibody for use of claim 7, wherein said animal is human.

9. The αVβ6 antibody for use or the HER2 antibody for use of any one of claims 7 or 8, wherein the breast cancer cells or ovarian cancer cells are resistant to trastuzumab treatment.

**10.** The αVβ6 antibody for use of any one of claims 1, 3, 5 or 7-9, or the HER2 antibody for use of any one of claims 2, 4, or 6-9, wherein the αVβ6 antibody is a monoclonal antibody.

**11.** The αVβ6 antibody for use of any one of claims 1, 3, 5 or 7-10 or the HER2 antibody for use of any one of claims 2, 4 or 6-10, wherein the αVβ6 antibody comprises: a heavy chain variable region according to SEQ ID NO: 75 and a light chain variable region according to SEQ ID NO: 77.

**12.** The αVβ6 antibody for use of any one of claims 1, 3, 5 or 7-11 or the HER2 antibody for use of any one of claims 2, 4, or 6-11, wherein αVβ6 and HER2 are inhibited, optionally wherein the level of at least one of αVβ6, HER2, HER3, and B6 or at least one downstream target of αVβ6 and/or HER2, optionally Akt2 or Smad2, is downregulated, in the tumor or tumor cells.

**13.** The αVβ6 antibody for use of any one of claims 1, 3, 5 or 7-12 or the HER2 antibody for use of any one of claims 2, 4, or 6-12, wherein the αVβ6 antibody binds αVβ6 with a Kd of less than 35 nanomolar (nM), wherein binding affinity is determined by Biacore analysis or FACS.

## Patentansprüche

**1.** αVβ6-Antikörper zur Verwendung in einem Verfahren zum Behandeln eines bösartigen Tumors bei einem Tier, wobei der αVβ6-Antikörper gleichzeitig mit einem HER2-Antikörper oder darauffolgend verabreicht wird,
wobei der αVβ6-Antikörper eine Schwerkette und eine Leichtkette umfasst, wobei die variable Region der Schwerkette des αVβ6-Antikörpers eine CDR1 der Aminosäuresequenz GGSISSGGYYWS, eine CDR2 der Aminosäuresequenz YIYYSGRTYNNPSLKS und eine CDR3 der Aminosäuresequenz VATGRADYHFYAMDV umfasst, und wobei die variable Region der Leichtkette des αVβ6-Antikörpers eine CDR1 der Aminosäuresequenz SGDVLAKKSAR, eine CDR2 der Aminosäuresequenz KDSERPS und eine CDR3 der Aminosäuresequenz YSAADNNLV umfasst; und
wobei der HER2-Antikörper Trastuzumab ist,
wobei der bösartige Tumor αVβ6 und HER2 überexprimiert.

**2.** HER2-Antikörper zur Verwendung in einem Verfahren zum Behandeln eines bösartigen Tumors bei einem Tier, wobei der HER2-Antikörper gleichzeitig mit einem αVβ6-Antikörper oder darauffolgend verabreicht wird,
wobei der αVβ6-Antikörper eine Schwerkette und eine Leichtkette umfasst, wobei die variable Region der Schwerkette des αVβ6-Antikörpers eine CDR1 der Aminosäuresequenz GGSISSGGYYWS, eine CDR2 der Aminosäuresequenz YIYYSGRTYNNPSLKS und eine CDR3 der Aminosäuresequenz VATGRADYHFYAMDV umfasst, und wobei die variable Region der Leichtkette des αVβ6-Antikörpers eine CDR1 der Aminosäuresequenz SGDVLAKKSAR, eine CDR2 der Aminosäuresequenz KDSERPS und eine CDR3 der Aminosäuresequenz YSAADNNLV umfasst; und
wobei der HER2-Antikörper Trastuzumab ist,
wobei der bösartige Tumor αVβ6 und HER2 überexprimiert.

**3.** αVβ6-Antikörper zur Verwendung in einem Verfahren durch Hemmen des Wachstums von Tumorzellen bei einem Tier, wobei der αVβ6-Antikörper gleichzeitig mit einem HER2-Antikörper oder darauffolgend verabreicht wird,
wobei der αVβ6-Antikörper eine Schwerkette und eine Leichtkette umfasst, wobei die variable Region der Schwerkette des αVβ6-Antikörpers eine CDR1 der Aminosäuresequenz GGSISSGGYYWS, eine CDR2 der Aminosäuresequenz YIYYSGRTYNNPSLKS und eine CDR3 der Aminosäuresequenz VATGRADYHFYAMDV umfasst, und wobei die variable Region der Leichtkette des αVβ6-Antikörpers eine CDR1 der Aminosäuresequenz SGDVLAKKSAR, eine CDR2 der Aminosäuresequenz KDSERPS und eine CDR3 der Aminosäuresequenz YSAADNNLV umfasst; und
wobei der HER2-Antikörper Trastuzumab ist,
wobei die Tumorzellen αVβ6 und HER2 überexprimieren.

**4.** HER2-Antikörper zur Verwendung in einem Verfahren durch Hemmen des Wachstums von Tumorzellen bei einem Tier, wobei der HER2-Antikörper gleichzeitig mit einem αVβ6-Antikörper oder darauffolgend verabreicht wird,
wobei der αVβ6-Antikörper eine Schwerkette und eine Leichtkette umfasst, wobei die variable Region der Schwerkette des αVβ6-Antikörpers eine CDR1 der Aminosäuresequenz GGSISSGGYYWS, eine CDR2 der Aminosäuresequenz YIYYSGRTYNNPSLKS und eine CDR3 der Aminosäuresequenz VATGRADYHFYAMDV umfasst, und wobei die variable Region der Leichtkette des αVβ6-Antikörpers eine CDR1 der Aminosäuresequenz SGDVLAKKSAR, eine CDR2 der Aminosäuresequenz KDSERPS und eine CDR3 der Aminosäuresequenz YSAADNNLV umfasst; und
wobei der HER2-Antikörper Trastuzumab ist,
wobei die Tumorzellen αVβ6 und HER2 überexprimieren.

**5.** αVβ6-Antikörper zur Verwendung in einem Verfahren durch Unterdrücken des Wachstums von Trastuzumabresistenten Tumorzellen bei einem Tier, wobei der αVβ6-Antikörper gleichzeitig mit einem HER2-Antikörper oder darauffolgend verabreicht wird,
wobei der αVβ6-Antikörper eine Schwerkette und eine Leichtkette umfasst, wobei die variable Region der Schwerkette des αVβ6-Antikörpers eine CDR1 der Aminosäuresequenz GGSISSGGYYWS, eine CDR2 der Aminosäuresequenz YIYYSGRTYNNPSLKS und eine CDR3 der Aminosäuresequenz VATGRADYHFYAMDV umfasst, und wobei die variable Region der Leichtkette des αVβ6-Antikörpers eine CDR1 der Aminosäuresequenz SGDVLAKKSAR, eine CDR2 der Aminosäuresequenz KDSERPS und eine CDR3 der Aminosäuresequenz YSAADNNLV umfasst; und
wobei der HER2-Antikörper Trastuzumab ist,
wobei die Tumorzellen αVβ6 und HER2 überexprimieren.

**6.** HER2-Antikörper zur Verwendung in einem Verfahren durch Unterdrücken des Wachstums von Trastuzumabresistenten Tumorzellen bei einem Tier, wobei der HER2-Antikörper gleichzeitig mit einem αVβ6-Antikörper oder darauffolgend verabreicht wird,
wobei der αVβ6-Antikörper eine Schwerkette und eine Leichtkette umfasst, wobei die variable Region der Schwerkette des αVβ6-Antikörpers eine CDR1 der Aminosäuresequenz GGSISSGGYYWS, eine CDR2 der Aminosäuresequenz YIYYSGRTYNNPSLKS und eine CDR3 der Aminosäuresequenz VATGRADYHFYAMDV umfasst, und wobei die variable Region der Leichtkette des αVβ6-Antikörpers eine CDR1 der Aminosäuresequenz SGDVLAKKSAR, eine CDR2 der Aminosäuresequenz KDSERPS und eine CDR3 der Aminosäuresequenz YSAADNNLV umfasst; und
wobei der HER2-Antikörper Trastuzumab ist,
wobei die Tumorzellen αVβ6 und HER2 überexprimieren.

**7.** αVβ6-Antikörper zur Verwendung nach einem der Ansprüche 1, 3 oder 5 oder HER2-Antikörper zur Verwendung nach einem der Ansprüche 2, 4 oder 6, wobei der bösartige Tumor oder die Tumorzellen Tumorzellen umfassen, ausgewählt aus Brustkrebszellen, Eierstockkrebszellen, Bauchspeicheldrüsenkrebszellen, Lungenkrebszellen, Darmkrebszellen, Kopf- und Halskrebszellen, Speiseröhrenkrebszellen, Magenkrebszellen und hepatozellulärer Krebszellen.

**8.** αVβ6-Antikörper zur Verwendung oder HER2-Antikörper zur Verwendung nach Anspruch 7, wobei das Tier ein Mensch ist.

**9.** αVβ6-Antikörper zur Verwendung oder HER2-Antikörper zur Verwendung nach einem der Ansprüche 7 oder 8, wobei die Brustkrebszellen oder Eierstockkrebszellen gegen Behandlung mit Trastuzumab resistent sind.

**10.** αVβ6-Antikörper zur Verwendung nach einem der Ansprüche 1, 3, 5 oder 7-9 oder HER2-Antikörper zur Verwendung nach einem der Ansprüche 2, 4 oder 6-9, wobei der αVβ6-Antikörper ein monoklonaler Antikörper ist.

**11.** αVβ6-Antikörper zur Verwendung nach einem der Ansprüche 1, 3, 5 oder 7-10 oder HER2-Antikörper zur Verwendung nach einem der Ansprüche 2, 4 oder 6-10, wobei der αVβ6-Antikörper eine variable Region der Schwerkette gemäß SEQ ID NO: 75 und eine variable Region der Leichtkette gemäß SEQ ID NO: 77 umfasst.

**12.** αVβ6-Antikörper zur Verwendung nach einem der Ansprüche 1, 3, 5 oder 7-11 oder HER2-Antikörper zur Verwendung nach einem der Ansprüche 2, 4 oder 6-11, wobei αVβ6 und HER2 gehemmt werden, optional wobei der Spiegel von mindestens einem von αVβ6, HER2, HER3 und B6 oder mindestens einem stromabwärts gelegenen Ziel von αVβ6 und/oder HER2, optional Akt2 oder Smad2, in dem Tumor oder in den Tumorzellen herunterreguliert ist.

**13.** αVβ6-Antikörper zur Verwendung nach einem der Ansprüche 1, 3, 5 oder 7-12 oder HER2-Antikörper zur Verwendung nach einem der Ansprüche 2, 4 oder 6-12, wobei der αVβ6-Antikörper αVβ6 mit einem Kd von weniger als 35 Nanomolar (nM) bindet, wobei die Bindungsaffinität durch Biacore-Analyse oder FACS bestimmt wird.

## Revendications

1. Anticorps d'αVβ6 destiné à être utilisé dans un procédé de traitement d'une tumeur maligne chez un animal, l'anticorps d'αVβ6 étant administré simultanément ou séquentiellement avec un anticorps de HER2,
l'anticorps d'αVβ6 comprenant une chaîne lourde et une chaîne légère, dans lequel la région variable de chaîne lourde de l'anticorps d'αVβ6 comprend une CDR1 de séquence d'acides aminés GGSISSGGYYWS, une CDR2 de séquence d'acides aminés YIYYSGRTYNNPSLKS et une CDR3 de séquence d'acides aminés VATGRADYHFYAMDV, et dans lequel la région variable de chaîne légère de l'anticorps d'αVβ6 comprend une CDR1 de séquence d'acides aminés SGDVLAKKSAR, une CDR2 de séquence d'acides aminés KDSERPS et une CDR3 de séquence d'acides aminés YSAADNNLV ; et
dans lequel l'anticorps de HER2 est le trastuzumab, dans lequel la tumeur maligne surexprime les αVβ6 et HER2.

2. Anticorps de HER2 destiné à être utilisé dans un procédé de traitement d'une tumeur maligne chez un animal, l'anticorps de HER2 étant administré simultanément ou séquentiellement avec un anticorps d'αVβ6,
dans lequel l'anticorps d'αVβ6 comprend une chaîne lourde et une chaîne légère, dans lequel la région variable de chaîne lourde de l'anticorps d'αVβ6 comprend une CDR1 de séquence d'acides aminés GGSISSGGYYWS, une CDR2 de séquence d'acides aminés YIYYSGRTYNNPSLKS et une CDR3 de séquence d'acides aminés VATGRADYHFYAMDV, et dans lequel la région variable de chaîne légère de l'anticorps d'αVβ6 comprend une CDR1 de séquence d'acides aminés SGDVLAKKSAR, une CDR2 de séquence d'acides aminés KDSERPS et une CDR3 de séquence d'acides aminés YSAADNNLV ; et
l'anticorps de HER2 étant le trastuzumab,
dans lequel la tumeur maligne surexprime les αVβ6 et HER2.

3. Anticorps d'αVβ6 destiné à être utilisé dans un procédé de traitement par une inhibition de la croissance de cellules tumorales chez un animal, l'anticorps d'αVβ6 étant administré simultanément ou séquentiellement avec un anticorps de HER2,
l'anticorps d'αVβ6 comprenant une chaîne lourde et une chaîne légère, dans lequel la région variable de chaîne lourde de l'anticorps d'αVβ6 comprend une CDR1 de séquence d'acides aminés GGSISSGGYYWS, une CDR2 de séquence d'acides aminés YIYYSGRTYNNPSLKS et une CDR3 de séquence d'acides aminés VATGRADYHFYAMDV, et dans lequel la région variable de chaîne légère de l'anticorps d'αVβ6 comprend une CDR1 de séquence d'acides aminés SGDVLAKKSAR, une CDR2 de séquence d'acides aminés KDSERPS et une CDR3 de séquence d'acides aminés YSAADNNLV ; et
dans lequel l'anticorps de HER2 est le trastuzumab, dans lequel les cellules tumorales surexpriment les αVβ6 et HER2.

4. Anticorps de HER2 destiné à être utilisé dans un procédé de traitement par une inhibition de la croissance de cellules tumorales chez un animal, l'anticorps de HER2 étant administré simultanément ou séquentiellement avec un anticorps d'αVβ6,
dans lequel l'anticorps d'αVβ6 comprend une chaîne lourde et une chaîne légère, dans lequel la région variable de chaîne lourde de l'anticorps d'αVβ6 comprend une CDR1 de séquence d'acides aminés GGSISSGGYYWS, une CDR2 de séquence d'acides aminés YIYYSGRTYNNPSLKS et une CDR3 de séquence d'acides aminés VATGRADYHFYAMDV, et dans lequel la région variable de chaîne légère de l'anticorps d'αVβ6 comprend une CDR1 de séquence d'acides aminés SGDVLAKKSAR, une CDR2 de séquence d'acides aminés KDSERPS et une CDR3 de séquence d'acides aminés YSAADNNLV ; et
l'anticorps de HER2 étant le trastuzumab,
dans lequel les cellules tumorales surexpriment les αVβ6 et HER2.

5. Anticorps d'αVβ6 destiné à être utilisé dans un procédé de traitement par une suppression de la croissance de cellules tumorales résistantes au trastuzumab chez un animal, l'anticorps d'αVβ6 étant administré simultanément ou séquentiellement avec un anticorps de HER2,
l'anticorps d'αVβ6 comprenant une chaîne lourde et une chaîne légère, dans lequel la région variable de chaîne lourde de l'anticorps d'αVβ6 comprend une CDR1 de séquence d'acides aminés GGSISSGGYYWS, une CDR2 de séquence d'acides aminés YIYYSGRTYNNPSLKS et une CDR3 de séquence d'acides aminés VATGRADYHFYAMDV, et dans lequel la région variable de chaîne légère de l'anticorps d'αVβ6 comprend une CDR1 de séquence d'acides aminés SGDVLAKKSAR, une CDR2 de séquence d'acides aminés KDSERPS et une CDR3 de séquence d'acides aminés YSAADNNLV ; et
dans lequel l'anticorps de HER2 est le trastuzumab,
dans lequel les cellules tumorales surexpriment les αVβ6 et HER2.

6. Anticorps de HER2 destiné à être utilisé dans un procédé de traitement par une suppression de la croissance de cellules tumorales résistantes au trastuzumab chez un animal, l'anticorps de HER2 étant administré simultanément ou séquentiellement avec un anticorps d'αVβ6,
dans lequel l'anticorps d'αVβ6 comprend une chaîne lourde et une chaîne légère, dans lequel la région variable de chaîne lourde de l'anticorps d'αVβ6 comprend une CDR1 de séquence d'acides aminés GGSISSGGYYWS, une CDR2 de séquence d'acides aminés YIYYSGRTYNNPSLKS et une CDR3 de séquence d'acides aminés VATGRADYHFYAMDV, et dans lequel la région variable de chaîne légère de l'anticorps d'αVβ6 comprend une CDR1 de séquence d'acides aminés SGDVLAKKSAR, une CDR2 de séquence d'acides aminés KDSERPS et une CDR3 de séquence d'acides aminés YSAADNNLV ; et
l'anticorps de HER2 étant le trastuzumab,
dans lequel les cellules tumorales surexpriment les αVβ6 et HER2.

7. Anticorps d'αVβ6 destiné à l'utilisation de l'une quelconque des revendications 1, 3 ou 5 ou bien anticorps de HER2 destiné à l'utilisation de l'une quelconque des revendications 2, 4 ou 6, dans lequel la tumeur maligne ou les cellules tumorales comprend/comprennent des cellules tumorales choisies parmi des cellules d'un cancer du sein, cellules d'un cancer de l'ovaire, cellules d'un cancer du pancréas, cellules d'un cancer du poumon, cellules d'un cancer colorectal, cellules d'un cancer de la tête et du cou, cellules d'un cancer de l'œsophage, cellules d'un cancer gastrique ou cellules d'un carcinome hépatocellulaire.

8. Anticorps d'αVβ6 destiné à l'utilisation ou anticorps de HER2 destiné à l'utilisation de la revendication 7, dans lequel ledit animal est humain.

9. Anticorps d'αVβ6 destiné à l'utilisation ou anticorps de HER2 destiné à l'utilisation de l'une quelconque des revendications 7 ou 8, dans lequel les cellules d'un cancer du sein ou les cellules d'un cancer de l'ovaire sont résistantes à un traitement par trastuzumab.

10. Anticorps d'αVβ6 destiné à l'utilisation de l'une quelconque des revendications 1, 3, 5 ou 7 à 9, ou bien anticorps de HER2 destiné à l'utilisation de l'une quelconque des revendications 2, 4 ou 6 à 9, l'anticorps d'αVβ6 étant un anticorps monoclonal.

11. Anticorps d'αVβ6 destiné à l'utilisation de l'une quelconque des revendications 1, 3, 5 ou 7 à 10, ou bien anticorps de HER2 destiné à l'utilisation de l'une quelconque des revendications 2, 4 ou 6 à 10, l'anticorps d'αVβ6 comprenant : une région variable de chaîne lourde selon la SEQ ID n° : 75 et une région variable de chaîne légère selon la SE ID n° : 77.

12. Anticorps d'αVβ6 destiné à l'utilisation de l'une quelconque des revendications 1, 3, 5 ou 7 à 11, ou bien anticorps de HER2 destiné à l'utilisation de l'une quelconque des revendications 2, 4 ou 6 à 11, dans lequel les αVβ6 et HER2 sont inhibés, en option dans lequel le taux d'au moins l'un d'entre les αVβ6, HER2, HER3 et B6 ou d'au moins une cible en aval des αVβ6 et/ou HER2, en option Akt2 ou Smad2, est régulé en baisse dans la tumeur ou les cellules tumorales.

13. Anticorps d'αVβ6 destiné à l'utilisation de l'une quelconque des revendications 1, 3, 5 ou 7 à 12, ou bien anticorps de HER2 destiné à l'utilisation de l'une quelconque des revendications 2, 4 ou 6 à 12, l'anticorps d'αVβ6 se liant au αVβ6 avec une Kd inférieure à 35 nanomolaire (nM), dans lequel l'affinité de liaison est déterminée par une analyse Biacore ou une FACS.

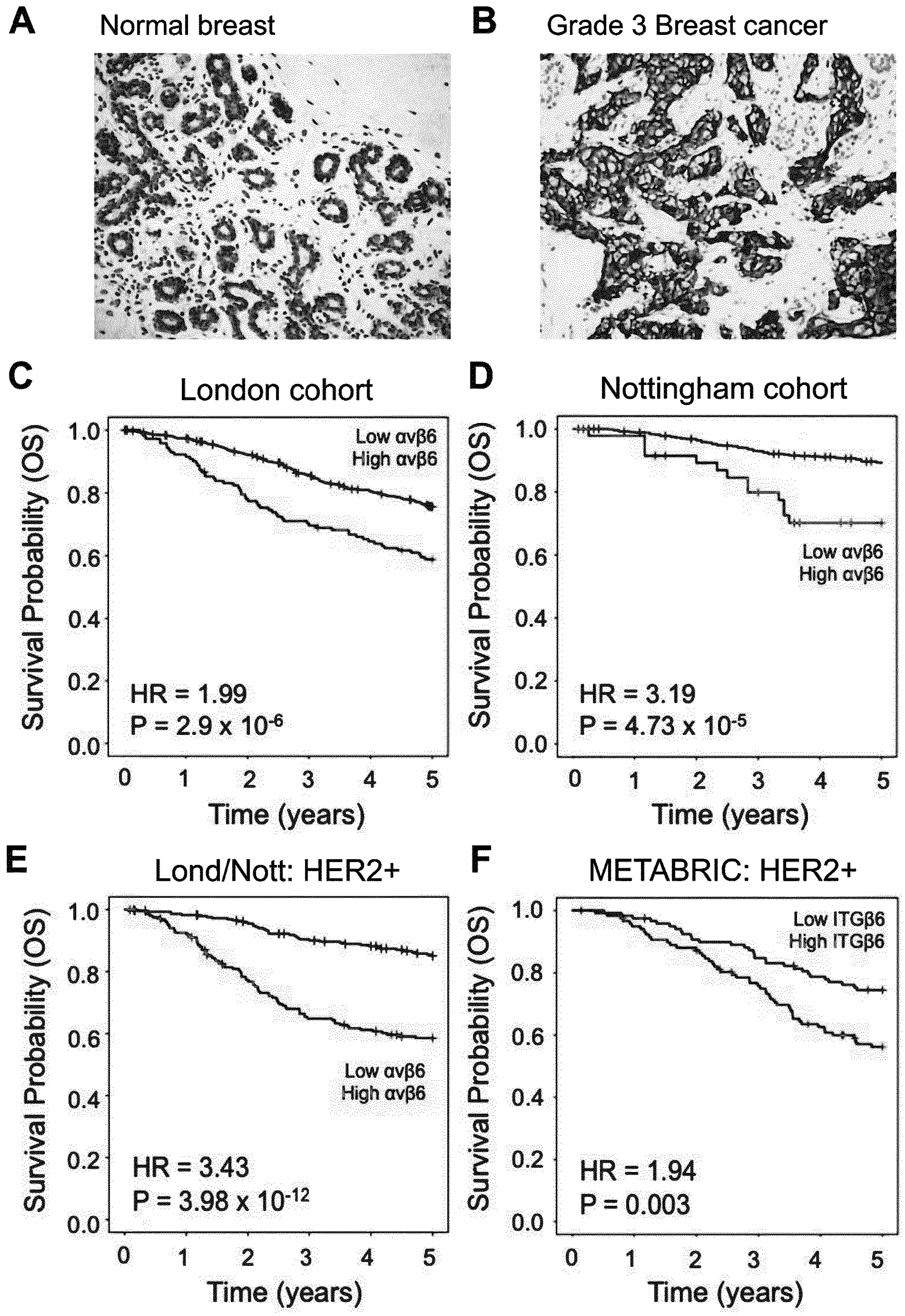
A
Normal breast
B
Grade 3 Breast cancer
C
London cohort
Low αvβ6
High αvβ6
HR = 1.99
P = 2.9 x 10-6
Survival Probability (OS)
Time (years)
D
Nottingham cohort
Low αvβ6
High αvβ6
HR = 3.19
P = 4.73 x 10-5
E
Lond/Nott: HER2+
Low αvβ6
High αvβ6
HR = 3.43
P = 3.98 x 10-12
F
METABRIC: HER2+
Low ITGβ6
High ITGβ6
HR = 1.94
P = 0.003

Figure 1

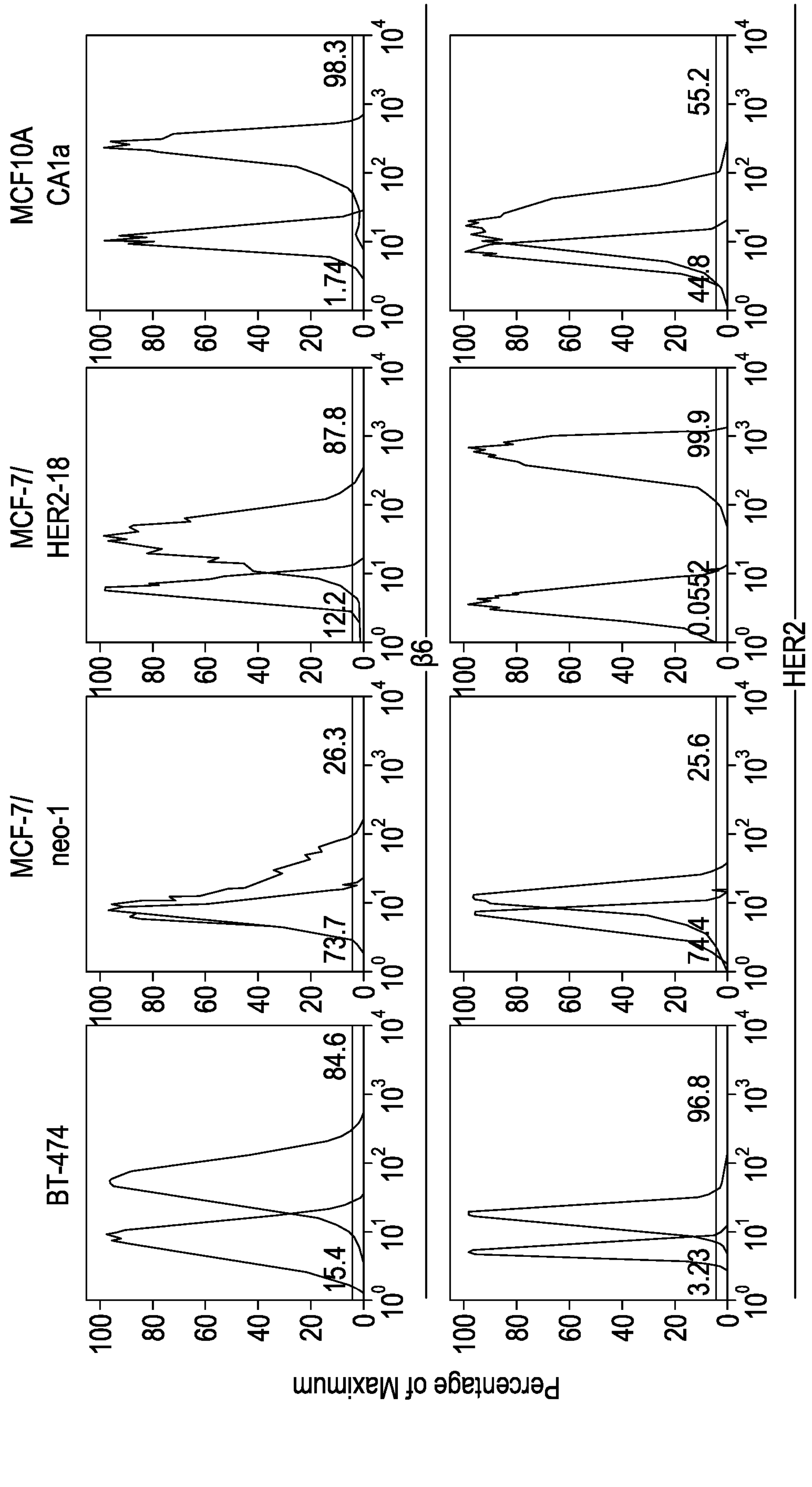
BT-474
MCF-7/
neo-1
MCF-7/
HER2-18
MCF10A
CA1a
Percentage of Maximum
15.4
84.6
73.7
26.3
12.2
87.8
1.74
98.3
3.23
96.8
74.4
25.6
0.0552
99.9
44.8
55.2
β6
HER2

Figure 2A

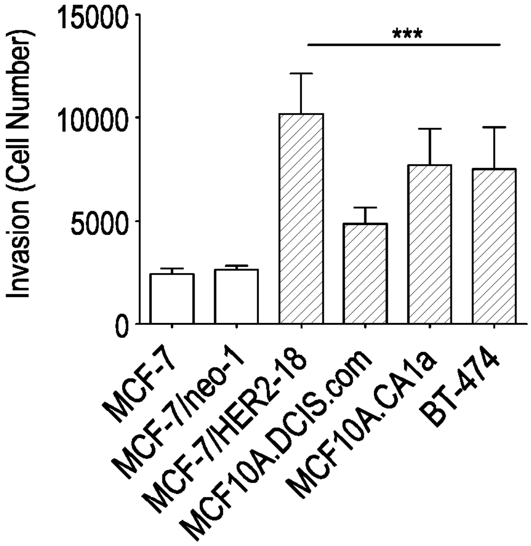


Figure 2B

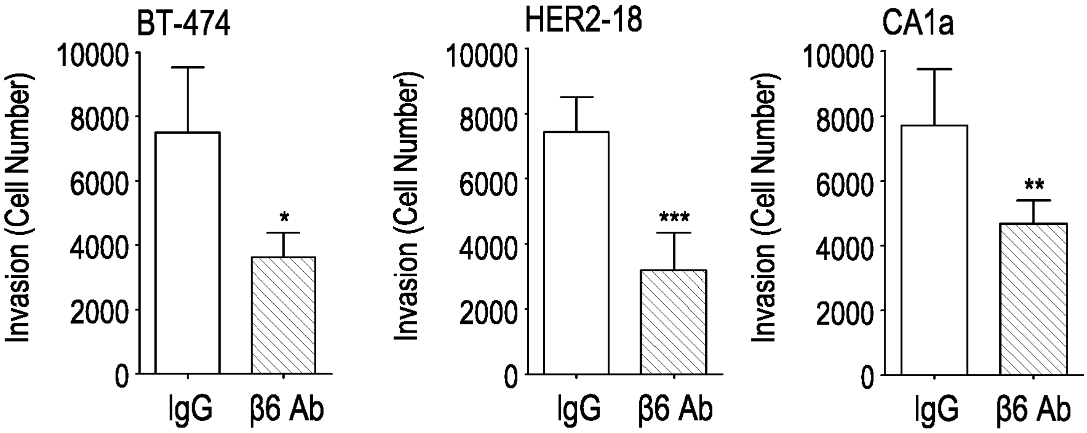


Figure 2C

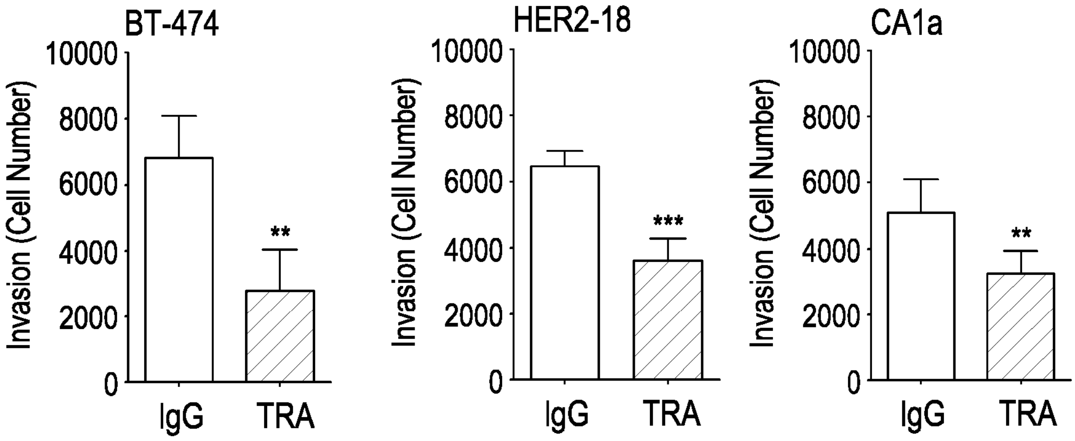


Figure 2D

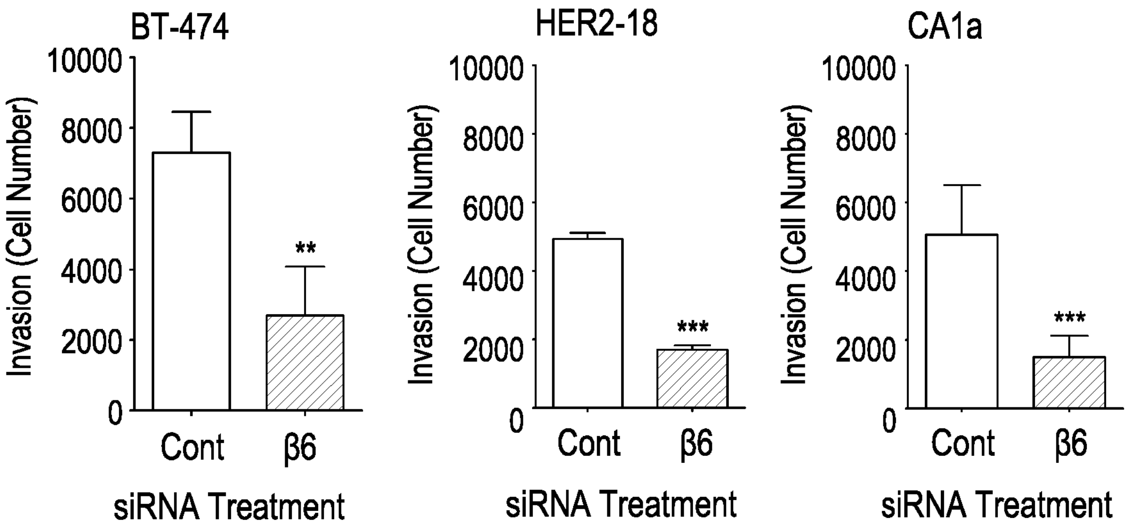


Figure 2E

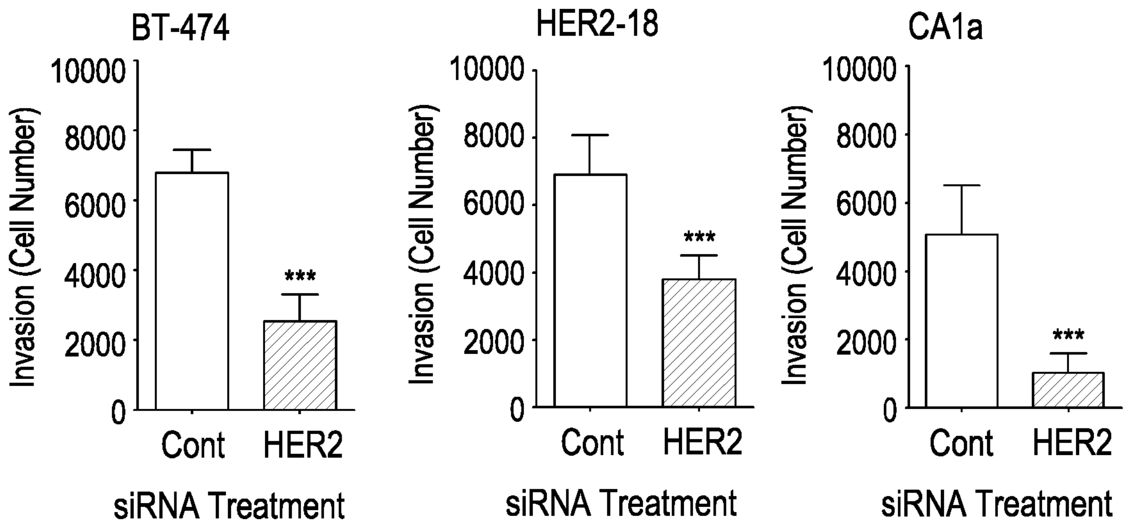


Figure 2F

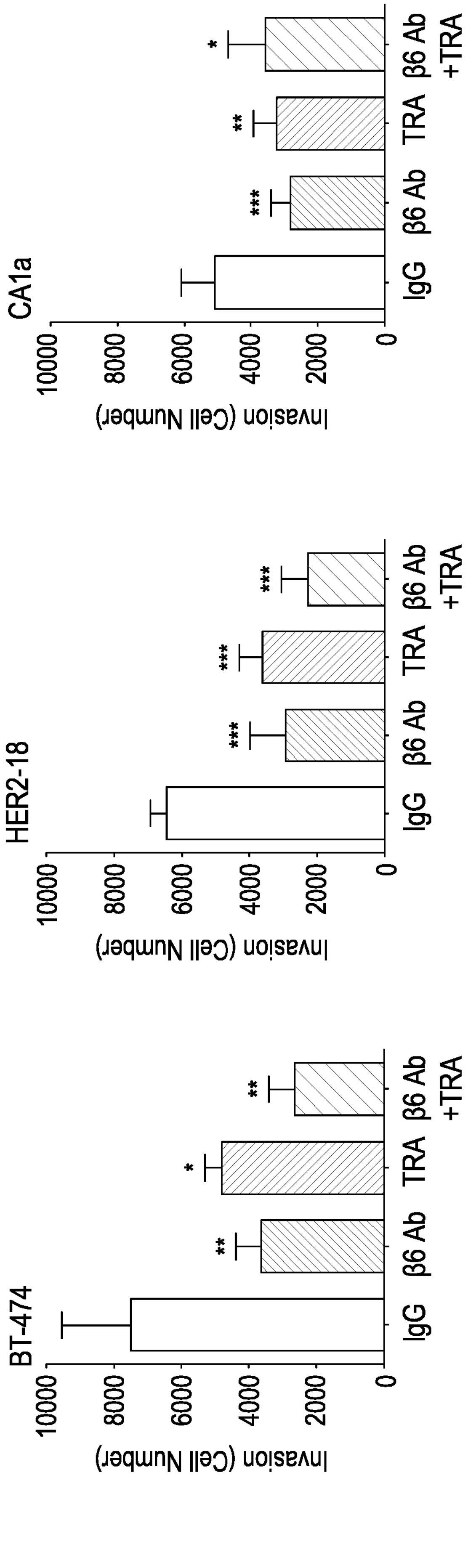
BT-474
HER2-18
CA1a
Invasion (Cell Number)
10000
8000
6000
4000
2000
0
IgG
β6 Ab
TRA
β6 Ab +TRA
*
**
***

Figure 2G

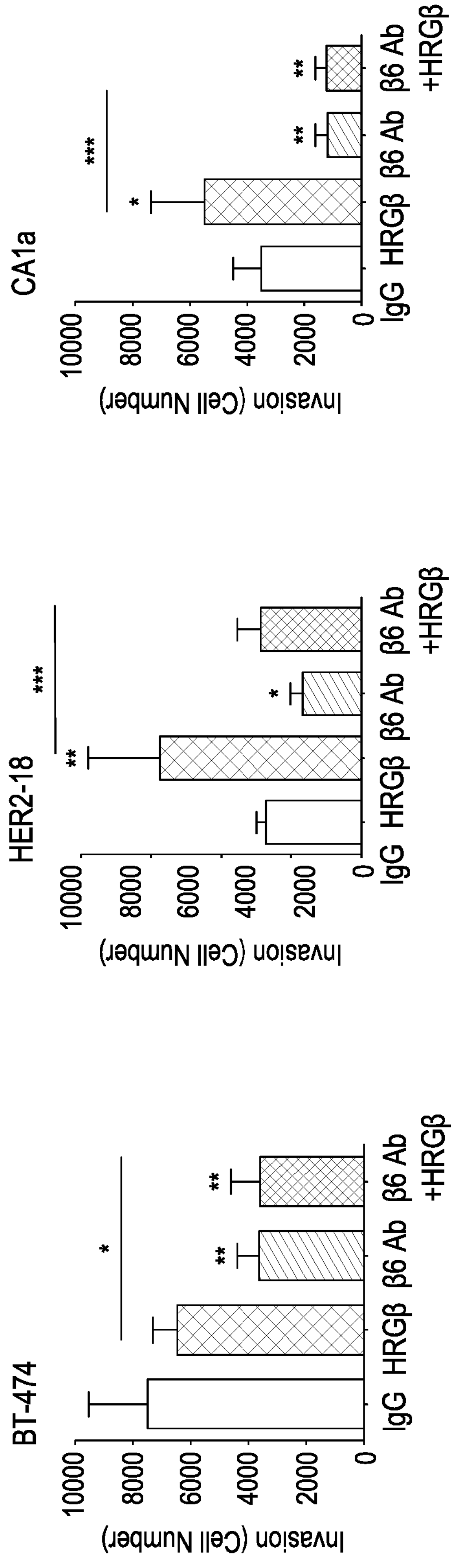


Figure 3A

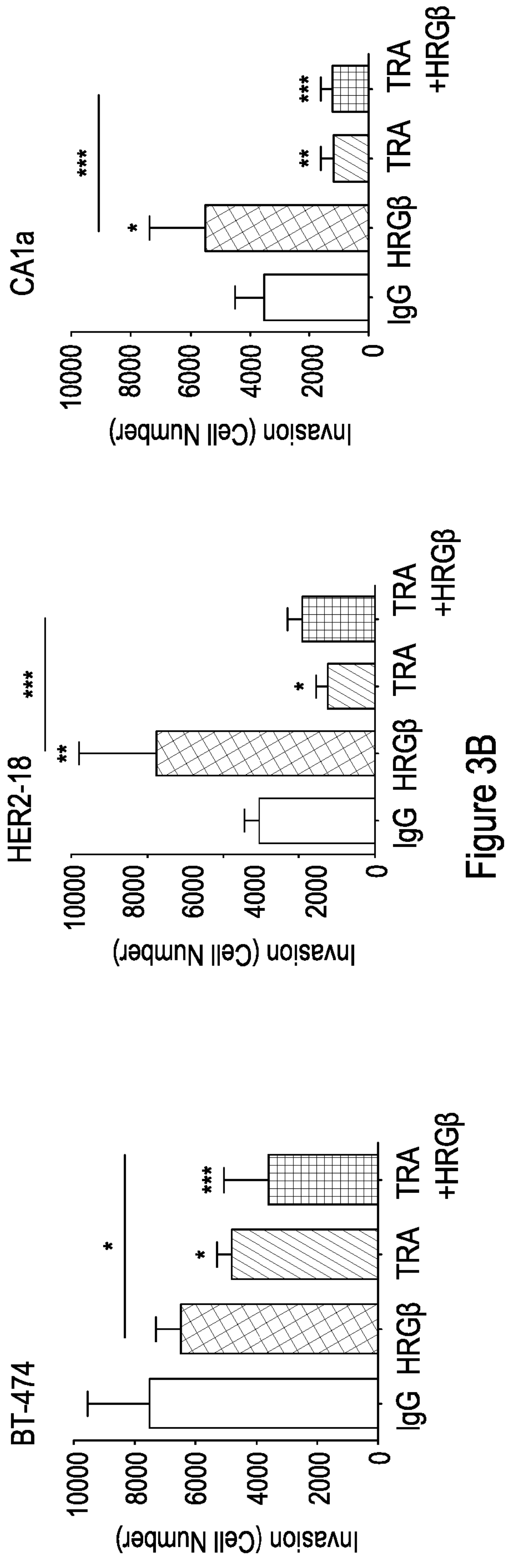


Figure 3B

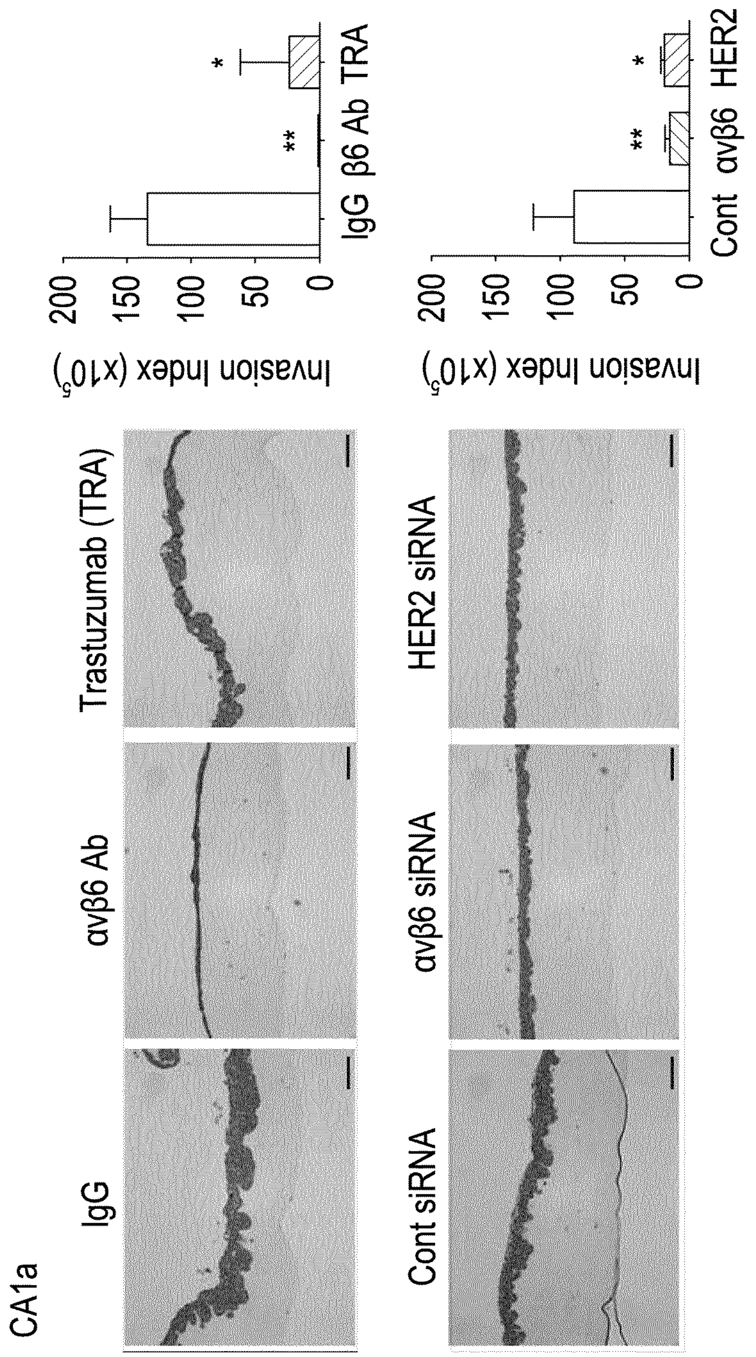
CA1a
IgG
αvβ6 Ab
Trastuzumab (TRA)
Cont siRNA
αvβ6 siRNA
HER2 siRNA
Invasion Index (x10⁵)
200
150
100
50
0
IgG β6 Ab TRA
**
*
Cont αvβ6 HER2


Figure 3C

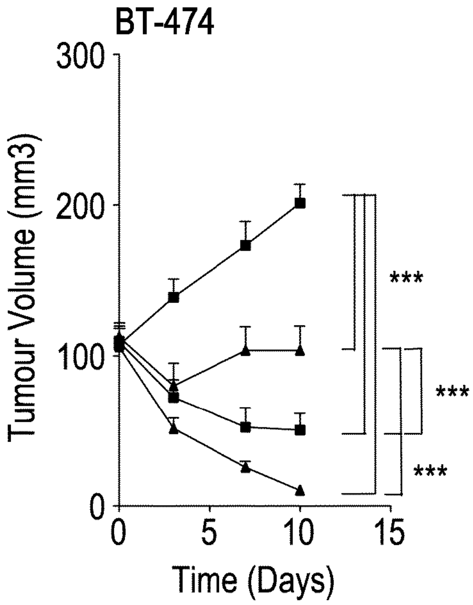


Figure 4A

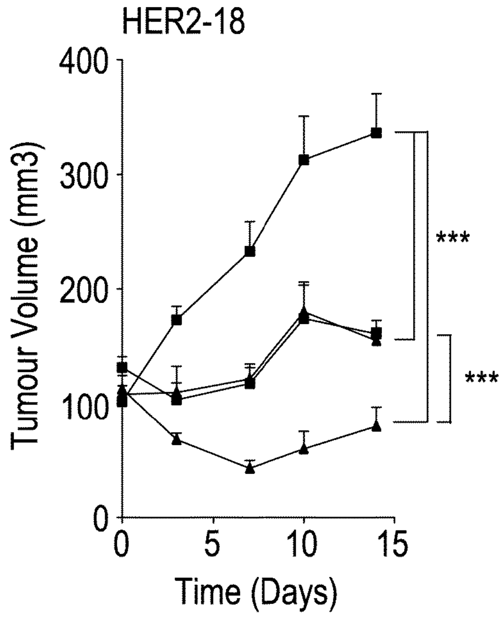


Figure 4B

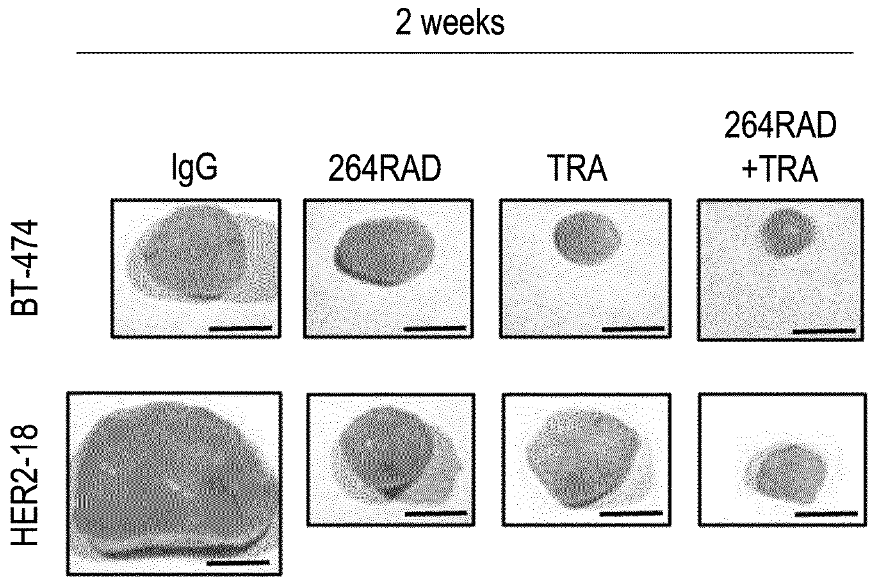


Figure 4C

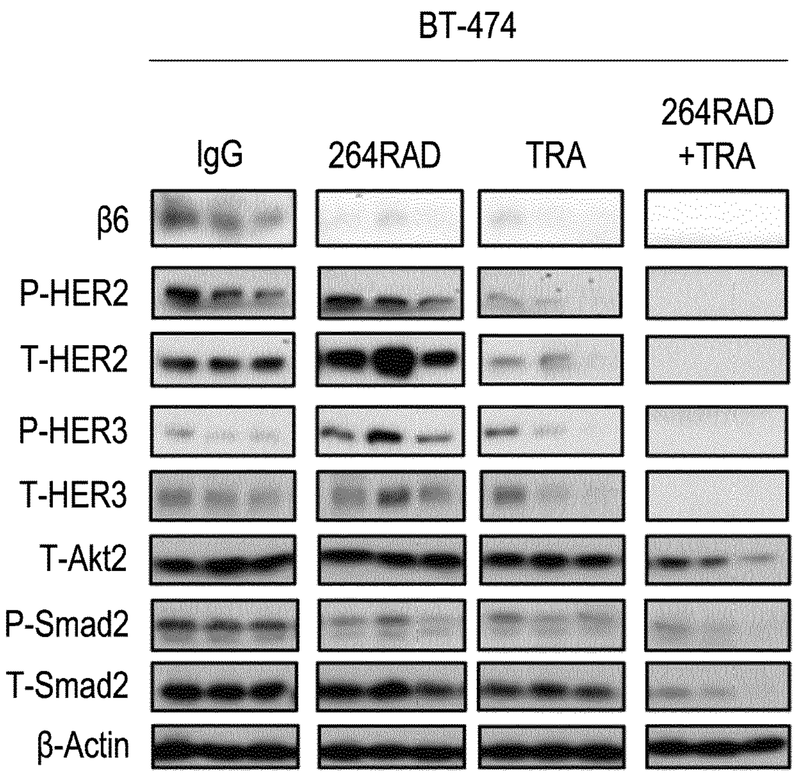
BT-474
IgG
264RAD
TRA
264RAD +TRA
β6
P-HER2
T-HER2
P-HER3
T-HER3
T-Akt2
P-Smad2
T-Smad2
β-Actin

Figure 4D

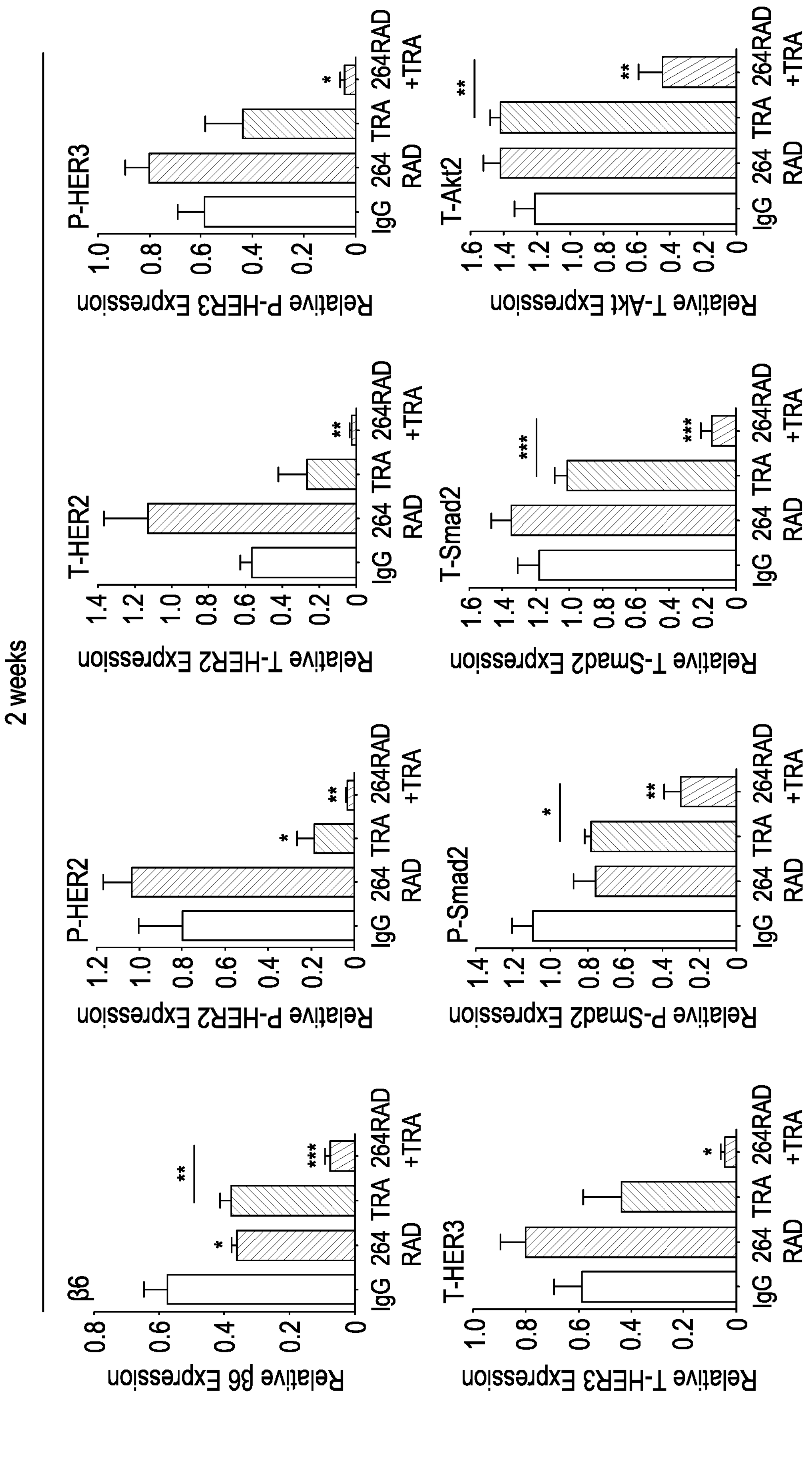
2 weeks
β6
Relative β6 Expression
P-HER2
Relative P-HER2 Expression
T-HER2
Relative T-HER2 Expression
P-HER3
Relative P-HER3 Expression
T-HER3
Relative T-HER3 Expression
P-Smad2
Relative P-Smad2 Expression
T-Smad2
Relative T-Smad2 Expression
T-Akt2
Relative T-Akt Expression
IgG
264 RAD
TRA
264RAD +TRA

Figure 4E

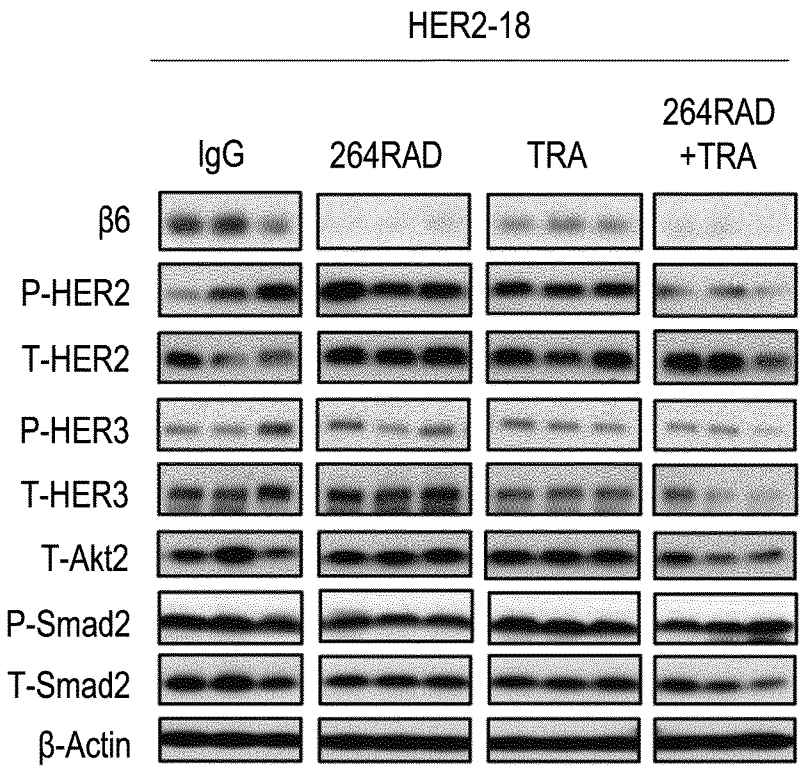
HER2-18
IgG
264RAD
TRA
264RAD
+TRA
β6
P-HER2
T-HER2
P-HER3
T-HER3
T-Akt2
P-Smad2
T-Smad2
β-Actin

Figure 4F

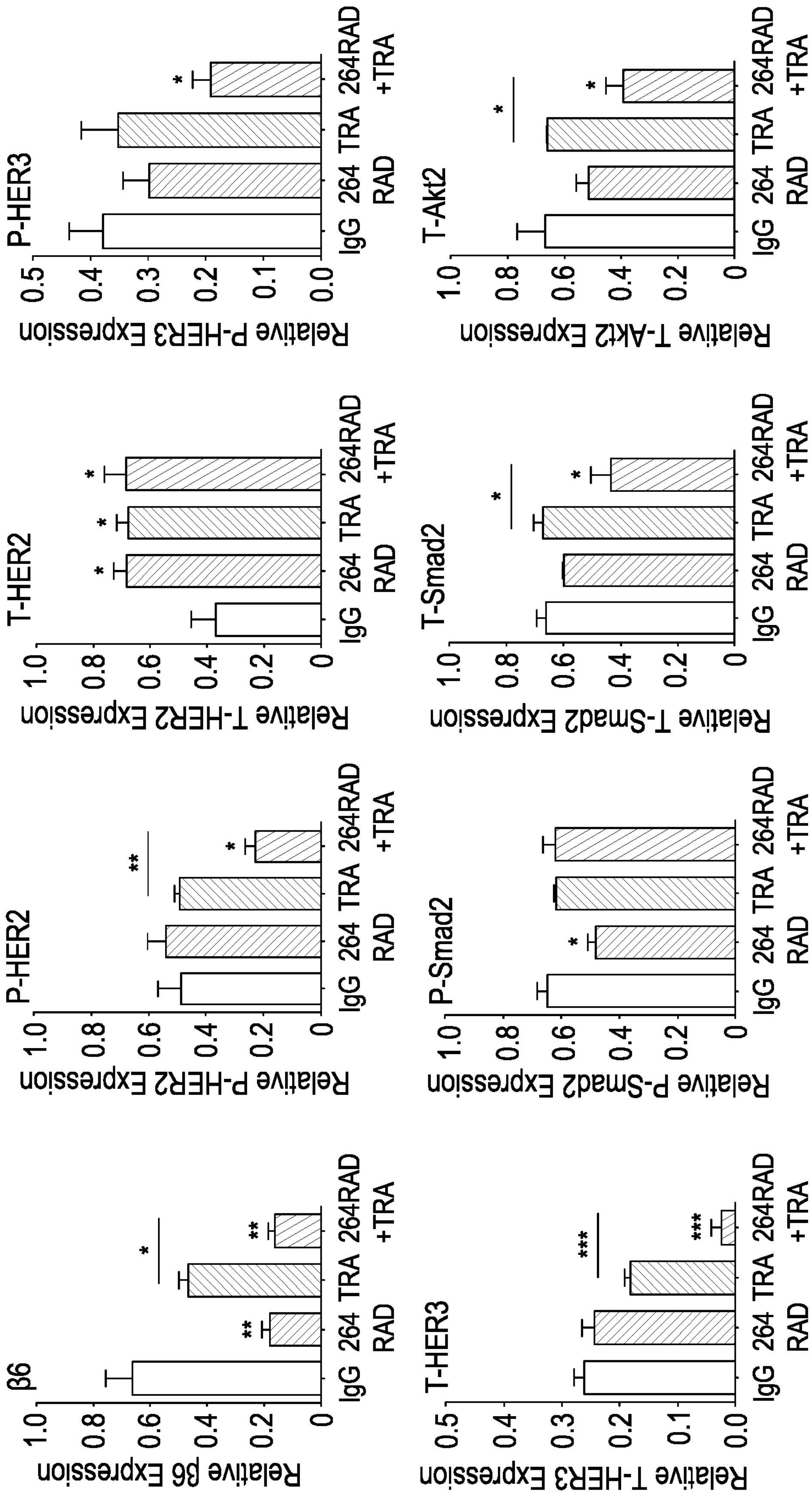
β6
Relative β6 Expression
1.0
0.8
0.6
0.4
0.2
0
IgG
264 RAD
TRA
264RAD +TRA
P-HER2
Relative P-HER2 Expression
T-HER2
Relative T-HER2 Expression
P-HER3
Relative P-HER3 Expression
0.5
0.4
0.3
0.2
0.1
0.0
T-HER3
Relative T-HER3 Expression
P-Smad2
Relative P-Smad2 Expression
T-Smad2
Relative T-Smad2 Expression
T-Akt2
Relative T-Akt2 Expression
*
**
***

Figure 4G

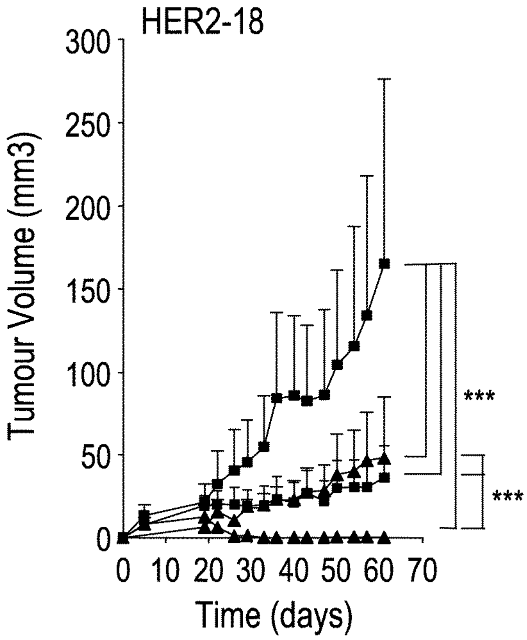


Figure 5A

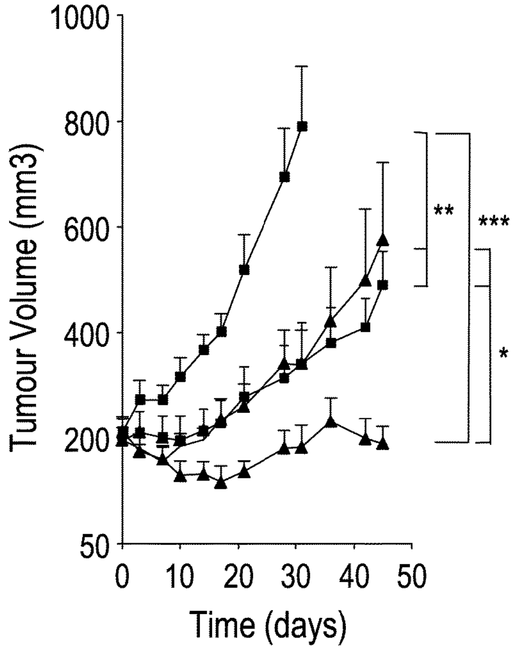


Figure 5B

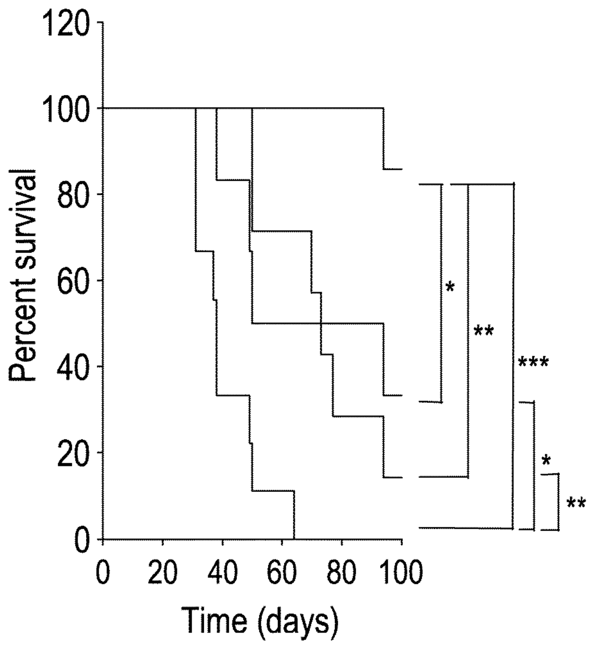


Figure 5C

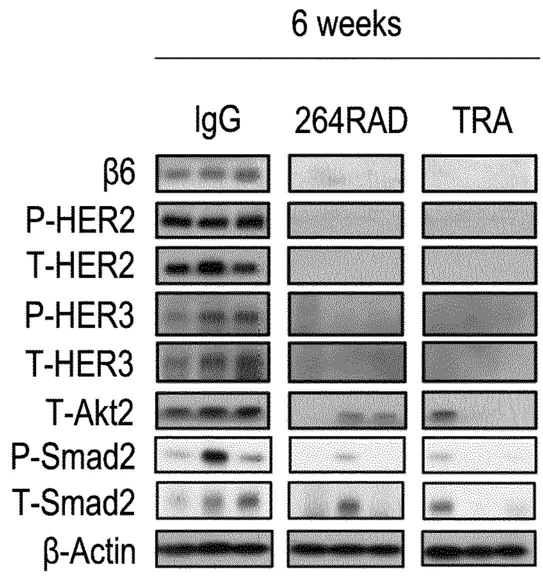


Figure 5D

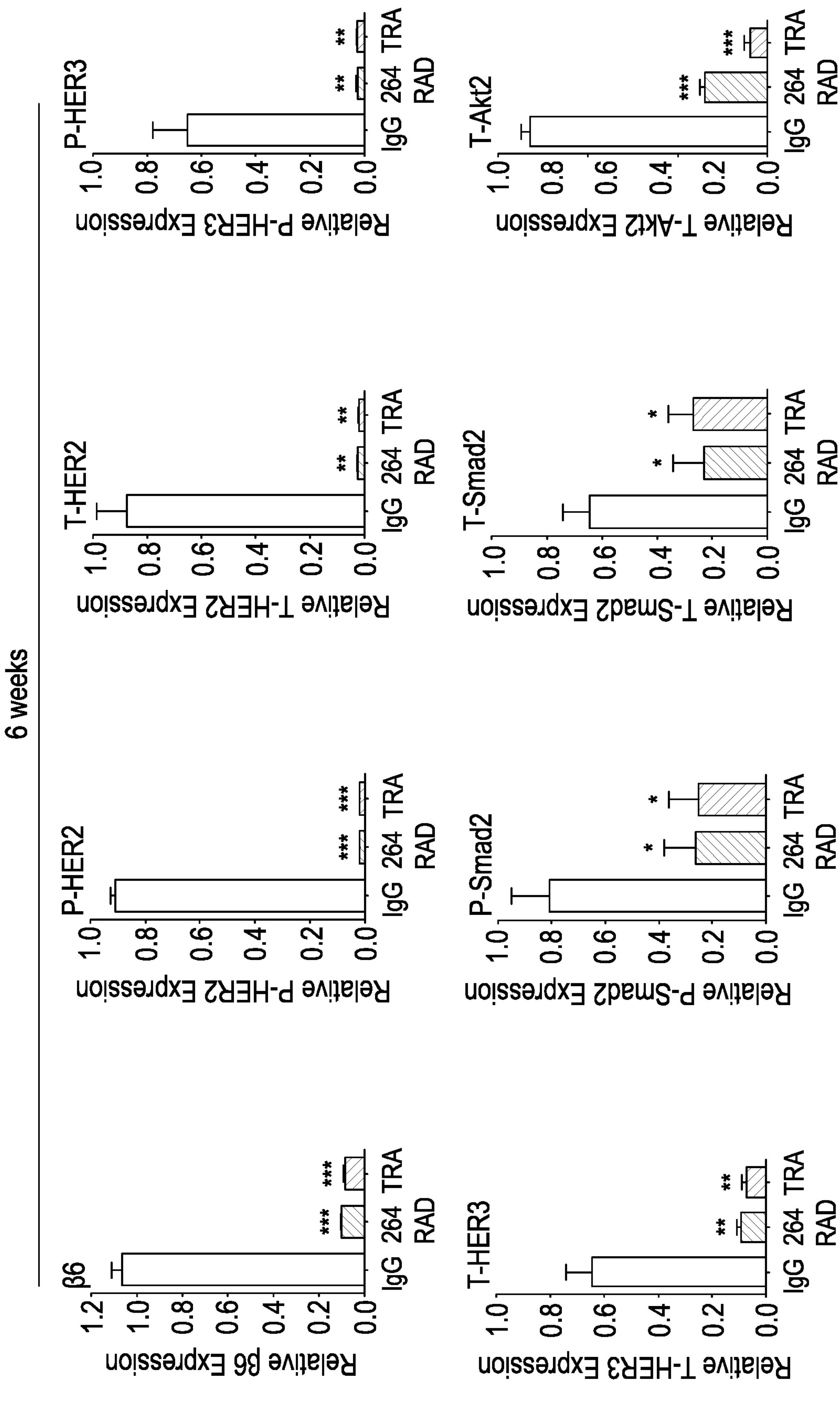
6 weeks
β6
Relative β6 Expression
P-HER2
Relative P-HER2 Expression
T-HER2
Relative T-HER2 Expression
P-HER3
Relative P-HER3 Expression
T-HER3
Relative T-HER3 Expression
P-Smad2
Relative P-Smad2 Expression
T-Smad2
Relative T-Smad2 Expression
T-Akt2
Relative T-Akt2 Expression
IgG 264 TRA
RAD

Figure 5E

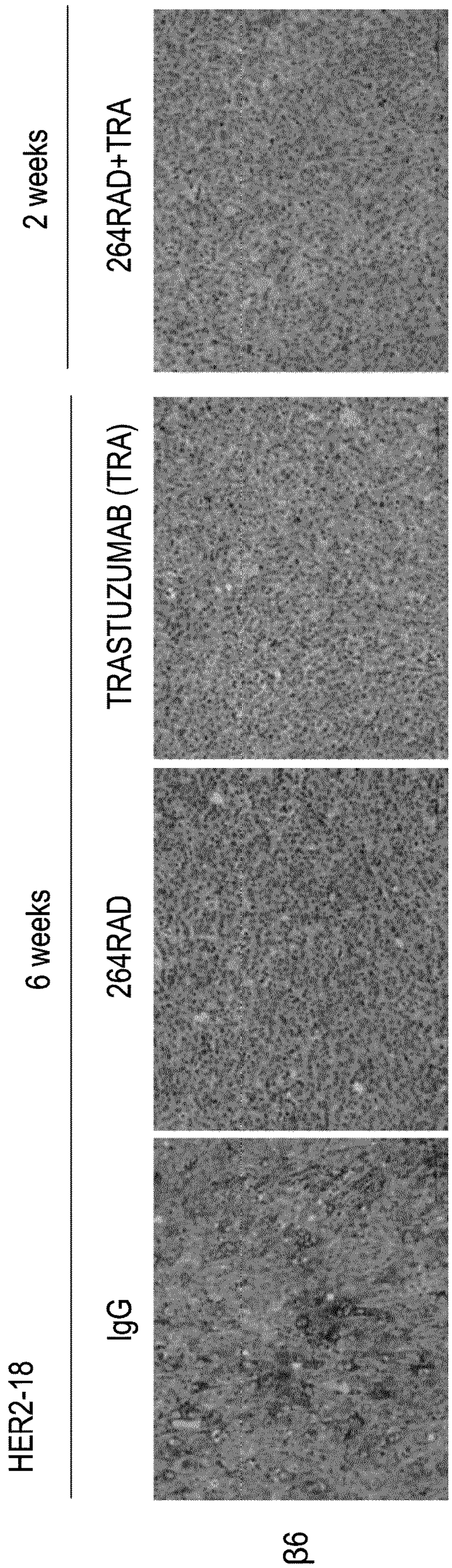
HER2-18
6 weeks
2 weeks
IgG
264RAD
TRASTUZUMAB (TRA)
264RAD+TRA
β6

Figure 5F

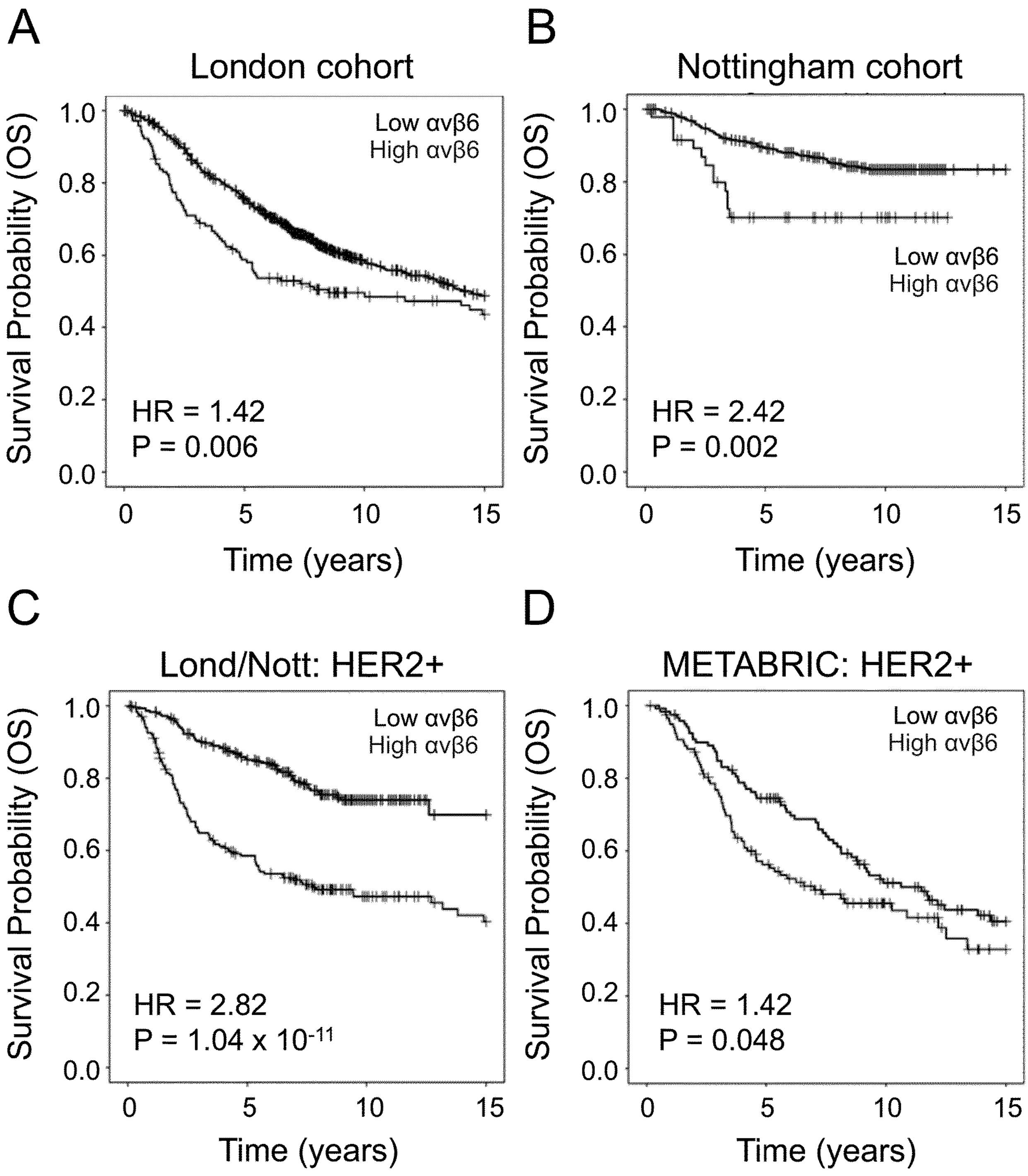
A
London cohort
Low αvβ6
High αvβ6
HR = 1.42
P = 0.006
Survival Probability (OS)
Time (years)
B
Nottingham cohort
Low αvβ6
High αvβ6
HR = 2.42
P = 0.002
Survival Probability (OS)
Time (years)
C
Lond/Nott: HER2+
Low αvβ6
High αvβ6
HR = 2.82
P = 1.04 x 10-11
Survival Probability (OS)
Time (years)
D
METABRIC: HER2+
Low αvβ6
High αvβ6
HR = 1.42
P = 0.048
Survival Probability (OS)
Time (years)

Figure 6

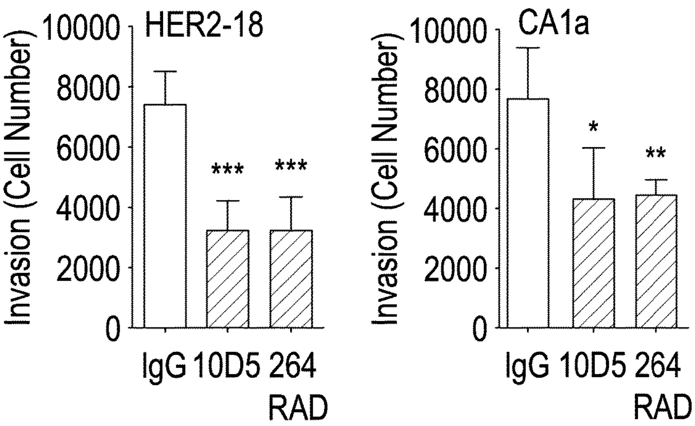


Figure 7A

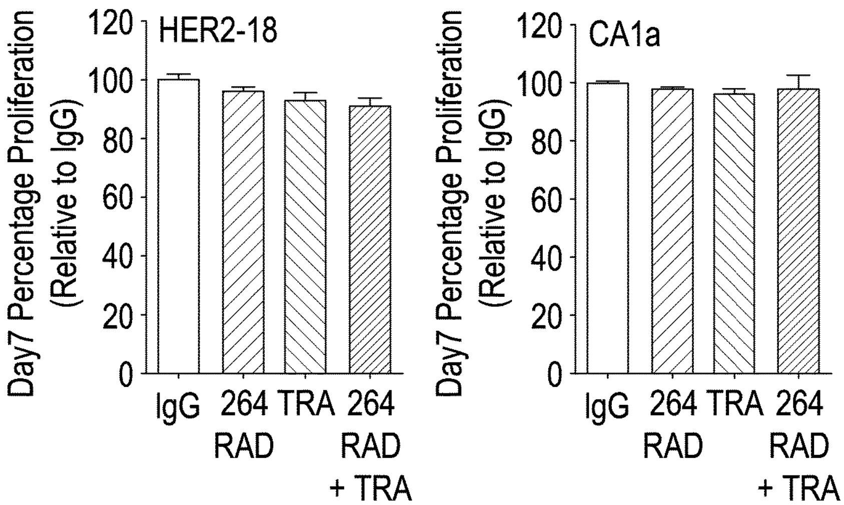


Figure 7B

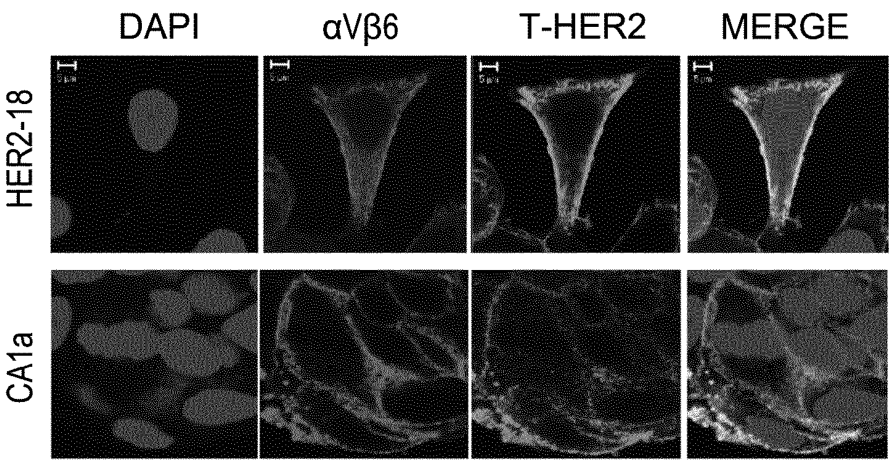


Figure 7C

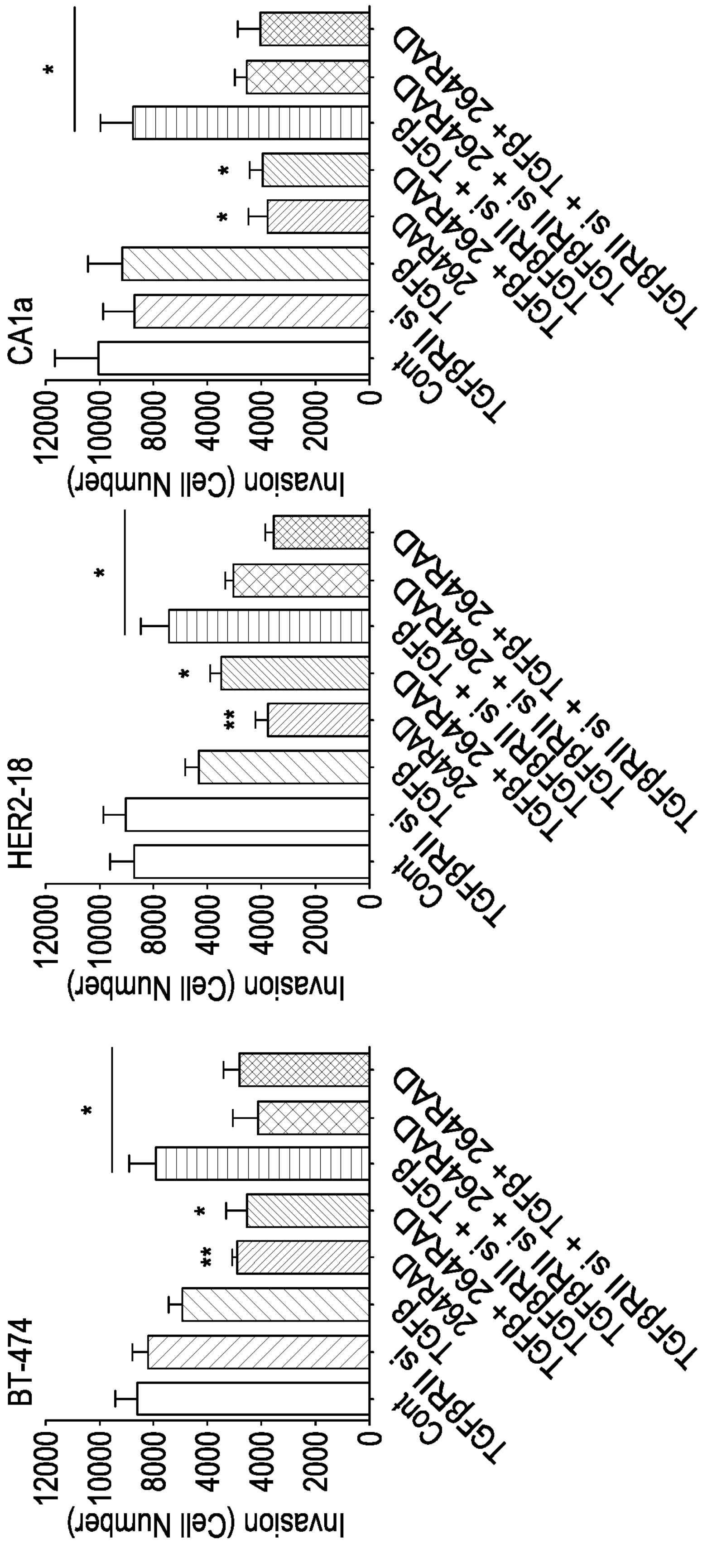
BT-474
HER2-18
CA1a
Invasion (Cell Number)
12000
10000
8000
4000
2000
0
Cont
TGFBRII si
TGFB
264RAD
TGFB+ 264RAD
TGFBRII si + TGFB
TGFBRII si + 264RAD
TGFBRII si + TGFB+ 264RAD
*
**

Figure 8

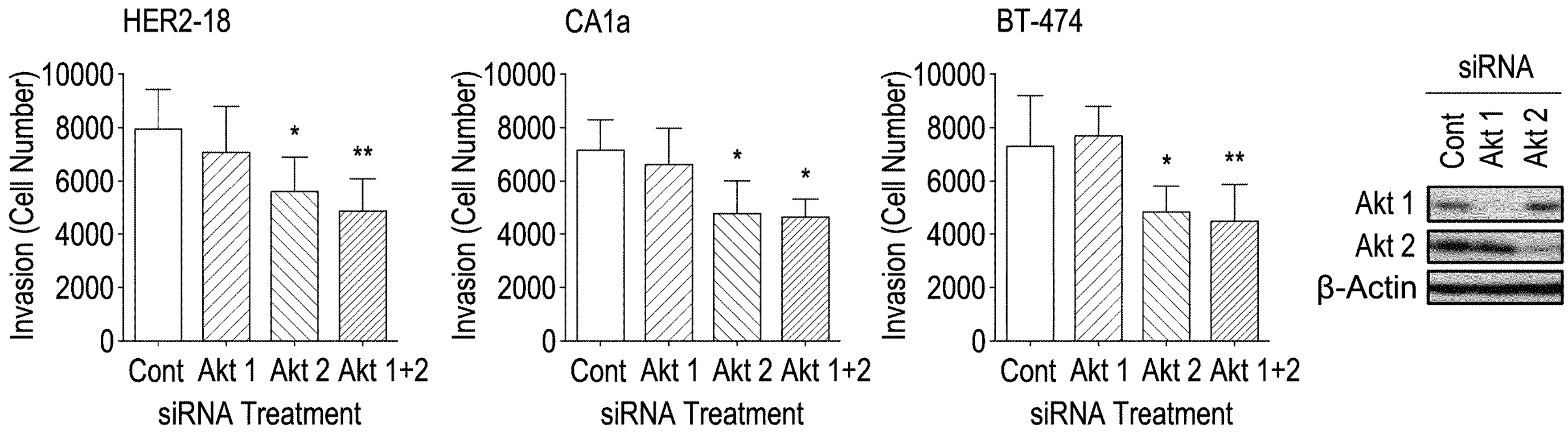


Figure 9A

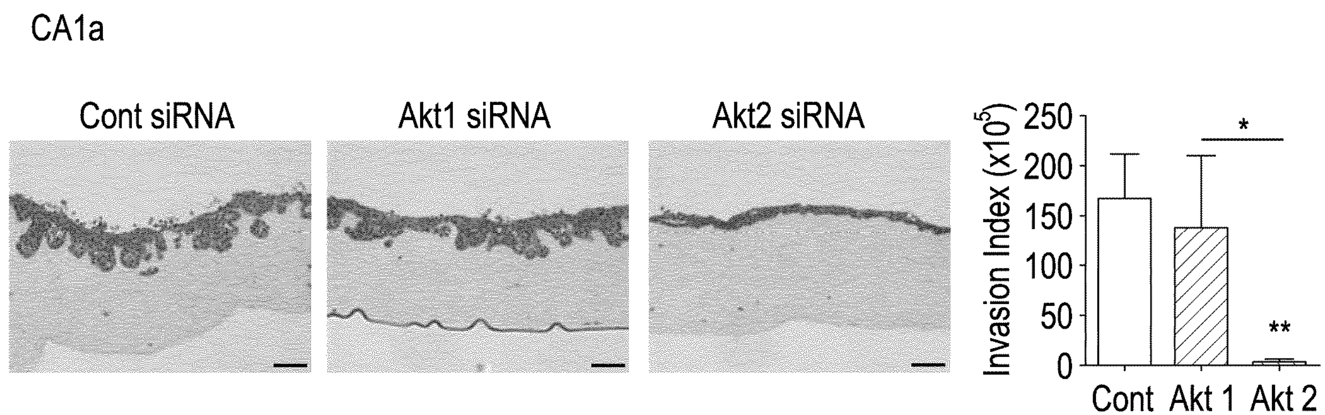


Figure 9B

| Clinico pathological features of the Nottingham and London cohorts of Breast Cancer Tissue Microarrays | | | | | |
|---|---|---|---|---|---|
| Factor | Category | Cases (% of total with info.) | Breast cancer deaths (% of cases) | Cases (% of total with info.) | Breast cancer deaths (% of cases) |
| | | Nottingham Cohort | | London Cohort | |
| Age (yrs) | ≤40 | 157 (9) | 54 (34) | 110 (10) | 58 (53) |
| | 41-50 | 501 (28) | 133 (27) | 248 (22) | 92 (37) |
| | 51-60 | 591 (33) | 154 (26) | 304 (27) | 144 (47) |
| | 61-70 | 528 (29) | 139 (26) | 278 (24) | 135 (49) |
| | 70+ | 14 (1) | 4 (29) | 190 (17) | 88 (46) |
| | Not known | 4 | 0 | 0 | 0 |
| Tumour size (mm) | ≤10 | 218 (12) | 26 (11) | 108 (10) | 20 (9) |
| | 11-20 | 905 (51) | 220 (24) | 213 (19) | 74 (35) |
| | 21-30 | 483 (27) | 164 (34) | 235 (22) | 85 (36) |
| | 31-50 | 146 (8) | 69 (47) | 360 (33) | 193 (54) |
| | >50 | 30 (2) | 0 (0) | 177 (16) | 125 (71) |
| | Not known | 13 | 2 | 37 | 20 |
| Node status | Negative | 987 (63) | 175 (18) | 440 (52) | 117 (27) |
| | Positive | 581 (37) | 215 (37) | 403 (48) | 189 (47) |
| | Not known | 227 | 94 | 287 | 211 |
| Grade | 1 | 330 (19) | 32 (10) | 151 (14) | 30 (20) |
| | 2 | 595 (33) | 125 (21) | 484 (45) | 220 (45) |
| | 3 | 857 (48) | 325 (38) | 451 (41) | 234 (52) |
| | Not known | 13 | 2 | 44 | 33 |
| ER status | Negative | 481 (29) | 165 (34) | 307 (32) | 164 (53) |
| | Positive | 1167 (71) | 285 (24) | 661 (68) | 301 (46) |
| | Not known | 147 | 34 | 162 | 52 |
| PR status | Negative | 735 (45) | 246 (33) | 484 (50) | 265 (55) |
| | Positive | 896 (55) | 201 (22) | 484 (50) | 197 (41) |
| | Not known | 164 | 484 | 162 | 55 |
| αvβ6 status | Negative/moderate | 1034 (83) | 277 (27) | 817 (85) | 393 (48) |
| | Strong | 207 (17) | 74 (36) | 142 (15) | 79 (56) |
| | Not known | 554 | 133 | 238 | 77 |
| Triple negative | No | 1256 (79) | 321 (26) | 719 (80) | 342 (48) |
| | Yes | 331 (21) | 113 (34) | 175 (20) | 99 (57) |
| | Not known | 208 | 50 | 303 | 108 |

Figure 10

| Factor | Category | αvβ6 -ve/moderate (%) | αvβ6 strong (%) | Significance |
|---|---|---|---|---|
| **Nottingham series** | | | | |
| **Tumour size (mm)** | ≤20 | 642 (63) | 116 (57) | p=0·1 |
| | >20 | 384 (37) | 89 (43) | |
| **Node status** | Negative | 549 (62) | 103 (58) | p=0·4 |
| | Positive | 341 (38) | 74 (42) | |
| **Grade** | 1 | 185 (18) | 33 (16) | p=0·006 |
| | 2 | 361 (35) | 52 (25) | |
| | 3 | 480 (47) | 120 (59) | |
| **ER status** | Negative | 241 (25) | 87 (46) | p<0·001 |
| | Positive | 713 (75) | 102 (54) | |
| **PR status** | Negative | 397 (42) | 113 (60) | p<0·001 |
| | Positive | 553 (58) | 76 (40) | |
| **HER2 status** | Negative | 931 (92) | 160 (78) | p<0·001 |
| | Positive | 81 (8) | 46 (22) | |
| **Triple-Neg/Basal** | Negative | 750 (81) | 138 (75) | p=0·05 |
| | Positive | 176 (19) | 47 (25) | |
| **London series** | | | | |
| **Tumour size (mm)** | ≤20 | 226 (29) | 28 (21) | p=0·06 |
| | >20 | 567 (71) | 107 (79) | |
| **Node status** | Negative | 312 (52) | 51 (52) | p=0·9 |
| | Positive | 290 (48) | 47 (48) | |
| **Grade** | 1 | 95 (12) | 3 (2) | p<0·001 |
| | 2 | 407 (51) | 57 (41) | |
| | 3 | 289 (37) | 80 (57) | |
| **ER status** | Negative | 196 (26) | 80 (63) | p<0·001 |
| | Positive | 545 (74) | 48 (37) | |
| **PR status** | Negative | 330 (45) | 106 (83) | p<0·001 |
| | Positive | 406 (55) | 21 (17) | |
| **HER2 status** | Negative | 671 (92) | 81 (63) | p<0·001 |
| | Positive | 55 (8) | 48 (37) | |
| **Triple-Neg/Basal** | Negative | 581 (83) | 86 (68) | p<0·001 |
| | Positive | 121 (17) | 40 (32) | |

Figure 11

| Molecular Subtype[1] | Cell Line | Receptor Status | Invasive Propensity[2] | Expression[3] αvB6 | Expression[3] HER2 |
|---|---|---|---|---|---|
| Normal breast epithelium | MCF10A | | - | ++++ | - |
| Basal A | BT-20 | TN | ++ | ++++ | - |
| | HCC1937 | TN | ND | ++++ | - |
| | HCC1954 | ER-, PR-, HER2+ | +++ | ++++ | ++++ |
| | MDA MB-468 | TN | ++++ | ++++ | - |
| Basal B | HCC38 | TN | ++ | ++++ | - |
| | HS578T | TN | ++ | - | - |
| | MCF10AT1k.c12 | ER+ | ND | ++++ | - |
| | MCF10A.neo.T | ER+, | + | ++++ | - |
| | MCF10A.CA1a | ER+ | ++++ | ++++ | +++ |
| | MCF10A.CA1h | ER+, | ND | ++++ | - |
| | MCF10A.DCIS.com | ER+ | ++++ | ++++ | +++ |
| | SUM159 | ER-, PR- | +++ | ++++ | - |
| | GI-101 | ER+ | ++++ | ++++ | - |
| Luminal | BT-474 | ER+, PR+, HER2+ | +++ | ++++ | ++++ |
| | MCF-7 | ER+, PR+ | - | - | + |
| | MCF-7/neo-1 | ER+, PR+ | - | - | + |
| | MCF-7/HER2-18 | ER+, PR+, HER2+ | ++++ | ++++ | ++++ |
| | SKBR-3 | ER-, PR- | ND | ++++ | ++++ |
| | ZR-75 | ER+, PR+ | ND | - | ++ |

Figure 12

| Target | Antibody Name/Catalogue Number | Supplier |
|---|---|---|
| **αvβ6** | Anti-integrin αvβ6 Antibody, clone 10D5 (#MAB2077Z) | Millipore |
| **αvβ6** | 264RAD | Oncology iMED, AstraZeneca |
| **αvβ6** | 6.2G2 | Stromedix Inc |
| **αvβ6** | Integrin Beta 6 (C-19) | Santa Cruz |
| **Total Akt (pan, 1, 2, 3) & Phospho-Akt** | Akt (pan) (C67E7) (#4691)<br>Akt1 (C73H10) (#2938)<br>Akt 2 (D6G4) (#3063)<br>Akt 3 (62A8) (#3788)<br>Phospho-Akt (Ser473) (D9E) XP (#4060) & Phospho-Akt (Thr308) (#9275) | Cell Signaling Technology (CST) |
| **β-Actin** | β-Actin (13E5) (#4970) | CST |
| **Total & Phospho-HER2** | HER2/ErbB2 (29D8) (#2165)<br>Phospho-HER2/ErbB2 (Tyr1221/1222) (6B12) (#2243) | CST |
| **Total & Phospho-HER3** | HER3/ErbB3 (1B2E) (#4754)<br>Phospho-HER3/ErbB3 (Tyr1289) (21D3) (#4791) | CST |
| **Total & Phospho-Smad2** | Smad2 (D43B4) (#5339), Phospho-Smad2 (Ser465/467) (#3101) | CST |

Figure 13

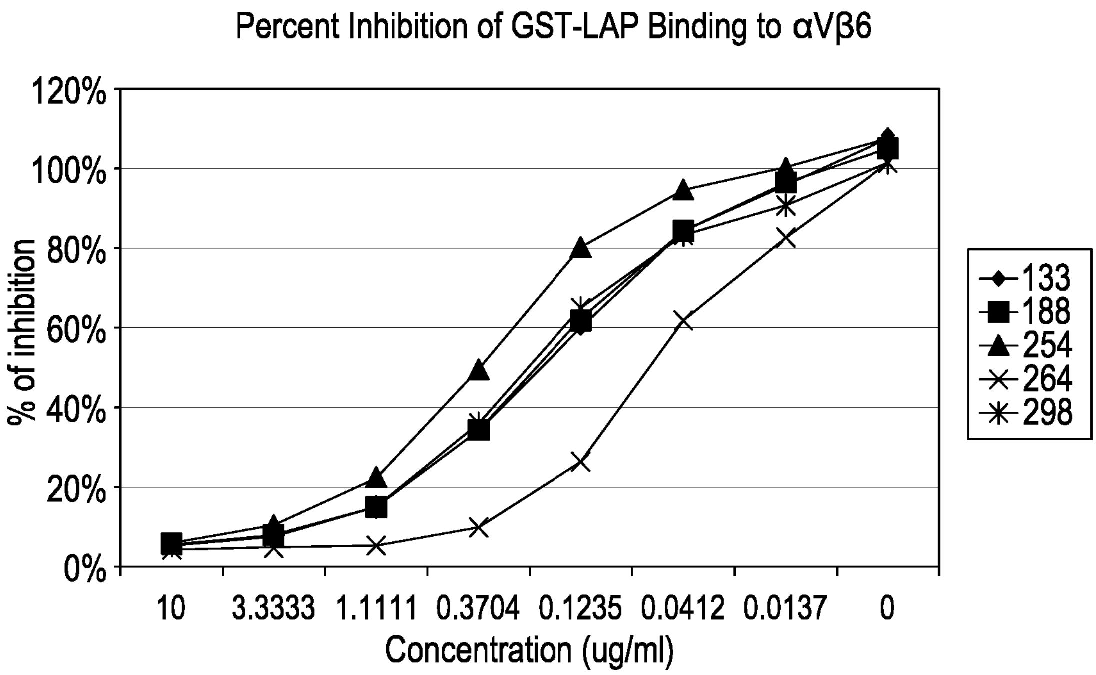
Percent Inhibition of GST-LAP Binding to αVβ6
120%
100%
80%
60%
40%
20%
0%
% of inhibition
10
3.3333
1.1111
0.3704
0.1235
0.0412
0.0137
0
Concentration (ug/ml)
133
188
254
264
298

Figure 14

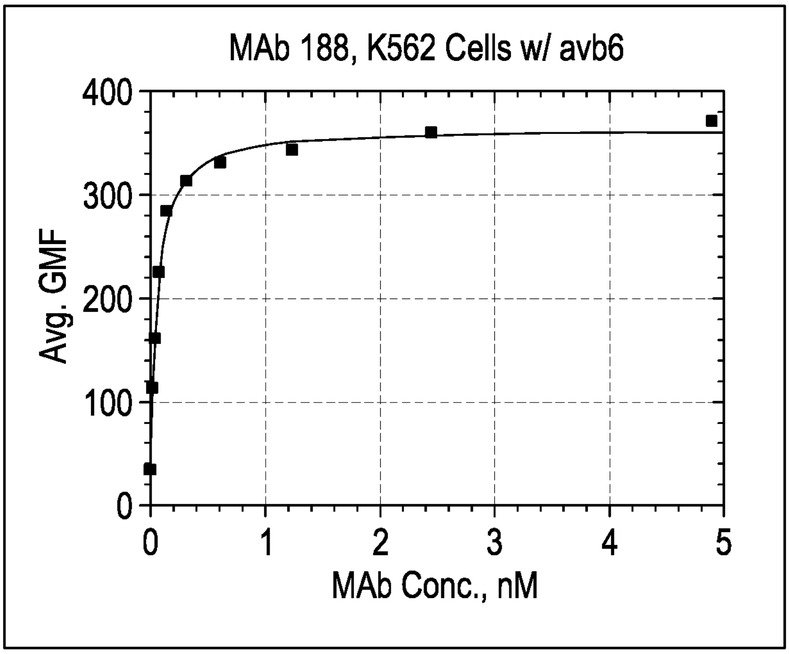


Figure 15A

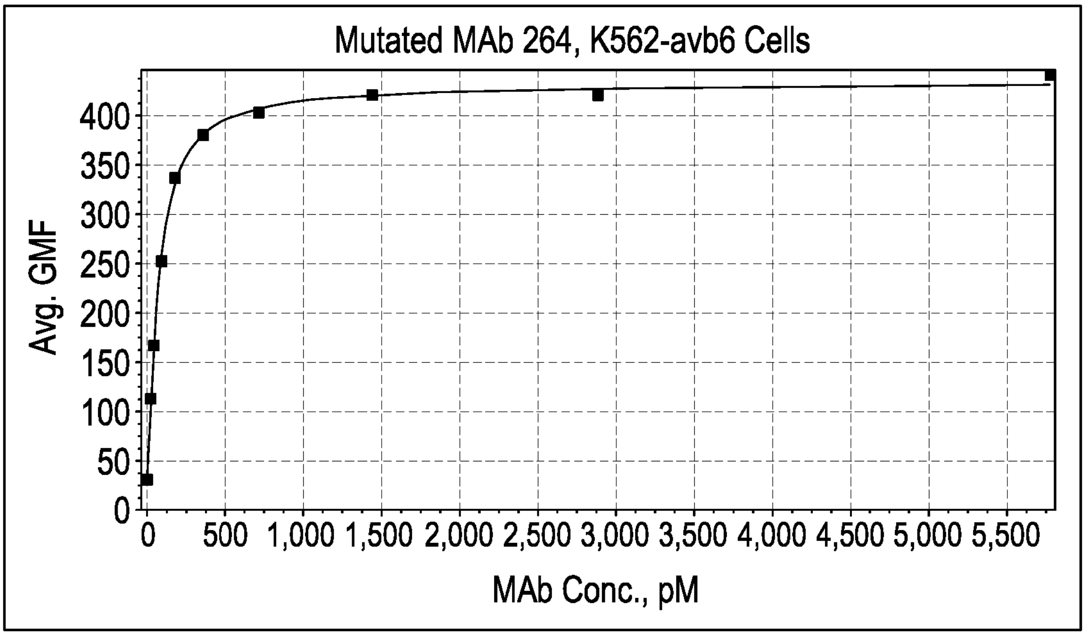


Figure 15B

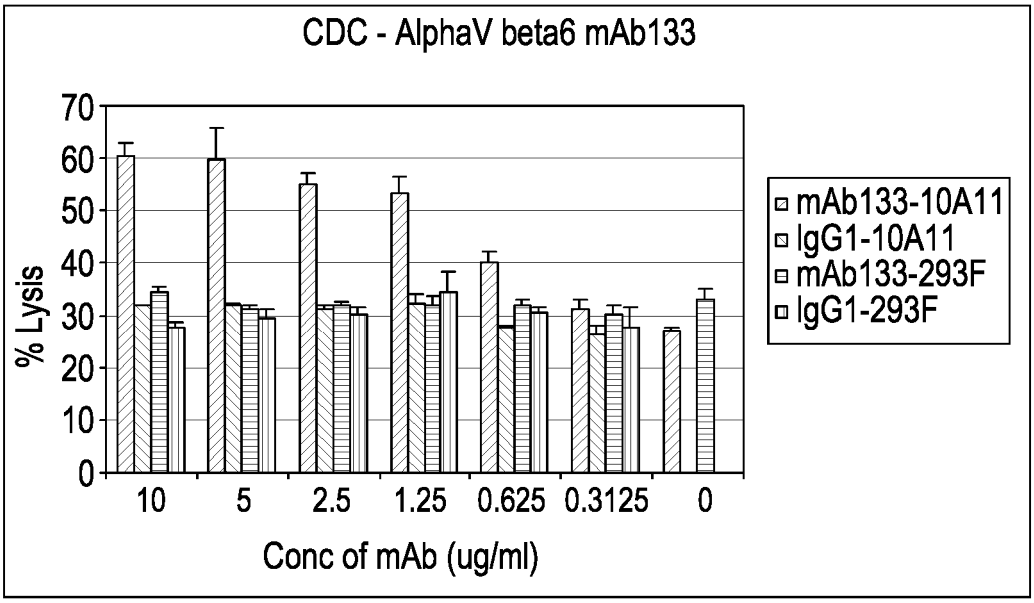


Figure 16A

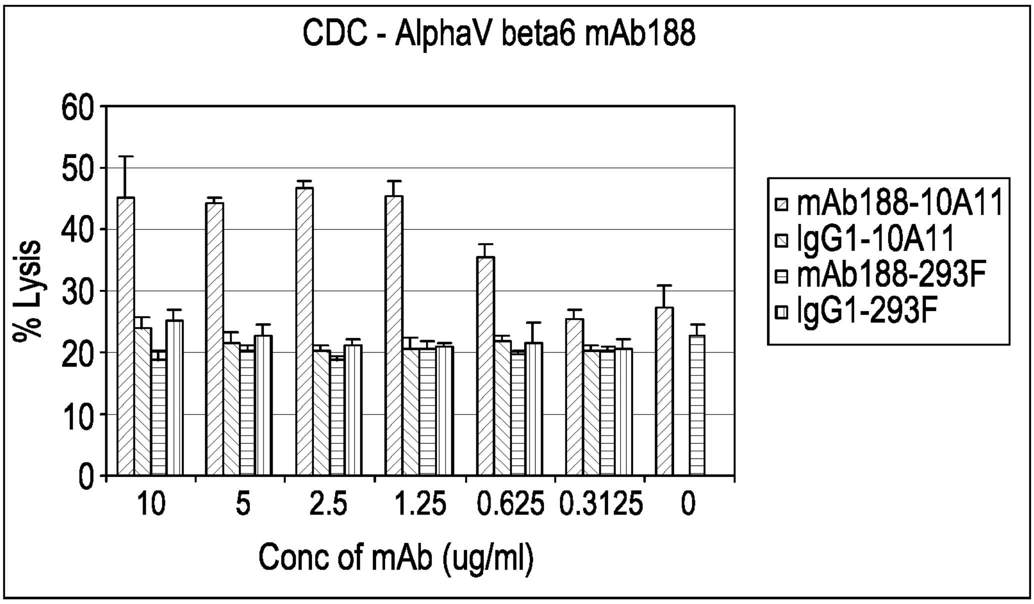


Figure 16B

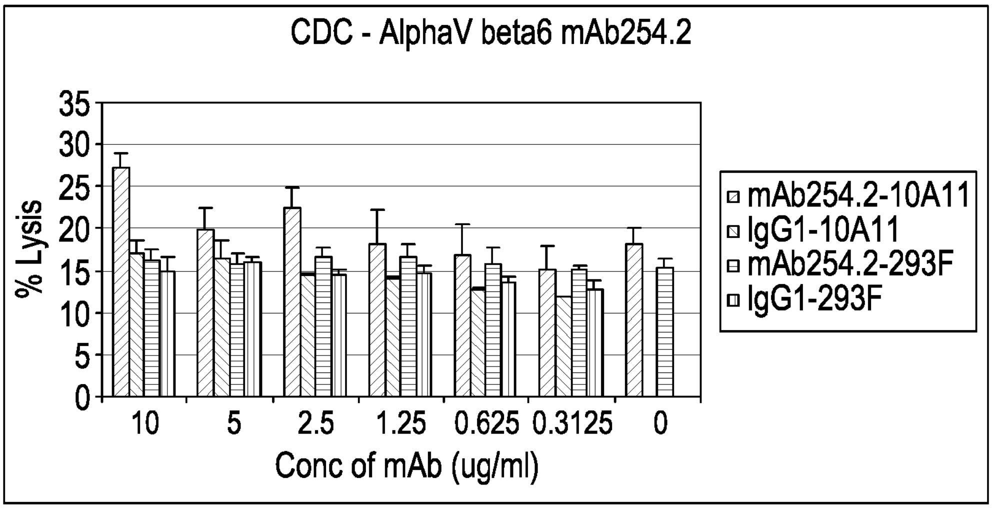


Figure 16C

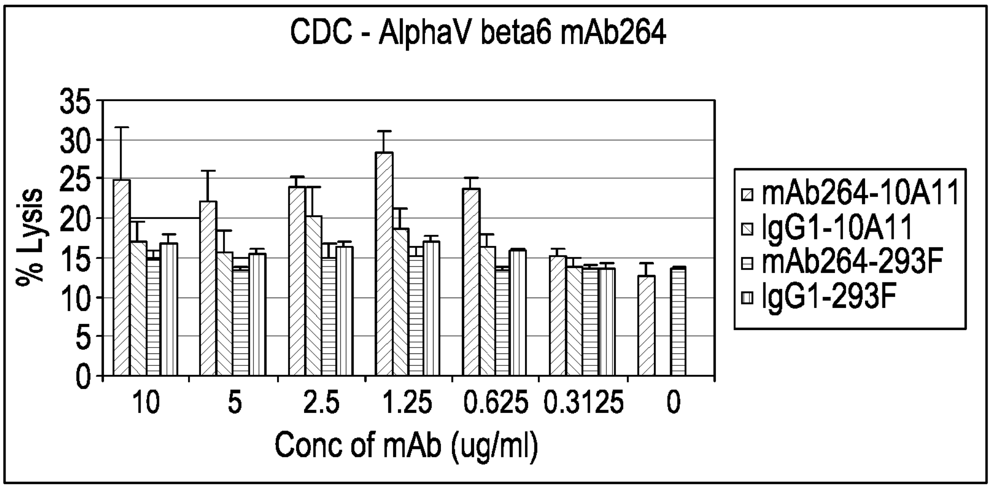


Figure 16D

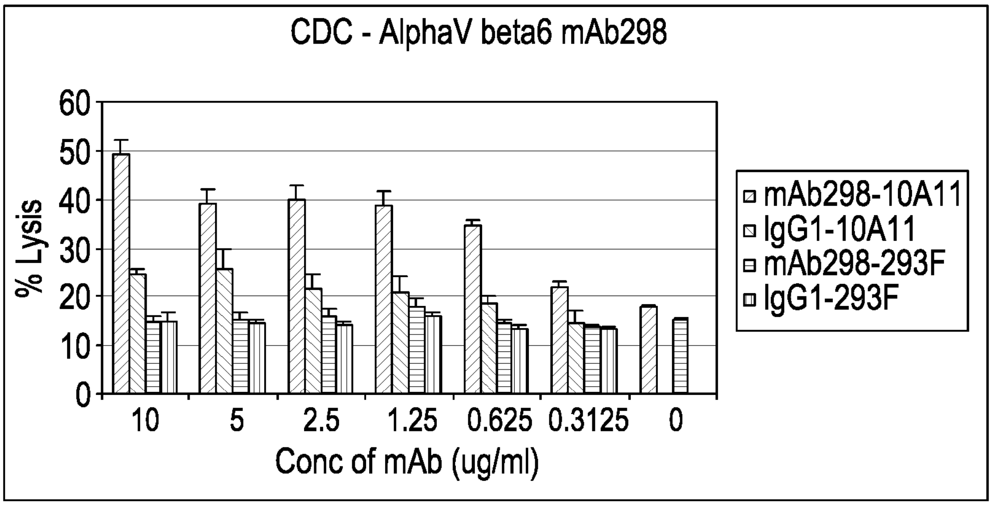


Figure 16E

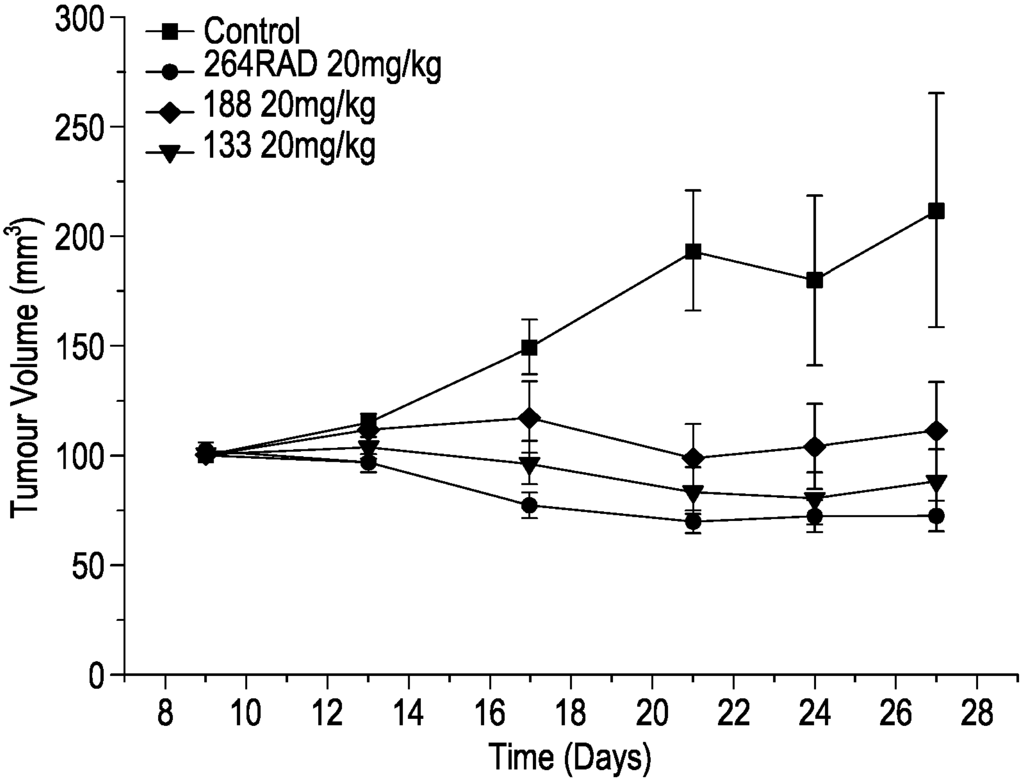
Control
264RAD 20mg/kg
188 20mg/kg
133 20mg/kg
Tumour Volume (mm³)
300
250
200
150
100
50
0
8
10
12
14
16
18
20
22
24
26
28
Time (Days)

Figure 17

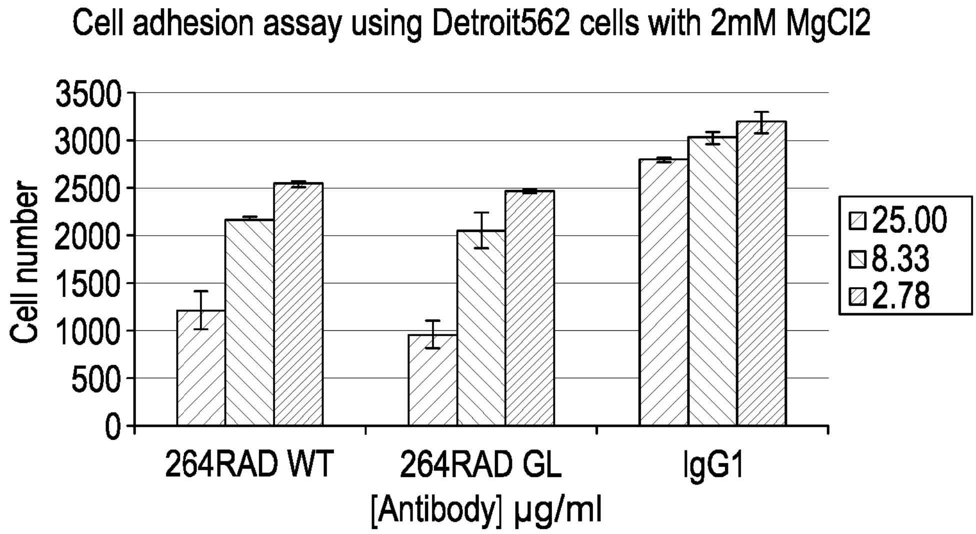
Cell adhesion assay using Detroit562 cells with 2mM MgCl2
3500
3000
2500
2000
1500
1000
500
0
Cell number
25.00
8.33
2.78
264RAD WT
264RAD GL
IgG1
[Antibody] µg/ml

Figure 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5151510 A, Stec
- US 9209965 W
- WO 9413804 A
- WO 9722596 A
- WO 9730035 A
- WO 9732856 A
- WO 9813354 A
- WO 9902166 A
- WO 0040529 A
- WO 0041669 A
- WO 0192224 A
- WO 0204434 A
- WO 0208213 A
- US 46600890
- US 07610515 B
- US 07919297 B
- US 07922649 B
- US 08031801 B
- US 08112848 B
- US 08234145 B
- US 08376279 B
- US 08430 A
- US 938 A
- US 08464584 B
- US 08464582 B
- US 08463191 B
- US 08462837 B
- US 08486853 B
- US 08486857 B
- US 08486859 B
- US 08462513 B
- US 08724752 B
- US 08759620 B
- US 20030093820 A
- US 6162963 A
- US 6150584 A
- US 6114598 A
- US 6075181 A
- US 5939598 A
- JP 3068180 B
- JP 3068506 B
- JP 3068507 B
- EP 0463151 B1
- WO 9402602 A
- WO 9634096 A
- WO 9824893 A
- WO 0076310 A
- US 5545807 A, Surani
- US 5545806 A
- US 5625825 A
- US 5625126 A
- US 5633425 A
- US 5661016 A
- US 5770429 A
- US 5789650 A
- US 5814318 A
- US 5877397 A
- US 5874299 A
- US 6255458 B, Lonberg and Kay
- US 5591669 A
- US 6023010 A, Krimpenfort and Berns
- US 5612205 A
- US 5721367 A
- US 5789215 A, Berns
- US 5643763 A, Choi and Dunn
- US 57474890
- US 07575962 B
- US 07810279 B
- US 07853408 B
- US 07904068 B
- US 07990860 B
- US 08053131 B
- US 08096762 B
- US 08155301 B
- US 08161739 B
- US 08165699 B
- US 08209741 B
- EP 0546073 B1
- WO 9203918 A
- WO 9222645 A
- WO 9222647 A
- WO 9222670 A
- WO 9312227 A
- WO 9400569 A
- WO 9425585 A
- WO 9614436 A
- WO 9713852 A
- WO 9824884 A
- US 5981175 A
- EP 773288 A
- EP 843961 A
- US 75962096
- US 4399216 A
- US 4912040 A
- US 4740461 A
- US 4959455 A
- US 3773919 A
- EP 58481 A

- EP 133988 A
- WO 9734631 A
- WO 02060919 A
- DE 3218121
- EP 52322 A
- EP 36676 A
- EP 88046 A
- EP 143949 A
- EP 142641 A
- JP 58118008 A
- US 4485045 A
- US 4544545 A
- EP 102324 A
- US 5194594 A
- US 4681581 A
- US 4735210 A
- US 5101827 A
- US 5102990 A
- US RE35500 E
- US 5648471 A
- US 5697902 A
- WO 0034784 A
- US 61885302 B


**Non-patent literature cited in the description**


- **J. J. BARRON et al.** HER2 Testing and Subsequent Trastuzumab Treatment for Breast Cancer in a Managed Care Environment. *The Oncologist,* 14 August 2009
- **SAMBROOK et al.** Molecular Cloning: A Laboratory Manual. Cold Spring Harbor Laboratory Press, 2001
- **MILHAVET O ; GARY DS ; MATTSON MP.** *Pharmacol Rev.,* December 2003, vol. 55 (4), 629-48
- **OPALINSKA JB ; GEWIRTZ AM.** *Sci STKE,* 28 October 2003, vol. 2003 (206), e47
- **LAPLANCHE et al.** *Nucl. Acids Res.,* 1986, vol. 14, 9081
- **STEC et al.** *J. Am. Chem. Soc.,* 1984, vol. 106, 6077
- **STEIN et al.** *Nucl. Acids Res.,* 1988, vol. 16, 3209
- **ZON et al.** *Anti-Cancer Drug Design,* 1991, vol. 6, 539
- **ZON et al.** Oligonucleotides and Analogues: A Practical Approach. Oxford University Press, 1991, 87-108
- **UHLMANN ; PEYMAN.** *Chemical Reviews,* 1990, vol. 90, 543
- **KABAT, E.A. et al.** Sequences of Proteins of Immunological Interest. US Department of Health and Human Services, Public Service, 1991
- **SEGAL et al.** *PNAS,* 1974, vol. 71, 4298-4302
- **AMIT et al.** *Science,* 1986, vol. 233, 747-753
- **CHOTHIA et al.** *J. Mol. Biol.,* 1987, vol. 196, 901-917
- **CHOTHIA et al.** *Nature,* 1989, vol. 342, 877-883
- **CATON et al.** *J. Immunol.,* 1990, vol. 144, 1965-1968
- **SHARON et al.** *PNAS,* 1990, vol. 87, 4814-4817
- **SHARON et al.** *J. Immunol.,* 1990, vol. 144, 4863-4869
- **KABAT et al.** *J. Immunol.,* 1991, vol. 147, 1709-1719
- **DAYHOFF, M.O.** Atlas of Protein Sequence and Structure. National Biomedical Research Foundation, 1972, vol. 5, 101-110
- Immunology - A Synthesis. Sinauer Associates, 1991
- **BOWIE et al.** *Science,* 1991, vol. 253, 164
- **ALTSCHUL et al.** *J. Mol. Biol.,* 1990, vol. 215, 405-410
- **PEARSON ; LIPMAN.** *PNAS USA,* 1988, vol. 85, 2444-2448
- **SMITH ; WATERMAN.** *J. Mol Biol.,* 1981, vol. 147, 195-197
- Proteins, Structures and Molecular Principles. W. H. Freeman and Company, 1984
- Introduction to Protein Structure. Garland Publishing, 1991
- **THORNTON.** *Nature,* 1991, vol. 354, 105
- **WOLD et al.** Multivariate data analysis in chemistry. Chemometrics -Mathematics and Statistics in Chemistry. D. Reidel Publishing Company, 1984
- **NORMAN et al.** Applied Regression Analysis. Wiley-Interscience, April 1998
- Computer-Assisted Reasoning. **KANDEL, ABRAHAM ; BACKER, ERIC.** Cluster Analysis. Prentice Hall PTR, 11 May 1995
- Principles of Multivariate Analysis: A User's Perspective. **KRZANOWSKI ; WOJTEK.** Oxford Statistical Science Series. Oxford University Press, December 2000
- **WITTEN, IAN H. ; FRANK, EIBE.** Data Mining: Practical Machine Learning Tools and Techniques with Java Implementations. Morgan Kaufmann, 11 October 1999
- Methods for Nonlinear Classification and Regression. **CHRISTOPHER C. HOLMES ; BANI K. MALLICK ; ADRIAN F. M. ; SMITH. BAYESIAN.** Wiley Series in Probability and Statistics. John Wiley & Sons, July 2002
- **GHOSE, ARUP K. ; VISWANADHAN, VELLARKAD N.** Combinatorial Library Design and Evaluation Principles, Software, Tools, and Applications. *Drug Discovery*

- **FAUCHERE.** *J. Adv. Drug Res.,* 1986, vol. 15, 29
- **VEBER ; FREIDINGER.** *TINS,* 1985, 392
- **EVANS et al.** *J. Med. Chem.,* 1987, vol. 30, 1229
- **RIZO ; GIERASCH.** *Ann. Rev. Biochem.,* 1992, vol. 61, 387
- **WARD, E.S. et al.** *Nature,* 1989, vol. 341, 544-546
- **MCCAFFERTY et al.** *Nature,* 1990, vol. 348, 552-554
- **HOLT et al.** *Trends in Biotechnology,* 2003, vol. 21, 484-490
- **BIRD et al.** *Science,* 1988, vol. 242, 423-426
- **HUSTON et al.** *PNAS USA,* 1988, vol. 85, 5879-5883
- **HOLLIGER, P. et al.** *Proc. Natl. Acad. Sci. USA,* 1993, vol. 90, 6444-6448
- **REITER, Y. et al.** *Nature Biotech,* 1996, vol. 14, 1239-1245
- **HU, S. et al.** *Cancer Res.,* 1996, vol. 56, 3055-3061
- The McGraw-Hill Dictionary of Chemical Terms. McGraw-Hill, 1985
- **STERN et al.** *Critical reviews in oncology/haematology,* 2005, vol. 54, 11-29
- **ANDERSEN ; REILLY.** *Current Opinion in Biotechnology,* 2004, vol. 15, 456-462
- **MENDEZ et al.** *Nature Genetics,* 1997, vol. 15, 146-156
- **GREEN ; JAKOBOVITS.** *J. Exp. Med.,* 1998, vol. 188, 483-495
- **ISHIDA et al.** *Cloning Stem Cells,* 2002, vol. 4, 91-102
- **BABCOOK et al.** *Proc. Natl. Acad. Sci. USA,* 1996, vol. 93, 7843-48
- Remington: The Science and Practice of Pharmacy. Lippincott Williams & Wilkens Publishers, 2003
- **LANGER et al.** *J. BiomedMater. Res.,* 1981, vol. 15, 167-277
- **LANGER.** *Chem. Tech.,* 1982, vol. 12, 98-105
- **SIDMAN et al.** *Biopolymers,* 1983, vol. 22, 547-556
- **EPSTEIN et al.** *Proc. Natl. Acad. Sci. USA,* 1985, vol. 82, 3688-3692
- **HWANG et al.** *Proc. Natl. Acad. Sci. USA,* 1980, vol. 77, 4030-4034
- **BALDRICK P.** Pharmaceutical excipient development: the need for preclinical guidance. *Regul. Toxicol. Pharmacol.,* 2000, vol. 32 (2), 210-8
- **WANG W.** Lyophilization and development of solid protein pharmaceuticals. *Int. J. Pharm.,* 2000, vol. 203 (1-2), 1-60
- **CHARMAN WN.** Lipids, lipophilic drugs, and oral drug delivery-some emerging concepts. *J Pharm Sci .,* 2000, vol. 89 (8), 967-78
- **POWELL et al.** Compendium of excipients for parenteral formulations. *PDA J Pharm Sci Technol.,* 1998, vol. 52, 238-311
- **FANGER et al.** *Immunol Methods,* 1994, vol. 4, 72-81
- **TRAUNECKER et al.** *Int. J. Cancer,* 1992, vol. 7, 51-52
- **DEO et al.** *Immunol. Today,* 1997, vol. 18, 127
- **VALERIUS et al.** *Blood,* 1997, vol. 90, 4485-4492
- **VITETTA.** *Immunol Today,* 1993, vol. 14, 252
- **JUNGHANS et al.** Cancer Chemotherapy and Biotherapy. Lippincott Raven, 1996, 655-686
- **HAAN ; MAGGOS.** *BioCentury,* 2004, vol. 12 (5), A1-A6
- **KOIDE et al.** *Journal of Molecular Biology,* vol. 284, 1141-1151
- **NYGREN et al.** *Current Opinion in Structural Biology,* 1997, vol. 7, 463-469
- **WESS, L.** *BioCentury, The Bernstein Report on BioBusiness,* 2004, vol. 12 (42), A1-A7
- **VARDEN Y ; SLIWKOWSKI MX.** Untangling the ErbB signalling network. *Nat Rev Mol Cell Bioi.,* 2001, vol. 2, 127-137
- **SLAMON DJ ; CLARK GM ; WONG SG et al.** Human breast cancer: correlation of relapse and survival with amplification of the HER-2/neu oncogene. *Science,* 1987, vol. 235, 177-182
- **SLAMON DJ ; GODOLPHIN W ; JONES LA et al.** Studies of the HER-2/neu proto-oncogene in human breast and ovarian cancer. *Science,* 1989, vol. 244, 707-712
- **WONG AL ; LEE SC.** Mechanisms of Resistance to Trastuzumab and Novel Therapeutic Strategies in HER2-Positive Breast Cancer. *Int J Breast Cancer,* 2012, vol. 2012, 415170
- **PICCART-GEBHART MJ ; PROCTER M ; LEYLAND-JONES B et al.** Trastuzumab after adjuvant chemotherapy in HER2-positive breast cancer. *N Engl J Med.,* 2005, vol. 353, 1659-1672
- **ROMOND EH ; PEREZ EA ; BRYANT J et al.** Trastuzumab plus adjuvant chemotherapy for operable HER2-positive breast cancer. *N Engl J Med.,* 2005, vol. 353, 1673-1684
- **ARRIBAS J ; BASELGA J ; PEDERSEN K ; PARRA-PALAU JL.** p95HER2 and breast cancer. *Cancer Res.,* 2011, vol. 71, 1515-1519
- **GAJRIA D ; CHANDARLAPATY S.** HER2-amplified breast cancer: mechanisms of trastuzumab resistance and novel targeted therapies. *Expert Rev Anticancer Ther.,* 2011, vol. 11, 263-275
- **LINDSLEY CW ; BARNETT SF ; LAYTON ME ; BILODEAU MT.** The P13K/Akt pathway: recent progress in the development of ATP-competitive and allosteric Akt kinase inhibitors. *Curr Cancer Drug Targets,* 2008, vol. 8, 7-18

- **UEDA Y ; WANG S ; DUMONT N et al.** Overexpression of HER2 (erbB2) in human breast epithelial cells unmasks transforming growth factor betainduced cell motility. *J Bioi Chem.,* 2004, vol. 279, 24505-24513
- **WANG SE ; SHIN I ; WU FY ; FRIEDMAN DB ; ARTEAGA CL.** HER2/Neu (ErbB2) signaling to Rac1-Pak1 is temporally and spatially modulated by transforming growth factor beta. *Cancer Res.,* 2006, vol. 66, 9591-9600
- **MURAOKA RS ; KOH Y ; ROEBUCK LR et al.** Increased malignancy of Neuinduced mammary tumors overexpressing active transforming growth factor betal. *Mol Cell Bioi.,* 2003, vol. 23, 8691-8703
- **LYONS RM ; GENTRY LE ; PURCHIO AF ; MOSES HL.** Mechanism of activation of latent recombinant transforming growth factor beta 1 by plasmin. *J Cell Bioi.,* 1990, vol. 110, 1361-1367
- **MUNGER JS ; HUANG X ; KAWAKATSU H et al.** The integrin alpha v beta 6 binds and activates latent TGF beta 1: a mechanism for regulating pulmonary inflammation and fibrosis. *Cell,* 1999, vol. 96, 319-328
- **HAZELBAG S ; KENTER GG ; GORTER A et al.** Overexpression of the alpha v beta 6 integrin in cervical squamous cell carcinoma is a prognostic factor for decreased survival. *J Pathol.,* 2007, vol. 212, 316-324
- **BATES RC ; BELLOVIN D1 ; BROWN C et al.** Transcriptional activation of integrin beta6 during the epithelial-mesenchymal transition defines a novel prognostic indicator of aggressive colon carcinoma. *J Clin Invest.,* 2005, vol. 115, 339-347
- **ELAYADI AN ; SAMLI KN ; PRUDKIN L et al.** A peptide selected by biopanning identifies the integrin alphavbeta6 as a prognostic biomarker for nonsmall cell lung cancer. *Cancer Res.,* 2007, vol. 67, 5889-5895
- **BREUSS JM ; GALLO J ; DELISSER HM et al.** Expression of the beta 6 integrin subunit in development, neoplasia and tissue repair suggests a role in epithelial remodeling. *J Cell Sci.,* 1995, vol. 108, 2241-2251
- **VAN AARSEN LA ; LEONE DR ; HO S et al.** Antibody-mediated blockade of integrin alpha v beta 6 inhibits tumor progression in vivo by a transforming growth factor-beta-regulated mechanism. *Cancer Res.,* 2008, vol. 68, 561-570
- **HU M ; YAO J ; CARROLL DK et al.** Regulation of in situ to invasive breast carcinoma transition. *Cancer Cell,* 2008, vol. 13, 394-406
- **HYNES RO.** Integrins: bidirectional, allosteric signaling machines. *Cell,* 2002, vol. 110, 673-687
- **KUMAR CC.** Signaling by integrin receptors. *Oncogene,* 1998, vol. 17, 1365-1373
- **MCSHANE LM ; ALTMAN DG ; SAUERBREI W et al.** Reporting recommendations for tumour MARKer prognostic studies (REMARK). *Eur J Cancer.,* 2005, vol. 41, 1690-1696
- **RAKHA EA ; EI-REHIM DA ; PAISH C et al.** Basal phenotype identifies a poor prognostic subgroup of breast cancer of clinical importance. *Eur J Cancer,* 2006, vol. 42, 3149-3156
- **ABD EI-REHIM OM ; PINDER SE ; PAISH CE et al.** Expression of luminal and basal cytokeratins in human breast carcinoma. *J Pathol.,* 2004, vol. 203, 661-671
- **CURTIS C ; SHAH SP ; CHIN SF et al.** The genomic and transcriptomic architecture of 2,000 breast tumours reveals novel subgroups. *Nature,* 2012, vol. 486, 346-352
- **CROWDER MJ HD.** Monographs on Statistics and Applied Probability. Chapman & Hall, 1990
- **LEWIS GO ; LOFGREN JA ; MCMURTREY AE et al.** Growth regulation of human breast and ovarian tumor cells by heregulin: Evidence for the requirement of ErbB2 as a critical component in mediating heregulin responsiveness. *Cancer Res.,* 1996, vol. 56, 1457-1465
- **EBERLEIN C ; KENDREW J ; MCDAID K et al.** A human monoclonal antibody 264RAD targeting alphavbeta6 integrin reduces tumour growth and metastasis, and modulates key biomarkers in vivo. *Oncogene,* 2012
- **SLAMON OJ ; LEYLAND-JANES B ; SHAK S et al.** Use of chemotherapy plus a monoclonal antibody against HER2 for metastatic breast cancer that overexpresses HER2. *N Eng/ J Med.,* 2001, vol. 344, 783-792
- **VARDEN V.** Biology of HER2 and its importance in breast cancer. *Oncology,* 2001, vol. 61 (2), 1-13
- **CLARK AS ; WEST K ; STREICHER S ; DENNIS PA.** Constitutive and inducible Akt activity promotes resistance to chemotherapy, trastuzumab, or tamoxifen in breast cancer cells. *Mol Cancer Ther.,* 2002, vol. 1, 707-717
- **MURAOKA-COOK RS ; SHIN I ; VI JV et al.** Activated type I TGFbeta receptor kinase enhances the survival of mammary epithelial cells and accelerates tumor progression. *Oncogene,* 2006, vol. 25, 3408-3423
- **FALCIONI R ; ANTONINI A ; NISTICO P et al.** Alpha 6 beta 4 and alpha 6 beta 1 integrins associate with ErbB-2 in human carcinoma cell lines. *Exp Cell Res.,* 1997, vol. 236, 76-85
- **GUO W ; PYLAYEVA Y ; PEPE A et al.** Beta 4 integrin amplifies ErbB2 signaling to promote mammary tumorigenesis. *Cell,* 2006, vol. 126, 489-502

- **VIOLETTE S ; SHEPPARD, D ; ROSAS, 10 ; ARJOMANDI, M ; RICE, T ; GILMAN, M ; KAMINSKI, N ; PRASSE, A ; MARONI, B.** Identification Of Biomarkers To Monitor The Activity Of STX-100, A Humanized Anti-αVβ6 Antibody, In A Phase 2a Trial In Idiopathic Pulmonary Fibrosis. *Am J Respir Crit Care Med.,* 2012, 185
- **BENZ CC ; SCOTT GK ; SARUP JC et al.** Estrogen-dependent, tamoxifen resistant tumorigenic growth of MCF-7 cells transfected with HER2/neu. *Breast Cancer Res Treat,* 1992, vol. 24 (2), 85-95
- **MEYER T ; MARSHALL JF ; HART IR.** Expression of alphav integrins and vitronectin receptor identity in breast cancer cells. *Br J Cancer,* 1998, vol. 77 (4), 530-6
- **SANTNER SJ ; DAWSON PJ ; TAIT L et al.** Malignant MCF10CA1 cell lines derived from premalignant human breast epithelial MCF10AT cells. *Breast Cancer Res Treat.,* 2001, vol. 65 (2), 101-10
- **NAHTA R ; ESTEVA FJ.** Herceptin: mechanisms of action and resistance. *Cancer Lett.,* 2006, vol. 232 (2), 23-38
- **NEVE RM ; CHIN K ; FRIDLYAND J et al.** A collection of breast cancer cell lines for the study of functionally distinct cancer subtypes. *Cancer Cell,* 2006, vol. 1 0 (6), 515-27
- **SUBIK K ; LEE JF ; BAXTER L et al.** The Expression Patterns of ER, PR, HER2, CK5/6, EGFR, Ki-67 and AR by Immunohistochemical Analysis in Breast Cancer Cell Lines. *Breast Cancer (Auckl),* 2010, vol. 4, 35-41